# EUROPEAN PATENT APPLICATION

(11) **EP 4 745 236 A1**
(43) Date of publication of application: **20.05.2026**
(21) Application number: 24838720.1
(22) Date of filing: 05.07.2024
(51) Int. Cl.: C12N 15/10, C12N 9/12, C12N 15/11, A61K 38/47

(54) **ENGINEERED MAMMALIAN GENE WRITING SYSTEM**

(30) Priority: 10.07.2023 CN 202310842342; 08.02.2024 CN 202410178308
(71) Applicant: Beijing Institute for Stem Cell and Regenerative Medicine, Beijing 100101 (CN); Institute Of Zoology, Chinese Academy Of Sciences, Beijing 100101 (CN)
(72) Inventor: LI, Wei, Beijing 100101 (CN); ZHOU, Qi, Beijing 100101 (CN); CHEN, Yangcan, Beijing 100101 (CN); LUO, Shengqiu, Beijing 100101 (CN); HU, Yanping, Beijing 100101 (CN); WANG, Xinge, Beijing 100101 (CN); MAO, Bangwei, Beijing 100101 (CN)
(74) Representative: Lavoix
(86) International application number: PCT/CN2024/103894
(87) International publication number: WO 2025/011467

(57) **Abstract**

Provided is a retrotransposase, comprising a target DNA binding domain containing a zinc finger binding motif, a reverse transcriptase domain, and an endonuclease domain, and capable of reversely transcribing an RNA into a DNA. Also provided is a system for modifying a DNA. The system comprises the retrotransposase or a nucleic acid encoding the retrotransposase; and a donor RNA or a nucleic acid encoding the donor RNA, wherein the donor RNA contains a sequence binding to the retrotransposase, and a heterologous sequence.

## Description

### TECHNICAL FIELD

The present application relates to the field of biotechnology. More specifically, the present application relates to a retrotransposon/retrotransposase capable of site-specific insertion of large DNA fragments into the human genome and the use thereof, as well as the system utilizing said enzyme.

### CROSS REFERENCE

This application claims the benefit of Chinese Patent Application No. CN2023108423427 filed on July 10, 2023 and Chinese Patent Application No. CN2024101783089 filed on February 8, 2024, the contents of which are incorporated herein by reference in their entirety.

### SEQUENCE LISTING DOCUMENT SUBMITTED SIMULTANEOUSLY

The entire contents of the following XML document are incorporated herein by reference in their entirety: sequence listing in computer readable format (CRF) (file name: FH01065PCT-sequence listing.xml, date: 20240705, size: 154KB).

### BACKGROUND ART

Site-specific insertion of large DNA fragments into the genome is an important and useful technique in genomic research.

The integration of large DNA fragments into mammalian cell genomes is generally inefficient and non-specific. Several existing genome editing methods, such as the Clustered Regularly Interspaced Short Palindromic Repeats (CRISPR)-Cas system and the like, are not applicable to the integration of long DNA fragments. For instance, the CRISPR/Cas9 system is only limited to editing small-scale systems and exhibits low efficiency when used for integrating longer sequences. Some other methods for site-specific insertion of large DNA fragments have their own deficiencies. For example, large-fragment insertion via homologous recombination exhibits low efficiency, and the introduction of double-stranded DNA breaks poses safety concerns; recombinase systems such as Cre/loxP often require the prior insertion of a loxP site before the second-step integration can be performed; in addition, most current technologies rely heavily on the supply of DNA donors, making it difficult to address issues such as in vivo delivery.

Retrotransposase effectively address the gap in site-specific integration of large DNA fragments, providing biological research with a more versatile and convenient tool. The novel retrotransposase system primarily consists of two components: first, retrotransposase polypeptide itself; second, the donor RNA carrying new genetic information. The retrotransposase forms a protein-RNA complex by recognizing and binding to the donor RNA sequence, and can convert the RNA into DNA for integration into genomic sites through its reverse transcriptase activity. The DNA integration method based on retrotransposase avoids the generation of double-stranded DNA breaks and the associated risks, while also eliminating the need for preparation and reliance on DNA donors in practical applications. Such method needs only a single reverse transcriptional step to reverse transcribe RNA into DNA, enabling the integration of large DNA fragments, which is highly advantageous for broad application in various scenarios.

### SUMMARY

Based on the above problems present in the prior art, there is an urgent need in the art to develop a more effective retrotransposase system, the present application involves the following contents:
1. An engineered retrotransposase, comprising one, two, or three mutations of the wild-type retrotransposase TgR2, or is a truncated form of the wild-type retrotransposase TgR2; or comprising one, two, or three mutations of the wild-type retrotransposase TgR2 on a truncated form of the wild-type retrotransposase TgR2, wherein the mutations are selected from a group consisting of:
   (i) amino acid residues in the wild-type retrotransposase TgR2 are mutated to the corresponding amino acids identified at those positions in a multiple sequence alignment;
   (ii) amino acid residues in the wild-type retrotransposase TgR2 that were predicted to potentially interact with DNA or RNA in its tertiary structure are mutated to positively charged amino acids; and
   (iii) cysteine residues in the wild-type retrotransposase TgR2 are mutated to serine.
2. The engineered retrotransposase according to item 1, wherein the amino acid sequence of the wild-type retrotransposase TgR2 is shown as SEQ ID NO.1 or the amino acid position numbering of the engineered retrotransposase is defined as SEQ ID NO.1; or
   the truncated form of the wild-type retrotransposase TgR2 is an N-terminal truncation, a C-terminal truncation and/or an internal sequence truncation of the wild-type retrotransposase TgR2,
   preferably, the N-terminal truncation is the truncation of the 1^{st} to 500^{th} amino acids of the wild-type retrotransposase TgR2, e.g., the truncation of the 1^{st} to 80^{th} amino acids, the 1^{st} to 190^{th} amino acids, or the 1^{st} to 220^{th} amino acids;
   preferably, the internal sequence truncation involves deletion of the 300^{th} to 1200^{th} amino acids of the wild-type retrotransposase TgR2, e.g., deletion of the 390^{th} to 500^{th} amino acids, the 1100^{th} to 1120^{th} amino acids.
3. The engineered retrotransposase according to item 1, wherein,
   the amino acids at the corresponding sites of the wild-type retrotransposase TgR2 in the multiple sequence alignment are identified using multiple sequence alignment softwares (such as MAFFT and the like), and the amino acid position numbering is defined as SEQ ID NO.1;
   preferably, the corresponding sites of the wild-type retrotransposase TgR2 in the multiple sequence alignment are selected from a group consisting of any one, two, three, four, five, six, seven, eight, nine, ten or more of the following sites:
      K700, L704, V727, S732, L737, A747, A748, T759, I760, W762, S763, H792, A793, Q795, E798, P801, V807, S808, E812, S815, T819, T820, H825, K828, S860, Y864, H865, R866, Q868, S869, N889, N891, T892, S895, Q911 or D957;
      more preferably, the amino acids at the corresponding sites of the wild-type retrotransposase TgR2 in the multiple sequence alignment are mutated into other amino acids corresponding to those sites. The mutations are selected from any one, two, three, four, five, six, seven, eight, nine, ten or more of the following:
         K700D, L704Q, V727L, S732T, L737I, A747S, A748S, A748P, T759L, I760L, W762R, W762K, W762Q, S763N, S763R, H792T, H792W, A793V, Q795K, Q795R, E798G, P801Q, V807I, S808R, E812K, E812R, S815N, T819D, T820L, H825Q, K828P, S860E, Y864F, H865Q, H865R, R866V, Q868S, S869K, N889G, N891Q, T892K, S895K, Q911E or D957G;
         more preferably, the amino acids at the corresponding sites of the wild-type retrotransposase TgR2 in the multiple sequence alignment are mutated into other amino acids corresponding to those sites. The mutations are selected from any of the following:
         K700D, W762Q, H792W, A793V, R866V and D957G.
4. The engineered retrotransposase according to item 1, wherein,
   the amino acids in the tertiary structure of the wild-type retrotransposase TgR2 that potentially interact with DNA or RNA are mutated to arginine; the amino acid position numbering is defined as SEQ ID NO.1;
   preferably, the amino acids in the tertiary structure of the wild-type retrotransposase TgR2 that potentially interact with DNA or RNA are those selected from a group consisting of any one, two, three, four, five, six, seven, eight, nine, ten or more of the following:
      E153, G154, E160, E163, Q164, Q168, D227, K228, N229, N240, K267, E268, N269, P291, E294, H341, K342, C344, E350, E361, N363, N365, N367, A379, Q381, S383, D384, S390, D573, G575, K576, K579, I585, L588, K635, E636, E638, K639, N640, Q642, E643, S645, K646, G647, S648, V661, V693, I695, P696, S699, K700, I713, I715, T728, S732, K733, S751, E752, N753, L754, T819, N847, K950, Q955, D957, D990, K993, T994, Y995, P998, I1001, D1005, H1006, T1044, D1046, T1052, Q1062, Q1071, S1072, S1073, D1074, D1075, G1098, G1099, D1100, E1102, N1103, P1105, S1106, E1109, S1113, S1114, E1115, N1118, N1123, E1125, E1127, Q1131, D1133, K1134, K1137, K1210, D1216, D1218, E1220, A1223, H1224, G1227, N1228, Q1233, D1234, I1237, K1238, H1240, N1241, E1245, E1249, N1268, E1270, E1302, E1306, K1307 or K1310;
      more preferably, the amino acids in the tertiary structure of the wild-type retrotransposase TgR2 that potentially interact with DNA or RNA are those selected from a group consisting of any one, two, three, four, five, six, seven, eight, nine, ten or more of the following:
      D227, K228, N229, N240, K267, N269, P291, E294, H341, K342, C344, E361, N365, N367, A379, Q381, S383, D384, S390, K576, K579, K639, K646, K733, T819, K950, Q955, D990, K993, T1044, T1052, Q1062, Q1071, D1074, D1075, G1098, G1099, P1105, E1109, E1125, E1127, Q1131, A1223, I1237, E1245, E1249 or E1306.
5. The engineered retrotransposase according to item 1, wherein,
   the cysteine(s) in the wild-type retrotransposase TgR2 is/are selected from a group consisting of any one, two, three, four, five, six, seven, eight, nine, ten or more of the following amino acid positions, with the amino acid position numbering defined as SEQ ID NO.1;
   C4, C145, C170, C282, C344, C404, C518, C521, C690, C735, C750, C855, C901, C913, C930, C1034, C1036, C1079, C1139 or C1244;
   preferably, the cysteine(s) in the wild-type retrotransposase TgR2 is/are selected from any one, two, three or four of the following:
      C344, C1034, C1036 and C1244.
6. The engineered retrotransposase according to any one of items 1-5, wherein, the amino acid position numbering is defined as SEQ ID NO.1; the engineered retrotransposase comprises one or more sets of mutations selected from the following:
   (1)G1098R; (2) G1098R+H341R; (3) G1098R+K700D; (4)G1098R+W762Q; (5)G1098R+H792W; (6)G1098R+A793V; (7)G1098R+R866V; (8)G1098R+Q955R; (9)G1098R+T1052R; (10)G1098R+Q1062R; (11)G1098R+P1105R; (12)G1098R+C1034S; (13)G1098R+I1237R; (14)G1098R+K1310R; (15)G1098R+D957G; (16)H341R+C1036S; (17)H341R+Q1062R; (18)H341R+I1237R; (19)H341R+K1310R; (20)I1237R+K1310R; (21)C1034S+C1036S; (22)K700D+R866V; (23)W762Q+R866V; (24)H792W+R866V; (25)A793V+R866V; (26)G1098R+A793V+K700D; (27)G1098R+A793V+W762Q; (28)G1098R+A793V+H792W; (29)G1098R+A793V+Q955R; (30)G1098R+A793V+W762Q+Q955R; (31)G1098R+A793V+W762Q+H341R; (32)G1098R+A793V+Q955R+H341R; (33)G1098R+A793V+W762Q+Q955R+H341R;
   preferably, the engineered retrotransposase comprises one or more sets of mutations selected from the following:
      G1098R+H341R; G1098R+K700D; G1098R+W762Q; G1098R+H792W, G1098R+A793V;
   G1098R+R866V; G1098R+Q955R; G1098R+A793V+W762Q; G1098R+A793V+Q955R; G1098R+A793V+Q955R+H341R; and G1098R+A793V+W762Q+Q955R+H341R.
7. An engineered retrotransposase, comprising any one or more sets of mutations selected from the following:
   (1)G1098R; (2) G1098R+H341R; (3) G1098R+K700D; (4)G1098R+W762Q; (5)G1098R+H792W; (6)G1098R+A793V; (7)G1098R+R866V; (8)G1098R+Q955R; (9)G1098R+T1052R; (10)G1098R+Q1062R; (11)G1098R+P1105R; (12)G1098R+C1034S; (13)G1098R+I1237R; (14)G1098R+K1310R; (15)G1098R+D957G; (16)H341R+C1036S; (17)H341R+Q1062R; (18)H341R+I1237R; (19)H341R+K1310R; (20)I1237R+K1310R; (21)C1034S+C1036S; (22)K700D+R866V; (23)W762Q+R866V; (24)H792W+R866V; (25)A793V+R866V; (26)G1098R+A793V+K700D; (27)G1098R+A793V+W762Q; (28)G1098R+A793V+H792W; (29)G1098R+A793V+Q955R; (30)G1098R+A793V+W762Q+Q955R; (31)G1098R+A793V+W762Q+H341R; (32)G1098R+A793V+Q955R+H341R; (33)G1098R+A793V+W762Q+Q955R+H341R;
   preferably, the engineered retrotransposase comprises one or more sets of mutations selected from the following:
      G1098R+H341R; G1098R+K700D; G1098R+W762Q; G1098R+H792W, G1098R+A793V; G1098R+R866V; G1098R+Q955R; G1098R+A793V+W762Q; G1098R+A793V+Q955R; G1098R+A793V+Q955R+H341R; G1098R+A793V+W762Q+Q955R+H341R;
      the amino acid position numbering is defined as SEQ ID NO.1.
8. An engineered retrotransposase, which is the engineered retrotransposase according to any one of items 1 to 7 or an engineered retrotransposase obtained by further conjugating the wild-type retrotransposase TgR2 with other amino acid sequences.
9. The engineered retrotransposase according to item 8, wherein, the other amino acid sequence(s) is/are selected from any one, two, three, four, five, six, or seven of the following: an exonuclease, a single-stranded DNA-binding protein, a chromatin-modulating peptide segment, a high mobility peptide segment, an RNaseH domain, a nuclear localization signal (NLS) for nuclear import, and a nuclear localization signal for nuclear export.
10. The engineered retrotransposase according to item 9, wherein the other amino acid sequence is an exonuclease, and the exonuclease is inserted at the N-terminal, after amino acid no. 191, after amino acid no. 432, after amino acid no. 1112, or at the C-terminal of the retrotransposase according to any one of items 1 to 7 or the wild-type retrotransposase TgR2;
   preferably, the exonuclease is an exonuclease from bacteriophage T5;
   more preferably, the amino acid sequence of the exonuclease is shown as SEQ ID NO.8 or has at least 70%, 75%, 80%, 85%, 90%, 95%, 96%, 97%, 98%, or 99% identity with the amino acid sequence of SEQ ID No. 8.
11. The engineered retrotransposase according to item 9, wherein, the other amino acid sequence is a single-stranded DNA-binding protein, and the single-stranded DNA-binding protein is inserted at the N-terminal, after amino acid no. 191, after amino acid no. 432, after amino acid no. 1112, or at the C-terminal of the retrotransposase according to any one of items 1 to 7 or the wild-type retrotransposase TgR2;
   preferably, the single-stranded DNA-binding protein is a single-stranded DNA-binding protein from the bacterium Sulfolobus tokodaii;
   more preferably, the sequence of the single-stranded DNA-binding protein is shown as SEQ ID NO.9 or has at least 70%, 75%, 80%, 85%, 90%, 95%, 96%, 97%, 98%, or 99% identity with the amino acid sequence of SEQ ID No. 9.
12. The engineered retrotransposase according to item 9, wherein the other amino acid sequence is a chromatin-modulating peptide segment or a high mobility peptide, and the chromatin-modulating peptide segment or high mobility peptide segment is inserted at the N-terminal, after amino acid no. 191, after amino acid no. 432, after amino acid no. 1112, or at the C-terminal of the retrotransposase according to any one of items 1 to 7 or the wild-type retrotransposase TgR2;
   preferably, the chromatin-modulating peptide segment or high mobility peptide segment is a chromatin-modulating peptide segment or high mobility peptide segment from human;
   preferably, the sequence of the chromatin-modulating peptide segment or high mobility peptide segment is shown as SEQ ID NO.10 or has at least 70%, 75%, 80%, 85%, 90%, 95%, 96%, 97%, 98%, or 99% identity with the amino acid sequence of SEQ ID No. 10.
13. The engineered retrotransposase according to item 9, wherein the other amino acid sequence is an RNaseH domain, and the RNaseH domain is inserted at the N-terminal, after amino acid no. 191, after amino acid no. 432, after amino acid no. 1112, or at the C-terminal of the retrotransposase according to any one of items 1 to 7 or the wild-type retrotransposase TgR2;
   preferably, the RNaseH domain is the RNaseH domain from MLV reverse transcriptase;
   more preferably, the sequence of the RNaseH domain of the MLV reverse transcriptase is shown as SEQ ID NO.11 or has at least 70%, 75%, 80%, 85%, 90%, 95%, 96%, 97%, 98%, or 99% identity with the amino acid sequence of SEQ ID No.11.
14. The engineered retrotransposase according to item 9, wherein,
   any two or three or four of the exonuclease and/or single-stranded DNA-binding protein and/or chromatin-mudulating peptide segment (CMP) and/or RNaseH domain are inserted at the N-terminal, after amino acid no. 191, after amino acid no. 432, after amino acid no. 1112, or at the C-terminal of the retrotransposase according to any one of items 1 to 7 or the wild-type retrotransposase TgR2;
   preferably, the exonuclease is inserted at the N-terminal of the retrotransposase according to any one of items 1 to 7 or the wild-type retrotransposase TgR2, and the exonuclease or single-stranded DNA-binding protein or chromatin-modulating peptide segment (CMP) or RNaseH domain is inserted after amino acid no. 432 of the retrotransposase according to any one of items 1 to 7 or the wild-type retrotransposase TgR2; or
   more preferably, the single-stranded DNA-binding protein is inserted at the N-terminal of the retrotransposase according to any one of items 1 to 7 or the wild-type retrotransposase TgR2, and the exonuclease or single-stranded DNA-binding protein or chromatin-modulating peptide segment (CMP) or RNaseH domain is inserted after amino acid no. 432 of the retrotransposase according to any one of items 1 to 7 or the wild-type retrotransposase TgR2; or
   more preferably, the chromatin-modulating peptide segment (CMP) is inserted at the N-terminal of the retrotransposase according to any one of items 1 to 7 or the wild-type retrotransposase TgR2, and the exonuclease or single-stranded DNA-binding protein or chromatin-modulating peptide segment (CMP) or RNaseH domain is inserted after amino acid no. 432 of the retrotransposase according to any one of items 1 to 7 or the wild-type retrotransposase TgR2; or
   more preferably, the single-stranded DNA-binding protein is inserted at the N-terminal of the retrotransposase according to any one of items 1 to 7 or the wild-type retrotransposase TgR2, and the chromatin-modulating peptide segment (CMP) is inserted after amino acid no. 432 of the retrotransposase according to any one of items 1 to 7 or the wild-type retrotransposase TgR2, and the chromatin-modulating peptide segment (CMP) or RNaseH domain is inserted after amino acid no. 1112 of the retrotransposase according to any one of items 1 to 7 or the wild-type retrotransposase TgR2; or
   more preferably, the single-stranded DNA-binding protein is inserted at the N-terminal of the retrotransposase according to any one of items 1 to 7 or wild-type retrotransposase TgR2, and the RNaseH domain is inserted after amino acid no. 432 of the retrotransposase according to any one of items 1 to 7 or wild-type retrotransposase TgR2, and the chromatin-modulating peptide segment (CMP) or RNaseH domain is inserted after amino acid no. 1112 of the retrotransposase according to any one of items 1 to 7 or wild-type retrotransposase TgR2; or
   more preferably, the chromatin-modulating peptide segment (CMP) is inserted at the N-terminal of the retrotransposase according to any one of items 1 to 7 or wild-type retrotransposase TgR2, and the chromatin-modulating peptide segment (CMP) is inserted after amino acid no. 432 of the retrotransposase according to any one of items 1 to 7 or wild-type retrotransposase TgR2, and the chromatin-modulating peptide segment (CMP) or RNaseH domain is inserted after amino acid no. 1112 of the retrotransposase according to any one of items 1 to 7 or wild-type retrotransposase TTgR2; or
   more preferably, the chromatin-modulating peptide segment (CMP) is inserted at the N-terminal of the retrotransposase according to any one of items 1 to 7 or wild-type retrotransposase TgR2, and the RNaseH domainis inserted after amino acid no. 432 of the retrotransposase according to any one of items 1 to 7 or wild-type retrotransposase TgR2, and the chromatin-modulating peptide segment (CMP) or RNaseH domain is inserted after amino acid no. 1112 of the retrotransposase according to any one of items 1 to 7 or wild-type retrotransposase TgR2.
15. The engineered retrotransposase accordimg to item 14, wherein,
   the sequence of the exonuclease is shown as SEQ ID NO.8 or has at least 70%, 75%, 80%, 85%, 90%, 95%, 96%, 97%, 98%, or 99% identity with the amino acid sequence of SEQ ID No. 8; or
   the sequence of the single-stranded DNA-binding protein is shown as SEQ ID NO.9 or has at least 70%, 75%, 80%, 85%, 90%, 95%, 96%, 97%, 98%, or 99% identity with the amino acid sequence of SEQ ID No. 9; or
   the sequence of the chromatin-modulating peptide segment or high mobility peptide segment is shown as SEQ ID NO.10 or has at least 70%, 75%, 80%, 85%, 90%, 95%, 96%, 97%, 98%, or 99% identity with the amino acid sequence of SEQ ID No. 10; or
   the sequence of the RNaseH domain is shown as SEQ ID NO.11 or has at least 70%, 75%, 80%, 85%, 90%, 95%, 96%, 97%, 98%, or 99% identity with the amino acid sequence of SEQ ID No.11.
16. The engineered retrotransposase according to any one of items 1 to 15, wherein, the engineered retrotransposase comprises an amino acid sequence as shown in any one of SEQ ID NO.2-7, 12, 13, 38, 40, 42, 43, 45, 47, 49, 51, 55-84; or comprises an amino acid sequence having at least 70%, 75%, 80%, 85%, 90%, 95%, 96%, 97%, 98%, or 99% identity with the amino acid sequence as shown in any one of SEQ ID No. 2-7, 12, 13, 38, 40, 42, 43, 45, 47, 49, 51, 55-84; or
   the engineered retrotransposase is a truncated form of any one of the amino acid sequences as shown in SEQ ID NOs. 1-7, 13, 38, 40, 42, 43, 45, 47, 49, 51, and 55-84, preferably a sequence obtained by truncating any one of the amino acid sequences of SEQ ID NO. 1-7, 13, 38, 40, 42, 43, 45, 47, 49, 51, and 55-84, specifically by removing amino acids corresponding to the 1^{st} to 80^{th}, or 1^{st} to 190^{th}, or 1^{st} to 220^{th}, or 390^{th} to 500^{th}, or 1100^{th} to 1120^{th} amino acids of the wild-type sequence SEQ ID NO. 1.
17. A system for modifying DNA, comprising:
   a retrotransposase, the retrotransposase is the wild-type retrotransposase TgR2 as shown in SEQ ID NO.1 or the retrotransposase according to any one of items 1 to 16, or a nucleic acid encoding the wild-type transposase TgR2 as shown in SEQ ID NO. 1 or the retrotransposase according to any one of items 1 to 16; and
   a donor RNA or a nucleic acid encoding the donor RNA, the donor RNA comprises: a sequence that binds to the retrotransposase and a heterologous sequence,
   preferably, the heterologous sequence is of at least 1-50000 bases, e.g., 1nt and above, 10nt and above, 50nt and above, 60nt and above, 70nt and above, 80nt and above, 90nt and above, 100nt and above, 150nt and above, 200nt and above, 250nt and above, 300nt and above, 350nt and above, 400nt and above, 450nt and above, 500nt and above, 550nt and above, 600nt and above, 650nt and above, 700nt and above, 750nt and above, 800nt and above, 850nt and above, 900nt and above, 950nt and above, 1000nt and above, 1100nt and above, 1200nt and above, 1300nt and above, 1400nt and above, 1500nt and above, 1600nt and above, 1700nt and above, 1800nt and above, 1900nt and above, 2000nt and above, 2100nt and above, 2200nt and above, 2300nt and above, 2400nt and above, 2500nt and above, 2600nt and above, 2700nt and above, 2800nt and above, 2900nt and above, 3000nt and above, 3500nt and above, 4000nt and above, 4500nt and above, 5000nt and above, 5500nt and above, 6000nt and above, 6500nt and above, 7000nt and above, 7500nt and above, 8000nt and above, 8500nt and above, 9000nt and above, 9500nt and above, 10000nt and above, 15000nt and above, 20000nt and above, 25000nt and above, 30000nt and above, 35000nt and above, 40000nt and above, 45000nt and above;
   more preferably, the system comprises a nuleic acid encoding VPX protein or the VPX protein itself.
18. The system according to item 17, wherein, the heterologous sequence comprises one or more of the following: a sequence encoding a polypeptide or a non-coding RNA sequence, a sequence containing a promoter or an enhancer, a sequence encoding one or more introns, and a transcription termination sequence;
   preferably, the peptide is a therapeutic polypeptide or a mammalian polypeptide; more preferably the peptide is a therapeutic polypeptide, a membrane protein, an intracellular protein, an extracellular protein, a structural protein, a signaling protein, a regulatory protein, a transport protein, an organelle protein, a sensory protein, a motor protein, a defense protein, a storage protein, a reporter protein, an antibody, an enzyme, or a coagulation factor; more preferably the polypeptide comprises 20-10000 amino acids, e.g., 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 550, 600, 650, 700, 750, 800, 850, 900, 950, 1000, 1100, 1200, 1300, 1400, 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 4000, 4100, 4200, 4300, 4400, 4500, 4600, 4700, 4800, 4900, 5000, 5100, 5200, 5300, 5400, 5500, 5600, 5700, 5800, 5900, 6000, 6100, 6200, 6300, 6400, 6500, 6600, 6700, 6800, 6900, 7000, 7100, 7200, 7300, 7400, 7500, 7600, 7700, 7800, 7900, 8000, 8100, 8200, 8300, 8400, 8500, 8600, 8700, 8800, 8900, 9000, 9100, 9200, 9300, 9400, 9500, 9600, 9700, 9800, or 9900 amino acids;
   preferably, the intracellular protein is selected from cytoplasmic proteins, nuclear proteins, organelle proteins, mitochondrial proteins, or lysosomal proteins,
   more preferably, the sequence encoding the polypeptide contains one or more introns.
19. The system according to item 17 or 18, wherein, the donor RNA further comprises a homologous domain, preferably the homologous domain comprises a first homologous domain and a second homologous domain;
   more preferably, the first homologous domain is defined as 3 or more bases located at the 5' end of the donor RNA that has 100% identity with the target DNA strand, and the second homologous domain is defined as 3 or more bases located at the 3' end of the donor RNA that has 100% identity with the target DNA strand; preferably the target DNA is a genomic safe harbor (GSH) site or a genomic Natural Harbor^{™} site.
20. The system according to any one of items 17 to 19, wherein, the nucleic acid encoding the retrotransposase according to any one of items 1 to 16 and the donor RNA or the nucleic acid encoding the donor RNA are separated nuleic acids, preferably the donor RNA does not encode retrotransposase; more preferably, the donor RNA comprises one or more chemical modifications; or
   the nucleic acid encoding the retrotransposase according to any one of items 1 to 16 is covalently linked to the donor RNA or the nucleic acid encoding the donor RNA, preferably, the nucleic acid encoding the retrotransposase according to any one of items 1 to 16 forms fused nucleic acid with the donor RNA or the nucleic acid encoding the donor RNA, more preferably, the fused nucleic acid comprises RNA or DNA;
   more preferably, the nucleic acid encoding the retrotransposase according to any one of items 1 to 16, the donor RNA or the nucleic acid encoding the donor RNA, and the nucleic acid encoding VPX protein are separated nuleic acids; or
   the nucleic acid encoding the retrotransposase according to any one of items 1 to 16 and the nucleic acid encoding VPX protein are covanlently linked or fused nucleic acids, and are separated nucleic acid from the donor RNA or the nucleic acid encoding the donor RNA;
   the nucleic acid encoding the retrotransposase according to any one of items 1 to 16 is covanlently linked or fused to the donor RNA or the nucleic acid encoding the donor RNA, and is separated nucleic acid from the nucleic acid encoding the VPX protein;
   the donor RNA or the nucleic acid encoding the donor RNA is covanlently linked or fused with the nucleic acid encoding VPX protein, and is separated from the nucleic acid encoding the retrotransposase according to any one of items 1 to 16; or
   the nucleic acid encoding the retrotransposase according to any one of items 1 to 16, the donor RNA or the nucleic acid encoding the donor RNA, and the nucleic acid encoding the VPX protein are covanlently linked or fusedtogether,
   more preferably, the fused nucleic acids comprise RNA or DNA.
21. The system according to any one of items 17 to 20, wherein the donor RNA comprises:
   an optional 5' untranslated region (5'UTR) that binds to the retrotransposase,
   a 3' untranslated region (3'UTR) that binds to the retrotransposase,
   a heterologous sequence, and
   a promoter that operably linked to the heterologous sequence,
   preferably, the promoter is located between the 5' untranslated region (5'UTR) that binds to the retrotransposase and the heterologous sequence; or preferably, the promoter is located between the 3' untranslated region (3'UTR) that binds to the retrotransposase and the heterologous sequence.
22. The system according to item 21, wherein, the heterologous sequence comprises an open reading frame oriented in the 5' to 3' direction on the donor RNA or its reverse complementary sequence; or the heterologous sequence comprises an open reading frame oriented in the 3' to 5' direction on the donor RNA or its reverse complementary sequence.
23. The system according to any one of items 17 to 14, wherein, the donor RNA further comprises a nuclear localization signal, or the nucleic acid encoding the retrotransposase according to any one of items 1 to 8 comprises a nuclear localization signal and/or a nucleolar localisation signal and/or a nuclear export signal.
24. The system according to any one of items 9 to 23, wherein, the nucleic acid encoding the retrotransposase according to any one of items 1 to 16 and the nucleic acid encoding the donor RNA are present in a ratio of 10:1-1:10, e.g., in a ratio of 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, or 1:10.
25. The system according to any one of items 17 to 24, wherein, the donor RNA comprises a stem-loop sequence or helix located 5' to the pseudoknot sequence; preferably comprises one or more (e.g., 2, 3 or more) stem-loop sequences or helices located 3' to the pseudoknot sequence, for example, at the 3' end of the pseudoknot sequence and the 5' end of the heterologous sequence; more preferably, the donor RNA of the pseudoknot has catalytic activity, e.g., RNA cleavage activity, e.g., cis-RNA cleavage activity, or
   the donor RNA comprises at least one stem-loop sequence or helix, for example, 1, 2, 3, 4, 5 or more stem-loop sequences, hairpin loops, or helix sequences, located, for instance, at the 3' end of the heterologous sequence.
26. The system according to any one of items 17 to 25, wherein,
   the 5' untranslated region (5'UTR) in the donor RNA that binds to the retrotransposase has at least 70%, 75%, 80%, 85%, 90%, 95%, 96%, 97%, 98%, 99%, 100% identity with the nucleotide sequence as defined in any one of SEQ ID No. 16, 27, 29, 31, 33, 35, 37, 39, and 41;
   the 3' untranslated region (3'UTR) in the donor RNA that binds to the retrotransposase has at least 70%, 75%, 80%, 85%, 90%, 95%, 96%, 97%, 98%, 99%, or 100% identity with the nucleotide sequence as defined in any one of SEQ ID No. 17, 32, 34, 44, 46, 48, 50, 52, and 54; or
   the amino acid sequence of the VPX protein has at least 70%, 75%, 80%, 85%, 90%, 95%, 96%, 97%, 98%, 99%, or 100% identity with the amino acid sequence as defined in any one of SEQ ID No. 85, 86 and 87.
27. The system according to any one of items 17 to 26, wherein, the donor RNA comprises the following structures from its 5' end to 3' end:
   a first homologous domain,
   a 5' untranslated region (5'UTR) that binds to the retrotransposase,
   a heterologous sequence,
   a 3' untranslated region (3'UTR) that binds to the retrotransposase, and
   a second homologous domain;
   preferably, the first homologous domain is 1 and above, or 2 and above, or 5 and above, or 10 and above, or 20 and above, or 30 and above, or 40 and above, or 50 and above, or 60 and above, or 70 and above, or 80 and above, or 90 and above, or 100 and above, or 110 and above, or 120 and above, or 130 and above, or 140 and above, or 150 and above bases located at the 5' end of the donor RNA and has 100% identity with target DNA strand; the second homologous domain is 1 and above, or 2 and above, or 5 and above, or 10 and above, or 20 and above, or 30 and above, or 40 and above, or 50 and above, or 60 and above, or 70 and above, or 80 and above, or 90 and above, or 100 and above, or 110 and above, or 120 and above, or 130 and above, or 140 and above, or 150 and above bases located at the 3' end of the donor RNA and has 100% identity with target DNA strand;
   more preferably, the 5' untranslated region (5' UTR) in the donor RNA that binds to the retrotransposase has at least 70%, 75%, 80%, 85%, 90%, 95%, 96%, 97%, 98%, 99%, or 100% identity with any one of the nucleotide sequences as defined in SEQ ID No. 16, 27, 29, 31, 33, 35, 37, 39, and 41;
   the 3' untranslated region (3'UTR) in the donor RNA that binds to the retrotransposase has at least 70%, 75%, 80%, 85%, 90%, 95%, 96%, 97%, 98%, 99%, 100% identity with any one of the nucleotide sequences as defined in SEQ ID No. 17, 32, 34, 44, 46, 48, 50, 52, and 54.
28. The system according to any one of items 17 to 27, wherein,
   the 5' untranslated region (5'UTR) that binds to the retrotransposase is a non-natural 5' untranslated region (5'UTR); or
   the 3' untranslated region (5'UTR) that binds to the retrotransposase is a non-natural 3' untranslated region (3'UTR);
   more preferably, the non-natural 5' untranslated region (5'UTR) comprises nucleotide insertions, deletions, and/or substitutions, relative to the natural 5'UTR sequence;
   more preferably, the non-natural 3' untranslated region (3'UTR) comprises nucleotide insertions, deletions, and/or substitutions, relative to the natural 3'UTR sequence;
   more preferably, the non-natural 5' untranslated region (5'UTR) has at least 70%, 75%, 80%, 85%, 90%, 95%, 96%, 97%, 98%, 99%, or 100% identity with the nucleotide sequence as defined in one of SEQ ID No. 16, 27, 29, 31, 33, 35, 37, 39, and 41;
   more preferably, the non-natural 3' untranslated region (3'UTR) has at least 70%, 75%, 80%, 85%, 90%, 95%, 96%, 97%, 98%, 99%, or 100% identity with the nucleotide sequence as defined in any one of SEQ ID No. 17, 32, 34, 44, 46, 48, 50, 52, and 54.
29. The system according to any one of items 17 to 27, wherein, the heterologous sequence is inserted into target sites within the cellular genome at an average copy number of at least 0.01, 0.025, 0.05, 0.075, 0.1, 0.15, 0.2, 0.25, 0.3, 0.4, 0.5, 0.75, 1, 1.25, 1.5, 1.75, 2, 2.5, 3, 4 or 5 copies per genome, preferably inserted at only one target site within the genome.
30. The system according to any one of items 17 to 29, wherein the system enables the heterologous sequences to be inserted into the the target sites of about 1%-100% of the cells (e.g., about 1%-10%, 10%-20%, 20%-30%, 30%-40%, 40%-50%, 50%-60%, 60%-70%, 70%-80%, 80%-90% or 90%-100% of the cells) within a cell population that contacts with the system (e.g., in an insterted copy number of one or more than one), e.g., as measured using single-cell ddPCR, or
   the system enables the heterologous sequences to be inserted into the target sites of about 1%-100% of the cells (e.g., about 1%-10%, 10%-20%, 20%-30%, 30%-40%, 40%-50%, 50%-60%, 60%-70%, 70%-80%, 80%-90% or 90%-100% of the cells) within the cell population that contacts with the system in an insterted copy number of 1, e.g., as measured using colony isolation and ddPCR.
31. The system according to any one of items 17 to 30, wherein the system enables the heterologous sequence to be inserted into the target sites (on-target insertion) within the cell population at a rate greater than into non-target sites (off-target insertion) within the cell population, wherein the ratio of the on-target insertion to the off-target insertion is greater than 10:1, 20:1, 30:1, 40:1, 50:1, 60:1, 70:1, 80:1, 90:1, 100:1, 200:1, 500:1 or 1,000:1.
32. The system according to any one of items 17 to 31, wherein,
   the sequence of the first homologous domain is shown as SEQ ID NO.15, 19, 21, 23, 25 or 28, or has at least 70%, 75%, 80%, 85%, 90%, 95%, 96%, 97%, 98%, 99% identity with the nucleotide sequence shown as SEQ ID No. 15, 19, 21, 23, 25 or 28; or
   the sequence of the second homologous domain is shown as SEQ ID NO.18, 20, 22, 24, 26 or 36, or has at least 70%, 75%, 80%, 85%, 90%, 95%, 96%, 97%, 98%, 99% identity with the nucleotide sequence shown as SEQ ID NO.18, 20, 22, 24, 26 or 36.
33. A non-natural 5' untranslated region (5'UTR), it has nucleotide insertions, deletions, and/or substitutions, relative to natural 5'UTR sequence; preferably has at least 70%, 75%, 80%, 85%, 90%, 95%, 96%, 97%, 98%, 99%, or 100% identity with the nucleotide sequence shown as SEQ ID No. 16, 27, 29, 31, 33, 35, 37, 39, or 41.
34. A non-natural 3' untranslated region (3'UTR), it has nucleotide insertions, deletions, and/or substitutions, relative to natural 3'UTR sequence; preferably has at least 70%, 75%, 80%, 85%, 90%, 95%, 96%, 97%, 98%, 99%, or 100% identity with the nucleotide sequence shown as SEQ ID No. 17, 32, 34, 44, 46, 48, 50, 52, or 54.
35. An engineered transposable element, comprising the following from the 5' end to 3' end:
   a 5' untranslated region (5'UTR), a heterologous sequence and a 3' untranslated region (3'UTR),
   wherein the 5' untranslated region comprises a sequence selected from the nucleotide sequence having at least 70%, 75%, 80%, 85%, 90%, 95%, 96%, 97%, 98%, 99%, or 100% identity with SEQ ID No. 16, 27, 29, 31, 33, 35, 37, 39, or 41; or
   wherein the 3' untranslated region comprises a sequence selected from the nucleotide sequence having at least 70%, 75%, 80%, 85%, 90%, 95%, 96%, 97%, 98%, 99%, or 100% identity with SEQ ID No. 17, 32, 34, 44, 46, 48, 50, 52, or 54,
   preferably, comprising the following from the 5' end to 3' end: a first homologous domain, a 5' untranslated region (5'UTR) that binds to the retrotransposase, a heterologous sequence, a 3' untranslated region (3'UTR) that binds to the retrotransposase, and a second homologous domain,
   more preferably, the sequence of the first homologous domain has at least 70%, 75%, 80%, 85%, 90%, 95%, 96%, 97%, 98%, or 99% identity with the nucleotide sequence shown as SEQ ID NO.15, 19, 21, 23, 25 or 28; or
   the sequence of the second homologous domain has at least 70%, 75%, 80%, 85%, 90%, 95%, 96%, 97%, 98%, or 99% identity with the nucleotide sequence of SEQ ID NO.18, 20, 22, 24, 26 or 36.
36. The element according to item 35, wherein, the element is the donor RNA or the nucleic acid encoding the donor RNA as defined in items 17 to 32.
37. A host cell, comprising the engineered retrotransposase according to any one of items 1 to 16 or the element according to item 35 or 36 or the system according to any one of items 17 to 32; preferably the host cell is a mammalian cell or a plant cell; more preferably is a human cell.
38. A method for modifying a target DNA strand in a cell, tissue or subject, comprising administering to the cell, tissue or subject the engineered retrotransposase according to any one of items 1 to 16, the element according to item 35 or 36, or the system according to any one of items 17 to 32, wherein the system reverse transcribes the donor RNA sequence into the target DNA strand, thereby modifying the target DNA strand in the cell, tissue or subject.
39. The method according to item 38, wherein the cell or tissue is a mammalian cell or tissue, preferably a human cell or tissue, and the subject is a mammal, preferably a human.
40. The method according to item 39, wherein, the cell is a fibroblast, a primary cell or a non-immortalized cell.
41. The method according to any one of items 38 to 40, wherein the method is performed *in vivo* or *in vitro.*
42. A method for modifying the genome of a mammalian cell or inserting DNA into the mammalian genome, comprising applying the engineered retrotransposase according to any one of items 1 to 16, the element according to item 35 or 36, or the system according to any one of items 17 to 32 to the cell, preferably the mammalian is a human.
43. The method according to item 42, wherein the method results in the addition of an exogenous DNA sequence of at least 5, 10, 20, 50, 100, 200, 500, 1000, 2000, 3000, 4000, 5000, 6000, 7000, 8000, 9000, or 10000 base pairs to the genome of the mammal.
44. The method according to any one of items 38 to 43, wherein the cell is part of a tissue; or the mammalian cell is euploid, is not immortalized, is part of an organism, is a primary cell, is non-dividing, is a hepatocyte, or is derived from a subject with a genetic disease.
45. The method according to any one of items 38 to 44, wherein,
   the method comprises contacting a cell, tissue or subject with the retrotransposase according to any one of items 1 to 16 or a nucleic acid encoding the retrotransposase according to any one of items 1 to 16 and a donor RNA or the nucleic acid encoding the donor RNA,
   preferably, the contacting comprises contacting the cell, tissue or subject with a plasmid, virus, virus-like particle, virion, liposome, vesicle, exosome, or lipid nanoparticle;
   more preferably, the contacting comprises using a non-viral delivery method, e.g., electroporation.
46. The method according to item 45, wherein,
   the contacting comprises intravenous administration to the subject, preferably at least twice, the retrotransposase according to any one of items 1 to 16 or a nucleic acid encoding the retrotransposase according to any one of items 1 to 16 and a donor RNA or a nucleic acid encoding the donor RNA.
47. The method according to any one of items 38 to 46, wherein,
   the retrotransposase according to any one of items 1 to 16 or the nucleic acid encoding the retrotransposase according to any one of items 1 to 16, and the donor RNA or the nucleic acid encoding the donor RNA, are administrated separately; or
   the retrotransposase according to any one of items 1 to 16 or the nucleic acid encoding the retrotransposase according to any one of items 1 to 16, and the donor RNA or the nucleic acid encoding the donor RNA are administrated together.
48. A nucleic acid encoding the retrotransposase according to any one of items 1 to 16.
49. A vector comprising the nucleic acid according to item 48.
50. A host cell comprising the vector according to item 49.
51. A pharmaceutical composition, comprising the system according to any one of items 17 to 32, the nuleic acid according to item 48, the vector according to item 49, or the host cell according to item 37 or 50; preferably the system is formulated in a pharmaceutically acceptable carrier; more preferably, the vector is a vesicle (including liposomes, natural or synthetic lipid bilayers, exosomes), a lipid nanoparticle, a virus or a plasmid vector.

### Effects

The system and method established by the present application enable gene writing at the DNA-level using only RNA donors, representing a technical innovation. By integrating a coding gene into an RNA sequence template, the system and method herein can fulfill, but are not limited to:
The therapeutic needs, for example, providing expression of a therapeutic transgene in individuals with loss-of-function mutations, replacing gain-of-function mutations with a normal transgene, providing regulatory sequences to eliminate the expression of gain-of-function mutations, and/or controlling the expression of operably linked genes, transgenes, and the systems thereof. In certain embodiments, the RNA sequence template encodes a promoter region specific to the therapeutic needs of the host cell, such as a tissue-specific promoter or enhancer. In other embodiments, the promoter may be operably linked to the coding sequence.

The preparation needs for functional cells, e.g., the integration of specific functional biomacromolecules (e.g., chimeric antigen receptors (CARs) and the like) into immune cells to confer novel tumor-killing functions.

New needs in crop breeding. For example, by integrating specific genes into plant callus tissues, new economic traits (e.g., stress tolerance, insect resistance, etc.) can be conferred to plants.

### BRIEF DESCRIPTION OF THE FIGURES

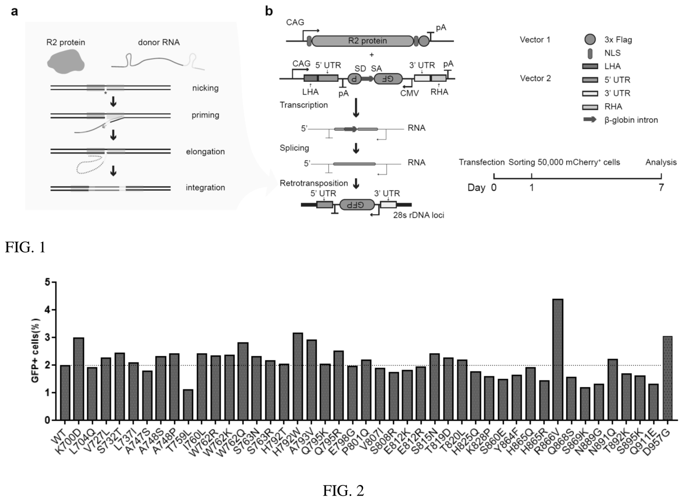

FIG. 1a: shows the integration mechanism of the R2 retrotransposase system. The R2 retrotransposase system consists of two parts, i.e., the retrotransposase and the donor RNA. The R2 retrotransposase has domains for DNA binding, endonuclease activity and retrotranscriptase activity; the donor RNA comprises a left homology arm, a 5'UTR sequence, the target sequence, a 3'UTR sequence, and a right homology arm. When the donor RNA is carried by the R2 retrotransposase to the target DNA sequence, the endonuclease domain of the R2 retrotransposase can cleave the target DNA, generating a nicked strand. This cleavage subsequently initiates reverse transcription of the donor RNA from the exposed 3' end of the nick, leading to the integration of the newly synthesized DNA strand into the genomic DNA.
FIG. 1b: shows the estabilishment of the GFP reporting system. The GFP reporting system consists of an R2 retrotransposase expression plasmid and a donor RNA expression plasmid, wherein the donor RNA ccomprises a left homology arm, a 5'UTR sequence, a 3'UTR sequence, a reverse-oriented expression cassette between the 5'UTR sequence and the 3'UTR sequence, and a right homology arm, wherein the expression cassette is driven by a CMV promoterand contains a GFP coding sequence interrupted by a reverse-oriented intron sequence. Transfection of this reporting system results in the separate expression of the R2 retrotransposase and the donor RNA. Following transcription initiated by the CAG promoter, the intron sequence within the donor RNA is spliced out.Consequently, the GFP coding sequence within the CMV promotor-driven expression cassette is restored to normal expression at the RNA level. However, at this stage, the GFP sequence exists as an antisense RNA strand incapable of translating into GFP protein. Only when the retrotransposase permanently integrates the donor RNA lacking the intron into the cellular DNA via its reverse transcriptase activity, can the reverse expression cassette initiated by the CMV promotor express normal intron-free GFP mRNA, therby expressing GFP protein with green fluorescence. Therefore, after transfecting this GFP reporting system into 293T cells and culturing for 24 hours, 50,000 mCherry-positive cells were sorted by flow cytometry. Following an additional 6 days of culture, fluorescence microscopy and flow cytometry were employed to detect the presence and proportion of the GFP-positive cells.
FIG. 2: shows the improved gene integration efficiency of wild-type TgR2 through introducing amino acid point mutations according to homologous sequence alignment. Multiple sequence alignment results from the same branch were extracted based on the phylogenetic position of the TgR2 protein. Amino acids in TgR2 were mutated to other amino acid sequences corresponding to the corresponding positions in the multiple sequence alignment. The test results of GFP reporting system indicated that, compared to the wild-type TgR2, among the 36 selected amino acid sites subjected to mutation, 6 mutants with specific amino acid substitutions - K700D, W762Q, H792W, A793V, R866V, D957G - significantly increased gene integration efficiency, and the remaining mutants showed no significant increase, minimal increase, or at decrease in efficiency.

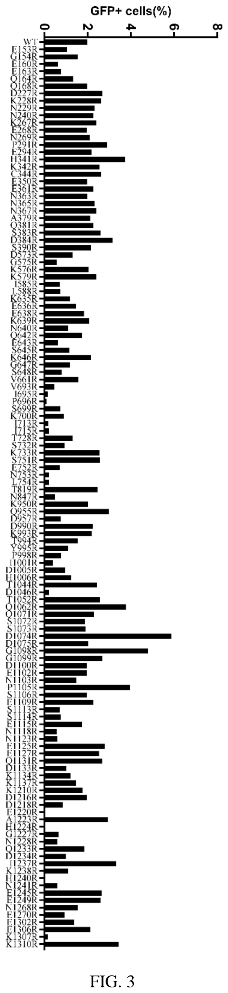

FIG. 3: shows introduction of arginine point mutations based on predicted 3D structure enhances the gene integration efficiency of wild-type TgR2. Testing using a GFP reporting system revealed that among the 132 selected amino acid residuess: E153, G154, E160, E163, Q164, Q168, D227R, K228R, N229R, N240R, K267R, E268R, N269R, P291R, E294R, H341R, K342R, C344R, E350R, E361R, N363R, N365R, N367R, A379R, Q381R, S383R, D384R, S390R, D573, G575, K576, K579, I585, L588, K635, E636, E638, K639, N640, Q642, E643, S645, K646, G647, S648, V661, V693, I695, P696, S699, K700, I713, I715, T728, S732, K733, S751, E752, N753, L754, T819, N847, K950, Q955, D957, D990, K993, T994, Y995, P998, I1001, D1005, H1006, T1044, D1046, T1052, Q1062, Q1071, S1072, S1073, D1074, D1075, G1098, G1099, D1100, E1102, N1103, P1105, S1106, E1109, S1113, S1114, E1115, N1118, N1123, E1125, E1127, Q1131, D1133, K1134, K1137, K1210R, D1216R, D1218R, E1220R, A1223R, H1224R, G1227R, N1228R, Q1233R, D1234R, I1237R, K1238R, H1240R, N1241R, E1245R, E1249R, N1268R, E1270R, E1302R, E1306R, K1307R, and K1310R, the gene integration efficiency was significantly enhanced in 10 arginine-substituted mutants (Q168R, T819R, Q955R, Q1062R, D1074R, G1098R, P1105R, E1125R, E1127R, Q1131R) compared to wild-type TgR2. For the remaining mutants, the enhancement was negligible, non-significant, or the efficiency was even reduced.

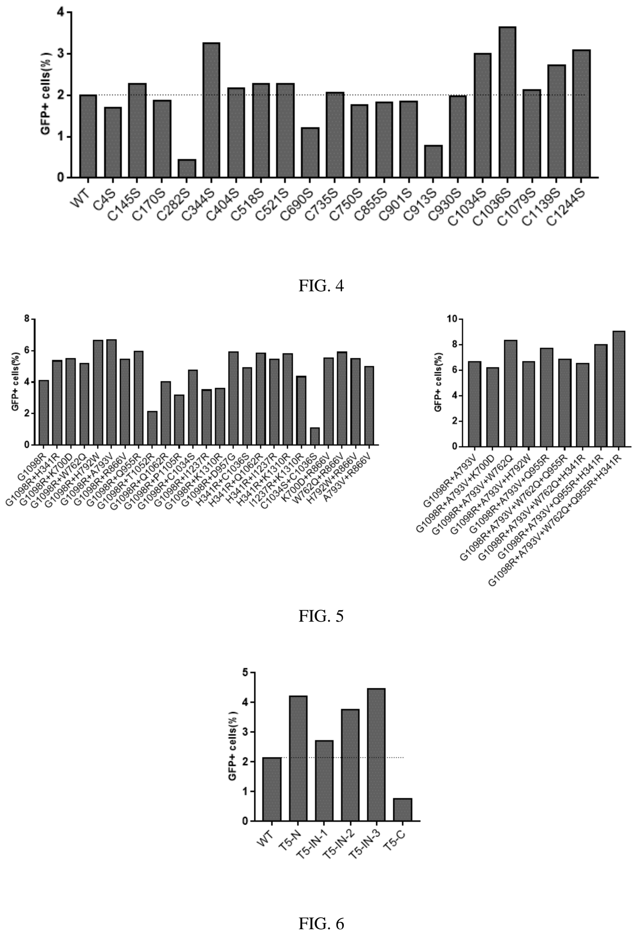

FIG. 4: To enhance the integration efficiency of TgR2, cysteine (C) residues in the protein sequence were substituted with serine (S). 20 cysteine (C) residues in the R2Tg protein were individually mutated to serine (S), generating the corresponding mutant proteins: C4, C145, C170, C282, C344, C404, C518, C521, C690, C735, C750, C855, C901, C913, C930, C1034, C1036, C1079, C1139, and C1244. Using the GFP reporting system designed in Example 1, compared to the wild-type TgR2 protein, the mutant wherein there were 4 mutant proteins: C344S, C1034S, C1036S, C1244S that significantly increased gene integration efficiency, and the remaining mutants exhibited either no improvement, minimal enhancement, or a decrease in efficiency.
FIG. 5: The combination of point mutations which were screened for increased efficiency yielded combinational proteins having significantly increased integration. The most efficient mutant G1098R, identified in Example 2, Example 3, Example 4, was selected as the baseline and combined with other high-efficiency mutant proteins. Compared with the single-point mutant G1098R, several mutant combinations: G1098R+H341R, G1098R+K700D, G1098R+W762Q, G1098R+H792W, G1098R+A793V, G1098R+R866V, an G1098R+Q955R significantly increased gene integration efficiency, demonstrating an obvious superimposed effect. The G1098R+A793V mutant having the highest integration efficiency was further combined with additional point mutations. Compared with the G1098R+A793V mutant, the mutants G1098R+A793V+W762Q, G1098R+A793V+Q955R, G1098R+A793V+Q955R+H341R, and G1098R+A793V+W762Q+Q955R+H341R exhibited a significant increase in gene integration efficiency.
FIG. 6: Conjugation of T5 exonuclease increases the gene integration efficiency of wild-type TgR2. T5 exonuclease was conjugated to the N-terminal or inserted after amino acid no. 191 (designated as IN-1), amino acid no. 432 (designated as IN-2), amino acid no. 1112 (designated as IN-3), or conjugated to the C-terminal of wild-type TgR2. It should be noted that two additional glycines (-GG-) were added flanking the T5 exonuclease inserted after amino acid no. 191, amino acid no. 432, and amino acid no. 1112. Compared to wild-type TgR2, results from the GFP reporting system designed in Example 1 showed that conjugation or insertion of T5 exonuclease at N-terminal, IN-2 and IN-3 positions of wild-type TgR2 significantly increased gene integration efficiency. Insertion of T5 exonuclease at the IN-1 position of wild-type TgR2 resulted in a modest increase in gene integration efficiency, whereas conjugation to T5 exonuclease at the C-terminal of the wild-type TgR2 led to a decrease in gene integration efficiency.

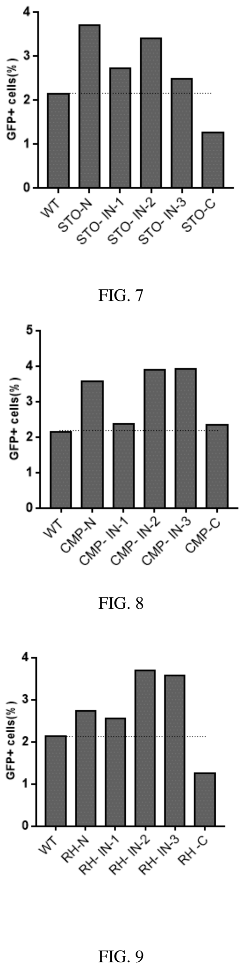

FIG. 7: Conjugation of the Sto single-stranded DNA-binding protein can increases the gene integration efficiency of wild-type TgR2. The Sto single-stranded DNA-binding protein was conjugated or inserted at N-terminal position, after amino acid no. 191 (designated as IN-1), after amino acid no. 432 (designated as IN-2), after amino acid no. 1112 (designated as IN-3) or at C-terminal position of wild-type TgR2, respectively. It should be noted that, two additional glycines (-GG-) were added flanking the Sto single-stranded DNA-binding protein inserted after amino acid no. 191, amino acid no. 432, and amino acid no. 1112, respectively. Compared to wild-type TgR2, results from the GFP reporting system designed in Example 1 showed that conjugation or insertion of Sto single-stranded DNA-binding protein at the N-terminal, IN-1 and IN-2 positions of wild-type TgR2 significantly increased gene integration efficiency. Insertion of Sto single-stranded DNA-binding protein at the IN-3 position of wild-type TgR2 resulted in a modest increase in gene integration efficiency, whereas conjugation to Sto single-stranded DNA-binding protein at the C-terminal of wild-type TgR2 led to a decrease in gene integration efficiency.
FIG. 8: Conjugation of the CMP epigenetic peptide segment can increase the gene integration efficiency of wild-type TgR2. The CMP epigenetic peptide segment was conjugated or inserted at N-terminal position, after amino acid no. 191 (designated as IN-1), after amino acid no. 432 (designated as IN-2), after amino acid no. 1112 (designated as IN-3) or at C-terminal position of wild-type TgR2, respectively. It should be noted that, two additional glycines (-GG-) were added flanking the CMP chromatin-modulating peptide segment inserted after amino acid no. 191, amino acid no. 432, and amino acid no. 1112, respectively. Compared to wild-type TgR2, results from the GFP reporting system designed in Example 1 showed that, conjugation or inserting of CMP epigenetic peptide segment at the N-terminal, IN-2 and IN-3 positions of wild-type TgR2 significantly increased gene integration efficiency. Conjugation or insertion of CMP epigenetic peptide segment at the IN-1 and C-terminal positions of wild-type TgR2 slightly increased gene integration efficiency.
FIG. 9: Conjugation of the RNaseH domain of MLV reverse transcriptase can increase the gene integration efficiency of wild-type TgR2. The RNaseH domain of MLV reverse transcriptase was conjugated to or inserted at the N-terminal, after amino acid no. 191 (designated as IN-1), after amino acid no. 432 (designated as IN-2), after amino acid no. 1112 (designated as IN-3) or at C-terminal of wild-type TgR2, respectively. It should be noted that, two additional glycines (-GG-) were added flanking the RNaseH domain inserted after amino acid no. 191, amino acid no. 432, and amino acid no. 1112, respectively. Compared to wild-type TgR2, results from the GFP reporting system designed in Example 1 showed that insertion of the MLV reverse transcriptase RNaseH domain at the IN-2 and IN-3 positions of wild-type TgR2 significantly increased gene integration efficiency. Conjugation or insertion at the MLV reverse transcriptase RNaseH domain at the N-terminal and IN-1 positions of wild-type TgR2 resulted in a modest increase in gene integration efficiency, whereas conjugation of the MLV reverse transcriptase RNaseH domain at the C-terminal of wild-type TgR2 led to a decrease in gene integration efficiency.

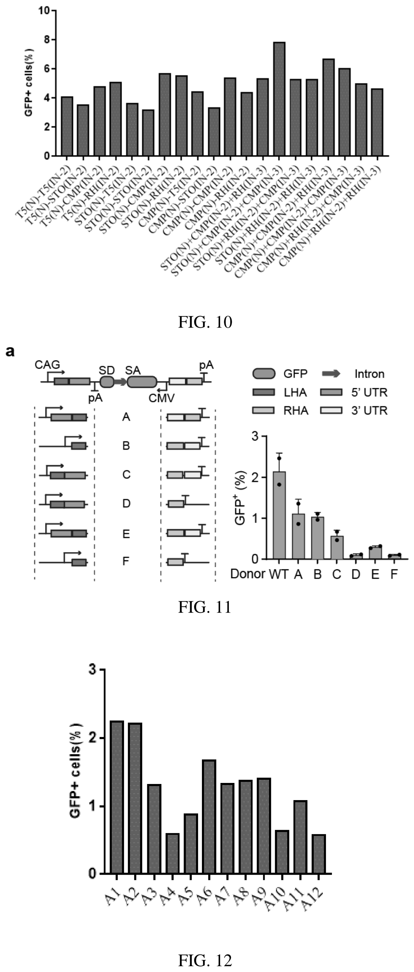

FIG. 10: Conjugation of multiple domains further increases the gene integration efficiency of wild-type TgR2. Any two selected from the group consisting of T5 exonuclease, the single-stranded DNA-binding protein Sto7d from Sulfolobus tokodaii bacteria (abbreviated as Sto), a chromatin-modulating peptide segment (CMP), and the RNaseH domain of MLV reverse transcriptase (abbreviated as RH)(SEQ) were conjugated or inserted at either the N-terminal or after amino acid no. 432 position (designated as IN-2) of wild-type TgR2. Alternatively, CMP or RH was optionally inserted at three positions: the N-terminal, after amino acid no. 432 (designated as IN-2), and after amino acid no. 1112 (designated as IN-3) of TgR2. Wherein the gene integration efficiencies were significantly increased for the combinations T5(N)-CMP(IN-2), T5(N)-RH(IN-2), STO(N)-CMP(IN-2), STO(N)-RH(IN-2), and CMP(N)-CMP(IN-2). Furthermore, the combinations STO(N)+CMP(IN-2)+CMP(IN-3), CMP(N)+CMP(IN-2)+RH(IN-3), and CMP(N)+CMP(IN-2)+CMP(IN-3) demonstrated a significant increase in gene integration efficiency compared to the previous combinations. The remaining combinations showed minimal or no increase.
FIG. 11: Effect of changing the order of the 5'homology arm, 5'UTR, 3'homology arm, and 3'UTR on the gene integration efficiency of wild-type TgR2. In this figure, WT represents the Donor used for wild-type R2Tg, A represents the donor with the order of the 5'homology arm and 5'UTR swapped, B represents the donor with the 5'UTR deleted, C represents the donor with the order of the 3'homology arm and 3'UTR swapped, D represents the donor with the 3'UTR deleted, E represents the donor with the oder of both the 5'homology arm/5'UTR and the order of 3'homology arm/3'UTR swapped, F represents the donor with both the 5'UTR and 3'UTR deleted. Based on the GFP reporting system designed in Example 1, the results showed that, compared to the wild-type donor, the gene integration efficiency was significantly reduced for all six donors (A, B, C, D, E, F) in which the order of these elements was altered or the UTR elements were deleted.
FIG. 12: Effect of changing the length of the 5'homology arm and 3'homology arm on the gene integration efficiency of wild-type TgR2. Wherein A1 represents a donor with a 150bp 5'homology arm (LHA) and a 150bp 3'homology arm (RHA), A2 represents a donor with a 100bp 5'homology arm (LHA) and a 100bp 3'homology arm (RHA), A3 represents a donor with a 50bp 5'homology arm (LHA) and a 50bp 3'homology arm (RHA), A4 represents a donor with a 20bp 5'homology arm (LHA) and a 20bp 3'homology arm (RHA), A5 represents a donor with a 10bp 5'homology arm (LHA) and a 10bp 3'homology arm (RHA), A6 represents a donor with a 100bp 5'homology arm (LHA) and a 50bp 3'homology arm (RHA), A7 represents a donor with a 100bp 5'homology arm (LHA) and a 20bp 3'homology arm (RHA), A8 represents a donor with a 100bp 5'homology arm (LHA) and a 10bp 3'homology arm (RHA), A9 represents a donor with a 50bp 5'homology arm (LHA) and a 100bp 3'homology arm (RHA), A10 represents a donor with a 20bp 5'homology arm (LHA) and a 100bp 3'homology arm (RHA), A11 represents a donor with a 10bp 5'homology arm (LHA) and a 100bp 3'homology arm (RHA), A12 represents a donor with no 5'homology arm (LHA) or 3'homology arm (RHA). Based on GFP reporting system designed in Example 1, the results showed that, the experimental groups using the length of homology arms of A1 and A2 exhibited the highest gene integration efficiency.

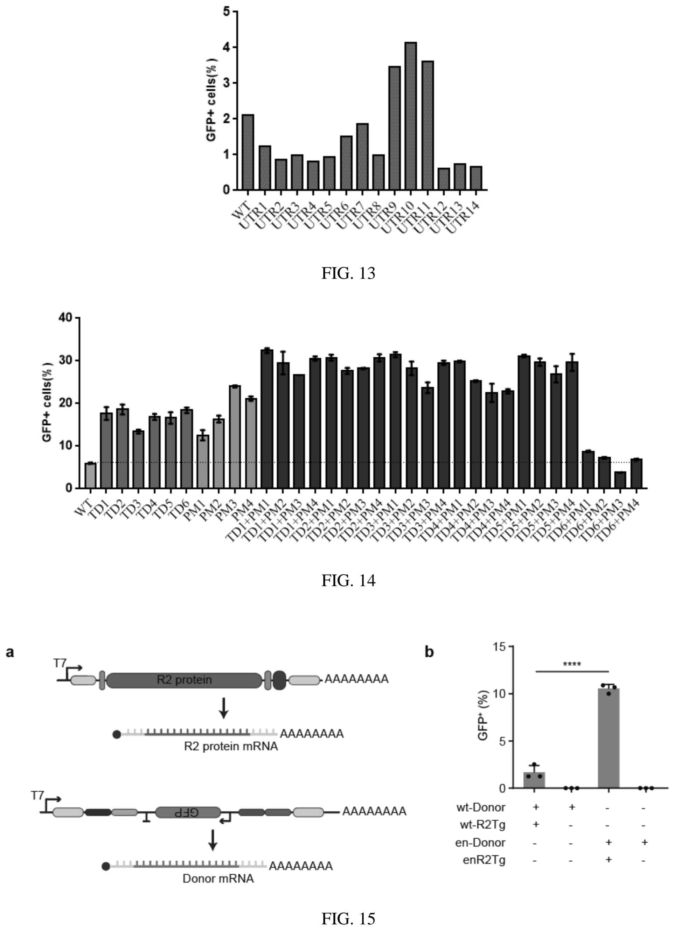

FIG. 13: Effect of 5'UTR and 3'UTR modifications on the gene integration efficiency of wild-type TgR2. The 5'UTR or 3'UTR sequences of the donor RNA for the wild-type TgR2 retrotransposase were modified, generating a series of modified UTR sequences designated as 1, 2, 3, 4, 5, 6, 7, 8, 9, 10, 11, 12, 13, and 14. Using the GFP reporting system designed in Example 1 in conjunction with the wild-type TgR2 retrotransposase, the gene integration efficiency of systems utilizing either the wild-type donor RNA or donor RNAs incorporating these modified UTR sequences was assessed. Wherein donor RNAs incorporating modified UTR sequences 9, 10, and 11 demonstrated a significant increase in gene integration efficiency. In contrast, the integration efficiency of donor RNAs containing the other modified sequences showed either no significant improvement or a decrease.
FIG. 14: Engineered proteins synergize with engineered RNA donors to enhance gene writing efficiency. Based on the engineered RNA donor with the optimal donor element order determined in Example 12, the optimal donor homology arm length determined in Example 13, and the optimal engineered 5'UTR and 3'UTR determined in Example 14, and by utilizing the GFP reporting system designed in Example 1, the TgR2 retrotransposase systems combining the engineered proteins G1098R+A793V+Q955R, G1098R+A793V+W762Q+Q955R, G1098R+A793V+W762Q+Q955R+H341R, and G1098R+A793V+Q955R+H341R (abbreviated sequentially as PM1, PM2, PM3, PM4) screened in Example 5, with T5(N)-CMP(IN-2), T5(N)-RH(IN-2), STO(N)-CMP(IN-2), STO(N)-RH(IN-2), CMP(N)-CMP(IN-2), and CMP(N)+CMP(IN-2)+CMP(IN-3) (abbreviated sequentially as TD1, TD2, TD3, TD4, TD5, TD6) screened in Example 10, were tested. The wild-type TgR2 retrotransposase system and the uncombined optimized proteins (TD1, TD2, TD3, TD4, TD5, TD6, PM1, PM2, PM3, PM4) served as references, wherein the combinations TD1+PM1, TD1+PM2, TD1+PM4, TD2+PM1, TD2+PM2, TD2+PM4, TD3+PM1, TD3+PM2, TD3+PM4, TD4+PM1, TD4+PM2, TD5+PM1, TD5+PM2, and TD5+PM4 demonstrated a significant enhancement in gene integration efficiency, whereas the remaining combinations showed either no significant improvement or a decrease in efficiency.
FIG. 15a: The schematic diagram of the in vitro transcription vector for the mRNA encoding the TgR2 protein and the donor RNA.
FIG. 15b: Delivery of the mRNA encoding the R2 retrotransposase protein and the donor RNA using Lipo3000 liposomes. The mRNA, which was *in vitro* transcribed and purified as described in Example 16, was transfected into HEK 293T cells using Lipo3000 liposomes. At 96 hours post-transfection, the presence and proportion of GFP-positive cells were assessed by fluorescence microscopy and flow cytometry. The engineered RNA donor containing the 5'UTR(SEQ ID NO.16) and a non-natural 3'UTR(SEQ ID NO.46) was designated as en-Donor, and the engineered R2 retrotransposase protein was designated as en-TgR2 (SEQ ID NO.77). The results showed that the use of the engineered retrotransposase protein in combination with the engineered RNA donor significantly enhanced the gene integration efficiency.

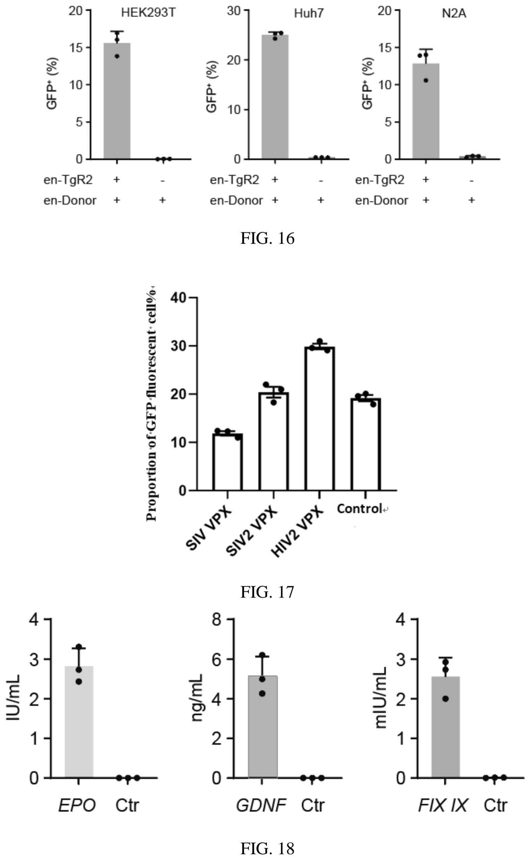

FIG. 16: Efficient gene integration was achieved in HEK 293T cell line, Huh7 cell line and N2A cell line following transfection with LNPs encapsulating mRNA encoding an engineered protein (SEQ ID NO.77) and an engineered RNA donor (5' UTR, SEQ ID NO.16; 3'UTR, SEQ ID NO.46).
FIG. 17: Co-transfection with a plasmid expressing the VPX protein increases the gene integration efficiency of the retrotransposase. Using the GFP reporting system designed in Example 1, the effect of co-transfection with additional plasmids expressing VPX proteins from different viral sources (SIV-VPX (SEQ ID No. 85), SIV2-VPX (SEQ ID No. 86), HIV2-VPX (SEQ ID No. 87)) on gene write efficiency was tested. Wherein co-transfection with the VPX plasmid derived from HIV-2 significantly increased gene integration efficiency.
FIG. 18: The engineered TgR2 system can integrate multiple genes. Based on the GFP reporting system in Example 1, the GFP gene sequence in the donor RNA plasmid was replaced with the corresponding gene sequences for GDNF (SEQ ID No.88), EPO (SEQ ID No.89) and Factor VI (SEQ ID No.53). The integration of the GDNF, EPO and Factor VI genes was then tested in cells using this reporting system. Results from ELISA and Biophen assays demonstrated that the engineered TgR2 system effectively integrated the GDNF, EPO and Factor VI genes.

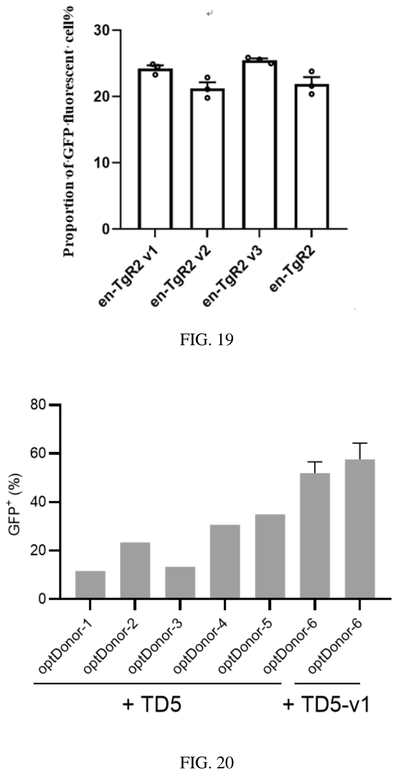

FIG. 19: Truncated en-TgR2 proteins can increase the gene integration efficiency. The gene writing efficiency of different truncated versions of the en-TgR2 protein was tested using the GFP reporting system designed in Example 1. The truncated variants were constructed by deleting amino acids corresponding to positions 1-80, 1-190 and 1-220 of the wild-type TgR2 protein sequence, the truncated proteins were designated as en-TgR2-v1 (SEQ ID No.91), en-TgR2-v2 (SEQ ID No.92) and en-TgR2-v3(SEQ ID No.93), respectively. The results demonstrated that, compared to the en-TgR2 protein, the en-TgR2-v1 and en-TgR2-v3 truncations were both capable of increasing gene writing efficiency.
FIG. 20: Integration of the GFP gene by engineered TgR2: TD5 and TD5-v1 using RNA donors with different architectures.

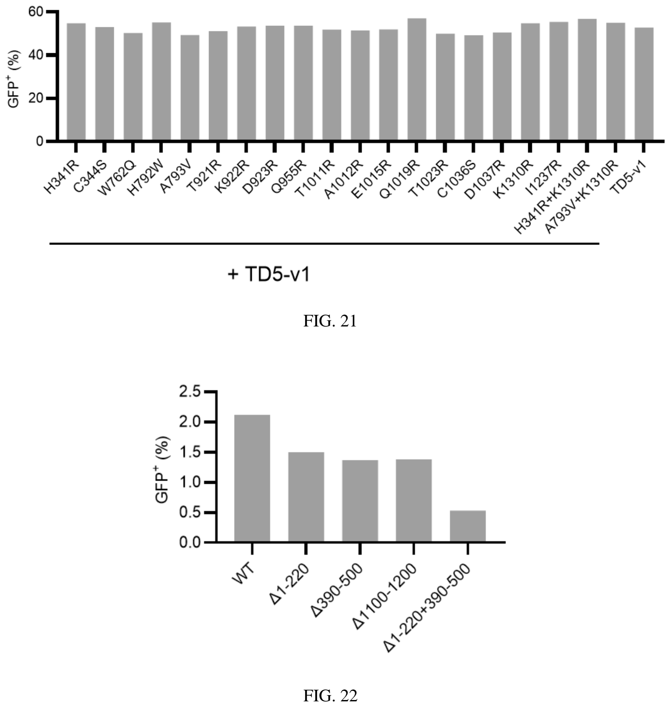

FIG. 21: Gene integration efficiency of amino acid point mutants introduced into the engineered TgR2: TD5-v1 background.
FIG. 22 shows multiple truncated variants of TgR2 are functional in mammalian cell, indicating the presence of a minimal core structure within the TgR2 protein responsible for its activity.

### DETAIL DESCRIPTION OF THE INVENTION

It should be noted that certain terms are used in the description and claims to refer to specific components. Those skilled in the art can understand that, the skilled persion may use different terms to refer to the same component. This specification and the appended claims do not distinguish components based on differences in terminology, but rather on differences in their functions as the criterion for differentiation. Terms such as "comprise" or "include" used throughout the specification and claims are open-ended expressions and should be interpreted as "including but not limited to." The subsequent description in the specification sets forth preferred embodiments for implementing the application. However, such description is intended to illustrate the general principles of the specification and is not intended to limit the scope of the application. The scope of protection of the present application shall be determined by the appended claims. Unless otherwise defined, all technical and scientific terms used herein shall have the same meanings as commonly understood by those skilled in the art to which the present disclosure pertains.

The terms "nucleic acid," "polynucleotide," and "nucleotide sequence" can be used interchangeably and refer to any polymeric form of nucleotides of any length, including deoxyribonucleotides, ribonucleotides, combination thereof, and analogues thereof. The term "oligonucleotide" refers to a short polynucleotide containing no more than approximately 50 nucleotides. As used herein, "complementarity" denotes the ability of a nucleic acid to form hydrogen bonds with another nucleic acid via conventional Watson-Crick base pairing. The percentage of complementarity denotes the percentage of residues in a nucleic acid molecule capable of forming hydrogen bonds (i.e., Watson-Crick base pairs) with a second nucleic acid (e.g., 5/10, 6/10, 7/10, 8/10, 9/10, or 10/10 corresponds to approximately 50%, 60%, 70%, 80%, 90%, and 100% complementarity, respectively). "Perfectly complementary" or "fully complementary" denotes that all consecutive residues in a nucleic acid sequence form hydrogen bonds with an identical number of consecutive residues in a second nucleic acid sequence. As used herein, "substantially complementary" refers to a degree of complementarity of at least approximately 70%, 75%, 80%, 85%, 90%, 95%, 97%, 98%, 99%, or 100% over a region of approximately 40, 50, 60, 70, 80, 100, 150, 200, 250 or more nucleotides, or to two kinds of nucleic acid that hybridize under stringent conditions.

The "percent sequence identity (%)" for a nucleic acid sequence is defined as the percentage of nucleotides in a candidate sequence that are identical to the nucleotides in a specific nucleic acid sequence, after aligning the sequences (allowing gaps if necessary) to achieve the maximum percent sequence identity. The "percent sequence identity (%)" for a peptide, polypeptide, or protein sequence is defined as the percentage of amino acid residues in a candidate sequence that are identical to or conservatively substituted for the amino acid residues in a specific peptide or amino acid sequence, after aligning sequences (if necessary) by allowing gaps to achieve the maximum percent sequence homology. For the purpose of determining amino acid sequence identity percentage, alignments may be performed in various ways within the scope of the art, such as using publicly available computer software including BLAST, BLAST-2, ALIGN, or MEGALIGN^{™} (DNASTAR). Those skilled in the art can determine suitable parameters for measuring alignment, including any algorithm required to achieve maximum alignment over the full length of the sequences being compared.

The terms "polypeptide" and "peptide" are used interchangeably herein and refer to a polymer of amino acids of any length. The polymer may be linear or branched, may comprise modified amino acids, and may be interrupted by non-amino acid residues. A protein may consist of one or more polypeptides. This term also encompasses amino acid polymers that have been modified; such as through the formation of disulfide bonds, glycosylation, lipidation, acetylation, phosphorylation, or any other operation (such as conjugation with a labeling component).

As used herein, "variant" is interpreted as a polynucleotide or polypeptide that differs from a reference polynucleotide or polypeptide, respectively, while retaining essential properties. A typical variant of a polynucleotide differs in its nucleic acid sequence from another reference polynucleotide. Changes in the variant nucleic acid sequence may or may not alter the amino acid sequence of the polypeptide encoded by the reference polynucleotide. Nucleotide changes can result in amino acid substitutions, additions, deletions, fusions, and truncations within the polypeptide encoded by the reference sequence, as described below. A typical variant of a polypeptide differs in its amino acid sequence from another reference polypeptide. Typically, the differences are limited such that the sequences of the reference polypeptide and the variant are substantially similar overall and identical in many regions. The amino acid sequences of the variant and reference polypeptides may differ through any combination of one or more substitutions, additions, or deletions. The substituted or inserted amino acid residues may or may not be those amino acid residues encoded by the genetic code. Variants of the polynucleotide or polypeptide may be naturally occurring (such as allelic variants) or may be variants not known to occur naturally. Non-naturally occurring variants of polynucleotides and polypeptides may be prepared by mutagenesis techniques, direct synthesis, and other recombinant methods known to those skilled in the art.

As used herein, the term "wild-type" has the meaning commonly understood by those skilled in the art, and refers to the typical form of an organism, strain, gene, or characteristic as it exists in nature, distinguishing it from mutants or variants. It can be isolated from natural sources and has not been deliberately modified.

As used herein, the terms "non-naturally occurring" or "engineered" are used interchangeably to indicate artificial involvement. When these terms are applied to describe a nucleic acid molecule or polypeptide, they indicate that said nucleic acid molecule or peptide is at least substantially free from at least one other component with which it is naturally associated or naturally occurs.

The term "cell" as used herein should be understood to refer not only to a specific individual cell, but also to its progeny or potential progeny. Because certain modifications may occur in the progeny due to mutations or environmental influences, such progeny may in fact differ from the parental cell, yet they are still included within the scope of the term as used herein.

As used herein, the terms "transduction" and "transfection" encompass methods known in the art for introducing DNA into cells using infectious agents (such as viruses) or other means to express a target protein or molecule. In addition to viral or virus-like agents, there are chemical-based transfection methods, such as those using calcium phosphate, dendrimers, liposomes, or cationic polymers (e.g., DEAE-dextran or polyethyleneimine); non-chemical methods, such as electroporation, cell squeezing, sonoporation, optical transfection, impalefection, protoplast fusion, plasmid delivery, or transposon; particle-based methods, such as those employing a gene gun, magnetofection or magnet-assisted transfection, and particle bombardment; and hybrid methods (such as nucleofection).

As used herein, the terms "transfected," "transformed," or 'transduced' refer to the process of transferring or introducing exogenous nucleic acid into a host cell. "Transfected," "transformed," or "transduced" cells are those that have been transfected, transformed, or transduced with exogenous nucleic acid.

The term "in vivo" refers to within the organism from which the cells are obtained. "In vitro" or "ex vivo" refers to outside the organism from which the cells are obtained.

As used herein, the terms "treatment" or "treating" refer to a method employed to achieve a beneficial or desired outcome, including a clinical result. For the purposes of this application, beneficial or desired clinical results include, but are not limited to, one or more of the following: alleviating one or more symptoms caused by the disease; reducing the severity of the disease; stabilizing the disease (e.g., preventing or delaying its deterioration), preventing or delaying spread of the disease (e.g., metastasis), preventing or delaying disease recurrence, reducing the recurrence rate of the disease, delaying or slowing disease progression, improving the disease state, providing (partial or complete) remission of the disease, reducing the dosage of one or more other drugs required to treat the disease, delaying disease progression, improving quality of life, and/or prolonging survival. "Treatment" also encompasses reducing the pathological consequences of a symptom, condition, or disease. The methods of the present application address any one or more of these therapeutic aspects.

As used herein, the term "effective amount" refers to an amount of a compound or composition sufficient to treat a specific disorder, condition, or disease (such as to improve, alleviate, mitigate, and/or delay one or more of its symptoms). As understood in the art, an "effective amount" may be administered in a single dose or multiple doses, i.e., either a single administration or multiple administrations may be required to achieve the desired therapeutic endpoint.

The terms "subject," "individual," or "patient" are used interchangeably herein for therapeutic purposes and refer to any animal classified as a mammal, including humans, domestic and farm animals, as well as zoo, farm, or pet animals such as dogs, horses, cats, cattle, and the like. In some embodiments, the individual is a human individual.

It should be understood that the embodiments of the present application described herein include embodiments relating to "consist of" and/or embodiments relating to "consist essentially of." Reference herein to a value or parameter as "about" includes (and describes) variations directed to that value or parameter itself. For example, a description referring to "about X" includes a description of "X."

As used herein, reference to a value or parameter as "non- or not" typically implies and describes the value or parameter "except for...". For example, a method not used to treat cancer type X means the method is used to treat cancers other than type X.

As used herein, the term "about X-Y" has the same meaning as "about X to about Y".

As used herein and in the appended claims, the singular forms "a/an" and "the" include plural objects unless the context otherwise clearly indicates. It should also be noted that claims may be drafted to exclude any optional elements. Therefore, this statement is intended to serve as a basis for the use of exclusive terms such as "only" or 'solely' in conjunction with claim elements, or for the use of the limitation "not."

As used herein, the term "and/or" in phrases such as "A and/or B" is intended to include A and B; A or B; A (alone); and B (alone). Similarly, as used herein, the term "and/or" in phrases such as "A, B, and/or C" is intended to include each of the following embodiments: A, B, and C; A, B, or C; A or C; A or B; B or C; A and C; A and B; B and C; A (alone); B (alone); and C (alone).

Unless otherwise specified, the mutations described herein may include one or more of the following: insertion, deletion, or substitution, and may involve a single amino acid mutation or multiple amino acid mutations.

In the present application, when describing amino acid positions, SEQ ID No. 1, the sequence numbering of the wild-type TgR2 enzyme, is uniformly adopted as the reference sequence. Those skilled in the art will readily understand how to determine amino acid positions by referring to the numbering of the reference sequence. For example, when describing K700, it is intended to indicate that the 700^{th} amino acid in reference SEQ ID No. 1 is K. If modifications are made to the amino acid sequence of the enzyme, such as deletions or insertions of amino acids, resulting in changes to the sequence numbering, those skilled in the art should align with the reference sequence to confirm the position of that amino acid. For instance, if the 1^{st} to 10^{th} amino acids of the reference sequence are deleted, K700 in the modified sequence would then refer to the 690^{th} amino acid. Those skilled in the art can identify the specific modified amino acid based on the sequence alterations and by comparison with the reference sequence.

A "vector" is a composition of substances that contains isolated nucleic acids and can be used to deliver said isolated nucleic acids into the interior of a cell. Many vectors are known in the art, including but not limited to: linear polynucleotides, polynucleotides associated with ionic or amphiphilic compounds, plasmids, and viruses. Typically, a suitable vector contains at least one replication origin, promoter sequence, convenient restriction endonuclease site, and one or more selective markers that function in at least one organism. The term "vector" should also be interpreted to include non-plasmid and non-viral compounds that facilitate the transfer of nucleic acid into cells, such as polylysine compounds, liposomes, etc.

In some embodiments, the vector is a viral vector. Examples of viral vectors include but are not limited to: adenovirus vectors, adeno-associated virus vectors, lentiviral vectors, retroviral vectors, vaccinia virus vectors, herpes simplex virus vectors, and derivatives thereof. In some embodiments, the vector is a bacteriophage vector. Viral vector technology is well known in the art and is described, for example, in Sambrook et al. (2001, Molecular Cloning: A Laboratory Manual, Cold Spring Harbor Laboratory, New York), as well as in other virology and molecular biology manuals.

In some embodiments, rAAV construct can be administered to a subject enterally. In some embodiments, the rAAV construct may be administered to a subject parenterally. In some embodiments, the rAAV particle may be administered subcutaneously, intraocularly, intravitreally, subretinally, intravenously (IV), intracerebroventricularly, intramuscularly, intrathecally (IT), intracisternally, intraperitoneally, via inhalation, topically, or by direct injection into one or more cells, tissues, or organs. In some embodiments, the rAAV particles may be administered to a subject by injection into the hepatic artery or portal vein.

Methods for introducing vectors into mammalian cells are well known in the art. Vectors can be transferred into host cells via physical, chemical, or biological means.

Physical methods for introducing vectors into host cells include: calcium phosphate precipitation, lipofection, particle bombardment, microinjection, electroporation, etc. Methods for generating cells containing vectors and/or exogenous nucleic acids are widely established. See, for example, Sambrook et al. (2001) Molecular Cloning: A Laboratory Manual, Cold Spring Harbor Laboratory, New York. In some embodiments, the vector is introduced into the cells via electroporation.

The method described herein is applicable to any suitable cell type. In some embodiments, the cells are bacterial cells, yeast cells, fungal cells, algal cells, plant cells, or animal cells (e.g., mammalian cells such as human cells). In some embodiments, the cells are of natural origin, such as cells isolated from a tissue biopsy. In some embodiments, the cells are isolated from cell lines cultured ex vivo. In some embodiments, the cells originate from a primary cell line. In some embodiments, the cells originate from an immortalized cell line. In some embodiments, the cells are genetically engineered cells.

The nuclear localization signal (NLS) as described herein is a domain of a protein, typically a short amino acid sequence, which interacts with nuclear import carriers to facilitate the transport of the protein into the nucleus. Meanwhile, an nuclear localization signal may be an RNA sequence. In certain embodiments, the nuclear localization signal is located on the donor RNA. In some embodiments, the retrotransposase polypeptide is encoded on a first RNA, and the donor RNA is a second separate RNA, with the nuclear localization signal residing on the donor RNA rather than on the RNA encoding the retrotransposase polypeptide. Although not wishing to be bound by theory, in some embodiments, the RNA encoding the retrotransposase is primarily targeted to the cytoplasm to promote its translation, while the donor RNA is primarily targeted to the nucleus to promote its transposition and integration into the genome. In some embodiments, the nuclear localization signal is located at the 3' end, the 5' end or internally within the donor RNA. In some embodiments, the nuclear localization signal is located at the 3' end of the heterologous sequence (e.g., directly at the 3' end) or at the 5' end of the heterologous sequence (e.g., directly at the 5' end). In some embodiments, the nuclear localization signal is located outside the 5'UTR or outside the 3'UTR of the donor RNA. In some embodiments, the nuclear localization signal is located between the 5'UTR and the 3'UTR, wherein optionally, the nuclear localization signal is not transcribed with the transgene (e.g., the nuclear localization signal is in an antisense orientation or downstream of a transcription termination signal or a polyadenylation signal). In some embodiments, the nuclear localization sequence is located within an intron. In some embodiments, multiple identical or different nuclear localization signals are present in the RNA, e.g., in the donor RNA. In some embodiments, the nuclear localization signal has a length of less than 5, 10, 25, 50, 75, 100, 150, 200, 250, 300, 350, 400, 450, 500, 600, 700, 800, 900 or 1000bp. Various RNA nuclear localization sequences may be used.

The term "domain" as used herein refers to a structure of a biomolecule that contributes to a specific function of that biomolecule. A domain may comprise a contiguous region (e.g., a contiguous sequence) or distinct non-contiguous regions (e.g., non-contiguous sequences) of the biomolecule. Examples of protein domains include but are not limited to endonuclease domains, target DNA-binding domains, and reverse transcription domains. An example of a nucleic acid domain is a regulatory domain, such as a transcription factor-binding domain.

In some embodiments, the present application relates to target DNA-binding domains comprising zinc finger-binding motifs, reverse transcriptase domains, and endonuclease domains.

In some embodiments, the reverse transcriptase domain refers to a domain possessing reverse transcription functionality. Those skilled in the art can identify a reverse transcriptase domain based on its homology to other known reverse transcriptase domains using conventional tools such as the Basic Local Alignment Search Tool (BLAST). In some embodiments, the reverse transcriptase domain is modified, for example, through site-specific mutagenesis. In embodiments, the reverse transcriptase domain is engineered to bind to a heterologous sequence.

In some embodiments, the endonuclease domain refers to a domain possessing endonuclease activity. The endonuclease element is a heterologous endonuclease element, such as Fok1 nuclease, a type II restriction-like endonuclease (RLE nuclease), or another RLE-type nuclease (also known as REL). In some embodiments, the heterologous nucleases activity exhibits nickase activity and do not generate double-strand breaks. Those skilled in the art can identify endonuclease domain based on homology with other known endonuclease domains use tools such as the Basic Local Alignment Search Tools (BLAST).

In some embodiments, the target DNA-binding domain is a domain comprising a zinc finger binding motif, wherein the zinc finger binding motif is an amino acid sequence responsible for binding to specific sequence of target DNA.

The term "exogenous" as used herein, when used in reference to a biomolecule (e.g., a nucleic acid sequence or a polypeptide), refers to a biomolecule that has been introduced artificially into the genome, cell, or organism of a host. For example, a nucleic acid added to an existing genome, cell, tissue, or subject via recombinant DNA technology or other methods is exogenous relative to the existing nucleic acid sequence, cell, tissue, or subject.

The term "heterologous" as used herein means that when used to describe a first element in reference to a second element, the term indicates that the first element and second element do not exist in nature in the arrangement described. For example, a heterologous polypeptide, nucleic acid molecule, construct, or sequence refers to (a) a polypeptide, nucleic acid molecule, or portion thereof that is not native to the cell in which it is expressed, (b) a polypeptide or nucleic acid molecule, or a portion thereof, that has been altered or mutated relative to its native state, or (c) a polypeptide or nucleic acid molecule whose expression is altered compared to its natural expression level under analogous conditions. For instance, a heterologous regulatory sequence (e.g., promoter, enhancer) may be employed to regulate the expression of a gene or nucleic acid molecule in a manner distinct from how the gene or nucleic acid molecule is typically expressed in nature. In another embodiment, a heterologous domain of a polypeptide or nucleic acid sequence (e.g., a DNA-binding domain of a polypeptide or a nucleic acid encoding the DNA-binding domain of a polypeptide) may be arranged relative to other domains, or may be a different sequence or originated from a different source relative to other domains or regions of the polypeptide or its encoding nucleic acid. In certain embodiments, a heterologous nucleic acid molecule may be present in the genome of a natural host cell but may have an altered expression level, a different sequence, or both. In other embodiments, a heterologous nucleic acid molecule may not be endogenous to the host cell or host genome, but may instead have been introduced into the host cell via transformation (e.g., transfection, electroporation), wherein the added molecule may integrate into the host genome or may exist transiently (e.g., mRNA) or semi-stably for more than one generation (e.g., as episomal viral vectors, plasmids, or other self-replicating vectors) as extrachromosomal genetic material.

The term "gene expression unit" as used herein refers to a nucleic acid sequence comprising at least one regulatory nucleic acid sequence operatively connected to at least one effector sequence. A first nucleic acid sequence is operably connected to a second nucleic acid sequence when the first nucleic acid sequence is positioned in a functional relationship with the second nucleic acid sequence. For example, a promoter or enhancer is operably linked to a coding sequence if it influences the transcription or expression of that coding sequence. Operably linked DNA sequences may be contiguous or non-contiguous. When two protein-coding regions need to be linked, the operably linked sequences may reside within the same reading frame.

As used herein, the term "host genome or host cell" refers to a cell and/or its genome into which proteins and/or genetic material have been introduced. It should be understood that these terms are intended to encompass not only the specific subject cell and/or genome, but also the progeny of such a cell and/or the genome of such progeny. Because certain modifications may occur in successive generations due to mutation or environmental influences, such progeny may in fact differ from the parental cell, but are still included within the scope of the term "host cell" as used herein. The host genome or host cell may be an isolated cell or cell line grown in culture, genomic material isolated from such a cell or cell line, or a host cell or host genome constituting living tissue or an organism. In some instances, the host cell may be an animal cell or a plant cell, for example, as described herein. In certain instances, the host cells may be a bovine cell, equine cell, porcine cell, caprine cell, ovine cell, chicken cell, or turkey cell. In certain instances, the host cell may be a maize cell, soybean cell, wheat cell, or rice cell.

Genomic Safe Harbor Site (GSH site) used herein: Genomic safe harbor sites are sites within the host genome capable of accommodating the integration of new genetic material, such that the inserted genetic elements do not cause significant alterations to the host genome or pose risks to the host cell or organism. GSH sites typically meet 1, 2, 3, 4, 5, 6, 7, 8, or 9 of the following criteria: (i) >300 kb from cancer-associated genes; (ii) >300 kb from miRNAs/other functional small RNAs; (iii) >50 kb from the 5' end of a gene; (iv) >50 kb from a replication origin; (v) >50 kb from any highly conserved element; (vi) low transcriptional activity (i.e., no mRNA within +/-25 kb); (vii) not located within a copy number variable region; (viii) situated in open chromatin; and/or (ix) unique, with only one copy in the human genome. Examples of GSH sites in the human genome meeting some or all of these criteria include: (i) adeno-associated virus site 1 (AAVS1), a natural site for AAV viral integration on chromosome 19; (ii) the chemokine (C-C motif) receptor 5 (CCR5) gene, a chemokine receptor gene known as an HIV-1 co-receptor; (iii) the human ortholog of the mouse Rosa26 locus; and (iv) the rDNA locus. Additional GSH sites are known and have been described in, for example, Pellenz et al., electronically published on August 20, 2018 (https://doi.org/10. 1101/396390).

In some embodiments, the Genomic Safe Harbor Site is a Natural Harbor^{™} site. In some embodiments, the Natural Harbor^{™} site is a ribosome DNA (rDNA) locus. In some embodiments, the Natural Harbor^{™} site is 5SrDNA, 18S rDNA, 5.8S rDNA or 28S rDNA. In some embodiments, the Natural Harbor^{™} site is the Mutsu site in 5S rDNA. In some embodiments, the Natural Harbor^{™} site is the R2 site in 28S rDNA.

As used herein, the term "pseudoknot sequence" refers to a nucleic acid (e.g., RNA) containing a sequence with appropriate self-complementarity to form a pseudoknot structure.

As used herein, the term "stem-loop sequence" refers to a nucleic acid sequence (e.g., an RNA sequence) possessing sufficient self-complementarity to form a stem-loop structure. For example, such a structure comprises a stem comprising at least two (e.g., 3, 4, 5, 6, 7, 8, 9, or 10) base pairs, and the loop may comprise at least three (e.g., four) base pairs. The stem may comprise mismatches or bulges.

In some embodiments, the cell described is an animal cell selected from the following group of organisms: cattle, sheep, goats, horses, pigs, deer, chickens, ducks, geese, rabbits, and fish.

In some embodiments, the cell is mammalian cell. In some embodiments, the cell is a human cell. In some embodiments, the human cell is a human embryonic kidney 293T (HEK293T or 293T) cell or a HeLa cell. In some embodiments, the cell is a human embryonic kidney (HEK293T) cell. In some embodiments, the cell is a mouse Hepa1-6 cell. In some embodiments, the mammalian cell is selected from the following group: immune cells, hepatocytes, tumor cells, stem cells, blood cells, neuronal cells, zygotes, muscle cells (such as cardiomyocytes), and skin cells.

In some embodiments, the cell described is an immune cell selected from the group consisting of: cytotoxic T cells, helper T cells, natural killer (NK) T cells, iNK-T cells, NK-T-like cells, γδ T cells, tumor-infiltrating T cells, and dendritic cell (DC)-activated T cells. In some embodiments, the method generates modified immune cells, such as CAR-T cells or TCR-T cells.

In some embodiments, the cell is a cell selected from the group consisting of an embryonic stem (ES) cell, an induced pluripotent stem (iPS) cell, a gamete progenitor cell, a gamete, a zygote, or an embryo.

The retrotransposase herein comprises a target DNA-binding domain containing a zinc finger binding motif, a reverse transcriptase domain, and an endonuclease domain, and is capable of reverse transcribing RNA into DNA.

The present application relates to engineered retrotransposases, which are mutants of the wild-type retrotransposaseTgR2. All sites described herein as capable of being mutated can be combined, and mutations can be introduced at 1, 2, 3, 4, 5, 6, 7, 8, 9, 10, 11, 12, 13, 14, 15, 16, 17, 18, 19, 20, 30, 40, 50, 60, 70, 80, 90, 100 of these sites.

For example, this engineered retrotransposase is derived from the sequence of the wild-type retrotransposase TgR2 through sequence modifications, corresponding to the wild-type retrotransposase TgR2 with a mutation at amino acid position 1098, such as possessing a G1098R mutation. In one embodiment, this engineered retrotransposase is derived from the sequence of the wild-type retrotransposase TgR2 through sequence modifications, corresponding to the wild-type retrotransposase TgR2 with mutations at amino acids positions 1098 and 793, such as possessing G1098R and A793V mutations. In one embodiment, this engineered retrotransposase is derived from the sequence of the wild-type retrotransposase TgR2 through sequence modifications, corresponding to the wild-type retrotransposase TgR2 with mutations at amino acid positions 1098, 793 and 955, such as possessing G1098R, A793V and Q955R mutations (also referred to as PM1 herein). In one embodiment, this engineered retrotransposase is derived from the sequence of the wild-type retrotransposase TgR2 through sequence modifications, corresponding to the wild-type retrotransposase TgR2 with mutations at amino acids positions 1098, 793, 955 and 762, such as possessing G1098R, A793V, Q955R and W762Q mutations (also referred to as PM2 herein). In one embodiment, this engineered retrotransposase is derived from the wild-type retrotransposase TgR2 through sequence modifications, corresponding to the wild-type retrotransposase TgR2 with mutations at amino acid positions 1098, 793, 955, 762 and 341, such as possessing G1098R, A793V, Q955R, W762Q and H341R mutations (also referred to as PM3 herein). In one embodiment, this engineered retrotransposase is derived from the wild-type retrotransposase TgR2 through sequence modifications, corresponding to the wild-type retrotransposase TgR2 with mutations at amino acid positions 1098, 793, 955 and 341, such as possessing G1098R, A793V, Q955R and H341R mutations (also referred to as PM4 herein).

In the present application, in addition to the mutants described above, those skilled in the art may further perform conservative mutations on amino acids not involved in the active site based on their knowledge. Specifically, this involves mutating one amino acid to another amino acid with similar physicochemical properties.

In the present application, all amino acid numbering is based on the amino acid sequence of the wild-type transposase TgR2 (the sequence shown in SEQ ID No.1), those skilled in the art will readily understand that if the amino acid sequence undergoes truncation, insertion, or other modifications, the actual amino acid sequence will change. However, the descriptions herein are still made with reference to the original sequence of the wild-type transposase TgR2. In the present application, when describing mutations, the letter preceding the position number indicates the original amino acid, while the letter following the position number indicates the amino acid after mutation. For example, G1098R denotes that, relative to the wild-type transposase TgR2, the amino acid at position 1098 is mutated from G to R. All other mutations are described in the same manner.

In one specific embodiment, the amino acid sequence of the retrotransposase herein is shown as any one of SEQ ID No. 2-7, or has at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% identity with the amino acid sequence as defined in any one of SEQ ID No. 2-7.

The present application relates to an engineered retrotransposase, this engineered retrotransposase is a truncated version of the wild-type retrotransposase TgR2.

**In** one specific embodiment, the retrotransposase herein is a truncated form of the wild-type retrotransposase TgR2, e.g., amino acids 1-200 can be truncated from the N-terminus of the wild-type retrotransposase TgR2, such as truncating the 1^{st}-10^{th}, the 1^{st}-20^{th}, the 1^{st}-30^{th}, the 1^{st}-40^{th}, the 1^{st}-50^{th}, the 1^{st}-60^{th}, the 1^{st}-70^{th}, the 1^{st}-80^{th} , the 1^{st}-90^{th}, the 1^{st}-100^{th}, the 1^{st}-110^{th}, the 1^{st}-120^{th}, the 1^{st}-130^{th}, the 1^{st}-140^{th}, the 1^{st}-150^{th}, the 1^{st}-160^{th}, the 1^{st}-170^{th}, the 1^{st}-180^{th}, the 1^{st}-190^{th}, the 1^{st}-200^{th}, the 1^{st}-210^{th}, the 1^{st}-220^{th}, the 1^{st}-230^{th}, the 1^{st}-240^{th}, the 1^{st}-2S0^{th}, the 1^{st}-260^{th}, the 1^{st}-270^{th}, the 1^{st}-280^{th}, the 1^{st}-290^{th}, the 1^{st}-300^{th}, the 1^{st}-310^{th}, the 1^{st}-320^{th}, the 1^{st}-330^{th}, the 1^{st}-340^{th}, the 1^{st}-350^{th}, the 1^{st}-360^{th}, the 1^{st}-370^{th}, the 1^{st}-380^{th}, the 1^{st}-390^{th}, the 1^{st}-400^{th}, the 1^{st}-410^{th}, the 1^{st}-420^{th}, the 1^{st}-430^{th}, the 1^{st}-440^{th}, the 1^{st}-450^{th}, the 1^{st}-460^{th}, the 1^{st}-470^{th}, the 1^{st}-480^{th}, the 1^{st}-490^{th}, the 1^{st}-500^{th} amino acids. Also, the 200^{st}-500^{st}, the 210^{st}-500^{st}, the 220^{st}-500^{st}, the 230^{st}-500^{st}, the 240^{st}-500^{st}, the 250^{st} -500^{st}, the 260^{st}-500^{st}, the 270^{st}-500^{st}, the 280^{st}-500^{st}, the 290^{st}-500^{st}, the 300^{st}-500^{st}, the 310^{st}-500^{st}, the 320^{st}-500^{st}, the 330^{st}-500^{st}, the 340^{st}-500^{st}, the 350^{st}-500^{st}, the 360^{st}-500^{st}, the 370^{st}-500^{st}, the 380^{st}-500^{st}, the 390^{st}-500^{st}, the 400^{st}-500^{st} amino acids can be truncated. Furthermore, the 1000^{st}-1200^{st}, the 1010^{st}-1190^{st}, the 1020^{st}-1180^{st}, the 1030^{st}-1180^{st}, the 1040^{st}-1170^{st}, the 1050^{st}-1160^{st}, the 1060^{st}-1150^{st}, the 1070^{st}-1140^{st}, the 1080^{st}-1150^{st}, the 1090^{st}-1140^{st}, the 1100^{st}-1130^{st}, the 1100^{st}-1120^{st} amino acids can also be truncated.

When describing the truncation of specific amino acid positions, the amino acid numbering is based on that of the wild-type transposase TgR2, i.e., described according to the sequence shown in SEQ **ID** No. 1. For example, when describing an N-terminal truncation of amino acids 1-220 corresponds to amino acids 1-220 counted from the N-terminal of the sequence shown in SEQ **ID** No. 1. For example, when describing truncating the 390^{th}-550^{th} from N-terminal, this corresponds to the 390^{th}-550^{th} amino acids counted from the N-terminal of the sequence shown in SEQ ID No. 1. For example, when describing truncating the 1100^{th}-1120^{th} from N-terminal, this corresponds to the 1100^{th}-1120^{th} amino acids counted from the N-terminal of the sequence shown in SEQ ID No. 1.

In the present application, the truncation positions described above can be combined, as long as those skilled in the art confirm these positions will not affect the activity of the retrotransposase herein, e.g., the 1^{st}-10^{th} and 490^{th}-500^{th} amino acids can be truncated. For example, the 1^{st}-220^{th} amino acids and the 390^{th}-550^{th} amino acids counted from the N-terminal can be truncated simultaneously.

In one specific embodiment, the amino acid sequence of the retrotransposase herein is shown as any one of SEQ ID No. 13, 38, 40, and 51, or has at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identity with the amino acid sequence as defined in any one of SEQ ID No. 13, 38, 40, and 51.

In one specific embodiment, the retrotransposase herein can be a truncated form of the retrotransposase mutant described herein, e.g., amino acids 1-500 corresponding to the wild-type sequence SEQ ID NO. 1 can be truncated from a mutant of the wild-type retrotransposase TgR2, e.g., truncating the 1^{st}-10^{th} the 1^{st}-20^{th}, the 1^{st}-30^{th}, the 1^{st}-40^{th}, the 1^{st}-50^{th}, the 1^{st}-60^{th}, the 1^{st}-70^{th}, the 1^{st}-80^{th}, the 1^{st}-90^{th}, the 1^{st}-100^{th}, the 1^{st}-110^{th}, the 1^{st}-120^{th}, the 1^{st}-130^{th}, the 1^{st}-140^{th}, the 1^{st}-150^{th}, the 1^{st}-160^{th}, the 1^{st}-170^{th}, the 1^{st}-180^{th}, the 1^{st}-190^{th}, the 1^{st}-200^{th}, the 1^{st}-210^{th}, the 1^{st}-220^{th}, the 1^{st}-230^{th}, the 1^{st}-240^{th}, the 1^{st}-250^{th}, the 1^{st}-260^{th}, the 1^{st}-270^{th}, the 1^{st}-280^{th}, the 1^{st}-290^{th}, the 1^{st}-300^{th}, the 1^{st}-310^{th}, the 1^{st}-320^{th}, the 1^{st}-330^{th}, the 1^{st}-340^{th}, the 1^{st}-350^{th}, the 1^{st}-360^{th}, the 1^{st}-370^{th}, the 1^{st}-380^{th}, the 1^{st}-390^{th}, the 1^{st}-400^{th}, the 1^{st}-410^{th}, the 1^{st}-420^{th}, the 1^{st}-430^{th}, the 1^{st}-440^{th}, the 1^{st}-450^{th}, the 1^{st}-460^{th}, the 1^{st}-470^{th}, the 1^{st}-480^{th}, the 1^{st}-490^{th}, the 1^{st}-500^{th} amino acids. For example, the 200^{th}-500^{th}, the 210^{th}-500^{th}, the 220^{th}-500^{th}, the 230^{th}-500^{th}, the 240^{th}-500^{th}, the 250^{th}-500^{th}, the 260^{th}-500^{th}, the 270^{th}-500^{th}, the 280^{th}-500^{th}, the 290^{th}-500^{th}, the 300^{th}-500^{th}, the 310^{th}-500^{th}, the 320^{th}-500^{th}, the 330^{th}-500^{th}, the 340^{th}-500^{th}, the 350^{th}-500^{th}, the 360^{th}-500^{th}, the 370^{th}-500^{th}, the 380^{th}-500^{th}, the 390^{th}-500^{th}, the 400^{th}-500^{th} amino acids can also be truncated. For example, the 1000^{th}-1200^{th}, the 1010^{th}-1190^{th}, the 1020^{th}-1180^{th}, the 1030^{th}-1180^{th}, the 1040^{th}-1170^{th}, the 1050^{th}-1160^{th}, the 1060^{th}-1150^{th}, the 1070^{th}-1l40^{th}, the 1080^{th}-1150^{th}, the 1090^{th}-1140^{th}, the 1100^{th}-1130^{th}, the 1100^{th}-1120^{th} amino acids can also be truncated.

In the present application, those skilled in the art can completely understand that, the wild-type retrotransposase TgR2 can be truncated first and then mutated, or of course, the wild-type retrotransposase TgR2 can be mutated first and then truncated. However, all amino acid sequences described are based on the numbering of wild-type retrotransposase TgR2.

The engineered retrotransposase herein may also be obtained by further conjugation of other amino acid sequences to the wild-type retrotransposase TgR2. The engineered retrotransposase herein may also be obtained by further conjugation of other amino acid sequences to the truncation form of the wild-type retrotransposase TgR2. The engineered retrotransposase herein may also be obtained by further conjugation of other amino acid sequences to the mutant of the wild-type retrotransposase TgR2. The engineered retrotransposase herein may also be obtained by further conjugation of other amino acid sequences to the truncated form and mutant of the wild-type retrotransposase TgR2.

The term "other amino acids" as described herein denotes any other amino acid sequence having functions other than the retrotransposase function. This sequence may be any peptide sequence with regulatory function, any peptide sequence with a linking function, or a non-functional peptide sequence lacking any specific function.

For example, other amino acid sequence can be selected from exonucleases, single-stranded DNA-binding proteins, chromatin-modulating peptide segments, high mobility peptide segments, RNaseH domains, nuclear import nuclear localization signals (NLS), nuclear export nuclear localization signals.

In the present application, the above-mentiond other amino acid sequence can be inserted at the N-terminal position, or after amino acid no. 191, or after amino acid no. 432, or after amino acid no. 1112 of the wild-type retrotransposase TgR2.

In the present application, the above-mentiond other amino acid sequence can be inserted at the N-terminal position, or after amino acid no. 191, or after amino acid no. 432, or after amino acid no. 1112 of a mutant of the wild-type retrotransposase TgR2.

In the present application, the above-mentiond other amino acid sequence can be inserted at the N-terminal position, or after amino acid no. 191, or after amino acid no. 432, or after amino acid no. 1112 of a truncated form of the wild-type retrotransposase TgR2, the amino acid numbering described herein still corresponds to that of the wild-type retrotransposase TgR2.

In the present application, the above-mentiond other amino acid sequence can be inserted at the N-terminal position, or after amino acid no. 191, or after amino acid no. 432, or after amino acid no. 1112 of the truncated and mutated wild-type retrotransposase TgR2, the amino acid numbering described herein still corresponds to that of the wild-type retrotransposase TgR2.

In addition, those skilled in the art can also understand that other amino acid sequences are inserted in combination, the multiple other amino acid sequences can be inserted in a tandem arrangement at any of the above positions, such as the N-terminal position, or after amino acid no. 191, or after amino acid no. 432, or after amino acid no. 1112, where the amino acid numbering described herein still corresponds to that of the wild-type retrotransposase TgR2.

In the present application, when describing insertion at the N-terminal position, it can typically be inserted either before the 1^{st} amino acid at the N-terminal or after the 1^{st} amino acid. Those skilled in the art can select the appropriate approach based on the specific circumstances.

In one embidiment, the exonuclease as described herein is an exonuclease derived from T5 bacteriophage, e.g., is the one as shown in SEQ ID NO.8 or has at least 70%, 75%, 80%, 85%, 90%, 95%, 96%, 97%, 98%, or 99% identity with the amino acid sequence shown in SEQ ID No. 8.

In one embidiment, the single-stranded DNA-binding protein as described herein is a single-stranded DNA-binding protein derived from the bacterium Sulfolobus tokodaii, e.g., is the one as shown in SEQ ID NO.9 or has at least 70%, 75%, 80%, 85%, 90%, 95%, 96%, 97%, 98%, or 99% identity with the amino acid sequence shown in SEQ ID No. 9.

In one embidiment, the chromatin-modulating peptide segment or high mobility peptide as described herein is a chromatin-modulating peptide segment or high mobility peptide segment derived from human, e.g., as shown in SEQ ID NO.10 or has at least 70%, 75%, 80%, 85%, 90%, 95%, 96%, 97%, 98%, or 99% identity with the amino acid sequence shown in SEQ ID No. 10.

In one embidiment, the RNaseH domain as described herein is an RNaseH domain derived from the MLV reverse transcriptase, e.g., is the one as shown in SEQ ID NO.11 or has at least 70%, 75%, 80%, 85%, 90%, 95%, 96%, 97%, 98%, or 99% identity with the amino acid sequence shown in SEQ ID No. 11.

Those skilled in the art fully understand that the other amino acids mentioned in the above insertion may be inserted in combination, may be inserted randomly, or may be inserted at a single position, e.g., the RNaseH domain may be inserted at the N-terminal position and a chromatin-modulating peptide segment may be inserted after amino acid no. 191. In the present application, the engineered retrotransposase may be selected from the amino acid sequences as shown in any one of SEQ ID NO.2-7, 12, 13, 38, 40, 42, 43, 45, 47, 49, 51, and 55-84, or be selected from the amino acid sequence that has at least 70%, 75%, 80%, 85%, 90%, 95%, 96%, 97%, 98%, or 99% identity with the amino acid sequence as described in any one of SEQ ID No. 2-7, 12, 13, 38, 40, 42, 43, 45, 47, 49, 51, an 55-84.

In the present application, engineered retrotransposase is a truncated version of the amino acid sequence as shown in any one of SEQ ID NO. 1-7, 13, 38, 40, 42, 43, 45, 47, 49, 51, and 55-84. For example, it is a sequence obtained by truncating amino acids corresponding to positions 1-80, or 1-190, or 1-220, or 390-550, or 1100-1120 of the wild-type sequence SEQ ID NO. 1 from any one of the amino acid sequences shown in SEQ ID NO. 1-7, 13, 38, 40, 42, 43, 45, 47, 49, 51, and 55-84.

The present application relates to a system for modifying DNA, wherein the system comprises: a retrotransposase described herein (including a wild-type retrotransposase or an engineered retrotransposase) or a nuleic acid encoding the retrotransposase described herein (including a wild-type retrotransposase or an engineered retrotransposase); and a donor RNA or a nucleic acid encoding the donor RNA, wherein the donor RNA comprises: a sequence that binds to the retrotransposase and a heterologous sequence, preferably, the heterologous sequence is of at least 1-50000 base in length, e.g., 1nt and above, 10nt and above, 50nt and above, 60nt and above, 70nt and above, 80nt and above, 90nt and above, 100nt and above, 150nt and above, 200nt and above, 250nt and above, 300nt and above, 350nt and above, 400nt and above, 450nt and above, 500nt and above, 550nt and above, 600nt and above, 650nt and above, 700nt and above, 750nt and above, 800nt and above, 850nt and above, 900nt and above, 950nt and above, 1000nt and above, 1100nt and above, 1200nt and above, 1300nt and above, 1400nt and above, 1500nt and above, 1600nt and above, 1700nt and above, 1800nt and above, 1900nt and above, 2000nt and above, 2100nt and above, 2200nt and above, 2300nt and above, 2400nt and above, 2500nt and above, 2600nt and above, 2700nt and above, 2800nt and above, 2900nt and above, 3000nt and above, 3500nt and above, 4000nt and above, 4500nt and above, 5000nt and above, 5500nt and above, 6000nt and above, 6500nt and above, 7000nt and above, 7500nt and above, 8000nt and above, 8500nt and above, 9000nt and above, 9500nt and above, 10000nt and above, 15000nt and above, 20000nt and above, 25000nt and above, 30000nt and above, 35000nt and above, 40000nt and above, 45000nt and above, wherein the retrotransposase is the retrotransposase as shown in SEQ ID NO.1 or an engineered modified retrotransposase as described herein.

In one aspect, the system for modifying DNA as described herein comprises: a retrotransposase described herein (including a wild-type retrotransposase or an engineered retrotransposase) or a nucleic acid encoding the retrotransposase described herein (including a wild-type retrotransposase or an engineered retrotransposase); and a donor RNA or a nucleic adic encoding the donor RNA, wherein the donor RNA comprises: a sequence that binds to the retrotransposase and a heterologous sequence, wherein the heterologous sequence is of at least 1-50000 bases in length.

In one aspect, in addition to the retrotransposase described herein (including a wild-type retrotransposase or an engineered retrotransposase) or a nucleic acid encoding the retrotransposase (including a wild-type retrotransposase or an engineered retrotransposase); and a donor RNA or a nucleic adic encoding the donor RNA, the system for modifying DNA as described herein further comprises a nucleic acid encoding a VPX protein or the VPX protein per se.

In one aspect, the amino acid sequence of the VPX protein has at least 70%, 75%, 80%, 85%, 90%, 95%, 96%, 97%, 98%, 99%, or 100% identity with the amino acid sequence as defined in any one of SEQ ID No. 85, 86 or 87.

In one specific embodiment, as shown in FIG. 1, the retrotransposase and the donor RNA are encoded by 2 plasmids separately, the retrotransposase gene is expressed under the control of the CAG promoter, the the donor RNA is expressed under the control of the CAG promoter. Meanwhile, the expression cassette for the donor RNA additionally comprises a reverse-oriented expression cassette initiated by the CMV promoter. Within this CMV-driven expression cassette, GFP is expressed, but this GFP sequence herein is interrupted by a reverse-orientedly inserted intron sequence. Therefore, the ultimate action mode of the system described herein is as follows: after the donor RNA is expressed under the CAG promoter, the intron sequence contained within the donor RNA is spliced out from the expressed RNA. This restores the integrity of the GFP sequence from the CMV-driven expression cassette at the RNA level. However, the GFP sequence is now an antisense RNA strand and lacks the ability to being translated into GFP protein. Only when the retrotransposase permanently integrates the intron-deleted donor RNA into the cellular DNA via reverse transcriptase activity can the CMV-initated reverse expression cassette expresses normal intron-free GFP mRNA, thereby producing the green fluorescent GFP protein.

The heterologous sequence herein is selected from one or more of the following: a sequence encoding a polypeptide, a sequence containing a tissue-specific promoter or enhancer, and a sequence encoding one or more introns.

In one specific embodiment, the polypepteide is a therapeutic peptide or a mammalian peptide; more preferably, the polypeptide is a therapeutic protein, a membrane protein, an intracellular protein, an extracellular protein, a structural protein, a signaling protein, a regulatory protein, a transport protein, an organelle protein, a sensory protein, a motor protein, a defense protein, a storage protein, a reporter protein, an antibody, an enzyme, or a coagulation factor.

In one specific embodiment, the number of the amino acids of the polypeptide is 20-10000, e.g., the number of the amino acids is 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 550, 600, 650, 700, 750, 800, 850, 900, 950, 1000, 1100, 1200, 1300, 1400, 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 4000, 4100, 4200, 4300, 4400, 4500, 4600, 4700, 4800, 4900, 5000, 5100, 5200, 5300, 5400, 5500, 5600, 5700, 5800, 5900, 6000, 6100, 6200, 6300, 6400, 6500, 6600, 6700, 6800, 6900, 7000, 7100, 7200, 7300, 7400, 7500, 7600, 7700, 7800, 7900, 8000, 8100, 8200, 8300, 8400, 8500, 8600, 8700, 8800, 8900, 9000, 9100, 9200, 9300, 9400, 9500, 9600, 9700, 9800, or 9900.

In one specific embodiment, the intracellular protein is selected from the group cinsisting of cytoplasmic proteins, nuclear proteins, organelle proteins, mitochondrial proteins, or lysosomal proteins. In one specific embodiment, the sequence encoding the polypeptide contains one or more introns.

The system described herein can be used ex vivo or in vivo. In some embodiments, e.g., the system or its components are delivered to cells (e.g., mammalian cells, such as human cells) ex vivo or in vivo. In some embodiments, the cells are eukaryotic cells, such as cells of a multicellular organism, e.g., an animal, such as a mammal (e.g., human, pig, cattle), a bird (e.g., poultry, such as chicken, turkey, or duck), or a fish. In some embodiments, the cells are non-human animal cells (e.g., from laboratory animals, livestock, or companion animals). In some embodiments, the cells are stem cells (e.g., hematopoietic stem cells), fibroblasts, or T cells. In some embodiments, the cells are non-proliferating cells, such as non-proliferating fibroblasts or non-proliferating T cells. In some embodiments, the cells are plant cells. Those skilled in the art will appreciate that components of the Gene Writer system may be delivered in the form of polypeptides, nucleic acids (e.g., DNA, RNA), or combinations thereof.

In the present application, the delivery can use any following combination to deliver retrotransposase (e.g., as DNA encoding the retrotransposase protein, as RNA encoding the retrotransposase protein or as the protein per se) and the donor RNA (e.g., as DNA encoding the RNA, or as RNA):
1. retrotransposase DNA + donor DNA
2. retrotransposase RNA + donor DNA
3. retrotransposase DNA + donor RNA
4. retrotransposase RNA + donor RNA
5. retrotransposase protein + donor DNA
6. retrotransposase protein + donor RNA
7. retrotransposase virus + virus containing the donor RNA or DNA
8. retrotransposase virus + donor DNA
9. retrotransposase virus + donor RNA
10. retrotransposase DNA+ virus containing the donor RNA or DNA
11. retrotransposase RNA+ virus containing the donor RNA or DNA
12. retrotransposase protein + virus containing the donor RNA or DNA

As describer above, in some embodiments, a virus is used to deliver DNA or RNA encoding the transposase protein, and in some embodiments, a virus is used to deliver donor RNA (or DNA encoding the donor RNA).

In one embodiment, the system and/or components of the system are delivered in the form of nucleic acids. For example, the retrotransposase polypeptide may be delivered as DNA or RNA encoding said polypeptide, and the donor RNA may be delivered as RNA or its complementary DNA to be transcribed into RNA. In some embodiments, the system or components of the system are delivered on one, two, three, four, or more distinct nucleic acid molecules. In some embodiments, the system or components of the system are delivered as a combination of DNA and RNA. In some embodiments, the system or components of the system are delivered as a combination of DNA and protein. In some embodiments, the system or components of the system are delivered as a combination of RNA and protein. In some embodiments, the transposase polypeptide is delivered as a protein.

In some embodiments, a vector is used to deliver the system or components of the system to a cell, such as a mammalian cell or a human cell. The vector may be, for example, a plasmid or a virus. In some embodiments, the delivery is performed in vivo, in vitro, ex vivo, or in situ. In some embodiments, the virus is an adeno-associated virus (AAV), a lentivirus, or an adenovirus. In some embodiments, the system or components of the system are delivered to cells together with virus-like particles or virions. In some embodiments, delivery utilizes more than one types of virus, virus-like particle, or virion.

In one embodiment, the compositions and systems described herein may be formulated in liposomes or other similar vesicles. Liposomes are spherical vesicular structures composed of one or multiple lipid bilayers surrounding an internal aqueous compartment and a relatively impermeable external lipophilic phospholipid bilayer. Liposomes may be anionic, neutral, or cationic. Liposomes are biocompatible, non-toxic, and capable of delivering both hydrophilic and lipophilic drug molecules, protecting their cargo from degradation by plasma enzymes, and transporting their payload across biological membranes and the blood-brain barrier (BBB).

Vesicles can be formed from several different types of lipids. However, phospholipids are most commonly used to produce liposomes as drug carriers. Methods for preparing multilamellar vesicle lipids are known in the art (see, e.g., U.S. Patent No. 6,693,086, the teaching of which regarding the preparation of multilamellar vesicle lipids are incorporated herein by reference). Although vesicle formation is spontaneous when lipid membranes are mixed with an aqueous solution, it can be accelerated by applying force in an oscillatory form using a homogenizer, ultrasonicator, or extrusion apparatus. Extruded lipids can be prepared by extrusion through filters with progressively reduced pore sizes.

Lipid nanoparticles represent another example of carriers providing biocompatible and biodegradable delivery system for the pharmaceutical compositions described herein. Nanostructured lipid carriers (NLCs) are modified solid lipid nanoparticles (SLNs) that retain SLN characteristics while enhancing drug stability and loading capacity and preventing drug leakage. Polymeric nanoparticles (PNPs) constitute a vital component of drug delivery systems. These nanoparticles effectively direct drug delivery to specific targets while enhancing drug stability and enabling controlled release. Polymer-lipid hybrid nanoparticles (PLNs), a novel carrier combining liposomes and polymers, may also be employed. These nanoparticles possess the complementary advantages of both PNPs and liposomes. PLNs are composed of a core-shell structure: the polymeric core provides structural stability, while the phospholipid shell offers excellent biocompatibility. The two components enhance the drug encapsulation efficiency, facilitate surface modification, and prevent the leakage of water-soluble drugs.

In one aspect, the donor RNA as described herein comprises sequentially the following from the 5' end to the 3' end: a 5' untranslated region (5'UTR) that binds to the retrotransposase, a heterologous sequence, and a 3' untranslated region (3'UTR) that binds to the retrotransposase.

In one aspect, the donor RNA as described herein comprises sequentially the following from the 5' end to the 3' end: a 5' untranslated region (5'UTR) that binds to the retrotransposase, a promoter that operably linked to a heterologous sequence, the heterologous sequence, and a 3' untranslated region (3'UTR) that binds to the retrotransposase.

In one aspect, the donor RNA as described herein comprises sequentially the following from the 5' end to the 3' end: a 5' untranslated region (5'UTR) that binds to the retrotransposase, a heterologous sequence, a promoter that operably linked to the heterologous sequence, and a 3' untranslated region (3'UTR) that binds to the retrotransposase.

In one aspect, the 5' untranslated region (5'UTR) has at least 70%, 75%, 80%, 85%, 90%, 95%, 96%, 97%, 98%, 99%, or 100% identity with the nucleotide sequence as defined in any one of SEQ ID No. 16, 27, 29, 31, 33, 35, 37, 39, and 41.

In one aspect, the 3' untranslated region (3'UTR) has at least 70%, 75%, 80%, 85%, 90%, 95%, 96%, 97%, 98%, 99%, or 100% identity with the nucleotide sequence as defined in any one of SEQ ID No. 17, 32, 34, 44, 46, 48, 50, 52, and 54.

In one aspect, the donor RNA described herein comprises the following structure from its 5' end to 3' end: a first homologous domain, a 5' untranslated region (5'UTR) that binds to the retrotransposase, a heterologous sequence, and a 3' untranslated region (3'UTR) that binds to the retrotransposase, and a second homologous domain.

In one aspect, the sequence of the first homologous domain has at least 70%, 75%, 80%, 85%, 90%, 95%, 96%, 97%, 98%, or 99% identity with the nucleotide sequence as defined in SEQ ID NO.15, 19, 21, 23, 25 or 28.

In one aspect, the sequence of the second homologous domain has at least 70%, 75%, 80%, 85%, 90%, 95%, 96%, 97%, 98%, or 99% identity with the nucleotide sequence as defined in SEQ ID NO.18, 20, 22, 24, 26 or 36.

The donor RNA described herein can also become an engineered transposable element.

The sequences described herein are represented in the form of deoxyribonucleotides. If they represent RNA sequences, those skilled in the art may replace T with U. When describing sequences herein, it may represent RNA sequences as well as their corresponding DNA sequences.

### Examples

The specific embodiments of the present application will be described in greater detail below with reference to the accompanying drawings. Although the specific embodiments of the application are illustrated in the drawings, it should be understood that the application may be implemented in various forms and should not be limited by the embodiments described herein. Rather, these embodiments are provided to enable a thorough understanding of the application and to fully convey the scope of the application to those skilled in the art.

### Experimental Protocol

### Plasmid Construction

The protein sequence of the wild-type TgR2 retrotransposase was codon-optimized for human expression and synthesized. The DNA coding fragment was inserted between the XmaI and NheI restriction sites of the pCAG-SV40 poly(A) vector via Gibson cloning. Point mutations in the TgR2 retrotransposase were introduced via PCR using primers containing the desired mutation sequences. The resulting mutant DNA fragments were then cloned into the pCAG-SV40 poly(A) vector between the XmaI and NheI restriction sites via Gibson cloning. For the plasmid expressing the donor RNA, multiple segments - including GFP (N)-intron-GFP (C), a 5'-UTR, a 3'-UTR, and the CMV promoter - were individually amplified by overlap PCR. These fragments were subsequently assembled and inserted into the pSV40-mCherry vector via Gibson cloning to construct the final donor RNA expressing plasmid.

### Cell culture, Transfection, and Fluorescence-activated Cell Sorting (FACS)

HEK293T cells were cultivated in DMEM (Gibco) contaning 1% penicillin-streptomycin (Gibco) and 10% fetal bovine serum (Gibco). Cells were seeded into 24-well cell culture plates (Corning) for 16 hours until reaching 70%-90% confluency. For transfection, 250ng of plasmid encoding the retrotransposase protein and 250ng of plasmid expressing the donor RNA were transfected into each well of the 24-well plate usingLipofectamine 3000 (Invitrogen). 24 hours post-transfection, cells were detached using trypsin - EDTA (0.05%) (Gibco). Cells exhibiting mCherry signal were then sorted using a MoFlo XDP instrument (Beckman Coulter) and re-seeded into 12-well plates. After continued culture for 6 days, the cells were digested with trypsin-EDTA (0.05%)(Gibco) and then analyzed using a BD FACSAria^{™} Fusion Cell Sorter(BD) to analyze the proportion of GFP-positive cells. By comparing the proportion of GFP-positive cells with that of the negative control, together with observation under a fluorescence microscopy, it was confirmed whether the engineered retrotransposase system exhibited enhanced gene integration efficiency in mammalian cells.

### Example 1: Construction of the GFP reporting system

This example designed one reporting system capable of accurately detecting the activity of a novel retrotransposase system in mammalian cells (see FIG. 1). Specifically, the retrotransposase protein and donor RNA are encoded by 2 separate plasmids, demonstrating the modularity of the novel retrotransposase system. The donor RNA is expressed from a CAG promoter. Notably, the expression cassette initiated by the CAG promoter for donor RNA expression also contains an embedded, inversely oriented expression cassette initiated by a CMV promoter. Within this CMV-driven expression cassette, GFP is expressed, but the GFP coding sequence is interrupted by an intron sequence inserted in the reverse orientation.

Therefore, the ultimate mode of action of the reporting system constructed in Example 1 herein is as follows: after the donor RNA is initiated for expression by the CAG promoter, the intron sequence contained within the donor RNA is spliced out from the expressed RNA. At this point, the GFP sequence within the expression cassette initated by the CMV promoter is restored to normal at the RNA level. However, this GFP sequence is an antisense RNA strand and lacks the ability to be translated into GFP protein. Only when the novel retrotransposase permanently integrates the intron-deleted donor RNA into the cellular DNA via reverse transcriptase activity can the CMV-initated reverse expression cassette produce normal, intron-free GFP mRNA, and subsequently express the green fluorescent GFP protein. Therefore, by detecting the presence and proportion of final GFP-positive cells using fluorescence microscopy and flow cytometry, it is possible to determine whether the novel transposase is functional in mammalian cells and to assess its activity level.

### Example 2: Truncated forms of wild-type TgR2 retain activity

Based on the GFP reporting system designed in Example 1, this example tested the gene integration efficiency of the wild-type TgR2 retrotransposase system and multiple mutants thereof. It should be noted that, the donor RNA of a retrotransposase system generally comprises 5 parts, i.e., a left homology arm, a 5'UTR, a 3'UTR, a sequence carrying the new genetic information located between the 5'UTR and 3'UTR, and a right homology arm (see FIG. 1).

### Plasmid Construction, Cell culture, Transfection, and Fluorescence-Activated Cell Sorting (FACS)

The plasmids used in this example were constructed using the method described in the aforementioned experimental protocol. Cell culture, transfection, and FACS experiments for this example were performed according to the methods described in the aforementioned experimental protocol. Truncated variants of the wild-type TgR2 were constructed in this example: Δ1-220(SEQ ID No.51); Δ390-500(SEQ ID No.13); Δ1100-1200(SEQ ID No.38); Δ1-220+390-500(SEQ ID No.40).

The results revealed that, compared to the reference wild-type TgR2 (SEQ ID No.1), all truncated variants retained a certain level of activity. The experimental results from Example 2 are presented in Table 1 and FIG. 22:

**Table 1**

| Wild-type TgR2 and the truncated variants | GFP integration efficiency (%) |
|---|---|
| WT | 2.12 |
| Δ1-220 | 1.50 |
| Δ390-500 | 1.37 |
| Δ1100-1200 | 1.38 |
| Δ1-220+390-500 | 0.53 |

### Example 3: Introduction of amino acid point mutations based on homologous sequence alignment to enhance the gene integration efficiency of wild-type TgR2

Based on the GFP reporting system designed in Example 1, this example tested the gene integration efficiency of the wild-type TgR2 retrotransposase system and multiple mutants thereof. It should be noted that, the donor RNA of a retrotransposase system generally comprises 5 parts, i.e., a left homology arm, a 5'UTR sequence, a 3'UTR sequence, a sequence carrying the new genetic information between the 5'UTR and 3'UTR, and a right homology arm (see FIG. 1).

### Sequence Alignment

Multiple sequence alignment of the reverse transcriptase sequences within the R2 proteins was performed using the FFT-NS-I x1000 algorithm in MAFFT. The resulting alignment was used to determine the best-fit model based on the Bayesian Information Criterion (BIC) as implemented in ModelFinder. A total of 543 protein models were tested. The most suitable model identified was LG+R7+F. Subsequently, a maximum likelihood (ML) phylogenetic tree was constructed using the LG+R7+F model with the ultrafast bootstrap parameters in IQ-TREE. Based on the position of the TgR2 protein in the phylogenetic tree, the multiple sequence alignment results from the same clade were extracted. Amino acids in TgR2 were mutated to the corresponding alternative amino acid sequences present at the equivalent positions in the multiple sequence alignment.

### Plasmid Construction, Cell culture, Transfection, and Fluorescence-Activated Cell Sorting (FACS)

The plasmids used in this example were constructed following the methods described in the aforementioned experimental protocol. Cell culture, transfection, and FACS experiments for this example were performed using the methods described in the aforementioned experimental protocol.

Based on the sequence alignment results, 36 amino acid residues in TgR2 (SEQ ID No.1) were selected for mutation: K700, L704, V727, S732, L737, A747, A748, T759, I760, W762, S763, H792, A793, Q795, E798, P801, V807, S808, E812, S815, T819, T820, H825, K828, S860, Y864, H865, R866, Q868, S869, N889, N891, T892, S895, Q911, and D957. Each was mutated to the corresponding amino acid residue found at the equivalent position in the multiple sequence alignment. By utilizing the GFP reporting system from Example 1, engineered TgR2 systems harboring single amino acid mutations were expressed, respectively. The preferred amino acid substitution patterns and their corresponding gene writing efficiencies are shown in FIG. 2. The results revealed that, compared to the wild-type TgR2 reference, the following 6 amino acid substitution mutants: K700D, W762Q, H792W, A793V, R866V, and D957G, could significantly increase the gene integration efficiency. However, the efficiency of most mutants did not show a significant improvement, and some even decreased. The experimental results from Example 2 are presented in Table 2 and FIG. 2:

**Table 2**

| wild-type TgR2 and mutants | GFP integration efficiency (%) |
|---|---|
| WT | 1.99 |
| K700D | 2.99 |
| L704Q | 1.92 |
| V727L | 2.27 |
| S732T | 2.44 |
| L737I | 2.09 |
| A747S | 1.78 |
| A748S | 2.32 |
| A748P | 2.41 |
| T759L | 1.12 |
| I760L | 2.42 |
| W762R | 2.33 |
| W762K | 2.35 |
| W762Q | 2.82 |
| S763N | 2.31 |
| S763R | 2.16 |
| H792T | 2.05 |
| H792W | 3.15 |
| A793V | 2.92 |
| Q795K | 2.03 |
| Q795R | 2.52 |
| E798G | 1.97 |
| P801Q | 2.19 |
| V807I | 1.89 |
| S808R | 1.73 |
| E812K | 1.82 |
| E812R | 1.94 |
| S815N | 2.40 |
| T819D | 2.26 |
| T820L | 2.18 |
| H825Q | 1.75 |
| K828P | 1.59 |
| S860E | 1.48 |
| Y864F | 1.63 |
| H865Q | 1.92 |
| H865R | 1.43 |
| R866V | 4.38 |
| Q868S | 1.56 |
| S869K | 1.19 |
| N889G | 1.31 |
| N891Q | 2.22 |
| T892K | 1.69 |
| S895K | 1.61 |
| Q911E | 1.32 |
| D957G | 3.06 |

### Example 4: Introduction of amino acid point mutations based on predicted protein 3D structure alignment to enhance the gene integration efficiency of wild-type TgR2

Based on the GFP reporting system designed in Example 1, this example tested the gene integration efficiency of the wild-type TgR2 retrotransposase system and several mutants thereof. It should be noted that the donor RNA for a retrotransposase system generally comprises 5 parts, i.e., a left homology arm, a 5'UTR sequence, a 3'UTR sequence, a sequence carrying the new genetic information located between the 5'UTR and 3'UTR sequences, and a right homology arm (see FIG. 1).

### Protein 3D Structural Prediction And Simulation

The tertiary structure of the wild-type TgR2 protein was predicted using the default parameters of the ColabFold software (Mirdita, M., Schutze, K., Moriwaki, Y, Heo, L., Ovchinnikov, S., and Steinegger, M. (2022). ColabFold: making protein folding accessible to all. Nat Methods 19, 679-682.). Multiple sequence alignment was performed using the MAFFT software. The boundaries of the various domains of TgR2 were determined based on the results of the multiple sequence alignment and the predicted tertiary structure. The predicted TgR2 structure was fit to the reported structure of a group-II intron reverse transcriptase (Stamos, J.L., Lentzsch, A.M., and Lambowitz, A.M. (2017). Structure of a Thermostable Group II Intron Reverse Transcriptase with Template-Primer and Its Functional and Evolutionary Implications. Mol Cell 68, 926-939 e924) (PDB: 6AR3), and the DNA-RNA was docked into the predicted TgR2 structure.

### Mutant Design

Amino acids within the predicted tertiary structure of TgR2 that potentially interact with DNA or RNA were mutated to positively charged arginine (R), thereby increasing the affinity of TgR2 protein to nucleic acid substrates, thereby improving the efficiency of gene integration.

### Plasmid Construction, Cell culture, Transfection, and Fluorescence-Activated Cell Sorting (FACS)

The plasmids used in this example were constructed using the methods described in the aforementioned experimental protocol. Cell culture, transfection, and FACS experiments for this example were also performed following the method described in the aforementioned experimental protocol.

Based on the results of 3D structure prediction and multiple sequence alignment, 132 amino acid residues in TgR2 were selected for this experiment: E153, G154, E160, E163, Q164, Q168, D227, K228, N229, N240, K267, E268, N269, P291, E294, H341, K342, C344, E350, E361, N363, N365, N367, A379, Q381, S383, D384, S390, D573, G575, K576, K579, I585, L588, K635, E636, E638, K639, N640, Q642, E643, S645, K646, G647, S648, V661, V693, I695, P696, S699, K700, I713, I715, T728, S732, K733, S751, E752, N753, L754, T819, N847, K950, Q955, D957, D990, K993, T994, Y995, P998, I1001, D1005, H1006, T1044, D1046, T1052, Q1062, Q1071, S1072, S1073, D1074, D1075, G1098, G1099, D1100, E1102, N1103, P1105, S1106, E1109, S1113, S1114, E1115, N1118, N1123, E1125, E1127, Q1131, D1133, K1134, K1137, K1210, D1216, D1218, E1220, A1223, H1224, G1227, N1228, Q1233, D1234, I1237, K1238, H1240, N1241, E1245, E1249, N1268, E1270, E1302, E1306, K1307, and K1310, and site-directed mutagenesis to arginine (R) was performed for testing. Using the GFP reporting system in Example 1, engineered TgR2 systems harboring single amino mutations were expressed, respectively. The preferred amino acid substitution sites and their corresponding gene editing efficiencies are shown in FIG. 3. The results revealed that, several mutant versions of TgR2 significantly increased the efficiency of gene integration compared to the wild-type reference. The experimental results in Example 3 are presented in Table 3 and FIG. 3.

**Table 3**

| wild-type TgR2 and mutants | GFP integration efficiency (%) |
|---|---|
| WT | 1.99 |
| E153R | 1.05 |
| G154R | 1.54 |
| E160R | 0.62 |
| E163R | 0.76 |
| Q164R | 1.33 |
| Q168R | 1.97 |
| D227R | 2.68 |
| K228R | 2.63 |
| N229R | 2.32 |
| N240R | 2.27 |
| K267R | 2.40 |
| E268R | 1.95 |
| N269R | 2.09 |
| P291R | 2.91 |
| E294R | 2.18 |
| H341R | 3.74 |
| K342R | 2.55 |
| C344R | 2.61 |
| E350R | 1.97 |
| E361R | 2.27 |
| N363R | 1.98 |
| N365R | 2.31 |
| N367R | 2.40 |
| A379R | 2.11 |
| Q381R | 2.26 |
| S383R | 2.60 |
| D384R | 3.14 |
| S390R | 2.14 |
| D573R | 1.30 |
| G575R | 0.58 |
| K576R | 2.04 |
| K579R | 2.39 |
| I585R | 0.70 |
| L588R | 0.73 |
| K635R | 1.18 |
| E636R | 1.45 |
| E638R | 1.83 |
| K639R | 2.06 |
| N640R | 1.09 |
| Q642R | 1.72 |
| E643R | 0.62 |
| S645R | 1.16 |
| K646R | 2.15 |
| G647R | 1.18 |
| S648R | 0.80 |
| V661R | 1.56 |
| V693R | 0.45 |
| I695R | 0.15 |
| P696R | 0.10 |
| S699R | 0.73 |
| K700R | 0.89 |
| I713R | 0.19 |
| I715R | 0.21 |
| T728R | 1.30 |
| S732R | 0.93 |
| K733R | 2.55 |
| S751R | 2.57 |
| E752R | 0.70 |
| N753R | 0.20 |
| L754R | 0.20 |
| T819R | 2.46 |
| N847R | 0.48 |
| K950R | 2.01 |
| Q955R | 2.97 |
| D957R | 0.75 |
| D990R | 2.22 |
| K993R | 2.19 |
| T994R | 1.54 |
| Y995R | 1.10 |
| P998R | 0.77 |
| I1001R | 0.39 |
| D1005R | 0.96 |
| H1006R | 1.23 |
| T1044R | 2.42 |
| D1046R | 0.21 |
| T1052R | 2.57 |
| Q1062R | 3.77 |
| Q1071R | 2.30 |
| S1072R | 1.88 |
| S1073R | 1.91 |
| D1074R | 5.86 |
| D1075R | 2.02 |
| G1098R | 4.80 |
| G1099R | 2.68 |
| D1100R | 1.96 |
| E1102R | 1.95 |
| N1103R | 1.47 |
| P1105R | 3.96 |
| S1106R | 1.96 |
| E1109R | 2.25 |
| S1113R | 0.72 |
| S1114R | 0.75 |
| E1115R | 1.72 |
| N1118R | 0.56 |
| N1123R | 0.59 |
| E1125R | 2.78 |
| E1127R | 2.52 |
| Q1131R | 2.66 |
| D1133R | 1.00 |
| K1134R | 1.21 |
| K1137R | 1.45 |
| K1210R | 1.76 |
| D1216R | 1.96 |
| D1218R | 0.84 |
| E1220R | 0.01 |
| A1223R | 2.94 |
| H1224R | 0.02 |
| G1227R | 0.65 |
| N1228R | 0.61 |
| Q1233R | 1.84 |
| D1234R | 1.00 |
| I1237R | 3.32 |
| K1238R | 1.11 |
| H1240R | 0.02 |
| N1241R | 0.60 |
| E1245R | 2.64 |
| E1249R | 2.59 |
| N1268R | 1.54 |
| E1270R | 0.93 |
| E1302R | 1.37 |
| E1306R | 2.12 |
| K1307R | 0.17 |
| K1310R | 3.44 |

### Example 5: Substitution of cysteine (C) with serine (S) in the protein sequence increases the integration efficiency of TgR2

Based on the GFP reporting system designed in Example 1, this example tested the gene integration efficiency of the wild-type TgR2 retrotransposase system and several mutants thereof. It should be noted that, the donor RNA for the retrotransposase system generally comprises 5 parts, i.e., a left homology arm, a 5'UTR sequence, a 3'UTR sequence, a sequence carrying the new genetic information located between the 5'UTR and 3'UTR sequences, and a right homology arm (see FIG. 1).

### Plasmid Construction, Cell culture, Transfection, and Fluorescence-activated Cell Sorting (FACS)

The plasmids used in this example were constructed using the method described in the aforementioned experimental protocol. Cell culture, transfection, and FACS experiments in this example were performed using the method described in the aforementioned experimental protocol.

In this example, 20 cysteine (C) residues were selected and mutated to serine (S): C4, C145, C170, C282, C344, C404, C518, C521, C690, C735, C750, C855, C901, C913, C930, C1034, C1036, C1079, C1139, and C1244. Based on the GFP reporting system designed in Example 1, the experimental results were as follows (FIG. 4, Table 4): compared to the wild-type TgR2 protein, four mutant proteins: C344S, C1034S, C1036S, and C1244S significantly increased gene integration efficiency, the remaining mutants showed either no significant improvement, no improvement, or even a decrease in efficiency.

**Table 4**

| wild-type TgR2 and mutant | GFP integration efficiency (%) |
|---|---|
| WT | 1.99 |
| C4S | 1.69 |
| C145S | 2.27 |
| C170S | 1.88 |
| C282S | 0.44 |
| C344S | 3.26 |
| C404S | 2.18 |
| C518S | 2.28 |
| C521S | 2.27 |
| C690S | 1.20 |
| C735S | 2.06 |
| C750S | 1.76 |
| C855S | 1.82 |
| C901S | 1.84 |
| C913S | 0.77 |
| C930S | 1.97 |
| C1034S | 3.00 |
| C1036S | 3.65 |
| C1079S | 2.13 |
| C1139S | 2.72 |
| C1244S | 3.09 |

### Example 6: Point mutations exhibit additive effects

The protein mutations identified in Example 2 and Example 3, which significantly increased gene integration efficiency, were combined. Based on the GFP reporting system designed in Example 1, this example tested the gene integration efficiency of single-point mutant TgR2 retrotransposase proteins and the combinational mutant proteins thereof. It should be noted that a donor RNA of the retrotransposase system generally comprises 5 components, i.e., a left homology arm, a 5'UTR sequence, a 3'UTR sequence, a sequence carrying the novel genetic information between the 5'UTR and 3'UTR sequences , and a right homology arm (see FIG. 1).

### Plasmid Construction, Cell culture, Transfection, and Fluorescence-activated Cell Sorting (FACS)

The plasmids used in this example were constructed following the methods described in the aforementioned experimental protocol. Cell culture, transfection, and FACS experiments in this example were also performed according to the methods described in the aforementioned experimental protocol.

The experimental results are as follows (FIG. 5, Table 5). Compared with the protein with the single-point mutatation G1098R, several mutant combinations - G1098R+H341R, G1098R+K700D, G1098R+W762Q, G1098R+H792W, G1098R+A793V, G1098R+R866V, and G1098R+Q955R - significantly increased gene integration efficiency, demonstrating a remarkable additive effect. The G1098R+A793V mutant, which exbibited the highest integration efficiency, was further combined with additional point mutations. The experimental data are shown in FIG. 5 and Table 6. Compared with the G1098R+A793V mutant, the mutants G1098R+A793V+W762Q, G1098R+A793V+Q955R, G1098R+A793V+Q955R+H341R, and G1098R+A793V+W762Q+Q955R+H341R further increased gene integration efficiency.

**Table 5**

| TgR2 mutants | GFP integration efficiency (%) |
|---|---|
| G1098R (SEQ ID No.2) | 4.09 |
| G1098R+H341R | 5.35 |
| G1098R+K700D | 5.47 |
| G1098R+W762Q | 5.16 |
| G1098R+H792W | 6.62 |
| G1098R+A793V (SEQ ID No.3) | 6.67 |
| G1098R+R866V | 5.43 |
| G1098R+Q955R | 5.94 |
| G1098R+T1052R | 2.13 |
| G1098R+Q1062R | 4.02 |
| G1098R+P1105R | 3.16 |
| G1098R+C1034S | 4.76 |
| G1098R+I1237R | 3.5 |
| G1098R+K1310R | 3.6 |
| G1098R+D957G | 5.9 |
| H341R+C1036S | 4.9 |
| H341R+Q1062R | 5.82 |
| H341R+11237R | 5.45 |
| H341R+K1310R | 5.81 |
| I1237R+K1310R | 4.35 |
| C1034S+C1036S | 1.08 |
| K700D+R866V | 5.52 |
| W762Q+R866V | 5.89 |
| H792W+R866V | 5.49 |
| A793V+R866V | 4.99 |

**Table 6**

| TgR2 mutants | GFP integration efficiency (%) |
|---|---|
| G1098R+A793V | 6.67 |
| G1098R+A793V+K700D | 6.2 |
| G1098R+A793V+W762Q | 8.34 |
| G1098R+A793V+H792W | 6.67 |
| G1098R+A793V+Q955R(SEQ ID No.4) | 7.7 |
| G1098R+A793V+W762Q+Q955R(SEQ ID No.5) | 6.86 |
| G1098R+A793V+W762Q+H341R | 6.54 |
| G1098R+A793V+Q955R+H341R(SEQ ID No.7) | 8 |
| G1098R+A793V+W762Q+Q955R+H341R(SEQ ID No.6) | 9.06 |

### Example 7: Conjugation with exonuclease to increase integration efficiency

Based on the GFP reporting system designed in Example 1, this example tested the gene integration efficiency of the wild-type TgR2 retrotransposase system compared to a system where the wild-type TgR2 transposase system was conjugated with T5 exonuclease at different positions on the protein. It should be noted that a donor RNA of the retrotransposase system generally comprises 5 parts, i.e., the left homology arm, the 5'UTR sequence, the 3'UTR sequence, the sequence carrying the novel genetic information located between the 5'UTR and 3'UTR sequences, and the right homology arm (see FIG. 1).

### Plasmid Construction, Cell culture, Transfection, and Fluorescence-Activated Cell Sorting (FACS)

The plasmids used in this example were constructed using the methods described in the aforementioned experimental protocol. Cell culture, transfection, and FACS experiments for this example were performed using the method described in the aforementioned experimental protocol.

The T5 exonuclease (SEQ ID No. 8) was conjugated or inserted at the N-terminal, after amino acid no. 191 (designated as IN-1), after amino acid no. 432 (designated as IN-2), after amino acid no. 1112 (designated as IN-3) and at the C-terminal of the wild-type TgR2, respectively. It should be noted that two additional glycine residues (-GG-) were added to both ends of the T5 exonuclease inserted after amino acid positions 191, 432, and 1112. Results from the GFP reporting system based on the design in Example 1 show that, compared with the wild-type TgR2, conjugating or inserting the T5 exonuclease at the N-terminal, IN-2 and IN-3 positions of the wild-type TgR2 significantly increased gene integration efficiency. Insertion of the T5 exonuclease at the IN-1 position of the wild-type TgR2 resulted in a modest improvement in gene integration efficiency, while conjugation of the T5 exonuclease at the C-terminal of the wild-type TgR2 resulted in a decrease in gene integration efficiency instead. The experimental data are shown in Table 7 and FIG. 6.

**Table 7**

| Wild-type TgR2 and TgR2 with T5 exonuclease conjugated or inserted at different positions | GFP integration efficiency (%) |
|---|---|
| WT | 2.14 |
| T5-N | 4.20 |
| T5-IN-1 | 2.72 |
| T5-IN-2 | 3.77 |
| T5-IN-3 | 4.46 |
| T5-C | 0.75 |

### Example 8: Conjugation with single-stranded DNA-binding protein to increases integration efficiency

Based on the GFP reporting system designed in Example 1, this example compared the gene integration efficiency of the wild-type TgR2 retrotransposase system with that of wild-type TgR2 retrotransposase systems conjugated at different positions with the single-stranded DNA-binding protein sto7d (abbreviated as Sto) (SEQ ID No. 9) from the bacterium *Sulfolobus tokodaii.* It should be noted that, the donor RNA of retrotransposase system generally comprises 5 parts, i.e., a left homology arm, a 5'UTR sequence, a 3'UTR sequence, a sequence carrying the novel genetic information located between the 5'UTR and 3'UTR sequences, and a right homology arm (see FIG. 1).

### Plasmid Construction, Cell culture, Transfection, and Fluorescence-activated Cell Sorting (FACS)

The plasmids used in this example were constructed using the methods described in the aforementioned experimental protocol. Cell culture, transfection, and FACS experiments for this example were performed using the methods described in the aforementioned experimental protocol.

The single-stranded DNA-binding protein sto7d from the bacterium *Sulfolobus tokodaii* (abbreviated as Sto) were conjugated or inserted at the N-terminal, after amino acid no. 191 (designated as IN-1), after amino acid no. 432 (designated as IN-2), after amino acid no. 1112 (designated as IN-3) and the C-terminal of the wild-type TgR2, respectively. It should be noted that, two additional glycine residues (-GG-) were added to both ends of the Sto single-stranded DNA-binding protein inserted after amino acids 191, 432, and 1112. The results from the GFP reporting system designed in Example 1 showed that, compared with the wild-type TgR2, conjugating or inserting the Sto single-stranded DNA-binding protein at the N-terminal, IN-1 and IN-2 positions of wild-type TgR2 significantly increased the efficiency of gene integration. Insertion of the Sto single-stranded DNA-binding protein at the IN-3 position of wild-type TgR2 resulted in a modest improvement in gene integration efficiency, while conjugation of the Sto single-stranded DNA-binding protein at the C-terminal of wild-type TgR2 led to a decrease in gene integration efficiency. The experimental data are shown in FIG. 7 and Table 8.

**Table 8**

| Wild-type TgR2 and TgR2 having Sto single-stranded DNA-binding protein conjugated or inserted at different positions | GFP integration efficiency (%) |
|---|---|
| WT | 2.14 |
| STO-N | 3.70 |
| STO- IN-1 | 2.72 |
| STO- IN-2 | 3.39 |
| STO- IN-3 | 2.47 |
| STO-C | 1.26 |

### Example 9: Conjugation of protein peptide segments to increase integration efficiency

Based on the GFP reporting system designed in Example 1, this example tested the gene integration efficiency of the wild-type TgR2 retrotransposase system and the wild-type TgR2 retrotransposase system conjugated with a high mobility peptide (CMP) segment at different positions. It should be noted that a donor RNA of the retrotransposase system generally comprises 5 parts, i.e., the left homology arm, the 5'UTR sequence, the 3'UTR sequence, the sequence carrying the new genetic information located between the 5'UTR and 3'UTR sequences, and the right homology arm (see FIG. 1).

### Plasmid Construction, Cell culture, Transfection, and Fluorescence-activated Cell Sorting (FACS)

The plasmids used in this example were constructed using the methods described in the aforementioned experimental protocol. Cell culture, transfection, and FACS experiments for this example were performed using the method described in the aforementioned experimental protocol.

A high mobility peptide segment (CMP) was conjugated or inserted at the N-terminal, after amino acid no. 191 (designated as IN-1), after amino acid no. 432 (designated as IN-2), after amino acid no. 1112 (designated as IN-3) and at the C-terminal of the wild-type TgR2, respectively (Ding, X., Seebeck, T., Feng, Y., Jiang, Y., Davis, G.D., and Chen, F. (2019). Improving CRISPR-Cas9 Genome Editing Efficiency by Fusion with Chromatin-Modulating Peptides. The CRISPR Journal 2, 51-63.) (SEQ ID No. 10). It should be noted that two additional glycine residues (-GG-) were added at both ends of the CMP chromatin-modulating peptide segment inserted after amino acid no. 191, after amino acid no. 432, or after amino acid no. 1112. Results from the GFP reporting system designed in Example 1 showed that, compared to the wild-type TgR2, conjugating or inserting the CMP epigenetic peptide segment at the N-terminal, IN-2 and IN-3 positions of the wild-type TgR2 significantly increased the gene integration efficiency. A modest improvement in gene integration efficiency was also observed when the CMP epigenetic peptide segment was conjugated or inserted at the IN-1 and C-terminal positions of the wild-type TgR2. The experimental data are shown in FIG. 8 and Table 9.

**Table 9**

| Wild-type TgR2 and TgR2 having CMP epigenetic peptide segment conjugated or inserted at different positions | GFP integration efficiency (%) |
|---|---|
| WT | 2.14 |
| CMP-N | 3.57 |
| CMP- IN-1 | 2.36 |
| CMP- IN-2 | 3.89 |
| CMP- IN-3 | 3.93 |
| CMP-C | 2.35 |

### Example 10: Conjugation of the RNaseH domain to increase integration efficiency

Based on the GFP reporting system designed in Example 1, this example tested the gene integration efficiency of the wild-type TgR2 retrotransposase system versus the wild-type TgR2 retrotransposase system conjugated with the RNaseH (abbreviated as RH) domain of MLV reverse transcriptase at different positions. It should be noted that a donor RNA of the retrotransposase system generally comprises 5 parts, i.e., a left homology arm, a 5'UTR sequence, a 3'UTR sequence, a sequence carrying the new genetic information located between the 5'UTR and 3'UTR sequences, and a right homology arm (see FIG. 1).

### Plasmid Construction, Cell culture, Transfection, and Fluorescence-activated Cell Sorting (FACS)

The plasmids used in this example were constructed using the methods described in the aforementioned experimental protocol. Cell culture, transfection, and FACS experiments for this example were performed using the methods described in the aforementioned experimental protocol.

The RNaseH domain (abbreviated as RH)(SEQ ID No. 11) of MLV reverse transcriptase was conjugated or inserted at the N-terminal, after amino acid no. 191 (designated as IN-1), after amino acid no. 432 (designated as IN-2), after amino acid no. 1112 (designated as IN-3) and at the C-terminal of wild-type TgR2. It should be noted that, two additional glycine residues (-GG-) were added at both ends of the RNaseH domain inserted after amino acid no. 191, after amino acid no. 432, or after amino acid no. 1112. Results from the GFP reporting system designed in Example 1 showed that, compared to wild-type TgR2, insertion of the MLV reverse transcriptase RNaseH domain at the IN-2 and IN-3 positions of wild-type TgR2 significantly increased gene integration efficiency. Conjugation of or insertion of the RNaseH domain of MLV reverse transcriptase at the N-terminal and the IN-1 positions of wild-type TgR2 resulted in a modest increase in gene integration efficiency, while conjugation of the RNaseH domain of MLV reverse transcriptase at the C-terminal of wild-type TgR2 led to a decrease in gene integration efficiency. The experimental data are shown in FIG. 9 and Table 10.

**Table 10:**

| Wild-type TgR2 and TgR2 having RNaseH domain conjugated or inserted at different positions | GFP integration efficiency (%) |
|---|---|
| WT | 2.14 |
| RH-N | 2.74 |
| RH- IN-1 | 2.56 |
| RH- IN-2 | 3.70 |
| RH- IN-3 | 3.57 |
| RH -C | 1.25 |

### Example 11: Superimposition modification of conjugated domains

Based on the GFP reporting system designed in Example 1, this example tested the gene integration efficiency of conjugating or inserting the RNaseH domains of any two of the following at the N-terminal or after amino acid no. 432 (designated as IN-2) of the wild-type TgR2: T5 exonuclease, the single-stranded DNA-binding protein sto7d from bacteria Sulfolobus tokodaii (abbreviated as Sto), a chromatin-modulating peptide segment (CMP), or a MLV reverse transcriptase (abbreviated as RH). Furthermore, the gene integration efficiency of optionally inserting the CMP or RH domain at three positions - the N-terminal, after amino acid no. 432 (designated as IN-2), or after amino acid no. 1112 (designated as IN-3) - of TgR2 was tested. It should be noted that the donor RNA for the retrotransposase system generally comprises 5 parts, i.e., a left homology arm, a 5'UTR sequence, a 3'UTR sequence, a sequence carrying the new genetic information located between the 5'UTR and 3'UTR sequences, and a right homology arm (see FIG. 1).

### Plasmid Construction, Cell culture, Transfection, and Fluorescence-Activated Cell Sorting (FACS)

The plasmids used in this example were constructed using the methods described in the aforementioned experimental protocol. Cell culture, transfection, and FACS experiments for this example were performed using the method described in the aforementioned experimental protocol.

The RNaseH domains of any two of T5 exonuclease, the single-stranded DNA-binding protein sto7d from bacteria Sulfolobus tokodaii (abbreviated as Sto), a chromatin-modulating peptide segment or high mobility peptide segment (CMP), and a MLV reverse transcriptase (abbreviated as RH) were selected to be conjugated or inserted at the N-terminal position, or after amino acid no. 432 (designated as IN-2) of the wild-type TgR2. Alternatively, CMP or RH was optionally inserted at three positions: at the N-terminal, after amino acid no. 432 (designated as IN-2), and after amino acid no. 1112 (designated as IN-3) of TgR2. Wherein the combinations T5(N)-CMP(IN-2), T5(N)-RH(IN-2), STO(N)-CMP(IN-2), STO(N)-RH(IN-2), and CMP(N)-CMP(IN-2) exhibited a significant increase in gene integration efficiency. Further enhancement in gene integration efficiency was observed with the combinations STO(N)+CMP(IN-2)+CMP(IN-3), CMP(N)+CMP(IN-2)+RH(IN-3), and CMP(N)+CMP(IN-2)+CMP(IN-3) compared to the original combinations. The remaining combinations showed either no significant improvement or no improvement at all. The experimental results are presented in Table 11 and FIG. 10.

**Table 11**

| Combination manners of conjugations | GFP integration efficiency (%) |
|---|---|
| T5(N)-T5(IN-2) | 4.10 |
| T5(N)-STO(IN-2) | 3.52 |
| T5(N)-CMP(IN-2) | 4.79 |
| T5(N)-RH(IN-2) | 5.06 |
| STO(N)-T5(IN-2) | 3.63 |
| STO(N)-STO(IN-2) | 3.15 |
| STO(N)-CMP(IN-2) | 5.67 |
| STO(N)-RH(IN-2) | 5.51 |
| CMP(N)-T5(IN-2) | 4.41 |
| CMP(N)-STO(IN-2) | 3.34 |
| CMP(N)-CMP(IN-2) | 5.35 |
| CMP(N)-RH(IN-2) | 4.39 |
| STO(N)+CMP(IN-2)+RH(IN-3) | 5.34 |
| STO(N)+CMP(IN-2)+CMP(IN-3) | 7.81 |
| STO(N)+RH(IN-2)+CMP(IN-3) | 5.29 |
| STO(N)+RH(IN-2)+RH(IN-3) | 5.28 |
| CMP(N)+CMP(IN-2)+RH(IN-3) | 6.69 |
| CMP(N)+CMP(IN-2)+CMP(IN-3) | 6.00 |
| CMP(N)+RH(IN-2)+CMP(IN-3) | 4.95 |
| CMP(N)+RH(IN-2)+RH(IN-3) | 4.60 |

### Example 12: Effect of changing the order of the 5'homology arm, 5'UTR, 3'homology arm, and 3'UTR on the gene integration efficiency of wild-type TgR2

Based on the GFP reporting system designed in Example 1, this example tested the gene integration efficiency of the wild-type TgR2 retrotransposase system and a system with modified UTR sequences in the donor RNA of the wild-type TgR2 retrotransposase. It should be noted that the donor RNA of a retrotransposase system generally comprises 5 parts, i.e., a left homology arm, a 5'UTR sequence, a 3'UTR sequence, a sequence carrying the new genetic information located between the 5'UTR and 3'UTR sequences, and a right homology arm (see FIG. 1).

### Plasmid Construction, Cell culture, Transfection, and Fluorescence-Activated Cell Sorting (FACS)

The plasmids used in this example were constructed using the methods described in the aforementioned experimental protocol. Cell culture, transfection, and FACS experiments for this example were performed using the methods described in the aforementioned experimental protocol.

By changing the order of the 5'homology arm, 5'UTR, 3'homology arm and 3'UTR, the effect on the gene integration efficiency of wild-type TgR2 was investigated. Wherein "WT" represents the donor used for wild-typeR2Tg. In Table 12 and FIG. 11, A represents the donor with altered order of the 5'homology arm and 5'UTR; B represents the donor with the 5'UTR deleted; C represents the donor with the altered order of the 3'homology arm and 3'UTR; D represents the donor with the 3'UTR deleted; E represents the donor with simultaneous alterations in the order of both the 5'homology arm/5'UTR and the order of 3'homology arm/3'UTR, and F represents the donor with both the 5'UTR and 3'UTR deleted. The experimental results demonstrated that compared to the wild-type donor, all six donors with altered element order or deleted UTR elements (A, B, C, D, E, F) showed a significant decrease in gene integration efficiency. The schematic diagram of the donor element order is shown in FIG. 11, and the experimental data are shown in Table 12.

**Table 12**

| Donors used | GFP integration efficiency (%) duplicate 1 | GFP integration efficiency (%) duplicate 2 |
|---|---|---|
| WT | 2.46 | 1.82 |
| A | 1.36 | 0.86 |
| B | 1.11 | 0.96 |
| C | 0.67 | 0.47 |
| D | 0.081 | 0.12 |
| E | 0.27 | 0.32 |
| F | 0.087 | 0.11 |

### Example 13: Effect of changing the length of the 5' and 3'homology arms on the gene integration efficiency of wild-type TgR2

Based on the GFP reporting system designed in Example 1, this example tested the gene integration efficiency of the wild-type TgR2 retrotransposase system as well as systems where the length of the 5' and 3'homology arms of the wild-type TgR2 retrotransposase donor RNA was altered. It should be noted that the donor RNA of the retrotransposase system generally comprises 5 parts, i.e., a wild-type or length-changed left homology arm, a 5'UTR sequence, a 3'UTR sequence, a sequence carrying the new genetic information located between the 5'UTR and 3'UTR sequences, and a wild-type or length-changed right homology arm (see FIG. 1).

### Plasmid Construction, Cell culture, Transfection, and Fluorescence-Activated Cell Sorting (FACS)

The plasmids used in this example were constructed using the methods described in the aforementioned experimental protocol. Cell culture, transfection, and FACS experiments for this example were performed using the methods described in the aforementioned experimental protocol.

The effect on the gene integration efficiency of the wild-type TgR2 was investigated by changing the lengths of the 5' and 3' homology arms. In Table 13 and FIG. 12, A1 represents a donor with a 150bp 5' left homology arm (LHA) (SEQ ID No. 15) and a 150bp 3' right homology arm (RHA) (SEQ ID No. 18); A2 represents a donor with a 100bp 5' left homology arm (LHA) (SEQ ID No. 19) and a 100bp 3' right homology arm (RHA) (SEQ ID No. 20); A3 represents a donor with a 50bp 5' left homology arm (LHA) (SEQ ID No. 21) and a 50bp 3' right homology arm (RHA) (SEQ ID No. 22); A4 represents a donor with a 20bp 5' left homology arm (LHA) (SEQ ID No. 23) and a 20bp 3' right homology arm (RHA) (SEQ ID No. 24); A5 represents a donor with a 10bp 5' left homology arm (LHA) (SEQ ID No. 25) and a 10bp 3' right homology arm (RHA) (SEQ ID No. 26); A6 represents a donor with a 100bp 5' left homology arm (LHA) (SEQ ID No. 19) and a 50bp 3' right homology arm (RHA) (SEQ ID No. 22); A7 represents a donor with a 100bp 5' left homology arm (LHA) (SEQ ID No. 19) and a 20bp 3' right homology arm (RHA) (SEQ ID No. 23); A8 represents a donor with a 100bp 5' left homology arm (LHA) (SEQ ID No. 19) and a 10bp 3' right homology arm (RHA) (SEQ ID No. 26); A9 represents a donor with a 50bp 5' left homology arm (LHA) (SEQ ID No. 21) and a 100bp 3' right homology arm (RHA) (SEQ ID No. 20); A10 represents a donor with a 20bp 5' left homology arm (LHA) (SEQ ID No. 23) and a 100bp 3' right homology arm (RHA) (SEQ ID No. 20); A11 represents a donor with a 10bp 5' left homology arm (LHA) (SEQ ID No. 25) and a 100bp 3' right homology arm (RHA) (SEQ ID No. 20); A12 represents a donor lacking both 5' left homology arm (LHA) and 3' right homology arm (RHA). The experimental data demonstrate that the experimental groups using donors A1 and A2, with the corresponding homology arm lengths, exhibited the highest gene integration efficiency. The specific experimental results are presented in Table 13 and FIG. 12.

**Table 13**

| Name of the donors used | Lengths of LHA/RHA (bp) | GFP integration efficiency (%) duplicate 1 | GFP integration efficiency (%) duplicate 2 |
|---|---|---|---|
| A1 | 150/150 | 2.13 | 2.35 |
| A2 | 100/100 | 2.12 | 2.31 |
| A3 | 50/50 | 1.39 | 1.23 |
| A4 | 20/20 | 0.69 | 0.5 |
| A5 | 10/10 | 0.72 | 1.04 |
| A6 | 100/50 | 1.85 | 1.5 |
| A7 | 100/20 | 1.27 | 1.37 |
| A8 | 100/10 | 1.36 | 1.38 |
| A9 | 50/100 | 1.21 | 1.58 |
| A10 | 20/100 | 0.75 | 0.53 |
| A11 | 10/100 | 0.83 | 1.31 |
| A12 | 0/0 | 0.66 | 0.5 |

### Example 14: Effect of modifying 5'UTR and 3'UTR on the gene integration efficiency of wild-type TgR2

Based on the GFP reporting system designed in Example 1, this example tested the gene integration efficiency of the wild-type TgR2 retrotransposase system as well as systems with modified UTR sequences in the donor RNA of the wild-type TgR2 retrotransposase. It should be noted that the donor RNA of a retrotransposase system generally comprises 5 parts, i.e., a left homology arm, the wild-type or modified 5'UTR sequence, the wild-type or modified 3'UTR sequence, a sequence carrying new genetic information located between the 5'UTR and 3'UTR sequences, and a right homology arm (see FIG. 1).

### Plasmid Construction, Cell culture, Transfection, and Fluorescence-Activated Cell Sorting (FACS)

The plasmids used in this example were constructed using the methods described in the aforementioned experimental protocol. Cell culture, transfection, and FACS experiments for this example were performed using the methods described in the aforementioned experimental protocol.

The 5'UTR and 3'UTR sequences of the wild-type TgR2 retrotransposase donor RNA were modified, generating constructs designated as UTR1, 2, 3, 4, 5, 6, 7, 8, 9, 10, 11, 12, 13, and 14, respectively (Table 14 and FIG. 13).
wherein:
the sequence of the 5'UTR used by UTR1 is shown as SEQ ID No. 27, the sequence of the 3'UTR used by UTR1 is shown as SEQ ID No. 17;
the sequence of the 5'UTR used by UTR2 is shown as SEQ ID No. 29, the sequence of the 3'UTR used by UTR2 is shown as SEQ ID No. 17;
the sequence of the 5'UTR used by UTR3 is shown as SEQ ID No. 31, the sequence of the 3'UTR used by UTR3 is shown as SEQ ID No. 17;
the sequence of the 5'UTR used by UTR4 is shown as SEQ ID No. 33, the sequence of the 3'UTR used by UTR4 is shown as SEQ ID No. 17;
the sequence of the 5'UTR used by UTR5 is shown as SEQ ID No. 35, the sequence of the 3'UTR used by UTR5 is shown as SEQ ID No. 17;
the sequence of the 5'UTR used by UTR6 is shown as SEQ ID No. 37, the sequence of the 3'UTR used by UTR6 is shown as SEQ ID No. 17;
the sequence of the 5'UTR used by UTR7 is shown as SEQ ID No. 39, the sequence of the 3'UTR used by UTR7 is shown as SEQ ID No. 17;
the sequence of the 5'UTR used by UTR8 is shown as SEQ ID No. 41, the sequence of the 3'UTR used by UTR8 is shown as SEQ ID No. 17;
the sequence of the 5'UTR used by UTR9 is shown as SEQ ID No.16, the sequence of the 3'UTR used by UTR9 is shown as SEQ ID No. 44;
the sequence of the 5'UTR used by UTR10 is shown as SEQ ID No.16, the sequence of the 3'UTR used by UTR10 is shown as SEQ ID No. 46;
the sequence of the 5'UTR used by UTR11 is shown as SEQ ID No.16, the sequence of the 3'UTR used by UTR11 is shown as SEQ ID No. 48;
the sequence of the 5'UTR used by UTR12 is shown as SEQ ID No.16, the sequence of the 3'UTR used by UTR12 is shown as SEQ ID No. 50;
the sequence of the 5'UTR used by UTR13 is shown as SEQ ID No.16, the sequence of the 3'UTR used by UTR13 is shown as SEQ ID No. 52;
the sequence of the 5'UTR used by UTR14 is shown as SEQ ID No.16, the sequence of the 3'UTR used by UTR14 is shown as SEQ ID No. 54.

Based on the GFP reporting system designed in Example 1, the gene integration efficiency of the wild-type TgR2 retrotransposase system and systems utilizing donor RNAs with engineered UTR sequences was tested. Experimental data showed that donors utilizing engineered UTR sequences 9, 10, and 11 all significantly increased gene integration efficiency, while the remaining engineered sequence donors showed either negligible increase or even a decrease in gene integration efficiency. The specific experimental results are presented in FIG. 13 and Table 14.

**Table 14**

| Wild-type and UTR modified donor sequences | GFP integration efficiency (%) duplicate 1 | GFP integration efficiency (%) duplicate 2 |
|---|---|---|
| WT | 2.23 | 1.98 |
| UTR1 | 1.12 | 1.33 |
| UTR2 | 0.98 | 0.76 |
| UTR3 | 0.97 | 1.02 |
| UTR4 | 0.93 | 0.69 |
| UTR5 | 1.09 | 0.8 |
| UTR6 | 1.3 | 1.71 |
| UTR7 | 1.89 | 1.81 |
| UTR8 | 0.81 | 1.16 |
| UTR9 | 3.41 | 3.5 |
| UTR10 | 3.81 | 4.46 |
| UTR11 | 3.21 | 4.01 |
| UTR12 | 0.6 | 0.61 |
| UTR13 | 0.6 | 0.85 |
| UTR14 | 0.65 | 0.65 |

### Example 15: Engineered proteins combined with engineered RNA donors synergistically increase gene writing efficiency

Using the engineered RNA donor with the optimal donor element order determined based on Example 12, the optimal donor homology arm length determined based on Example 13, and the optimal engineered 5'UTR and 3'UTR determined based on Example 14, we tested the TgR2 retrotransposase system combining the engineering strategies screened in Examples 6 and 11, employing the GFP reporting system designed in Example 1. It should be noted that the donor RNA for a retrotransposase system generally comprises 5 parts, i.e., a left homology arm, a wild-type or modified 5'UTR sequence, a wild-type or modified 3'UTR sequence, a sequence carrying the new genetic information located between the 5'UTR and 3'UTR sequences, and a right homology arm (see FIG. 1).

### Plasmid Construction, Cell culture, Transfection, and Fluorescence-Activated Cell Sorting (FACS)

The plasmids used in this example were constructed using the method described in the aforementioned experimental protocol. Cell culture, transfection, and FACS experiments for this example were performed using the methods described in the aforementioned experimental protocol.

Utilizing the engineered RNA donor with the optimal donor element order determined based on Example 12, the optimal donor homology arm length determined based on Example 13, and the optimized 5'UTR and 3'UTR determined based on Example 14, we tested the TgR2 retrotransposase system comprising combinations of the variants G1098R+A793V+Q955R, G1098R+A793V+W762Q+Q955R, G1098R+A793V+W762Q+Q955R+H341R, and G1098R+A793V+Q955R+H341R (subsequentially abbreviated as PM1, PM2, PM3, and PM4, corresponding to the following sequences, respectively: SEQ ID No.4, SEQ ID No.5, SEQ ID No.6, and SEQ ID No.7) screened in Example 6, with the variants T5(N)-RH(IN-2), CMP(N)+CMP(IN-2)+CMP(IN-3), STO(N)-RH(IN-2), T5(N)-CMP(IN-2), STO(N)-CMP(IN-2), and CMP(N)-CMP(IN-2)(subsequentially abbreviated as TD1, TD2, TD3, TD4, TD5, and TD6, corresponding to the following sequences, respectively: SEQ ID No.55, SEQ ID No.56, SEQ ID No.57, SEQ ID No.58, SEQ ID No.59, and SEQ ID No.60) screened in Example 11. The GFP reporting system designed in Example 1 was employed for this assessment. The combinations tested were as follows:
TD1+PM1 (SEQ ID No.61)
TD1+PM2 (SEQ ID No.62)
TD1+PM3 (SEQ ID No.63)
TD1+PM4 (SEQ ID No.64)
TD2+PM1 (SEQ ID No.65)
TD2+PM2 (SEQ ID No.66)
TD2+PM3 (SEQ ID No.67)
TD2+PM4 (SEQ ID No.68)
TD3+PM1 (SEQ ID No.69)
TD3+PM2 (SEQ ID No.70)
TD3+PM3 (SEQ ID No.71)
TD3+PM4 (SEQ ID No.72)
TD4+PM1 (SEQ ID No.73)
TD4+PM2 (SEQ ID No.74)
TD4+PM3 (SEQ ID No.75)
TD4+PM4 (SEQ ID No.76)
TD5+PM1 (SEQ ID No.77)
TD5+PM2 (SEQ ID No.78)
TD5+PM3 (SEQ ID No.79)
TD1+PM4 (SEQ ID No.80)
TD6+PM1 (SEQ ID No.81)
TD6+PM2 (SEQ ID No.82)
TD6+PM3 (SEQ ID No.83)
TD6+PM4 (SEQ ID No.84).

Compared to the wild-type TgR2 retrotransposase system and the uncombined preferred proteins (TD1, TD2, TD3, TD4, TD5, TD6, PM1, PM2, PM3, PM4), the combinations TD1+PM1, TD1+PM2, TD1+PM4, TD2+PM1, TD2+PM2, TD2+PM4, TD3+PM1, TD3+PM2, TD3+PM4, TD4+PM1, TD4+PM2, TD5+PM1, TD5+PM2, and TD5+PM4 significantly increased gene integration efficiency. In contrast, the remaining combinations showed either negligible increase or even a decrease in the gene integration efficiency. The experimental results are presented in Table 15 and FIG. 14.

**Table 15:**

| Wild-type and optimized proteins | GFP integration efficiency (%) duplicate 1 | GFP integration efficiency (%) duplicate 2 |
|---|---|---|
| WT | 5.77 | 6.09 |
| TD1 | 16.1 | 19.1 |
| TD2 | 17.4 | 19.7 |
| TD3 | 13.8 | 12.9 |
| TD4 | 17.5 | 16.1 |
| TD5 | 15.2 | 17.9 |
| TD6 | 17.7 | 19 |
| PM1 | 11.3 | 13.7 |
| PM2 | 15.5 | 17.1 |
| PM3 | 24.2 | 23.7 |
| PM4 | 20.6 | 21.6 |
| TD1+PM1 | 31.8 | 32.9 |
| TD1+PM2 | 26.8 | 32.1 |
| TD1+PM3 | 26.6 | 26.6 |
| TD1+PM4 | 31 | 30 |
| TD2+PM1 | 31.4 | 30 |
| TD2+PM2 | 28.3 | 26.9 |
| TD2+PM3 | 28.3 | 28 |
| TD2+PM4 | 29.8 | 31.5 |
| TD3+PM1 | 30.8 | 32 |
| TD3+PM2 | 29.8 | 26.6 |
| TD3+PM3 | 24.9 | 22.4 |
| TD3+PM4 | 29 | 30 |
| TD4+PM1 | 29.6 | 30 |
| TD4+PM2 | 25.4 | 25 |
| TD4+PM3 | 20.3 | 24.6 |
| TD4+PM4 | 23.3 | 22.3 |
| TD5+PM1 | 30.8 | 31.4 |
| TD5+PM2 | 28.8 | 30.5 |
| TD5+PM3 | 28.7 | 24.9 |
| TD5+PM4 | 27.6 | 31.6 |
| TD6+PM1 | 8.54 | 8.92 |
| TD6+PM2 | 6.98 | 7.37 |
| TD6+PM3 | 3.68 | 3.85 |
| TD6+PM4 | 6.63 | 7.02 |

### Example 16: Using Lipo3000 liposomes to deliver mRNA expressing the R2 retrotransposase protein and an RNA donor

This example established an *ex vivo* transcription system for mRNA expressing the TgR2 protein and donor RNA, and tested the gene integration efficiency when using Lipo3000 liposomes to deliver the mRNA of the TgR2 retrotransposase system along with the donor RNA.

### Plasmid Construction and Ex Vivo Transcription

Plasmids containing the sequences for en-TgR2 and en-Donor were used as templates for ex vivo transcription using the HiScribe^{™} T7 mRNA synthesis kit.

### Cell culture, Transfection, and Fluorescence-Activated Cell Sorting (FACS)

HEK293T cells were cultured in DMEM (Gibco) supplemented with 1% penicillin-streptomycin (Gibco) and 10% fetal bovine serum (Gibco). The cells were seeded into 24-well cell culture plates (Corning) for 16 hours until reaching 70%-90% confluency. Using Lipofectamine 3000 (Invitrogen), 1µg of mRNA encoding the retrotransposase protein and 1µg of donor RNA were transfected into each well of the 24-well plate. At 96 hours post-transfection, cells were detached with trypsin-EDTA (0.05%) (Gibco). The proportion of cells exhibiting GFP-positive signals was then analyzed using a BD FACSAria^{™} Fusion Cell Sorter (BD).

In this example, Lipo3000 liposomes were used to deliver mRNA encoding either the wild-type or the optimized TgR2 retrotransposase system (the TgR2 retrotransposase system constructed using SEQ ID NO. 77 was designated as en-TgR2). The engineered RNA donor containing the 5'UTR (SEQ ID NO.16) and a non-natural 3'UTR (SEQ ID NO.46) was designated as en-Donor. The gene writing efficiencies for both systems are shown in Table 16. The results showed that, compared to the wild-type TgR2 system, the preferred system utilizing en-Donor and en-TgR2 (SEQ ID NO. 77) significantly increased gene integration efficiency. The experimental results from Example 16 are shown in Table 16 and FIG. 15.

**Table 16:**

| TgR2 system used | GFP integration efficiency (%) duplicate 1 | GFP integration efficiency (%) duplicate 2 | GFP integration efficiency (%) duplicate 3 |
|---|---|---|---|
| wt-TgR2+ wt-Donor | 2.52 | 1.25 | 1.25 |
| wt-Donor | 0 | 0 | 0 |
| en-TgR2+en-Donor | 10 | 10.7 | 10.9 |
| en-Donor | 0 | 0 | 0 |

### Example 17: Transfection of HEK293T, Huh7 and N2A cell lines using LNP-encapsulated mRNA expressing the TgR2 retrotransposase system and donor RNA

Based on the *ex vivo* transcription system constructed in Example 16 comprising mRNA expressing the TgR2 protein (SEQ ID NO. 77) and donor RNA, the corresponding mRNAs were obtained through ex vivo transcription and purification steps. These mRNAs were then encapsulated using lipid nanoparticles (LNP). This example evaluated the gene integration efficiency following transfection of HEK293T, Huh7 and N2A cell lines with LNPs packaging the optimized mRNA of the TgR2 retrotransposase system.

### Cell culture, LNP transfection, and Fluorescence-Activated cell sorting (FACS)

HEK293T, Huh7 and N2A cell lines were cultured in DMEM (Gibco) supplemented with 1% penicillin-streptomycin (Gibco) and 10% fetal bovine serum (Gibco). Cells were seeded in 24-well cell culture plates (Corning) for 16 hours until reaching 70%-90% confluence. Transfection was performed by adding LNPs packaged with 1µg of mRNA encoding the retrotransposase protein and 2µg of donor RNA to each well of the 24-well plate. After 72 hours of transfection, cells were detached using trypsin-EDTA (0.05%)(Gibco). The proportion of cells exhibiting GFP-positive signals was then analyzed using a BD FACSAria^{™} Fusion Cell Sorter (BD). Enhanced gene integration efficiency of the engineered retrotransposase system in mammalian cells was confirmed by comparing the proportion of GFP-positive cells with that of the negative control, in conjunction with observations under a fluorescence microscopy.

In this example, LNPs were used to package the mRNA of an optimized TgR2 retrotransposase (SEQ ID NO. 77) system, which was subsequently used to transfect the HEK293T, Huh7 and N2A cell lines. The gene writing efficiencies of the two kinds of mRNA observed in HEK 293T, Huh7 and N2A cell lines are shown in Table 17. The results show that following LNP-mediated transfection, the optimized TgR2 retrotransposase system achieved gene integration efficiency of 16.9%, 25.4%, and 14% in the HEK 293T, Huh7 and N2A cell lines, respectively. The experimental results of Example 17 are shown in Table 17 and FIG. 16.

**Table 17:**

| Cell lines | TgR2 systems used | GFP integration efficiency (%) duplicate 1 | GFP integration efficiency (%) duplicate 2 | GFP integration efficiency (%) duplicate 3 |
|---|---|---|---|---|
| HEK293T | en-TgR2+en-Donor | 16 | 13.8 | 16.9 |
| HEK 293T | en-Donor | 0.062 | 0.021 | 0.057 |
| Huh7 | en-TgR2+en-Donor | 24.3 | 25.3 | 25.4 |
| Huh7 | en-Donor | 0.4 | 0.38 | 0.38 |
| N2A | en-TgR2+en-Donor | 14 | 13.9 | 10.6 |
| N2A | en-Donor | 0.43 | 0.47 | 0.27 |

### Example 18: Expression of VPX protein increases gene integration efficiency of the TgR2 system

Based on the GFP reporting system described in Example 1, this example tested the effect of co-transfecting plasmids expressing VPX protein from different viral sources on the gene integration efficiency of the TgR2 system.

### Plasmid Construction

Plasmids used in this example were constructed using the methods described in the aforementioned experimental protocol. In addition, the DNA encoding sequences for the VPX protein derived from SIV virus (SEQ ID NO. 85), the VPX protein derived from SIV2 virus (SEQ ID NO. 86), and the VPX protein derived from HIV2 virus (SEQ ID NO. 87) were obtained through synthesis. Subsequently, the DNA coding fragments were inserted between the XmaI and NheI restriction sites of the pCAG-SV40 poly(A) vector via Gibson cloning.

### Cell culture, Transfection, and Fluorescence-Activated Cell Sorting (FACS)

Experiments were conducted using the same cell culture conditions as described in the experimental protocol of Example 16. UsingLipofectamine 3000 (Invitrogen), 250ng of plasmid encoding the retrotransposase protein (en-TgR2) and 250ng of plasmid expressing the donor RNA (en-Donor) were co-transfected into each well of a 24-well plate, along with a VPX plasmid. At 120 hours post-transfection, cells were detached using trypsin-EDTA (0.05%)(Gibco). The proportion of cells exhibiting GFP-positive signals was subsequently analyzed using a BD FACSAria^{™} Fusion Cell Sorter (BD).

Based on the GFP reporting system described in Example 1, this example tested the gene integration efficiency of the TgR2 system when co-transfected with plasmids expressing VPX proteins derived from different viral sources (which were SIV-VPX(SEQ ID No.85), SIV2-VPX(SEQ ID No.86), and HIV2-VPX(SEQ ID No.87), respectively). The results showed that co-transfection with the SIV-VPX plasmid reduced the gene integration efficiency of the TgR2 system. Co-transfection with the SIV-VPX plasmid slightly increased the gene integration efficiency of TgR2 system, while co-transfection with the HIV2-VPX plasmid increased the gene integration efficiency of the TgR2 system. The experimental results from Example 18 are shown in Table 18 and FIG. 17.

**Table 18:**

| VPX protein used | GFP integration efficiency (%) duplicate 1 | GFP integration efficiency (%) duplicate 2 | GFP integration efficiency (%) duplicate 3 |
|---|---|---|---|
| SIV VPX | 12.4 | 11 | 12.3 |
| SIV2 VPX | 18.3 | 21 | 22 |
| HIV2 VPX | 29.6 | 29.1 | 31 |
| Control (no) | 17.9 | 20.1 | 19.6 |

### Example 19: The engineered TgR2 system can integrate genes other than GFP

This example tested the integration efficiency of the engineered TgR2 system for the GDNF (SEQ ID No.88), EPO (SEQ ID No.89) and Factor VI (SEQ ID No.53) genes. By replacing the GFP gene sequence in the donor RNA plasmid of the GFP reporting system described in Example 1 with the GDNF, EPO and Factor VI genes, the corresponding genes were integrated into cells using this system. Integration efficiency of the engineered TgR2 system (en-TgR2) for the GDNF, EPO and Factor VI genes was subsequently determined in combination with ELISA detection method.

### Plasmid Construction and In Vitro Transcription

The plasmids used in this example were constructed using the methods described in the experimental protocol of Example 16. Furthermore, the DNA coding sequences for the GDNF, EPO, and Factor VI genes were obtained by synthesis. Plasmids expressing donor RNA for the corresponding genes were genereated by replacing the original GFP gene via overlap PCR. Using the constructed *in vitro* transcription plasmids as templates, *in vitro* transcription was performed with the HiScribe^{™} T7 mRNA synthesis kit. The EPO content was measured using a human Erythropoietin (EPO) ELISA kit; the GDNF content was measured using a Human GDNF ELISA Kit, and the Factor IX content was measured using a Biophen kit.

### Cell culture, Transfection, and Fluorescence-Activated Cell Sorting (FACS)

The experiments were conducted using the same cell culture conditions as described in the experimental protocol of Example 16. For each well of a 24-well plate, 1µg of mRNA encoding the retrotransposase protein (en-TgR2) and 2µg of donor RNA (en-Donor) were transfected using Lipofectamine 3000 (Invitrogen). At 96 hours post-transfection, the cells were detached using trypsin-EDTA (0.05%) (Gibco).

This example tested the integration efficiency of the engineered TgR2 system for the GDNF, EPO and Factor VI genes. In the GFP reporting system described in Example 1, the GFP gene sequence in the donor RNA plasmid was replaced with the corresponding GDNF, EPO and Factor VI genes. This modified reporting system was then used to test the integration of these target genes in cells. Combined with ELISA detection, the results showed that the engineered TgR2 system successfully integrated the GDNF, EPO, and Factor VI genes. The experimental results from Example 19 are shown in FIG. 18.

### Example 20: en-TgR2 truncated proteins increase gene integration efficiency

Based on the GFP reporting system described in Example 1, this example tested the gene integration efficiency of different en-TgR2 truncate proteins (are SEQ ID No.42, SEQ ID No.43, and SEQ ID No.45, respectively). By referencing the wild-type TgR2 protein sequence, corresponding truncated proteins were designed and tested for gene integration efficiency in combination with the en-Donor.

### Plasmid Construction

The plasmids used in this example were constructed using the methods described in the aforementioned experimental protocol.

### Cell culture, Transfection, and Fluorescence-Activated Cell Sorting (FACS)

HEK293T cells were cultured in DMEM (Gibco) contaning 1% penicillin-streptomycin (Gibco) and 10% fetal bovine serum (Gibco). Cells were seeded into 24-well cell culture plates (Corning) for 16 hours until reaching 70%-90% confluency. UsingLipofectamine 3000 (Invitrogen), 250ng of plasmid encoding the retrotransposase protein and 250ng of plasmid expressing the donor RNA were transfected into each well of the 24-well plate. At 120 hours post-transfection, cells were detached with trypsin-EDTA (0.05%)(Gibco), and the proportion of cells exhibiting GFP-positive signals was analyzed using a BD FACSAria^{™} Fusion Cell Sorter(BD). Enhanced gene integration efficiency of the engineered retrotransposase system in mammalian cells was confirmed by comparing the proportion of GFP-positive cells with that of the negative control, combined with observations under a fluorescence microscopy.

This example tested the effects of different en-TgR2 truncated proteins on gene integration efficiency. By referencing the wild-type TgR2 protein sequence, amino acis at corresponding positions of 1-80, 1-190 and 1-220 were deleted. The corresponding en-TgR2 truncated proteins were designated as en-TgR2-v1 (SEQ ID No.42), en-TgR2-v2(SEQ ID No.43) and en-TgR2-v3(SEQ ID No.45). The results showed that, compared to the full-length en-TgR2 protein, the truncated variants en-TgR2-v1 and en-TgR2-v3 significantly increased gene writing efficiency. The results of Example 20 are presented in Table 19 and FIG. 19.

**Table 19:**

| Names of the TgR2 proteins used | GFP integration efficiency (%) duplicate 1 | GFP integration efficiency (%) duplicate 2 | GFP integration efficiency (%) duplicate 3 |
|---|---|---|---|
| en-TgR2-v1 | 24.9 | 24.5 | 23.3 |
| en-TgR2-v2 | 21.1 | 22.9 | 19.8 |
| en-TgR2-v3 | 25 | 25.9 | 25.6 |
| en-TgR2 | 20.4 | 23.8 | 21.6 |

### Example 21: Gene integration using engineered R2 protein with donor RNA containing modified bases

This example describes the construction of an in vitro transcription system for mRNA expressing the TgR2 protein and donor RNA. In this system, uracil (U) in the donor RNA was substituted with N1-methyl-pseudouridine (m1Ψ). The gene integration efficiency was tested upon delivery of the mRNA encoding the TgR2 transposase system and the donor RNA using Lipo3000 liposomes.

### Plasmid Construction and In Vitro Transcription

Plasmids containing the sequences for TgR2 and the donor were used as templates for *in vitro* transcription, which was performed using the HiScribe^{™} T7 mRNA synthesis kit.

### Cell culture, Transfection, and Fluorescence-Activated Cell Sorting (FACS)

HEK293T cells were cultured in DMEM (Gibco) contaning 1% penicillin-streptomycin (Gibco) and 10% fetal bovine serum (Gibco). Cells were seeded in 24-well plates (Corning) for 16 hours, until reaching 70%-90% confluency. UsingLipofectamine 3000 (Invitrogen), 500ng of mRNA encoding the retrotransposase protein and 1µg of donor RNA were transfected into each well of the 24-well plate. At 96 hours post-transfection, cells were detached with trypsin-EDTA (0.05%) (Gibco). The proportion of cells exhibiting GFP-positive signals was then analyzed using a BD FACSAria^{™} Fusion Cell Sorter (BD).

This example used engineered TgR2 sequences: TD5 (SEQ ID No.59) and TD5-v1 (SEQ ID No.12). The engineered donor RNA sequence (optDonor) employed in this example consists of the following components.

### First homologous domain:

A1 (SEQ ID No.19)
A2 (SEQ ID No.28);
   **a 5' untranslated region (5'UTR) that binds to the retrotransposase:**
B (SEQ ID No.16);
   **a heterologous sequence:**
C (SEQ ID No. 30);
   **a 3' untranslated region (3'UTR) that binds to retrotransposase:**
D1 (SEQ ID No.32)
D2 (SEQ ID No.34);
   **second homologous domain:**
E1 (SEQ ID No.20)
E2 (SEQ ID No.36).

The optDonor sequences were generated by sequentially combining the above sequences, wherein for optDonor-2, 3, 4, 5, and 6, 22 adenine residues were added to their 3' ends.
optDonor-1: A1+B+C+D1+E1
optDonor-2: A+B+C+D1+E2
optDonor-3: A1+B+C+D1+E1
optDonor-4: A2+B+C+D1+E2
optDonor-5: A1+B+C+D2+E2
optDonor-6: A2+B+C+D2+E2

The experimental results (Table 20, FIG. 20) demonstrated that T5D-v1 could achieve a GFP gene integration efficiency exceeding 60% in human cells.

**Table 20:**

| Experimental grous | | GFP integration efficiency (%) duplicate 1 | GFP integration efficiency (%) duplicate 2 |
|---|---|---|---|
| TD5 + | optDonor-1 | 11.6 | |
| | optDonor-2 | 23.4 | |
| | optDonor-3 | 13.4 | |
| | optDonor-4 | 30.7 | |
| | optDonor-5 | 34.9 | |
| | optDonor-6 | 48.7 | 55.2 |
| TD5-v1 + | optDonor-6 | 52.8 | 62.3 |

### Example 22: Further design of R2 protein mutants for gene integration in combination with donor RNA containing modified nucleotides

This example established an in vitro transcription system for mRNA encoding the TgR2 protein and donor RNA. In this system, uracil (U) in the donor RNA was substituted with N1-methyl-pseudouridine (m1Ψ). The gene integration efficiency was tested when the mRNA encoding the TgR2 transposase system and the donor RNA were delivered using Lipo3000 liposomes.

### Plasmid Construction and In Vitro Transcription

Plasmids containing the TgR2 sequence and the donor sequence were used as templates for in vitro transcription, which was performed using the HiScribe^{™} T7 mRNA synthesis kit.

### Cell culture, Transfection, and Fluorescence-Activated Cell Sorting (FACS)

HEK293T cells were cultured in DMEM (Gibco) contaning 1% penicillin-streptomycin (Gibco) and 10% fetal bovine serum (Gibco). Cells were seeded into 24-well cell culture plates (Corning) for 16 hours until reaching 70%-90% confluency. UsingLipofectamine 3000 (Invitrogen), 500ng of mRNA encoding the retrotransposase protein and 1µg of donor RNA were transfected into each well of the 24-well plate. At 96 hours post-transfection, cells were detached with trypsin-EDTA (0.05%) (Gibco). The proportion of cells exhibiting GFP-positive signals was then analyzed using a BD FACSAria^{™} Fusion Cell Sorter (BD).

This example used the engineered TgR2 sequence TD5-v1(SEQ ID No.12) and its further designed mutants based on TD5-v1. (For engineered TgR2 proteins with amino acid truncations or insertions, such as SEQ ID No.59 or SEQ ID No.12, the numbering of mutated amino acids follows the sequence of the reference wild-type protein (SEQ ID No.1).)

The experiments in this example were performed using the engineered optDonor-6 sequence from Example 20, to which 22 Adenines (A) were added at the end of optDonor-6. The experimental results are presented in Table 21 and FIG. 21.

**Table 21**

| | | GFP integration efficiency (%) |
|---|---|---|
| TD5-v1 + | H341R | 54.8 |
| | C344S | 53 |
| | W762Q | 50.3 |
| | H792W | 55.2 |
| | A793V | 49.3 |
| | T921R | 51.1 |
| | K922R | 53.3 |
| | D923R | 53.7 |
| | Q955R | 53.7 |
| | T1011R | 51.9 |
| | A1012R | 51.5 |
| | E1015R | 52 |
| | Q1019R | 57.1 |
| | T1023R | 50 |
| | C1036S | 49.2 |
| | D1037R | 50.6 |
| | K1310R | 54.8 |
| | I1237R | 55.4 |
| | H341R+K1310R | 56.8 |
| | A793V+K1310R | 55 |
| | TD5-v1 | 52.8 |

The sequences involved in the present application are shown as below:

| No. | descriptio n | sequence |
|---|---|---|
| 1 | Amino acid sequence of the wild-type TgR2 | MASCPKPGPPVSAGAMSLESGLTTHSVLAIERGPNSLANSGSDFGGGGLGLPLRLLRVSVGTQTSRSDWV DLVSWSHPGPTSKSQQVDLVSLFPKHRVDLLSKNDQVDLVAQFLPSKFPPNLAENDLALLVNLEFYRSDL HVYECVHFAAHWEGLSGLPEVYEQLAPQPCVGETLHSSLPRDSELFVPEEGSSEKESEDAPKTSPPTPGKH GLEQTGEEKVMVTVPDKNPPCPCCGTRVNSVLNLIEHLKVSHGKRGVCFRCAKCGKENSNYHSVVCHFP KCRGPETEKAPAGEWICEVCNRDFTTKIGLGQHKRLAHPAVRNQERIVASQPKETSNRGAHKRCWTKEE EELLIRLEAQFEGNKNINKLIAEHITTKTAKQISDKRRLLSRKPAEEPREEPGTCHHTRRAAASLRTEPEMS HHAQAEDRDNGPGRRPLPGRAAAGGRTMDEIRRHPDKGNGQQRPTKQKSEEQLQAYYKKTLEERLSAG ALNTFPRAFKQVMEGRDIKLVINQTAQDCFGCLESISQIRTATRDKKDTVTREKHPKKPFQKWMKDRAIK KGNYLRFQRLFYLDRGKLAKIILDDIECLSCDIPLSEIYSVFKTRWETTGSFKSLGDFKTYGKADNTAFREL ITAKEIEKNVQEMSKGSAPGPDGITLGDVVKMDPEFSRTMEIFNLWLTTGKIPDMVRGCRTVLIPKSSKPD RLKDINNWRPITIGSILLRLFSRIVTARLSKACPLNPRQRGFIRAAGCSENLKLLQTIIWSAKREHRPLGVVF VDIAKAFDTVSHQHIIHALQQREVDPHIVGLVSNMYENISTYITTKRNTHTDKIQIRVGVKQGDPMSPLLF NLAMDPLLCKLEESGKGYHRGQSSITAMAFADDLVLLSDSWENMNTNISILETFCNLTGLKTQGQKCHG FYIKPTKDSYTINDCAAWTINGTPLNMIDPGESEKYLGLQFDPWIGIARSGLSTKLDFWLQRIDQAPLKPL QKTDILKTYTIPRLIYIADHSEVKTALLETLDQKIRTAVKEWLHLPPCTCDAILYSSTRDGGLGITKLAGLIP SVQARRLHRIAQSSDDTMKCFMEKEKMEQLHKKLWIQAGGDRENIPSIWEAPPSSEPPNNVSTNSEWEAP TQKDKFPKPCNWRKNEFKKWTKLASQGRGIVNFERDKISNHWIQYYRRIPHRKLLTALQLRANVYPTRE FLARGRQDQYIKACRHCDADIESCAHIIGNCPVTQDARIKRHNYICELLLEEAKKKDWVVFKEPHIRDSN KELYKPDLIFVKDARALVVDVTVRYEAAKSSLEEAAAEKVRKYKHLETEVRHLTNAKDVTFVGFPLGAR GKWHQDNFKLLTELGLSKSRQVKMAETFSTVALFSSVDIVHMFASRARKSMVM |
| 2 | Amino acid sequence of the G1098R mutant | MASCPKPGPPVSAGAMSLESGLTTHSVLAIERGPNSLANSGSDFGGGGLGLPLRLLRVSVGTQTSRSDWV DLVSWSHPGPTSKSQQVDLVSLFPKHRVDLLSKNDQVDLVAQFLPSKFPPNLAENDLALLVNLEFYRSDL HVYECVHFAAHWEGLSGLPEVYEQLAPQPCVGETLHSSLPRDSELFVPEEGSSEKESEDAPKTSPPTPGKH GLEQTGEEKVMVTVPDKNPPCPCCGTRVNSVLNLIEHLKVSHGKRGVCFRCAKCGKENSNYHSVVCHFP KCRGPETEKAPAGEWICEVCNRDFTTKIGLGQHKRLAHPAVRNQERIVASQPKETSNRGAHKRCWTKEE EELLIRLEAQFEGNKNINKLIAEHITTKTAKQISDKRRLLSRKPAEEPREEPGTCHHTRRAAASLRTEPEMS HHAQAEDRDNGPGRRPLPGRAAAGGRTMDEIRRHPDKGNGQQRPTKQKSEEQLQAYYKKTLEERLSAG ALNTFPRAFKQVMEGRDIKLVINQTAQDCFGCLESISQIRTATRDKKDTVTREKHPKKPFQKWMKDRAIK KGNYLRFQRLFYLDRGKLAKIILDDIECLSCDIPLSEIYSVFKTRWETTGSFKSLGDFKTYGKADNTAFREL ITAKEIEKNVQEMSKGSAPGPDGITLGDVVKMDPEFSRTMEIFNLWLTTGKIPDMVRGCRTVLIPKSSKPD RLKDINNWRPITIGSILLRLFSRIVTARLSKACPLNPRQRGFIRAAGCSENLKLLQTIIWSAKREHRPLGVVF VDIAKAFDTVSHQHIIHALQQREVDPHIVGLVSNMYENISTYITTKRNTHTDKIQIRVGVKQGDPMSPLLF NLAMDPLLCKLEESGKGYHRGQSSITAMAFADDLVLLSDSWENMNTNISILETFCNLTGLKTQGQKCHG FYIKPTKDSYTINDCAAWTINGTPLNMIDPGESEKYLGLQFDPWIGIARSGLSTKLDFWLQRIDQAPLKPL QKTDILKTYTIPRLIYIADHSEVKTALLETLDQKIRTAVKEWLHLPPCTCDAILYSSTRDGGLGITKLAGLIP SVQARRLHRIAQSSDDTMKCFMEKEKMEQLHKKLWIQARGDRENIPSIWEAPPSSEPPNNVSTNSEWEAP |
| | | TQKDKFPKPCNWRKNEFKKWTKLASQGRGIVNFERDKISNHWIQYYRRIPHRKLLTALQLRANVYPTRE FLARGRQDQYIKACRHCDADIESCAHIIGNCPVTQDARIKRHNYICELLLEEAKKKDWVVFKEPHIRDSN KELYKPDLIFVKDARALVVDVTVRYEAAKSSLEEAAAEKVRKYKHLETEVRHLTNAKDVTFVGFPLGAR GKWHQDNFKLLTELGLSKSRQVKMAETFSTVALFSSVDIVHMFASRARKSMVM |
| 3 | Amino acid sequence of the G1098R+ A793V mutant | MASCPKPGPPVSAGAMSLESGLTTHSVLAIERGPNSLANSGSDFGGGGLGLPLRLLRVSVGTQTSRSDWV DLVSWSHPGPTSKSQQVDLVSLFPKHRVDLLSKNDQVDLVAQFLPSKFPPNLAENDLALLVNLEFYRSDL HVYECVHFAAHWEGLSGLPEVYEQLAPQPCVGETLHSSLPRDSELFVPEEGSSEKESEDAPKTSPPTPGKH GLEQTGEEKVMVTVPDKNPPCPCCGTRVNSVLNLIEHLKVSHGKRGVCFRCAKCGKENSNYHSVVCHFP KCRGPETEKAPAGEWICEVCNRDFTTKIGLGQHKRLAHPAVRNQERIVASQPKETSNRGAHKRCWTKEE EELLIRLEAQFEGNKNINKLIAEHITTKTAKQISDKRRLLSRKPAEEPREEPGTCHHTRRAAASLRTEPEMS HHAQAEDRDNGPGRRPLPGRAAAGGRTMDEIRRHPDKGNGQQRPTKQKSEEQLQAYYKKTLEERLSAG ALNTFPRAFKQVMEGRDIKLVINQTAQDCFGCLESISQIRTATRDKKDTVTREKHPKKPFQKWMKDRAIK KGNYLRFQRLFYLDRGKLAKIILDDIECLSCDIPLSEIYSVFKTRWETTGSFKSLGDFKTYGKADNTAFREL ITAKEIEKNVQEMSKGSAPGPDGITLGDVVKMDPEFSRTMEIFNLWLTTGKIPDMVRGCRTVLIPKSSKPD RLKDINNWRPITIGSILLRLFSRIVTARLSKACPLNPRQRGFIRAAGCSENLKLLQTIIWSAKREHRPLGVVF VDIAKAFDTVSHQHIIHVLQQREVDPHIVGLVSNMYENISTYITTKRNTHTDKIQIRVGVKQGDPMSPLLF NLAMDPLLCKLEESGKGYHRGQSSITAMAFADDLVLLSDSWENMNTNISILETFCNLTGLKTQGQKCHG FYIKPTKDSYTINDCAAWTINGTPLNMIDPGESEKYLGLQFDPWIGIARSGLSTKLDFWLQRIDQAPLKPL QKTDILKTYTIPRLIYIADHSEVKTALLETLDQKIRTAVKEWLHLPPCTCDAILYSSTRDGGLGITKLAGLIP SVQARRLHRIAQSSDDTMKCFMEKEKMEQLHKKLWIQARGDRENIPSIWEAPPSSEPPNNVSTNSEWEAP TQKDKFPKPCNWRKNEFKKWTKLASQGRGIVNFERDKISNHWIQYYRRIPHRKLLTALQLRANVYPTRE FLARGRQDQYIKACRHCDADIESCAHIIGNCPVTQDARIKRHNYICELLLEEAKKKDWVVFKEPHIRDSN KELYKPDLIFVKDARALVVDVTVRYEAAKSSLEEAAAEKVRKYKHLETEVRHLTNAKDVTFVGFPLGAR GKWHQDNFKLLTELGLSKSRQVKMAETFSTVALFSSVDIVHMFASRARKSMVM |
| 4 | Amino acid sequence of the G1098R+ A793V+Q 955R mutant (PM1) | MASCPKPGPPVSAGAMSLESGLTTHSVLAIERGPNSLANSGSDFGGGGLGLPLRLLRVSVGTQTSRSDWV DLVSWSHPGPTSKSQQVDLVSLFPKHRVDLLSKNDQVDLVAQFLPSKFPPNLAENDLALLVNLEFYRSDL HVYECVHFAAHWEGLSGLPEVYEQLAPQPCVGETLHSSLPRDSELFVPEEGSSEKESEDAPKTSPPTPGKH GLEQTGEEKVMVTVPDKNPPCPCCGTRVNSVLNLIEHLKVSHGKRGVCFRCAKCGKENSNYHSVVCHFP KCRGPETEKAPAGEWICEVCNRDFTTKIGLGQHKRLAHPAVRNQERIVASQPKETSNRGAHKRCWTKEE EELLIRLEAQFEGNKNINKLIAEHITTKTAKQISDKRRLLSRKPAEEPREEPGTCHHTRRAAASLRTEPEMS HHAQAEDRDNGPGRRPLPGRAAAGGRTMDEIRRHPDKGNGQQRPTKQKSEEQLQAYYKKTLEERLSAG ALNTFPRAFKQVMEGRDIKLVINQTAQDCFGCLESISQIRTATRDKKDTVTREKHPKKPFQKWMKDRAIK KGNYLRFQRLFYLDRGKLAKIILDDIECLSCDIPLSEIYSVFKTRWETTGSFKSLGDFKTYGKADNTAFREL ITAKEIEKNVQEMSKGSAPGPDGITLGDVVKMDPEFSRTMEIFNLWLTTGKIPDMVRGCRTVLIPKSSKPD RLKDINNWRPITIGSILLRLFSRIVTARLSKACPLNPRQRGFIRAAGCSENLKLLQTIIWSAKREHRPLGVVF VDIAKAFDTVSHQHIIHVLQQREVDPHIVGLVSNMYENISTYITTKRNTHTDKIQIRVGVKQGDPMSPLLF NLAMDPLLCKLEESGKGYHRGQSSITAMAFADDLVLLSDSWENMNTNISILETFCNLTGLKTQGQKCHG FYIKPTKDSYTINDCAAWTINGTPLNMIDPGESEKYLGLRFDPWIGIARSGLSTKLDFWLQRIDQAPLKPL QKTDILKTYTIPRLIYIADHSEVKTALLETLDQKIRTAVKEWLHLPPCTCDAILYSSTRDGGLGITKLAGLIP SVQARRLHRIAQSSDDTMKCFMEKEKMEQLHKKLWIQARGDRENIPSIWEAPPSSEPPNNVSTNSEWEAP TQKDKFPKPCNWRKNEFKKWTKLASQGRGIVNFERDKISNHWIQYYRRIPHRKLLTALQLRANVYPTRE FLARGRQDQYIKACRHCDADIESCAHIIGNCPVTQDARIKRHNYICELLLEEAKKKDWVVFKEPHIRDSN KELYKPDLIFVKDARALVVDVTVRYEAAKSSLEEAAAEKVRKYKHLETEVRHLTNAKDVTFVGFPLGAR GKWHQDNFKLLTELGLSKSRQVKMAETFSTVALFSSVDIVHMFASRARKSMVM |
| 5 | Amino acid sequence of the G1098R+ A793V+ W762Q+ Q955R mutant (PM2) | MASCPKPGPPVSAGAMSLESGLTTHSVLAIERGPNSLANSGSDFGGGGLGLPLRLLRVSVGTQTSRSDWV DLVSWSHPGPTSKSQQVDLVSLFPKHRVDLLSKNDQVDLVAQFLPSKFPPNLAENDLALLVNLEFYRSDL HVYECVHFAAHWEGLSGLPEVYEQLAPQPCVGETLHSSLPRDSELFVPEEGSSEKESEDAPKTSPPTPGKH GLEQTGEEKVMVTVPDKNPPCPCCGTRVNSVLNLIEHLKVSHGKRGVCFRCAKCGKENSNYHSVVCHFP KCRGPETEKAPAGEWICEVCNRDFTTKIGLGQHKRLAHPAVRNQERIVASQPKETSNRGAHKRCWTKEE EELLIRLEAQFEGNKNINKLIAEHITTKTAKQISDKRRLLSRKPAEEPREEPGTCHHTRRAAASLRTEPEMS HHAQAEDRDNGPGRRPLPGRAAAGGRTMDEIRRHPDKGNGQQRPTKQKSEEQLQAYYKKTLEERLSAG ALNTFPRAFKQVMEGRDIKLVINQTAQDCFGCLESISQIRTATRDKKDTVTREKHPKKPFQKWMKDRAIK KGNYLRFQRLFYLDRGKLAKIILDDIECLSCDIPLSEIYSVFKTRWETTGSFKSLGDFKTYGKADNTAFREL ITAKEIEKNVQEMSKGSAPGPDGITLGDVVKMDPEFSRTMEIFNLWLTTGKIPDMVRGCRTVLIPKSSKPD RLKDINNWRPITIGSILLRLFSRIVTARLSKACPLNPRQRGFIRAAGCSENLKLLQTIIQSAKREHRPLGVVF VDIAKAFDTVSHQHIIHVLQQREVDPHIVGLVSNMYENISTYITTKRNTHTDKIQIRVGVKQGDPMSPLLF NLAMDPLLCKLEESGKGYHRGQSSITAMAFADDLVLLSDSWENMNTNISILETFCNLTGLKTQGQKCHG FYIKPTKDSYTINDCAAWTINGTPLNMIDPGESEKYLGLRFDPWIGIARSGLSTKLDFWLQRIDQAPLKPL QKTDILKTYTIPRLIYIADHSEVKTALLETLDQKIRTAVKEWLHLPPCTCDAILYSSTRDGGLGITKLAGLIP SVQARRLHRIAQSSDDTMKCFMEKEKMEQLHKKLWIQARGDRENIPSIWEAPPSSEPPNNVSTNSEWEAP TQKDKFPKPCNWRKNEFKKWTKLASQGRGIVNFERDKISNHWIQYYRRIPHRKLLTALQLRANVYPTRE FLARGRQDQYIKACRHCDADIESCAHIIGNCPVTQDARIKRHNYICELLLEEAKKKDWVVFKEPHIRDSN KELYKPDLIFVKDARALVVDVTVRYEAAKSSLEEAAAEKVRKYKHLETEVRHLTNAKDVTFVGFPLGAR GKWHQDNFKLLTELGLSKSRQVKMAETFSTVALFSSVDIVHMFASRARKSMVM |
| 6 | Amino acid sequence of the G1098R+ A793V+ W762Q+ Q955R+H 341R mutant (PM3) | MASCPKPGPPVSAGAMSLESGLTTHSVLAIERGPNSLANSGSDFGGGGLGLPLRLLRVSVGTQTSRSDWV DLVSWSHPGPTSKSQQVDLVSLFPKHRVDLLSKNDQVDLVAQFLPSKFPPNLAENDLALLVNLEFYRSDL HVYECVHFAAHWEGLSGLPEVYEQLAPQPCVGETLHSSLPRDSELFVPEEGSSEKESEDAPKTSPPTPGKH GLEQTGEEKVMVTVPDKNPPCPCCGTRVNSVLNLIEHLKVSHGKRGVCFRCAKCGKENSNYHSVVCHFP KCRGPETEKAPAGEWICEVCNRDFTTKIGLGQHKRLAHPAVRNQERIVASQPKETSNRGARKRCWTKEE EELLIRLEAQFEGNKNINKLIAEHITTKTAKQISDKRRLLSRKPAEEPREEPGTCHHTRRAAASLRTEPEMS HHAQAEDRDNGPGRRPLPGRAAAGGRTMDEIRRHPDKGNGQQRPTKQKSEEQLQAYYKKTLEERLSAG ALNTFPRAFKQVMEGRDIKLVINQTAQDCFGCLESISQIRTATRDKKDTVTREKHPKKPFQKWMKDRAIK KGNYLRFQRLFYLDRGKLAKIILDDIECLSCDIPLSEIYSVFKTRWETTGSFKSLGDFKTYGKADNTAFREL ITAKEIEKNVQEMSKGSAPGPDGITLGDVVKMDPEFSRTMEIFNLWLTTGKIPDMVRGCRTVLIPKSSKPD RLKDINNWRPITIGSILLRLFSRIVTARLSKACPLNPRQRGFIRAAGCSENLKLLQTIIQSAKREHRPLGVVF VDIAKAFDTVSHQHIIHVLQQREVDPHIVGLVSNMYENISTYITTKRNTHTDKIQIRVGVKQGDPMSPLLF NLAMDPLLCKLEESGKGYHRGQSSITAMAFADDLVLLSDSWENMNTNISILETFCNLTGLKTQGQKCHG FYIKPTKDSYTINDCAAWTINGTPLNMIDPGESEKYLGLRFDPWIGIARSGLSTKLDFWLQRIDQAPLKPL QKTDILKTYTIPRLIYIADHSEVKTALLETLDQKIRTAVKEWLHLPPCTCDAILYSSTRDGGLGITKLAGLIP SVQARRLHRIAQSSDDTMKCFMEKEKMEQLHKKLWIQARGDRENIPSIWEAPPSSEPPNNVSTNSEWEAP TQKDKFPKPCNWRKNEFKKWTKLASQGRGIVNFERDKISNHWIQYYRRIPHRKLLTALQLRANVYPTRE FLARGRQDQYIKACRHCDADIESCAHIIGNCPVTQDARIKRHNYICELLLEEAKKKDWVVFKEPHIRDSN KELYKPDLIFVKDARALVVDVTVRYEAAKSSLEEAAAEKVRKYKHLETEVRHLTNAKDVTFVGFPLGAR GKWHQDNFKLLTELGLSKSRQVKMAETFSTVALFSSVDIVHMFASRARKSMVM |
| 7 | Amino acid sequence of the | MASCPKPGPPVSAGAMSLESGLTTHSVLAIERGPNSLANSGSDFGGGGLGLPLRLLRVSVGTQTSRSDWV DLVSWSHPGPTSKSQQVDLVSLFPKHRVDLLSKNDQVDLVAQFLPSKFPPNLAENDLALLVNLEFYRSDL HVYECVHFAAHWEGLSGLPEVYEQLAPQPCVGETLHSSLPRDSELFVPEEGSSEKESEDAPKTSPPTPGKH GLEQTGEEKVMVTVPDKNPPCPCCGTRVNSVLNLIEHLKVSHGKRGVCFRCAKCGKENSNYHSVVCHFP |
| | G1098R+ A793V+Q 955R+H3 41R mutant (PM4) | KCRGPETEKAPAGEWICEVCNRDFTTKIGLGQHKRLAHPAVRNQERIVASQPKETSNRGARKRCWTKEE EELLIRLEAQFEGNKNINKLIAEHITTKTAKQISDKRRLLSRKPAEEPREEPGTCHHTRRAAASLRTEPEMS HHAQAEDRDNGPGRRPLPGRAAAGGRTMDEIRRHPDKGNGQQRPTKQKSEEQLQAYYKKTLEERLSAG ALNTFPRAFKQVMEGRDIKLVINQTAQDCFGCLESISQIRTATRDKKDTVTREKHPKKPFQKWMKDRAIK KGNYLRFQRLFYLDRGKLAKIILDDIECLSCDIPLSEIYSVFKTRWETTGSFKSLGDFKTYGKADNTAFREL ITAKEIEKNVQEMSKGSAPGPDGITLGDVVKMDPEFSRTMEIFNLWLTTGKIPDMVRGCRTVLIPKSSKPD RLKDINNWRPITIGSILLRLFSRIVTARLSKACPLNPRQRGFIRAAGCSENLKLLQTIIWSAKREHRPLGVVF VDIAKAFDTVSHQHIIHVLQQREVDPHIVGLVSNMYENISTYITTKRNTHTDKIQIRVGVKQGDPMSPLLF NLAMDPLLCKLEESGKGYHRGQSSITAMAFADDLVLLSDSWENMNTNISILETFCNLTGLKTQGQKCHG FYIKPTKDSYTINDCAAWTINGTPLNMIDPGESEKYLGLRFDPWIGIARSGLSTKLDFWLQRIDQAPLKPL QKTDILKTYTIPRLIYIADHSEVKTALLETLDQKIRTAVKEWLHLPPCTCDAILYSSTRDGGLGITKLAGLIP SVQARRLHRIAQSSDDTMKCFMEKEKMEQLHKKLWIQARGDRENIPSIWEAPPSSEPPNNVSTNSEWEAP TQKDKFPKPCNWRKNEFKKWTKLASQGRGIVNFERDKISNHWIQYYRRIPHRKLLTALQLRANVYPTRE FLARGRQDQYIKACRHCDADIESCAHIIGNCPVTQDARIKRHNYICELLLEEAKKKDWVVFKEPHIRDSN KELYKPDLIFVKDARALVVDVTVRYEAAKSSLEEAAAEKVRKYKHLETEVRHLTNAKDVTFVGFPLGAR GKWHQDNFKLLTELGLSKSRQVKMAETFSTVALFSSVDIVHMFASRARKSMVM |
| 8 | Amino acid sequence of T5 exonuclea se | MSKSWGKFIEEEEAEMASRRNLMIVDGTNLGFRFKHNNSKKPFASSYVSTIQSLAKSYSARTTIVLGDKG KSVFRLEHLPEYKGNRDEKYAQRTEEEKALDEQFFEYLKDAFELCKTTFPTFTIRGVEADDMAAYIVKLI GHLYDHVWLISTDGDWDTLLTDKVSRFSFTTRREYHLRDMYEHHNVDDVEQFISLKAIMGDLGDNIRGV EGIGAKRGYNIIREFGNVLDIIDQLPLPGKQKYIQNLNASEELLFRNLILVDLPTYCVDAIAAVGQDVLDKF TKDILEIAEQ |
| 9 | Amino acid sequence of single-stra nded DNA-bind ing protein sto7d | MVTVKFKYKGEELEVDISHIKKVWRVGKMISFTYDDNGKTGRGAVSEKDAPKELLQMLEKSGKK |
| 10 | Amino acid sequence of high mobility peptide segment (CMP) | MPKRKVSSAEGAAKEEPKRRSARLSAKPPAKVEAKPKKAAAKDKSSDKKVQTKGKRGAKGKQAEVAN QETKEDLPAENGETKTEESPASDEAGEKEAKSD |
| 11 | Amino acid sequence of the RNaseH domain (RH) of MLV reverse transcripta se | DILAEAHGTRPDLTDQPLPDADHTWYTDGSSLLQEGQRKAGAAVTTETEVIWAKALPAGTSAQRAELIA LTQALKMAEGKKLNVYTDSRYAFATAHIHGEIYRRRGWLTSEGKEIKNKDEILALLKALFLPKRLSIIHCP GHQKGHSAEARGNRMADQAARKAAITETPDTSTLLIENSSPSGGSKRTADGSEFE |
| 12 | TD5-vl (SEQ ID No.12) | MPKRKVSSAEGAAKEEPKRRSARLSAKPPAKVEAKPKKAAAKDKSSDKKVQTKGKRGAKGKQAEVAN QETKEDLPAENGETKTEESPASDEAGEKEAKSDVMVTVPDKNPPCPCCGTRVNSVLNLIEHLKVSHGKR GVCFRCAKCGKENSNYHSVVCHFPKCRGPETEKAPAGEWICEVCNRDFTTKIGLGQHKRLAHPAVRNQE RIVASQPKETSNRGAHKRCWTKEEEELLIRLEAQFEGNKNINKLIAEHITTKTAKQISDKRRLLSRKPAEEP REEPGTCHHTRRAAASLRTEPEMSHHAQAEDRDNGGGMPKRKVSSAEGAAKEEPKRRSARLSAKPPAK VEAKPKKAAAKDKSSDKKVQTKGKRGAKGKQAEVANQETKEDLPAENGETKTEESPASDEAGEKEAKS DGGPGRRPLPGRAAAGGRTMDEIRRHPDKGNGQQRPTKQKSEEQLQAYYKKTLEERLSAGALNTFPRAF KQVMEGRDIKLVINQTAQDCFGCLESISQIRTATRDKKDTVTREKHPKKPFQKWMKDRAIKKGNYLRFQ RLFYLDRGKLAKIILDDIECLSCDIPLSEIYSVFKTRWETTGSFKSLGDFKTYGKADNTAFRELITAKEIEKN VQEMSKGSAPGPDGITLGDVVKMDPEFSRTMEIFNLWLTTGKIPDMVRGCRTVLIPKSSKPDRLKDINNW RPITIGSILLRLFSRIVTARLSKACPLNPRQRGFIRAAGCSENLKLLQTIIWSAKREHRPLGVVFVDIAKAFD TVSHQHIIHALQQREVDPHIVGLVSNMYENISTYITTKRNTHTDKIQIRVGVKQGDPMSPLLFNLAMDPLL CKLEESGKGYHRGQSSITAMAFADDLVLLSDSWENMNTNISILETFCNLTGLKTQGQKCHGFYIKPTKDS YTINDCAAWTINGTPLNMIDPGESEKYLGLQFDPWIGIARSGLSTKLDFWLQRIDQAPLKPLQKTDILKTY TIPRLIYIADHSEVKTALLETLDQKIRTAVKEWLHLPPCTCDAILYSSTRDGGLGITKLAGLIPSVQARRLH RIAQSSDDTMKCFMEKEKMEQLHKKLWIQAGGDRENIPSIWEAPPSSEPPNNVSTNSEWEAPTQKDKFPK PCNWRKNEFKKWTKLASQGRGIVNFERDKISNHWIQYYRRIPHRKLLTALQLRANVYPTREFLARGRQD QYIKACRHCDADIESCAHIIGNCPVTQDARIKRHNYICELLLEEAKKKDWVVFKEPHIRDSNKELYKPDLI FVKDARALVVDVTVRYEAAKSSLEEAAAEKVRKYKHLETEVRHLTNAKDVTFVGFPLGARGKWHQDN FKLLTELGLSKSRQVKMAETFSTVALFSSVDIVHMFASRARKSMVM |
| 13 | Δ390-500 (truncatio n of the wild-typ e) | MASCPKPGPPVSAGAMSLESGLTTHSVLAIERGPNSLANSGSDFGGGGLGLPLRLLRVSVGTQTSRSDWV DLVSWSHPGPTSKSQQVDLVSLFPKHRVDLLSKNDQVDLVAQFLPSKFPPNLAENDLALLVNLEFYRSDL HVYECVHFAAHWEGLSGLPEVYEQLAPQPCVGETLHSSLPRDSELFVPEEGSSEKESEDAPKTSPPTPGKH GLEQTGEEKVMVTVPDKNPPCPCCGTRVNSVLNLIEHLKVSHGKRGVCFRCAKCGKENSNYHSVVCHFP KCRGPETEKAPAGEWICEVCNRDFTTKIGLGQHKRLAHPAVRNQERIVASQPKETSNRGAHKRCWTKEE EELLIRLEAQFEGNKNINKLIAEHITTKTAKQISDKRRLLVMEGRDIKLVINQTAQDCFGCLESISQIRTATR DKKDTVTREKHPKKPFQKWMKDRAIKKGNYLRFQRLFYLDRGKLAKIILDDIECLSCDIPLSEIYSVFKTR WETTGSFKSLGDFKTYGKADNTAFRELITAKEIEKNVQEMSKGSAPGPDGITLGDVVKMDPEFSRTMEIF NLWLTTGKIPDMVRGCRTVLIPKSSKPDRLKDINNWRPITIGSILLRLFSRIVTARLSKACPLNPRQRGFIRA AGCSENLKLLQTIIWSAKREHRPLGVVFVDIAKAFDTVSHQHIIHALQQREVDPHIVGLVSNMYENISTYIT TKRNTHTDKIQIRVGVKQGDPMSPLLFNLAMDPLLCKLEESGKGYHRGQSSITAMAFADDLVLLSDSWE NMNTNISILETFCNLTGLKTQGQKCHGFYIKPTKDSYTINDCAAWTINGTPLNMIDPGESEKYLGLQFDPW IGIARSGLSTKLDFWLQRIDQAPLKPLQKTDILKTYTIPRLIYIADHSEVKTALLETLDQKIRTAVKEWLHL PPCTCDAILYSSTRDGGLGITKLAGLIPSVQARRLHRIAQSSDDTMKCFMEKEKMEQLHKKLWIQAGGDR ENIPSIWEAPPSSEPPNNVSTNSEWEAPTQKDKFPKPCNWRKNEFKKWTKLASQGRGIVNFERDKISNHWI QYYRRIPHRKLLTALQLRANVYPTREFLARGRQDQYIKACRHCDADIESCAHIIGNCPVTQDARIKRHNYI CELLLEEAKKKDWVVFKEPHIRDSNKELYKPDLIFVKDARALVVDVTVRYEAAKSSLEEAAAEKVRKYK HLETEVRHLTNAKDVTFVGFPLGARGKWHQDNFKLLTELGLSKSRQVKMAETFSTVALFSSVDIVHMFA SRARKSMVM |
| 14 | Flag tag sequence | DYKDHDGDYKDHDIDYKDDDDK |
| 15 | nucleotide sequence of the original left homology arm (LHA) (150bp) | gaatccgactgtttaattaaaacaaagcatcgcgaaggcccgcggcgggtgttgacgcgatgtgatttctgcccagtgctctgaatgtcaaagtgaagaaattcaatga agcgcgggtaaacggcgggagtaactatgactctcttaagg |
| 16 | wild-type 5' UTR sequence (5'UTR of UTR 9, 10, 11, 12, 13, 14) | Gtctagttacaactgggcatcgctgcagagatcgcacctcctcgtggtcccgctggtagcccttcgaagggtgactaagtcgatctctgccccaggtacggagccgtt gggactcaccagtccaacgtaactcctgcctaaattcggtgaaacaaattcctcggtaaaaagcccc |
| 17 | wild-type 3' UTR sequence (3'UTR of UTR 1, 2, 3, 4, 5, 6, 7, 8) | ttcaggttatttagatgcttagtttttgtacctttcttgttttgtttaggattttgatagtgttagtatttttatatttttgtacgattgcataatgttcttttttatacagttctgttttaataaa atagacgatagctagagacgttagggcagccacaagccagttaggtagcggatagtaggtaggaacagacttttactatttcataacgcgtcaattaccacctgatttgg accaattcacgggatttgtccaaggtggacgggccacctttacttaacccggaaaaggaacatatataatttatgtgtgttcgataaa |
| 18 | nucleotide sequence of the original right homology arm (RHA) (150bp) | tagccaaatgcctcgtcatctaattagtgacgcgcatgaatggatgaacgagattcccactgtccctacctactatccagcgaaaccacagccaagggaacgggcttg gcggaatcagcggggaaagaagaccctgttgagcttgactct |
| 19 20 | 5' homology arm (LHA) of 100bp 3' homology arm (RHA) of 100bp | gttgacgcgatgtgatttctgcccagtgctctgaatgtcaaagtgaagaaattcaatgaagcgcgggtaaacggcgggagtaactatgactctcttaagg tagccaaatgcctcgtcatctaattagtgacgcgcatgaatggatgaacgagattcccactgtccctacctactatccagcgaaaccacagccaagggaa |
| 21 | 5' homology arm (LHA) of 50bp | attcaatgaagcgcgggtaaacggcgggagtaactatgactctcttaagg |
| 22 | 3' homology arm (RHA) of 50bp | tagccaaatgcctcgtcatctaattagtgacgcgcatgaatggatgaacg |
| 23 | 5' homology arm (LHA) of 20bp | taactatgactctcttaagg |
| 24 | 3' homology arm (RHA) of 20bp | tagccaaatgcctcgtcatc |
| 25 | 5' homology arm (LHA) of 10bp | tctcttaagg |
| 26 | 3' homology arm (RHA) of 10bp | tagccaaatg |
| 27 | UTR1-5' UTR | Gtcaactgggcatcgctgcagagatcgcacctcctcgtggtcccgctggtagcccttcgaagggtgactaagtcgatctctgccccaggtacggagccgttgggact caccagtccaacgtaactcctgcctaaattcggtgaaacaaattcctcggtaaaaagcccc |
| 28 | A2 | ggcgggagtaactatgactctcttaagg |
| 29 | UTR2-5' UTR | aactgggcatcgctgcagagatcgcacctcctcgtggtcccgctggtagcccttcgaagggtgactaagtcgatctctgccccaggtacggagccgttgggactcac cagtccaacgtaactcctgcctaaattcggtgaaacaaattcctcggtaaaaagcccc |
| 30 | C | GAACAAACGACCCAACACCCGTGCGTTTTATTCTGTCTTTTTATTGCCGATCTTACTTGTACAGCTCG TCCATGCCGAGAGTGATCCCGGCGGCGGTCACGAACTCCAGCAGGACCATGTGATCGCGCTTCTCGT TGGGGTCTTTGCTCAGGGCGGACTGGGTGCTCAGGTAGTGGTTGTCGGGCAGCAGCACGGGGCCGTC GCCGATGGGGGTGTTCTGCTGGTAGTGGTCGGCCAGCTGCACGCTGCCGTCCTCGATGTTGTGGCGG ATCTTGAAGTTCACCTTGATGCCGTTCTTCTGCTTGTCGGCCATGATATAGACGTTGTGGCTGTTGTA GTTGTACTCCAGCTTGTGCCCCAGGATGTTGCCGTCCTCCTTGAAGTCGATGCCCTTCAGCTCGATGC GGTTCACCAGGGTGTCGCCCTCGAACTTCACCTCGGCGCGGGTCTTGTAGTTGCCGTCGTCCTTGAAG AAGATGGTGCGCTCCTGGACGTAGCCTTCGGGCATGGCGGACTTGAAGAAGTCGTGCTGCTTCATGT GGTCGGGGTAGCGGCTGAAGCACTGCACGCCGTAGGTCAGGGTGGTCACGAGGGTGGGCCAGGGCA CGGGCAGCTTGCCGGTGGTGCAGATGAACTTCAGGGTCAGCTTGCCGTAGGTGGCATCGCCCTCGCC CTCGCCGGACACGCTGAACTTGTGGCCGTTTACGTCGCCGTCCAGCTCGACCAGGATGGGCACCACC CCGGTGAACAGCTCCTCGCCCTTGCTCACCATGGTGGCGCTAGCGGATCTGACGGTTCACTAAACCA GCTCTGCTTATATAGACCTCCCACCGTACACGCCTACCGCCCATTTGCGTCAATGGGGCGGAGTTGTT ACGACATTTTGGAAAGTCCCGTTGATTTTGGTGCCAAAACAAACTCCCATTGACGTCAATGGGGTGG AGACTTGGAAATCCCCGTGAGTCAAACCGCTATCCACGCCCATTGATGTACTGCCAAAACCGCATCA CCATGGTAATAGCGATGACTAATACGTAGATGTACTGCCAAGTAGGAAAGTCCCATAAGGTCATGTA CTGGGCATAATGCCAGGCGGGCCATTTACCGTCATTGACGTCAATAGGGGGCGTACTTGGCATATGA TACACTTGATGTACTGCCAAGTGGGCAGTTTACCGTAAATACTCCACCCATTGACGTCAATGGAAAG TCCCTATTGGCGTTACTATGGGAACATACGTCATTATTGACGTCAATGGGCGGGGGTCGTTGGGCGG TCAGCCAGGCGGGCCATTTACCGTAAGTTATGTAACG |
| 31 | UTR3-5' UTR | Gtctagttacaactgggcatcgctcccaggtacggagccgttgggactcaccagtccaacgtaactcctgcctaaattcggtgaaacaaattcctcggtaaaaagccc c |
| 32 | D1 | cacgggatttgtccaaggtggacgggccacctttacttaacccggaaaaggaacatatataatttatgtgtgttcgataaa |
| 33 | UTR4-5' UTR | cccaggtacggagccgttgggactcaccagtccaacgtaactcctgcctaaattcggtgaaacaaattcctcggtaaaaagcccc |
| 34 | D2 | ggtggacgggccacctttacttaacccggaaaaggaacatatataatttatgtgtgttcgataaa |
| 35 | UTR5-5' UTR | GtctagttacaactgggcatcgctgcagagatcgcacctcctcgtggtcccgctggtagcccttcgaagggtgaTtaagtcgatctctgccccaggtacggagccgtt gggactcaccagtccaacgtaactcctgcctaaattcggtgaaacaaattcctcggtaaaaagcccc |
| 36 | E2 | tagc |
| 37 | UTR6-5' UTR | Gtctagttacaactgggcatcgctgcagagatcgcacctcctcgtggtcccgctggtagcccttcgaagggtgactaagtcgatctctgccccaaattcggtgaaacaa attcctcggtaaaaagcccc |
| 38 | Δ1100-11 20 (truncated form of the wild-type) | MASCPKPGPPVSAGAMSLESGLTTHSVLAIERGPNSLANSGSDFGGGGLGLPLRLLRVSVGTQTSRSDWV DLVSWSHPGPTSKSQQVDLVSLFPKHRVDLLSKNDQVDLVAQFLPSKFPPNLAENDLALLVNLEFYRSDL HVYECVHFAAHWEGLSGLPEVYEQLAPQPCVGETLHSSLPRDSELFVPEEGSSEKESEDAPKTSPPTPGKH GLEQTGEEKVMVTVPDKNPPCPCCGTRVNSVLNLIEHLKVSHGKRGVCFRCAKCGKENSNYHSVVCHFP KCRGPETEKAPAGEWICEVCNRDFTTKIGLGQHKRLAHPAVRNQERIVASQPKETSNRGAHKRCWTKEE EELLIRLEAQFEGNKNINKLIAEHITTKTAKQISDKRRLLSRKPAEEPREEPGTCHHTRRAAASLRTEPEMS HHAQAEDRDNGPGRRPLPGRAAAGGRTMDEIRRHPDKGNGQQRPTKQKSEEQLQAYYKKTLEERLSAG ALNTFPRAFKQVMEGRDIKLVINQTAQDCFGCLESISQIRTATRDKKDTVTREKHPKKPFQKWMKDRAIK KGNYLRFQRLFYLDRGKLAKIILDDIECLSCDIPLSEIYSVFKTRWETTGSFKSLGDFKTYGKADNTAFREL ITAKEIEKNVQEMSKGSAPGPDGITLGDVVKMDPEFSRTMEIFNLWLTTGKIPDMVRGCRTVLIPKSSKPD RLKDINNWRPITIGSILLRLFSRIVTARLSKACPLNPRQRGFIRAAGCSENLKLLQTIIWSAKREHRPLGVVF VDIAKAFDTVSHQHIIHALQQREVDPHIVGLVSNMYENISTYITTKRNTHTDKIQIRVGVKQGDPMSPLLF NLAMDPLLCKLEESGKGYHRGQSSITAMAFADDLVLLSDSWENMNTNISILETFCNLTGLKTQGQKCHG FYIKPTKDSYTINDCAAWTINGTPLNMIDPGESEKYLGLQFDPWIGIARSGLSTKLDFWLQRIDQAPLKPL QKTDILKTYTIPRLIYIADHSEVKTALLETLDQKIRTAVKEWLHLPPCTCDAILYSSTRDGGLGITKLAGLIP SVQARRLHRIAQSSDDTMKCFMEKEKMEQLHKKLWIQAGGSTNSEWEAPTQKDKFPKPCNWRKNEFKK WTKLASQGRGIVNFERDKISNHWIQYYRRIPHRKLLTALQLRANVYPTREFLARGRQDQYIKACRHCDA DIESCAHIIGNCPVTQDARIKRHNYICELLLEEAKKKDWVVFKEPHIRDSNKELYKPDLIFVKDARALVVD VTVRYEAAKSSLEEAAAEKVRKYKHLETEVRHLTNAKDVTFVGFPLGARGKWHQDNFKLLTELGLSKS RQVKMAETFSTVALFSSVDIVHMFASRARKSMVM |
| 39 | UTR7-5' UTR | Gtctagttacaactgggcatcgctgcagagatcgcacctcctcgtggtcccgctggtagcccttcgaagggtgactaagtcgatctctgccccaggtacggagccgtt gggactcaccagtccaacgtaactcctgcctaaa |
| 40 | Δ1-220+3 90-500 (truncate form of the wild-type) | VMVTVPDKNPPCPCCGTRVNSVLNLIEHLKVSHGKRGVCFRCAKCGKENSNYHSVVCHFPKCRGPETEK APAGEWICEVCNRDFTTKIGLGQHKRLAHPAVRNQERIVASQPKETSNRGAHKRCWTKEEEELLIRLEAQ FEGNKNINKLIAEHITTKTAKQISDKRRLLVMEGRDIKLVINQTAQDCFGCLESISQIRTATRDKKDTVTRE KHPKKPFQKWMKDRAIKKGNYLRFQRLFYLDRGKLAKIILDDIECLSCDIPLSEIYSVFKTRWETTGSFKS LGDFKTYGKADNTAFRELITAKEIEKNVQEMSKGSAPGPDGITLGDVVKMDPEFSRTMEIFNLWLTTGKI PDMVRGCRTVLIPKSSKPDRLKDINNWRPITIGSILLRLFSRIVTARLSKACPLNPRQRGFIRAAGCSENLKL LQTIIWSAKREHRPLGVVFVDIAKAFDTVSHQHIIHALQQREVDPHIVGLVSNMYENISTYITTKRNTHTD KIQIRVGVKQGDPMSPLLFNLAMDPLLCKLEESGKGYHRGQSSITAMAFADDLVLLSDSWENMNTNISIL ETFCNLTGLKTQGQKCHGFYIKPTKDSYTINDCAAWTINGTPLNMIDPGESEKYLGLQFDPWIGIARSGLS TKLDFWLQRIDQAPLKPLQKTDILKTYTIPRLIYIADHSEVKTALLETLDQKIRTAVKEWLHLPPCTCDAIL YSSTRDGGLGITKLAGLIPSVQARRLHRIAQSSDDTMKCFMEKEKMEQLHKKLWIQAGGDRENIPSIWEA PPSSEPPNNVSTNSEWEAPTQKDKFPKPCNWRKNEFKKWTKLASQGRGIVNFERDKISNHWIQYYRRIPH RKLLTALQLRANVYPTREFLARGRQDQYIKACRHCDADIESCAHIIGNCPVTQDARIKRHNYICELLLEEA KKKDWVVFKEPHIRDSNKELYKPDLIFVKDARALVVDVTVRYEAAKSSLEEAAAEKVRKYKHLETEVR HLTNAKDVTFVGFPLGARGKWHQDNFKLLTELGLSKSRQVKMAETFSTVALFSSVDIVHMFASRARKS MVM |
| 41 | UTR8-5' UTR | aaattcggtgaaacaaattcctcggtaaaaagcccc |
| 42 | en-TgR2-v1 | MPKRKVSSAEGAAKEEPKRRSARLSAKPPAKVEAKPKKAAAKDKSSDKKVQTKGKRGAKGKQAEVAN QETKEDLPAENGETKTEESPASDEAGEKEAKSDTSKSQQVDLVSLFPKHRVDLLSKNDQVDLVAQFLPSK FPPNLAENDLALLVNLEFYRSDLHVYECVHFAAHWEGLSGLPEVYEQLAPQPCVGETLHSSLPRDSELFV PEEGSSEKESEDAPKTSPPTPGKHGLEQTGEEKVMVTVPDKNPPCPCCGTRVNSVLNLIEHLKVSHGKRG VCFRCAKCGKENSNYHSVVCHFPKCRGPETEKAPAGEWICEVCNRDFTTKIGLGQHKRLAHPAVRNQER IVASQPKETSNRGAHKRCWTKEEEELLIRLEAQFEGNKNINKLIAEHITTKTAKQISDKRRLLSRKPAEEPR EEPGTCHHTRRAAASLRTEPEMSHHAQAEDRDNGGGMPKRKVSSAEGAAKEEPKRRSARLSAKPPAKV EAKPKKAAAKDKSSDKKVQTKGKRGAKGKQAEVANQETKEDLPAENGETKTEESPASDEAGEKEAKSD GGPGRRPLPGRAAAGGRTMDEIRRHPDKGNGQQRPTKQKSEEQLQAYYKKTLEERLSAGALNTFPRAFK QVMEGRDIKLVINQTAQDCFGCLESISQIRTATRDKKDTVTREKHPKKPFQKWMKDRAIKKGNYLRFQR LFYLDRGKLAKIILDDIECLSCDIPLSEIYSVFKTRWETTGSFKSLGDFKTYGKADNTAFRELITAKEIEKNV QEMSKGSAPGPDGITLGDVVKMDPEFSRTMEIFNLWLTTGKIPDMVRGCRTVLIPKSSKPDRLKDINNWR PITIGSILLRLFSRIVTARLSKACPLNPRQRGFIRAAGCSENLKLLQTIIWSAKREHRPLGVVFVDIAKAFDT VSHQHIIHVLQQREVDPHIVGLVSNMYENISTYITTKRNTHTDKIQIRVGVKQGDPMSPLLFNLAMDPLLC KLEESGKGYHRGQSSITAMAFADDLVLLSDSWENMNTNISILETFCNLTGLKTQGQKCHGFYIKPTKDSY TINDCAAWTINGTPLNMIDPGESEKYLGLRFDPWIGIARSGLSTKLDFWLQRIDQAPLKPLQKTDILKTYTI PRLIYIADHSEVKTALLETLDQKIRTAVKEWLHLPPCTCDAILYSSTRDGGLGITKLAGLIPSVQARRLHRI AQSSDDTMKCFMEKEKMEQLHKKLWIQARGDRENIPSIWEAPPSSEPPNNVSTNSEWEAPTQKDKFPKP CNWRKNEFKKWTKLASQGRGIVNFERDKISNHWIQYYRRIPHRKLLTALQLRANVYPTREFLARGRQDQ YIKACRHCDADIESCAHIIGNCPVTQDARIKRHNYICELLLEEAKKKDWVVFKEPHIRDSNKELYKPDLIF VKDARALVVDVTVRYEAAKSSLEEAAAEKVRKYKHLETEVRHLTNAKDVTFVGFPLGARGKWHQDNF KLLTELGLSKSRQVKMAETFSTVALFSSVDIVHMFASRARKSMVM |
| 43 | en-TgR2-v2 | MPKRKVSSAEGAAKEEPKRRSARLSAKPPAKVEAKPKKAAAKDKSSDKKVQTKGKRGAKGKQAEVAN QETKEDLPAENGETKTEESPASDEAGEKEAKSDGSSEKESEDAPKTSPPTPGKHGLEQTGEEKVMVTVPD KNPPCPCCGTRVNSVLNLIEHLKVSHGKRGVCFRCAKCGKENSNYHSVVCHFPKCRGPETEKAPAGEWI CEVCNRDFTTKIGLGQHKRLAHPAVRNQERIVASQPKETSNRGAHKRCWTKEEEELLIRLEAQFEGNKNI NKLIAEHITTKTAKQISDKRRLLSRKPAEEPREEPGTCHHTRRAAASLRTEPEMSHHAQAEDRDNGGGMP KRKVSSAEGAAKEEPKRRSARLSAKPPAKVEAKPKKAAAKDKSSDKKVQTKGKRGAKGKQAEVANQE TKEDLPAENGETKTEESPASDEAGEKEAKSDGGPGRRPLPGRAAAGGRTMDEIRRHPDKGNGQQRPTKQ KSEEQLQAYYKKTLEERLSAGALNTFPRAFKQVMEGRDIKLVINQTAQDCFGCLESISQIRTATRDKKDT VTREKHPKKPFQKWMKDRAIKKGNYLRFQRLFYLDRGKLAKIILDDIECLSCDIPLSEIYSVFKTRWETTG SFKSLGDFKTYGKADNTAFRELITAKEIEKNVQEMSKGSAPGPDGITLGDVVKMDPEFSRTMEIFNLWLT TGKIPDMVRGCRTVLIPKSSKPDRLKDINNWRPITIGSILLRLFSRIVTARLSKACPLNPRQRGFIRAAGCSE NLKLLQTIIWSAKREHRPLGVVFVDIAKAFDTVSHQHIIHVLQQREVDPHIVGLVSNMYENISTYITTKRN THTDKIQIRVGVKQGDPMSPLLFNLAMDPLLCKLEESGKGYHRGQSSITAMAFADDLVLLSDSWENMNT NISILETFCNLTGLKTQGQKCHGFYIKPTKDSYTINDCAAWTINGTPLNMIDPGESEKYLGLRFDPWIGIAR SGLSTKLDFWLQRIDQAPLKPLQKTDILKTYTIPRLIYIADHSEVKTALLETLDQKIRTAVKEWLHLPPCTC DAILYSSTRDGGLGITKLAGLIPSVQARRLHRIAQSSDDTMKCFMEKEKMEQLHKKLWIQARGDRENIPSI WEAPPSSEPPNNVSTNSEWEAPTQKDKFPKPCNWRKNEFKKWTKLASQGRGIVNFERDKISNHWIQYYR RIPHRKLLTALQLRANVYPTREFLARGRQDQYIKACRHCDADIESCAHIIGNCPVTQDARIKRHNYICELL LEEAKKKDWVVFKEPHIRDSNKELYKPDLIFVKDARALVVDVTVRYEAAKSSLEEAAAEKVRKYKHLET EVRHLTNAKDVTFVGFPLGARGKWHQDNFKLLTELGLSKSRQVKMAETFSTVALFSSVDIVHMFASRAR KSMVM |
| 44 | UTR9-3' UTR | tttggaccaattcacgggatttgtccaaggtggacgggccacctttacttaacccggaaaaggaacatatataatttatgtgtgttcgataaa |
| 45 | en-TgR2-v3 | MPKRKVSSAEGAAKEEPKRRSARLSAKPPAKVEAKPKKAAAKDKSSDKKVQTKGKRGAKGKQAEVAN QETKEDLPAENGETKTEESPASDEAGEKEAKSDVMVTVPDKNPPCPCCGTRVNSVLNLIEHLKVSHGKR GVCFRCAKCGKENSNYHSVVCHFPKCRGPETEKAPAGEWICEVCNRDFTTKIGLGQHKRLAHPAVRNQE RIVASQPKETSNRGAHKRCWTKEEEELLIRLEAQFEGNKNINKLIAEHITTKTAKQISDKRRLLSRKPAEEP REEPGTCHHTRRAAASLRTEPEMSHHAQAEDRDNGGGMPKRKVSSAEGAAKEEPKRRSARLSAKPPAK VEAKPKKAAAKDKSSDKKVQTKGKRGAKGKQAEVANQETKEDLPAENGETKTEESPASDEAGEKEAKS DGGPGRRPLPGRAAAGGRTMDEIRRHPDKGNGQQRPTKQKSEEQLQAYYKKTLEERLSAGALNTFPRAF KQVMEGRDIKLVINQTAQDCFGCLESISQIRTATRDKKDTVTREKHPKKPFQKWMKDRAIKKGNYLRFQ RLFYLDRGKLAKIILDDIECLSCDIPLSEIYSVFKTRWETTGSFKSLGDFKTYGKADNTAFRELITAKEIEKN VQEMSKGSAPGPDGITLGDVVKMDPEFSRTMEIFNLWLTTGKIPDMVRGCRTVLIPKSSKPDRLKDINNW RPITIGSILLRLFSRIVTARLSKACPLNPRQRGFIRAAGCSENLKLLQTIIWSAKREHRPLGVVFVDIAKAFD TVSHQHIIHVLQQREVDPHIVGLVSNMYENISTYITTKRNTHTDKIQIRVGVKQGDPMSPLLFNLAMDPLL CKLEESGKGYHRGQSSITAMAFADDLVLLSDSWENMNTNISILETFCNLTGLKTQGQKCHGFYIKPTKDS YTINDCAAWTINGTPLNMIDPGESEKYLGLRFDPWIGIARSGLSTKLDFWLQRIDQAPLKPLQKTDILKTY TIPRLIYIADHSEVKTALLETLDQKIRTAVKEWLHLPPCTCDAILYSSTRDGGLGITKLAGLIPSVQARRLH RIAQSSDDTMKCFMEKEKMEQLHKKLWIQARGDRENIPSIWEAPPSSEPPNNVSTNSEWEAPTQKDKFPK PCNWRKNEFKKWTKLASQGRGIVNFERDKISNHWIQYYRRIPHRKLLTALQLRANVYPTREFLARGRQD QYIKACRHCDADIESCAHIIGNCPVTQDARIKRHNYICELLLEEAKKKDWVVFKEPHIRDSNKELYKPDLI FVKDARALVVDVTVRYEAAKSSLEEAAAEKVRKYKHLETEVRHLTNAKDVTFVGFPLGARGKWHQDN FKLLTELGLSKSRQVKMAETFSTVALFSSVDIVHMFASRARKSMVM |
| 46 | UTR10-3' UTR(D1) | cacgggatttgtccaaggtggacgggccacctttacttaacccggaaaaggaacatatataatttatgtgtgttcgataaa |
| 47 | TgR2-v1 | TSKSQQVDLVSLFPKHRVDLLSKNDQVDLVAQFLPSKFPPNLAENDLALLVNLEFYRSDLHVYECVHFA AHWEGLSGLPEVYEQLAPQPCVGETLHSSLPRDSELFVPEEGSSEKESEDAPKTSPPTPGKHGLEQTGEEK VMVTVPDKNPPCPCCGTRVNSVLNLIEHLKVSHGKRGVCFRCAKCGKENSNYHSVVCHFPKCRGPETEK APAGEWICEVCNRDFTTKIGLGQHKRLAHPAVRNQERIVASQPKETSNRGAHKRCWTKEEEELLIRLEAQ FEGNKNINKLIAEHITTKTAKQISDKRRLLSRKPAEEPREEPGTCHHTRRAAASLRTEPEMSHHAQAEDRD NGPGRRPLPGRAAAGGRTMDEIRRHPDKGNGQQRPTKQKSEEQLQAYYKKTLEERLSAGALNTFPRAFK QVMEGRDIKLVINQTAQDCFGCLESISQIRTATRDKKDTVTREKHPKKPFQKWMKDRAIKKGNYLRFQR LFYLDRGKLAKIILDDIECLSCDIPLSEIYSVFKTRWETTGSFKSLGDFKTYGKADNTAFRELITAKEIEKNV QEMSKGSAPGPDGITLGDVVKMDPEFSRTMEIFNLWLTTGKIPDMVRGCRTVLIPKSSKPDRLKDINNWR PITIGSILLRLFSRIVTARLSKACPLNPRQRGFIRAAGCSENLKLLQTIIWSAKREHRPLGVVFVDIAKAFDT VSHQHIIHALQQREVDPHIVGLVSNMYENISTYITTKRNTHTDKIQIRVGVKQGDPMSPLLFNLAMDPLLC KLEESGKGYHRGQSSITAMAFADDLVLLSDSWENMNTNISILETFCNLTGLKTQGQKCHGFYIKPTKDSY TINDCAAWTINGTPLNMIDPGESEKYLGLQFDPWIGIARSGLSTKLDFWLQRIDQAPLKPLQKTDILKTYTI PRLIYIADHSEVKTALLETLDQKIRTAVKEWLHLPPCTCDAILYSSTRDGGLGITKLAGLIPSVQARRLHRI AQSSDDTMKCFMEKEKMEQLHKKLWIQAGGDRENIPSIWEAPPSSEPPNNVSTNSEWEAPTQKDKFPKP CNWRKNEFKKWTKLASQGRGIVNFERDKISNHWIQYYRRIPHRKLLTALQLRANVYPTREFLARGRQDQ YIKACRHCDADIESCAHIIGNCPVTQDARIKRHNYICELLLEEAKKKDWVVFKEPHIRDSNKELYKPDLIF VKDARALVVDVTVRYEAAKSSLEEAAAEKVRKYKHLETEVRHLTNAKDVTFVGFPLGARGKWHQDNF KLLTELGLSKSRQVKMAETFSTVALFSSVDIVHMFASRARKSMVM |
| 48 | UTR11-3' UTR | ttcaggttatttagatgcGAAAgcgtcaattaccacctgatttggaccaattcacgggatttgtccaaggtggacgggccacctttacttaacccggaaaaggaacat atataatttatgtgtgttcgataaa |
| 49 | TgR2-v2 | GSSEKESEDAPKTSPPTPGKHGLEQTGEEKVMVTVPDKNPPCPCCGTRVNSVLNLIEHLKVSHGKRGVCF RCAKCGKENSNYHSVVCHFPKCRGPETEKAPAGEWICEVCNRDFTTKIGLGQHKRLAHPAVRNQERIVA SQPKETSNRGAHKRCWTKEEEELLIRLEAQFEGNKNINKLIAEHITTKTAKQISDKRRLLSRKPAEEPREEP GTCHHTRRAAASLRTEPEMSHHAQAEDRDNGPGRRPLPGRAAAGGRTMDEIRRHPDKGNGQQRPTKQK SEEQLQAYYKKTLEERLSAGALNTFPRAFKQVMEGRDIKLVINQTAQDCFGCLESISQIRTATRDKKDTV TREKHPKKPFQKWMKDRAIKKGNYLRFQRLFYLDRGKLAKIILDDIECLSCDIPLSEIYSVFKTRWETTGS FKSLGDFKTYGKADNTAFRELITAKEIEKNVQEMSKGSAPGPDGITLGDVVKMDPEFSRTMEIFNLWLTT GKIPDMVRGCRTVLIPKSSKPDRLKDINNWRPITIGSILLRLFSRIVTARLSKACPLNPRQRGFIRAAGCSEN LKLLQTIIWSAKREHRPLGVVFVDIAKAFDTVSHQHIIHALQQREVDPHIVGLVSNMYENISTYITTKRNT HTDKIQIRVGVKQGDPMSPLLFNLAMDPLLCKLEESGKGYHRGQSSITAMAFADDLVLLSDSWENMNTN ISILETFCNLTGLKTQGQKCHGFYIKPTKDSYTINDCAAWTINGTPLNMIDPGESEKYLGLQFDPWIGIARS GLSTKLDFWLQRIDQAPLKPLQKTDILKTYTIPRLIYIADHSEVKTALLETLDQKIRTAVKEWLHLPPCTCD AILYSSTRDGGLGITKLAGLIPSVQARRLHRIAQSSDDTMKCFMEKEKMEQLHKKLWIQAGGDRENIPSI WEAPPSSEPPNNVSTNSEWEAPTQKDKFPKPCNWRKNEFKKWTKLASQGRGIVNFERDKISNHWIQYYR RIPHRKLLTALQLRANVYPTREFLARGRQDQYIKACRHCDADIESCAHIIGNCPVTQDARIKRHNYICELL LEEAKKKDWVVFKEPHIRDSNKELYKPDLIFVKDARALVVDVTVRYEAAKSSLEEAAAEKVRKYKHLET EVRHLTNAKDVTFVGFPLGARGKWHQDNFKLLTELGLSKSRQVKMAETFSTVALFSSVDIVHMFASRAR KSMVM |
| 50 | UTR12-3' UTR | ttcaggttatttagatgcttagtttttgtacctttcttgttttgtttaggattttgatagtgttagtatttttatatttttgtacgattgcataatgttcttttttatacagttctgttttaataaa atagacgatagctagagacgttagggcagccacaagccagttaggtagcggatagtaggtaggaacagacttttactatttcataacgcgtcaattaccacctgatttgg accaattcagaaaaggaacatatataatttatgtgtgttcgataaa |
| 51 | TgR2-v3 | VMVTVPDKNPPCPCCGTRVNSVLNLIEHLKVSHGKRGVCFRCAKCGKENSNYHSVVCHFPKCRGPETEK APAGEWICEVCNRDFTTKIGLGQHKRLAHPAVRNQERIVASQPKETSNRGAHKRCWTKEEEELLIRLEAQ FEGNKNINKLIAEHITTKTAKQISDKRRLLSRKPAEEPREEPGTCHHTRRAAASLRTEPEMSHHAQAEDRD NGPGRRPLPGRAAAGGRTMDEIRRHPDKGNGQQRPTKQKSEEQLQAYYKKTLEERLSAGALNTFPRAFK QVMEGRDIKLVINQTAQDCFGCLESISQIRTATRDKKDTVTREKHPKKPFQKWMKDRAIKKGNYLRFQR LFYLDRGKLAKIILDDIECLSCDIPLSEIYSVFKTRWETTGSFKSLGDFKTYGKADNTAFRELITAKEIEKNV QEMSKGSAPGPDGITLGDVVKMDPEFSRTMEIFNLWLTTGKIPDMVRGCRTVLIPKSSKPDRLKDINNWR PITIGSILLRLFSRIVTARLSKACPLNPRQRGFIRAAGCSENLKLLQTIIWSAKREHRPLGVVFVDIAKAFDT VSHQHIIHALQQREVDPHIVGLVSNMYENISTYITTKRNTHTDKIQIRVGVKQGDPMSPLLFNLAMDPLLC KLEESGKGYHRGQSSITAMAFADDLVLLSDSWENMNTNISILETFCNLTGLKTQGQKCHGFYIKPTKDSY TINDCAAWTINGTPLNMIDPGESEKYLGLQFDPWIGIARSGLSTKLDFWLQRIDQAPLKPLQKTDILKTYTI PRLIYIADHSEVKTALLETLDQKIRTAVKEWLHLPPCTCDAILYSSTRDGGLGITKLAGLIPSVQARRLHRI AQSSDDTMKCFMEKEKMEQLHKKLWIQAGGDRENIPSIWEAPPSSEPPNNVSTNSEWEAPTQKDKFPKP CNWRKNEFKKWTKLASQGRGIVNFERDKISNHWIQYYRRIPHRKLLTALQLRANVYPTREFLARGRQDQ YIKACRHCDADIESCAHIIGNCPVTQDARIKRHNYICELLLEEAKKKDWVVFKEPHIRDSNKELYKPDLIF VKDARALVVDVTVRYEAAKSSLEEAAAEKVRKYKHLETEVRHLTNAKDVTFVGFPLGARGKWHQDNF KLLTELGLSKSRQVKMAETFSTVALFSSVDIVHMFASRARKSMVM |
| 52 | UTR13-3' UTR | ttcaggttatttagatgcttagtttttgtacctttcttgttttgtttaggattttgatagtgttagtatttttatatttttgtacgattgcataatgttcttttttatacagttctgttttaataaa atagacgatagctagagacgttagggcagccacaagccagttaggtagcggatagtaggtaggaacagacttttactatttcataacgcgtcaattaccacctgatttgg accaattcacgggatttgtccaaggtggacgggccacctttacttaacccggaa |
| 53 | Factor VI | MQRVNMIMAESPGLITICLLGYLLSAECTVFLDHENANKILNRPKRYNSGKLEEFVQGNLERECMEEKCS FEEAREVFENTERTTEFWKQYVDGDQCESNPCLNGGSCKDDINSYECWCPFGFEGKNCELDVTCNIKNG RCEQFCKNSADNKVVCSCTEGYRLAENQKSCEPAVPFPCGRVSVSQTSKLTRAETVFPDVDYVNSTEAET ILDNITQSTQSFNDFTRVVGGEDAKPGQFPWQVVLNGKVDAFCGGSIVNEKWIVTAAHCVETGVKITVV AGEHNIEETEHTEQKRNVIRIIPHHNYNAAINKYNHDIALLELDEPLVLNSYVTPICIADKEYTNIFLKFGSG YVSGWGRVFHKGRSALVLQYLRVPLVDRATCLLSTKFTIYNNMFCAGFHEGGRDSCQGDSGGPHVTEV EGTSFLTGIISWGEECAMKGKYGIYTKVSRYVNWIKEKTKLT |
| 54 | UTR14-3' UTR | gaaaaggaacatatataatttatgtgtgttcgataaa |

### Sequences of the final combined mutations

| | |
|---|---|
| TD1 (SEQ ID No.55) | SKSWGKFIEEEEAEMASRRNLMIVDGTNLGFRFKHNNSKKPFASSYVSTIQSLAKSYSARTTIVLGDKGKSVFR LEHLPEYKGNRDEKYAQRTEEEKALDEQFFEYLKDAFELCKTTFPTFTIRGVEADDMAAYIVKLIGHLYDHV WLISTDGDWDTLLTDKVSRFSFTTRREYHLRDMYEHHNVDDVEQFISLKAIMGDLGDNIRGVEGIGAKRGYNI IREFGNVLDIIDQLPLPGKQKYIQNLNASEELLFRNLILVDLPTYCVDAIAAVGQDVLDKFTKDILEIAEQASCPK PGPPVSAGAMSLESGLTTHSVLAIERGPNSLANSGSDFGGGGLGLPLRLLRVSVGTQTSRSDWVDLVSWSHPG PTSKSQQVDLVSLFPKHRVDLLSKNDQVDLVAQFLPSKFPPNLAENDLALLVNLEFYRSDLHVYECVHFAAH WEGLSGLPEVYEQLAPQPCVGETLHSSLPRDSELFVPEEGSSEKESEDAPKTSPPTPGKHGLEQTGEEKVMVTV PDKNPPCPCCGTRVNSVLNLIEHLKVSHGKRGVCFRCAKCGKENSNYHSVVCHFPKCRGPETEKAPAGEWIC EVCNRDFTTKIGLGQHKRLAHPAVRNQERIVASQPKETSNRGAHKRCWTKEEEELLIRLEAQFEGNKNINKLI AEHITTKTAKQISDKRRLLSRKPAEEPREEPGTCHHTRRAAASLRTEPEMSHHAQAEDRDNGGGMPKRKVSSA EGAAKEEPKRRSARLSAKPPAKVEAKPKKAAAKDKSSDKKVQTKGKRGAKGKQAEVANQETKEDLPAENG ETKTEESPASDEAGEKEAKSDGGPGRRPLPGRAAAGGRTMDEIRRHPDKGNGQQRPTKQKSEEQLQAYYKKT LEERLSAGALNTFPRAFKQVMEGRDIKLVINQTAQDCFGCLESISQIRTATRDKKDTVTREKHPKKPFQKWMK DRAIKKGNYLRFQRLFYLDRGKLAKIILDDIECLSCDIPLSEIYSVFKTRWETTGSFKSLGDFKTYGKADNTAFR ELITAKEIEKNVQEMSKGSAPGPDGITLGDVVKMDPEFSRTMEIFNLWLTTGKIPDMVRGCRTVLIPKSSKPDR LKDINNWRPITIGSILLRLFSRIVTARLSKACPLNPRQRGFIRAAGCSENLKLLQTIIWSAKREHRPLGVVFVDIA KAFDTVSHQHIIHALQQREVDPHIVGLVSNMYENISTYITTKRNTHTDKIQIRVGVKQGDPMSPLLFNLAMDPL LCKLEESGKGYHRGQSSITAMAFADDLVLLSDSWENMNTNISILETFCNLTGLKTQGQKCHGFYIKPTKDSYTI NDCAAWTINGTPLNMIDPGESEKYLGLQFDPWIGIARSGLSTKLDFWLQRIDQAPLKPLQKTDILKTYTIPRLIY IADHSEVKTALLETLDQKIRTAVKEWLHLPPCTCDAILYSSTRDGGLGITKLAGLIPSVQARRLHRIAQSSDDT MKCFMEKEKMEQLHKKLWIQAGGDRENIPSIWEAPPSSEPPNNVSTNSEWEAPTQKDKFPKPCNWRKNEFKK WTKLASQGRGIVNFERDKISNHWIQYYRRIPHRKLLTALQLRANVYPTREFLARGRQDQYIKACRHCDADIES CAHIIGNCPVTQDARIKRHNYICELLLEEAKKKDWVVFKEPHIRDSNKELYKPDLIFVKDARALVVDVTVRYE AAKSSLEEAAAEKVRKYKHLETEVRHLTNAKDVTFVGFPLGARGKWHQDNFKLLTELGLSKSRQVKMAETF STVALFSSVDIVHMFASRARKSMVM |
| TD2 (SEQ ID No.56) | SKSWGKFIEEEEAEMASRRNLMIVDGTNLGFRFKHNNSKKPFASSYVSTIQSLAKSYSARTTIVLGDKGKSVFR LEHLPEYKGNRDEKYAQRTEEEKALDEQFFEYLKDAFELCKTTFPTFTIRGVEADDMAAYIVKLIGHLYDHV WLISTDGDWDTLLTDKVSRFSFTTRREYHLRDMYEHHNVDDVEQFISLKAIMGDLGDNIRGVEGIGAKRGYNI IREFGNVLDIIDQLPLPGKQKYIQNLNASEELLFRNLILVDLPTYCVDAIAAVGQDVLDKFTKDILEIAEQASCPK PGPPVSAGAMSLESGLTTHSVLAIERGPNSLANSGSDFGGGGLGLPLRLLRVSVGTQTSRSDWVDLVSWSHPG PTSKSQQVDLVSLFPKHRVDLLSKNDQVDLVAQFLPSKFPPNLAENDLALLVNLEFYRSDLHVYECVHFAAH WEGLSGLPEVYEQLAPQPCVGETLHSSLPRDSELFVPEEGSSEKESEDAPKTSPPTPGKHGLEQTGEEKVMVTV PDKNPPCPCCGTRVNSVLNLIEHLKVSHGKRGVCFRCAKCGKENSNYHSVVCHFPKCRGPETEKAPAGEWIC EVCNRDFTTKIGLGQHKRLAHPAVRNQERIVASQPKETSNRGAHKRCWTKEEEELLIRLEAQFEGNKNINKLI AEHITTKTAKQISDKRRLLSRKPAEEPREEPGTCHHTRRAAASLRTEPEMSHHAQAEDRDNGGGDILAEAHGT RPDLTDQPLPDADHTWYTDGSSLLQEGQRKAGAAVTTETEVIWAKALPAGTSAQRAELIALTQALKMAEGK KLNVYTDSRYAFATAHIHGEIYRRRGWLTSEGKEIKNKDEILALLKALFLPKRLSIIHCPGHQKGHSAEARGNR MADQAARKAAITETPDTSTLLIENSSPSGGSKRTADGSEFEGGPGRRPLPGRAAAGGRTMDEIRRHPDKGNGQ QRPTKQKSEEQLQAYYKKTLEERLSAGALNTFPRAFKQVMEGRDIKLVINQTAQDCFGCLESISQIRTATRDK KDTVTREKHPKKPFQKWMKDRAIKKGNYLRFQRLFYLDRGKLAKIILDDIECLSCDIPLSEIYSVFKTRWETTG SFKSLGDFKTYGKADNTAFRELITAKEIEKNVQEMSKGSAPGPDGITLGDVVKMDPEFSRTMEIFNLWLTTGKI PDMVRGCRTVLIPKSSKPDRLKDINNWRPITIGSILLRLFSRIVTARLSKACPLNPRQRGFIRAAGCSENLKLLQT IIWSAKREHRPLGVVFVDIAKAFDTVSHQHIIHALQQREVDPHIVGLVSNMYENISTYITTKRNTHTDKIQIRVG VKQGDPMSPLLFNLAMDPLLCKLEESGKGYHRGQSSITAMAFADDLVLLSDSWENMNTNISILETFCNLTGLK TQGQKCHGFYIKPTKDSYTINDCAAWTINGTPLNMIDPGESEKYLGLQFDPWIGIARSGLSTKLDFWLQRIDQA PLKPLQKTDILKTYTIPRLIYIADHSEVKTALLETLDQKIRTAVKEWLHLPPCTCDAILYSSTRDGGLGITKLAGL IPSVQARRLHRIAQSSDDTMKCFMEKEKMEQLHKKLWIQAGGDRENIPSIWEAPPSSEPPNNVSTNSEWEAPT QKDKFPKPCNWRKNEFKKWTKLASQGRGIVNFERDKISNHWIQYYRRIPHRKLLTALQLRANVYPTREFLAR GRQDQYIKACRHCDADIESCAHIIGNCPVTQDARIKRHNYICELLLEEAKKKDWVVFKEPHIRDSNKELYKPDL IFVKDARALVVDVTVRYEAAKSSLEEAAAEKVRKYKHLETEVRHLTNAKDVTFVGFPLGARGKWHQDNFKL LTELGLSKSRQVKMAETFSTVALFSSVDIVHMFASRARKSMVM |
| TD3 (SEQ ID No.57) | VTVKFKYKGEELEVDISKIKKVWRVGKMISFTYDDNGKTGRGAVSEKDAPKELLQMLEKSGKKASCPKPGPP VSAGAMSLESGLTTHSVLAIERGPNSLANSGSDFGGGGLGLPLRLLRVSVGTQTSRSDWVDLVSWSHPGPTSK SQQVDLVSLFPKHRVDLLSKNDQVDLVAQFLPSKFPPNLAENDLALLVNLEFYRSDLHVYECVHFAAHWEGL SGLPEVYEQLAPQPCVGETLHSSLPRDSELFVPEEGSSEKESEDAPKTSPPTPGKHGLEQTGEEKVMVTVPDKN PPCPCCGTRVNSVLNLIEHLKVSHGKRGVCFRCAKCGKENSNYHSVVCHFPKCRGPETEKAPAGEWICEVCN RDFTTKIGLGQHKRLAHPAVRNQERIVASQPKETSNRGAHKRCWTKEEEELLIRLEAQFEGNKNINKLIAEHIT TKTAKQISDKRRLLSRKPAEEPREEPGTCHHTRRAAASLRTEPEMSHHAQAEDRDNGGGMPKRKVSSAEGAA KEEPKRRSARLSAKPPAKVEAKPKKAAAKDKSSDKKVQTKGKRGAKGKQAEVANQETKEDLPAENGETKTE ESPASDEAGEKEAKSDGGPGRRPLPGRAAAGGRTMDEIRRHPDKGNGQQRPTKQKSEEQLQAYYKKTLEERL SAGALNTFPRAFKQVMEGRDIKLVINQTAQDCFGCLESISQIRTATRDKKDTVTREKHPKKPFQKWMKDRAIK KGNYLRFQRLFYLDRGKLAKIILDDIECLSCDIPLSEIYSVFKTRWETTGSFKSLGDFKTYGKADNTAFRELITA KEIEKNVQEMSKGSAPGPDGITLGDVVKMDPEFSRTMEIFNLWLTTGKIPDMVRGCRTVLIPKSSKPDRLKDIN NWRPITIGSILLRLFSRIVTARLSKACPLNPRQRGFIRAAGCSENLKLLQTIIWSAKREHRPLGVVFVDIAKAFDT VSHQHIIHALQQREVDPHIVGLVSNMYENISTYITTKRNTHTDKIQIRVGVKQGDPMSPLLFNLAMDPLLCKLE ESGKGYHRGQSSITAMAFADDLVLLSDSWENMNTNISILETFCNLTGLKTQGQKCHGFYIKPTKDSYTINDCA AWTINGTPLNMIDPGESEKYLGLQFDPWIGIARSGLSTKLDFWLQRIDQAPLKPLQKTDILKTYTIPRLIYIADHS EVKTALLETLDQKIRTAVKEWLHLPPCTCDAILYSSTRDGGLGITKLAGLIPSVQARRLHRIAQSSDDTMKCFM EKEKMEQLHKKLWIQAGGDRENIPSIWEAPPSSEPPNNVSTNSEWEAPTQKDKFPKPCNWRKNEFKKWTKLA SQGRGIVNFERDKISNHWIQYYRRIPHRKLLTALQLRANVYPTREFLARGRQDQYIKACRHCDADIESCAHIIG NCPVTQDARIKRHNYICELLLEEAKKKDWVVFKEPHIRDSNKELYKPDLIFVKDARALVVDVTVRYEAAKSSL EEAAAEKVRKYKHLETEVRHLTNAKDVTFVGFPLGARGKWHQDNFKLLTELGLSKSRQVKMAETFSTVALF SSVDIVHMFASRARKSMVM |
| TD4 (SEQ ID No.58) | VTVKFKYKGEELEVDISKIKKVWRVGKMISFTYDDNGKTGRGAVSEKDAPKELLQMLEKSGKKASCPKPGPP VSAGAMSLESGLTTHSVLAIERGPNSLANSGSDFGGGGLGLPLRLLRVSVGTQTSRSDWVDLVSWSHPGPTSK SQQVDLVSLFPKHRVDLLSKNDQVDLVAQFLPSKFPPNLAENDLALLVNLEFYRSDLHVYECVHFAAHWEGL SGLPEVYEQLAPQPCVGETLHSSLPRDSELFVPEEGSSEKESEDAPKTSPPTPGKHGLEQTGEEKVMVTVPDKN PPCPCCGTRVNSVLNLIEHLKVSHGKRGVCFRCAKCGKENSNYHSVVCHFPKCRGPETEKAPAGEWICEVCN RDFTTKIGLGQHKRLAHPAVRNQERIVASQPKETSNRGAHKRCWTKEEEELLIRLEAQFEGNKNINKLIAEHIT TKTAKQISDKRRLLSRKPAEEPREEPGTCHHTRRAAASLRTEPEMSHHAQAEDRDNGGGDILAEAHGTRPDLT DQPLPDADHTWYTDGSSLLQEGQRKAGAAVTTETEVIWAKALPAGTSAQRAELIALTQALKMAEGKKLNVY TDSRYAFATAHIHGEIYRRRGWLTSEGKEIKNKDEILALLKALFLPKRLSIIHCPGHQKGHSAEARGNRMADQA ARKAAITETPDTSTLLIENSSPSGGSKRTADGSEFEGGPGRRPLPGRAAGGRTMDEIRRHPDKGNGQQRPTKQ KSEEQLQAYYKKTLEERLSAGALNTFPRAFKQVMEGRDIKLVINQTAQDCFGCLESISQIRTATRDKKDTVTRE KHPKKPFQKWMKDRAIKKGNYLRFQRLFYLDRGKLAKIILDDIECLSCDIPLSEIYSVFKTRWETTGSFKSLGD FKTYGKADNTAFRELITAKEIEKNVQEMSKGSAPGPDGITLGDVVKMDPEFSRTMEIFNLWLTTGKIPDMVRG CRTVLIPKSSKPDRLKDINNWRPITIGSILLRLFSRIVTARLSKACPLNPRQRGFIRAAGCSENLKLLQTIIWSAKR EHRPLGVVFVDIAKAFDTVSHQHIIHALQQREVDPHIVGLVSNMYENISTYITTKRNTHTDKIQIRVGVKQGDP MSPLLFNLAMDPLLCKLEESGKGYHRGQSSITAMAFADDLVLLSDSWENMNTNISILETFCNLTGLKTQGQKC HGFYIKPTKDSYTINDCAAWTINGTPLNMIDPGESEKYLGLQFDPWIGIARSGLSTKLDFWLQRIDQAPLKPLQ KTDILKTYTIPRLIYIADHSEVKTALLETLDQKIRTAVKEWLHLPPCTCDAILYSSTRDGGLGITKLAGLIPSVQA RRLHRIAQSSDDTMKCFMEKEKMEQLHKKLWIQAGGDRENIPSIWEAPPSSEPPNNVSTNSEWEAPTQKDKFP KPCNWRKNEFKKWTKLASQGRGIVNFERDKISNHWIQYYRRIPHRKLLTALQLRANVYPTREFLARGRQDQY IKACRHCDADIESCAHIIGNCPVTQDARIKRHNYICELLLEEAKKKDWVVFKEPHIRDSNKELYKPDLIFVKDA RALVVDVTVRYEAAKSSLEEAAAEKVRKYKHLETEVRHLTNAKDVTFVGFPLGARGKWHQDNFKLLTELGL SKSRQVKMAETFSTVALFSSVDIVHMFASRARKSMVM |
| TD5 (SEQ ID No.59) | MPKRKVSSAEGAAKEEPKRRSARLSAKPPAKVEAKPKKAAAKDKSSDKKVQTKGKRGAKGKQAEVANQET KEDLPAENGETKTEESPASDEAGEKEAKSDASCPKPGPPVSAGAMSLESGLTTHSVLAIERGPNSLANSGSDFG GGGLGLPLRLLRVSVGTQTSRSDWVDLVSWSHPGPTSKSQQVDLVSLFPKHRVDLLSKNDQVDLVAQFLPSK FPPNLAENDLALLVNLEFYRSDLHVYECVHFAAHWEGLSGLPEVYEQLAPQPCVGETLHSSLPRDSELFVPEE GSSEKESEDAPKTSPPTPGKHGLEQTGEEKVMVTVPDKNPPCPCCGTRVNSVLNLIEHLKVSHGKRGVCFRCA KCGKENSNYHSVVCHFPKCRGPETEKAPAGEWICEVCNRDFTTKIGLGQHKRLAHPAVRNQERIVASQPKETS NRGAHKRCWTKEEEELLIRLEAQFEGNKNINKLIAEHITTKTAKQISDKRRLLSRKPAEEPREEPGTCHHTRRA AASLRTEPEMSHHAQAEDRDNGGGMPKRKVSSAEGAAKEEPKRRSARLSAKPPAKVEAKPKKAAAKDKSSD KKVQTKGKRGAKGKQAEVANQETKEDLPAENGETKTEESPASDEAGEKEAKSDGGPGRRPLPGRAAAGGRT MDEIRRHPDKGNGQQRPTKQKSEEQLQAYYKKTLEERLSAGALNTFPRAFKQVMEGRDIKLVINQTAQDCFG CLESISQIRTATRDKKDTVTREKHPKKPFQKWMKDRAIKKGNYLRFQRLFYLDRGKLAKIILDDIECLSCDIPLS EIYSVFKTRWETTGSFKSLGDFKTYGKADNTAFRELITAKEIEKNVQEMSKGSAPGPDGITLGDVVKMDPEFSR TMEIFNLWLTTGKIPDMVRGCRTVLIPKSSKPDRLKDINNWRPITIGSILLRLFSRIVTARLSKACPLNPRQRGFI RAAGCSENLKLLQTIIWSAKREHRPLGVVFVDIAKAFDTVSHQHIIHALQQREVDPHIVGLVSNMYENISTYITT KRNTHTDKIQIRVGVKQGDPMSPLLFNLAMDPLLCKLEESGKGYHRGQSSITAMAFADDLVLLSDSWENMNT NISILETFCNLTGLKTQGQKCHGFYIKPTKDSYTINDCAAWTINGTPLNMIDPGESEKYLGLQFDPWIGIARSGL STKLDFWLQRIDQAPLKPLQKTDILKTYTIPRLIYIADHSEVKTALLETLDQKIRTAVKEWLHLPPCTCDAILYS STRDGGLGITKLAGLIPSVQARRLHRIAQSSDDTMKCFMEKEKMEQLHKKLWIQAGGDRENIPSIWEAPPSSEP PNNVSTNSEWEAPTQKDKFPKPCNWRKNEFKKWTKLASQGRGIVNFERDKISNHWIQYYRRIPHRKLLTALQ LRANVYPTREFLARGRQDQYIKACRHCDADIESCAHIIGNCPVTQDARIKRHNYICELLLEEAKKKDWVVFKE PHIRDSNKELYKPDLIFVKDARALVVDVTVRYEAAKSSLEEAAAEKVRKYKHLETEVRHLTNAKDVTFVGFP LGARGKWHQDNFKLLTELGLSKSRQVKMAETFSTVALFSSVDIVHMFASRARKSMVM |
| TD6 (SEQ ID No.60) | MPKRKVSSAEGAAKEEPKRRSARLSAKPPAKVEAKPKKAAAKDKSSDKKVQTKGKRGAKGKQAEVANQET KEDLPAENGETKTEESPASDEAGEKEAKSDASCPKPGPPVSAGAMSLESGLTTHSVLAIERGPNSLANSGSDFG GGGLGLPLRLLRVSVGTQTSRSDWVDLVSWSHPGPTSKSQQVDLVSLFPKHRVDLLSKNDQVDLVAQFLPSK FPPNLAENDLALLVNLEFYRSDLHVYECVHFAAHWEGLSGLPEVYEQLAPQPCVGETLHSSLPRDSELFVPEE GSSEKESEDAPKTSPPTPGKHGLEQTGEEKVMVTVPDKNPPCPCCGTRVNSVLNLIEHLKVSHGKRGVCFRCA KCGKENSNYHSVVCHFPKCRGPETEKAPAGEWICEVCNRDFTTKIGLGQHKRLAHPAVRNQERIVASQPKETS NRGAHKRCWTKEEEELLIRLEAQFEGNKNINKLIAEHITTKTAKQISDKRRLLSRKPAEEPREEPGTCHHTRRA AASLRTEPEMSHHAQAEDRDNGGGMPKRKVSSAEGAAKEEPKRRSARLSAKPPAKVEAKPKKAAAKDKSSD KKVQTKGKRGAKGKQAEVANQETKEDLPAENGETKTEESPASDEAGEKEAKSDGGPGRRPLPGRAAAGGRT MDEIRRHPDKGNGQQRPTKQKSEEQLQAYYKKTLEERLSAGALNTFPRAFKQVMEGRDIKLVINQTAQDCFG CLESISQIRTATRDKKDTVTREKHPKKPFQKWMKDRAIKKGNYLRFQRLFYLDRGKLAKIILDDIECLSCDIPLS EIYSVFKTRWETTGSFKSLGDFKTYGKADNTAFRELITAKEIEKNVQEMSKGSAPGPDGITLGDVVKMDPEFSR TMEIFNLWLTTGKIPDMVRGCRTVLIPKSSKPDRLKDINNWRPITIGSILLRLFSRIVTARLSKACPLNPRQRGFI RAAGCSENLKLLQTIIWSAKREHRPLGVVFVDIAKAFDTVSHQHIIHALQQREVDPHIVGLVSNMYENISTYITT KRNTHTDKIQIRVGVKQGDPMSPLLFNLAMDPLLCKLEESGKGYHRGQSSITAMAFADDLVLLSDSWENMNT NISILETFCNLTGLKTQGQKCHGFYIKPTKDSYTINDCAAWTINGTPLNMIDPGESEKYLGLQFDPWIGIARSGL STKLDFWLQRIDQAPLKPLQKTDILKTYTIPRLIYIADHSEVKTALLETLDQKIRTAVKEWLHLPPCTCDAILYS STRDGGLGITKLAGLIPSVQARRLHRIAQSSDDTMKCFMEKEKMEQLHKKLWIQAGGDRENIPSIWEAPPGGM PKRKVSSAEGAAKEEPKRRSARLSAKPPAKVEAKPKKAAAKDKSSDKKVQTKGKRGAKGKQAEVANQETKE DLPAENGETKTEESPASDEAGEKEAKSDGGSSEPPNNVSTNSEWEAPTQKDKFPKPCNWRKNEFKKWTKLAS QGRGIVNFERDKISNHWIQYYRRIPHRKLLTALQLRANVYPTREFLARGRQDQYIKACRHCDADIESCAHIIGN CPVTQDARIKRHNYICELLLEEAKKKDWVVFKEPHIRDSNKELYKPDLIFVKDARALVVDVTVRYEAAKSSLE EAAAEKVRKYKHLETEVRHLTNAKDVTFVGFPLGARGKWHQDNFKLLTELGLSKSRQVKMAETFSTVALFS SVDIVHMFASRARKSMVM |
| TD1+PM1 (SEQ ID No.61) | SKSWGKFIEEEEAEMASRRNLMIVDGTNLGFRFKHNNSKKPFASSYVSTIQSLAKSYSARTTIVLGDKGKSVFR LEHLPEYKGNRDEKYAQRTEEEKALDEQFFEYLKDAFELCKTTFPTFTIRGVEADDMAAYIVKLIGHLYDHV WLISTDGDWDTLLTDKVSRFSFTTRREYHLRDMYEHHNVDDVEQFISLKAIMGDLGDNIRGVEGIGAKRGYNI IREFGNVLDIIDQLPLPGKQKYIQNLNASEELLFRNLILVDLPTYCVDAIAAVGQDVLDKFTKDILEIAEQASCPK PGPPVSAGAMSLESGLTTHSVLAIERGPNSLANSGSDFGGGGLGLPLRLLRVSVGTQTSRSDWVDLVSWSHPG PTSKSQQVDLVSLFPKHRVDLLSKNDQVDLVAQFLPSKFPPNLAENDLALLVNLEFYRSDLHVYECVHFAAH WEGLSGLPEVYEQLAPQPCVGETLHSSLPRDSELFVPEEGSSEKESEDAPKTSPPTPGKHGLEQTGEEKVMVTV PDKNPPCPCCGTRVNSVLNLIEHLKVSHGKRGVCFRCAKCGKENSNYHSVVCHFPKCRGPETEKAPAGEWIC EVCNRDFTTKIGLGQHKRLAHPAVRNQERIVASQPKETSNRGAHKRCWTKEEEELLIRLEAQFEGNKNINKLI AEHITTKTAKQISDKRRLLSRKPAEEPREEPGTCHHTRRAAASLRTEPEMSHHAQAEDRDNGGGMPKRKVSSA EGAAKEEPKRRSARLSAKPPAKVEAKPKKAAAKDKSSDKKVQTKGKRGAKGKQAEVANQETKEDLPAENG ETKTEESPASDEAGEKEAKSDGGPGRRPLPGRAAAGGRTMDEIRRHPDKGNGQQRPTKQKSEEQLQAYYKKT LEERLSAGALNTFPRAFKQVMEGRDIKLVINQTAQDCFGCLESISQIRTATRDKKDTVTREKHPKKPFQKWMK DRAIKKGNYLRFQRLFYLDRGKLAKIILDDIECLSCDIPLSEIYSVFKTRWETTGSFKSLGDFKTYGKADNTAFR ELITAKEIEKNVQEMSKGSAPGPDGITLGDVVKMDPEFSRTMEIFNLWLTTGKIPDMVRGCRTVLIPKSSKPDR LKDINNWRPITIGSILLRLFSRIVTARLSKACPLNPRQRGFIRAAGCSENLKLLQTIIWSAKREHRPLGVVFVDIA KAFDTVSHQHIIHVLQQREVDPHIVGLVSNMYENISTYITTKRNTHTDKIQIRVGVKQGDPMSPLLFNLAMDPL LCKLEESGKGYHRGQSSITAMAFADDLVLLSDSWENMNTNISILETFCNLTGLKTQGQKCHGFYIKPTKDSYTI NDCAAWTINGTPLNMIDPGESEKYLGLRFDPWIGIARSGLSTKLDFWLQRIDQAPLKPLQKTDILKTYTIPRLIY IADHSEVKTALLETLDQKIRTAVKEWLHLPPCTCDAILYSSTRDGGLGITKLAGLIPSVQARRLHRIAQSSDDT MKCFMEKEKMEQLHKKLWIQARGDRENIPSIWEAPPSSEPPNNVSTNSEWEAPTQKDKFPKPCNWRKNEFKK WTKLASQGRGIVNFERDKISNHWIQYYRRIPHRKLLTALQLRANVYPTREFLARGRQDQYIKACRHCDADIES CAHIIGNCPVTQDARIKRHNYICELLLEEAKKKDWVVFKEPHIRDSNKELYKPDLIFVKDARALVVDVTVRYE AAKSSLEEAAAEKVRKYKHLETEVRHLTNAKDVTFVGFPLGARGKWHQDNFKLLTELGLSKSRQVKMAETF STVALFSSVDIVHMFASRARKSMVM |
| TD1+PM2 (SEQ ID No.62) | PDKNPPCPCCGTRVNSVLNLIEHLKVSHGKRGVCFRCAKCGKENSNYHSVVCHFPKCRGPETEKAPAGEWIC EVCNRDFTTKIGLGQHKRLAHPAVRNQERIVASQPKETSNRGAHKRCWTKEEEELLIRLEAQFEGNKNINKLI AEHITTKTAKQISDKRRLLSRKPAEEPREEPGTCHHTRRAAASLRTEPEMSHHAQAEDRDNGGGMPKRKVSSA EGAAKEEPKRRSARLSAKPPAKVEAKPKKAAAKDKSSDKKVQTKGKRGAKGKQAEVANQETKEDLPAENG ETKTEESPASDEAGEKEAKSDGGPGRRPLPGRAAAGGRTMDEIRRHPDKGNGQQRPTKQKSEEQLQAYYKKT LEERLSAGALNTFPRAFKQVMEGRDIKLVINQTAQDCFGCLESISQIRTATRDKKDTVTREKHPKKPFQKWMK DRAIKKGNYLRFQRLFYLDRGKLAKIILDDIECLSCDIPLSEIYSVFKTRWETTGSFKSLGDFKTYGKADNTAFR ELITAKEIEKNVQEMSKGSAPGPDGITLGDVVKMDPEFSRTMEIFNLWLTTGKIPDMVRGCRTVLIPKSSKPDR LKDINNWRPITIGSILLRLFSRIVTARLSKACPLNPRQRGFIRAAGCSENLKLLQTIIWSAKREHRPLGVVFVDIA KAFDTVSHQHIIHVLQQREVDPHIVGLVSNMYENISTYITTKRNTHTDKIQIRVGVKQGDPMSPLLFNLAMDPL LCKLEESGKGYHRGQSSITAMAFADDLVLLSDSWENMNTNISILETFCNLTGLKTQGQKCHGFYIKPTKDSYTI NDCAAWTINGTPLNMIDPGESEKYLGLRFDPWIGIARSGLSTKLDFWLQRIDQAPLKPLQKTDILKTYTIPRLIY IADHSEVKTALLETLDQKIRTAVKEWLHLPPCTCDAILYSSTRDGGLGITKLAGLIPSVQARRLHRIAQSSDDT MKCFMEKEKMEQLHKKLWIQARGDRENIPSIWEAPPSSEPPNNVSTNSEWEAPTQKDKFPKPCNWRKNEFKK WTKLASQGRGIVNFERDKISNHWIQYYRRIPHRKLLTALQLRANVYPTREFLARGRQDQYIKACRHCDADIES CAHIIGNCPVTQDARIKRHNYICELLLEEAKKKDWVVFKEPHIRDSNKELYKPDLIFVKDARALVVDVTVRYE AAKSSLEEAAAEKVRKYKHLETEVRHLTNAKDVTFVGFPLGARGKWHQDNFKLLTELGLSKSRQVKMAETF STVALFSSVDIVHMFASRARKSMVM SKSWGKFIEEEEAEMASRRNLMIVDGTNLGFRFKHNNSKKPFASSYVSTIQSLAKSYSARTTIVLGDKGKSVFR LEHLPEYKGNRDEKYAQRTEEEKALDEQFFEYLKDAFELCKTTFPTFTIRGVEADDMAAYIVKLIGHLYDHV WLISTDGDWDTLLTDKVSRFSFTTRREYHLRDMYEHHNVDDVEQFISLKAIMGDLGDNIRGVEGIGAKRGYNI IREFGNVLDIIDQLPLPGKQKYIQNLNASEELLFRNLILVDLPTYCVDAIAAVGQDVLDKFTKDILEIAEQASCPK PGPPVSAGAMSLESGLTTHSVLAIERGPNSLANSGSDFGGGGLGLPLRLLRVSVGTQTSRSDWVDLVSWSHPG PTSKSQQVDLVSLFPKHRVDLLSKNDQVDLVAQFLPSKFPPNLAENDLALLVNLEFYRSDLHVYECVHFAAH WEGLSGLPEVYEQLAPQPCVGETLHSSLPRDSELFVPEEGSSEKESEDAPKTSPPTPGKHGLEQTGEEKVMVTV PDKNPPCPCCGTRVNSVLNLIEHLKVSHGKRGVCFRCAKCGKENSNYHSVVCHFPKCRGPETEKAPAGEWIC EVCNRDFTTKIGLGQHKRLAHPAVRNQERIVASQPKETSNRGAHKRCWTKEEEELLIRLEAQFEGNKNINKLI AEHITTKTAKQISDKRRLLSRKPAEEPREEPGTCHHTRRAAASLRTEPEMSHHAQAEDRDNGGGMPKRKVSSA EGAAKEEPKRRSARLSAKPPAKVEAKPKKAAAKDKSSDKKVQTKGKRGAKGKQAEVANQETKEDLPAENG ETKTEESPASDEAGEKEAKSDGGPGRRPLPGRAAAGGRTMDEIRRHPDKGNGQQRPTKQKSEEQLQAYYKKT LEERLSAGALNTFPRAFKQVMEGRDIKLVINQTAQDCFGCLESISQIRTATRDKKDTVTREKHPKKPFQKWMK DRAIKKGNYLRFQRLFYLDRGKLAKIILDDIECLSCDIPLSEIYSVFKTRWETTGSFKSLGDFKTYGKADNTAFR ELITAKEIEKNVQEMSKGSAPGPDGITLGDVVKMDPEFSRTMEIFNLWLTTGKIPDMVRGCRTVLIPKSSKPDR LKDINNWRPITIGSILLRLFSRIVTARLSKACPLNPRQRGFIRAAGCSENLKLLQTIIQSAKREHRPLGVVFVDIAK AFDTVSHQHIIHVLQQREVDPHIVGLVSNMYENISTYITTKRNTHTDKIQIRVGVKQGDPMSPLLFNLAMDPLL CKLEESGKGYHRGQSSITAMAFADDLVLLSDSWENMNTNISILETFCNLTGLKTQGQKCHGFYIKPTKDSYTIN DCAAWTINGTPLNMIDPGESEKYLGLRFDPWIGIARSGLSTKLDFWLQRIDQAPLKPLQKTDILKTYTIPRLIYI ADHSEVKTALLETLDQKIRTAVKEWLHLPPCTCDAILYSSTRDGGLGITKLAGLIPSVQARRLHRIAQSSDDTM KCFMEKEKMEQLHKKLWIQARGDRENIPSIWEAPPSSEPPNNVSTNSEWEAPTQKDKFPKPCNWRKNEFKKW TKLASQGRGIVNFERDKISNHWIQYYRRIPHRKLLTALQLRANVYPTREFLARGRQDQYIKACRHCDADIESC AHIIGNCPVTQDARIKRHNYICELLLEEAKKKDWVVFKEPHIRDSNKELYKPDLIFVKDARALVVDVTVRYEA AKSSLEEAAAEKVRKYKHLETEVRHLTNAKDVTFVGFPLGARGKWHQDNFKLLTELGLSKSRQVKMAETFS TVALFSSVDIVHMFASRARKSMVM |
| TD1+PM3 (SEQ | SKSWGKFIEEEEAEMASRRNLMIVDGTNLGFRFKHNNSKKPFASSYVSTIQSLAKSYSARTTIVLGDKGKSVFR |
| ID No.63) | LEHLPEYKGNRDEKYAQRTEEEKALDEQFFEYLKDAFELCKTTFPTFTIRGVEADDMAAYIVKLIGHLYDHV WLISTDGDWDTLLTDKVSRFSFTTRREYHLRDMYEHHNVDDVEQFISLKAIMGDLGDNIRGVEGIGAKRGYNI IREFGNVLDIIDQLPLPGKQKYIQNLNASEELLFRNLILVDLPTYCVDAIAAVGQDVLDKFTKDILEIAEQASCPK PGPPVSAGAMSLESGLTTHSVLAIERGPNSLANSGSDFGGGGLGLPLRLLRVSVGTQTSRSDWVDLVSWSHPG PTSKSQQVDLVSLFPKHRVDLLSKNDQVDLVAQFLPSKFPPNLAENDLALLVNLEFYRSDLHVYECVHFAAH WEGLSGLPEVYEQLAPQPCVGETLHSSLPRDSELFVPEEGSSEKESEDAPKTSPPTPGKHGLEQTGEEKVMVTV PDKNPPCPCCGTRVNSVLNLIEHLKVSHGKRGVCFRCAKCGKENSNYHSVVCHFPKCRGPETEKAPAGEWIC EVCNRDFTTKIGLGQHKRLAHPAVRNQERIVASQPKETSNRGARKRCWTKEEEELLIRLEAQFEGNKNINKLIA EHITTKTAKQISDKRRLLSRKPAEEPREEPGTCHHTRRAAASLRTEPEMSHHAQAEDRDNGGGMPKRKVSSAE GAAKEEPKRRSARLSAKPPAKVEAKPKKAAAKDKSSDKKVQTKGKRGAKGKQAEVANQETKEDLPAENGE TKTEESPASDEAGEKEAKSDGGPGRRPLPGRAAAGGRTMDEIRRHPDKGNGQQRPTKQKSEEQLQAYYKKTL EERLSAGALNTFPRAFKQVMEGRDIKLVINQTAQDCFGCLESISQIRTATRDKKDTVTREKHPKKPFQKWMKD RAIKKGNYLRFQRLFYLDRGKLAKIILDDIECLSCDIPLSEIYSVFKTRWETTGSFKSLGDFKTYGKADNTAFRE LITAKEIEKNVQEMSKGSAPGPDGITLGDVVKMDPEFSRTMEIFNLWLTTGKIPDMVRGCRTVLIPKSSKPDRL KDINNWRPITIGSILLRLFSRIVTARLSKACPLNPRQRGFIRAAGCSENLKLLQTIIQSAKREHRPLGVVFVDIAK AFDTVSHQHIIHVLQQREVDPHIVGLVSNMYENISTYITTKRNTHTDKIQIRVGVKQGDPMSPLLFNLAMDPLL CKLEESGKGYHRGQSSITAMAFADDLVLLSDSWENMNTNISILETFCNLTGLKTQGQKCHGFYIKPTKDSYTIN DCAAWTINGTPLNMIDPGESEKYLGLRFDPWIGIARSGLSTKLDFWLQRIDQAPLKPLQKTDILKTYTIPRLIYI ADHSEVKTALLETLDQKIRTAVKEWLHLPPCTCDAILYSSTRDGGLGITKLAGLIPSVQARRLHRIAQSSDDTM KCFMEKEKMEQLHKKLWIQARGDRENIPSIWEAPPSSEPPNNVSTNSEWEAPTQKDKFPKPCNWRKNEFKKW TKLASQGRGIVNFERDKISNHWIQYYRRIPHRKLLTALQLRANVYPTREFLARGRQDQYIKACRHCDADIESC AHIIGNCPVTQDARIKRHNYICELLLEEAKKKDWVVFKEPHIRDSNKELYKPDLIFVKDARALVVDVTVRYEA AKSSLEEAAAEKVRKYKHLETEVRHLTNAKDVTFVGFPLGARGKWHQDNFKLLTELGLSKSRQVKMAETFS TVALFSSVDIVHMFASRARKSMVM |
| TD1+PM4 (SEQ ID No.64) | SKSWGKFIEEEEAEMASRRNLMIVDGTNLGFRFKHNNSKKPFASSYVSTIQSLAKSYSARTTIVLGDKGKSVFR LEHLPEYKGNRDEKYAQRTEEEKALDEQFFEYLKDAFELCKTTFPTFTIRGVEADDMAAYIVKLIGHLYDHV WLISTDGDWDTLLTDKVSRFSFTTRREYHLRDMYEHHNVDDVEQFISLKAIMGDLGDNIRGVEGIGAKRGYNI IREFGNVLDIIDQLPLPGKQKYIQNLNASEELLFRNLILVDLPTYCVDAIAAVGQDVLDKFTKDILEIAEQASCPK PGPPVSAGAMSLESGLTTHSVLAIERGPNSLANSGSDFGGGGLGLPLRLLRVSVGTQTSRSDWVDLVSWSHPG PTSKSQQVDLVSLFPKHRVDLLSKNDQVDLVAQFLPSKFPPNLAENDLALLVNLEFYRSDLHVYECVHFAAH WEGLSGLPEVYEQLAPQPCVGETLHSSLPRDSELFVPEEGSSEKESEDAPKTSPPTPGKHGLEQTGEEKVMVTV PDKNPPCPCCGTRVNSVLNLIEHLKVSHGKRGVCFRCAKCGKENSNYHSVVCHFPKCRGPETEKAPAGEWIC EVCNRDFTTKIGLGQHKRLAHPAVRNQERIVASQPKETSNRGARKRCWTKEEEELLIRLEAQFEGNKNINKLIA EHITTKTAKQISDKRRLLSRKPAEEPREEPGTCHHTRRAAASLRTEPEMSHHAQAEDRDNGGGMPKRKVSSAE GAAKEEPKRRSARLSAKPPAKVEAKPKKAAAKDKSSDKKVQTKGKRGAKGKQAEVANQETKEDLPAENGE TKTEESPASDEAGEKEAKSDGGPGRRPLPGRAAAGGRTMDEIRRHPDKGNGQQRPTKQKSEEQLQAYYKKTL EERLSAGALNTFPRAFKQVMEGRDIKLVINQTAQDCFGCLESISQIRTATRDKKDTVTREKHPKKPFQKWMKD RAIKKGNYLRFQRLFYLDRGKLAKIILDDIECLSCDIPLSEIYSVFKTRWETTGSFKSLGDFKTYGKADNTAFRE LITAKEIEKNVQEMSKGSAPGPDGITLGDVVKMDPEFSRTMEIFNLWLTTGKIPDMVRGCRTVLIPKSSKPDRL KDINNWRPITIGSILLRLFSRIVTARLSKACPLNPRQRGFIRAAGCSENLKLLQTIIWSAKREHRPLGVVFVDIAK AFDTVSHQHIIHVLQQREVDPHIVGLVSNMYENISTYITTKRNTHTDKIQIRVGVKQGDPMSPLLFNLAMDPLL CKLEESGKGYHRGQSSITAMAFADDLVLLSDSWENMNTNISILETFCNLTGLKTQGQKCHGFYIKPTKDSYTIN DCAAWTINGTPLNMIDPGESEKYLGLRFDPWIGIARSGLSTKLDFWLQRIDQAPLKPLQKTDILKTYTIPRLIYI ADHSEVKTALLETLDQKIRTAVKEWLHLPPCTCDAILYSSTRDGGLGITKLAGLIPSVQARRLHRIAQSSDDTM KCFMEKEKMEQLHKKLWIQARGDRENIPSIWEAPPSSEPPNNVSTNSEWEAPTQKDKFPKPCNWRKNEFKKW TKLASQGRGIVNFERDKISNHWIQYYRRIPHRKLLTALQLRANVYPTREFLARGRQDQYIKACRHCDADIESC AHIIGNCPVTQDARIKRHNYICELLLEEAKKKDWVVFKEPHIRDSNKELYKPDLIFVKDARALVVDVTVRYEA AKSSLEEAAAEKVRKYKHLETEVRHLTNAKDVTFVGFPLGARGKWHQDNFKLLTELGLSKSRQVKMAETFS TVALFSSVDIVHMFASRARKSMVM |
| TD2+PM1 (SEQ ID No.65) | SKSWGKFIEEEEAEMASRRNLMIVDGTNLGFRFKHNNSKKPFASSYVSTIQSLAKSYSARTTIVLGDKGKSVFR LEHLPEYKGNRDEKYAQRTEEEKALDEQFFEYLKDAFELCKTTFPTFTIRGVEADDMAAYIVKLIGHLYDHV WLISTDGDWDTLLTDKVSRFSFTTRREYHLRDMYEHHNVDDVEQFISLKAIMGDLGDNIRGVEGIGAKRGYNI IREFGNVLDIIDQLPLPGKQKYIQNLNASEELLFRNLILVDLPTYCVDAIAAVGQDVLDKFTKDILEIAEQASCPK PGPPVSAGAMSLESGLTTHSVLAIERGPNSLANSGSDFGGGGLGLPLRLLRVSVGTQTSRSDWVDLVSWSHPG PTSKSQQVDLVSLFPKHRVDLLSKNDQVDLVAQFLPSKFPPNLAENDLALLVNLEFYRSDLHVYECVHFAAH WEGLSGLPEVYEQLAPQPCVGETLHSSLPRDSELFVPEEGSSEKESEDAPKTSPPTPGKHGLEQTGEEKVMVTV PDKNPPCPCCGTRVNSVLNLIEHLKVSHGKRGVCFRCAKCGKENSNYHSVVCHFPKCRGPETEKAPAGEWIC EVCNRDFTTKIGLGQHKRLAHPAVRNQERIVASQPKETSNRGAHKRCWTKEEEELLIRLEAQFEGNKNINKLI AEHITTKTAKQISDKRRLLSRKPAEEPREEPGTCHHTRRAAASLRTEPEMSHHAQAEDRDNGGGDILAEAHGT RPDLTDQPLPDADHTWYTDGSSLLQEGQRKAGAAVTTETEVIWAKALPAGTSAQRAELIALTQALKMAEGK KLNVYTDSRYAFATAHIHGEIYRRRGWLTSEGKEIKNKDEILALLKALFLPKRLSIIHCPGHQKGHSAEARGNR MADQAARKAAITETPDTSTLLIENSSPSGGSKRTADGSEFEGGPGRRPLPGRAAAGGRTMDEIRRHPDKGNGQ QRPTKQKSEEQLQAYYKKTLEERLSAGALNTFPRAFKQVMEGRDIKLVINQTAQDCFGCLESISQIRTATRDK KDTVTREKHPKKPFQKWMKDRAIKKGNYLRFQRLFYLDRGKLAKIILDDIECLSCDIPLSEIYSVFKTRWETTG SFKSLGDFKTYGKADNTAFRELITAKEIEKNVQEMSKGSAPGPDGITLGDVVKMDPEFSRTMEIFNLWLTTGKI PDMVRGCRTVLIPKSSKPDRLKDINNWRPITIGSILLRLFSRIVTARLSKACPLNPRQRGFIRAAGCSENLKLLQT IIWSAKREHRPLGVVFVDIAKAFDTVSHQHIIHVLQQREVDPHIVGLVSNMYENISTYITTKRNTHTDKIQIRVG VKQGDPMSPLLFNLAMDPLLCKLEESGKGYHRGQSSITAMAFADDLVLLSDSWENMNTNISILETFCNLTGLK TQGQKCHGFYIKPTKDSYTINDCAAWTINGTPLNMIDPGESEKYLGLRFDPWIGIARSGLSTKLDFWLQRIDQA PLKPLQKTDILKTYTIPRLIYIADHSEVKTALLETLDQKIRTAVKEWLHLPPCTCDAILYSSTRDGGLGITKLAGL IPSVQARRLHRIAQSSDDTMKCFMEKEKMEQLHKKLWIQARGDRENIPSIWEAPPSSEPPNNVSTNSEWEAPT QKDKFPKPCNWRKNEFKKWTKLASQGRGIVNFERDKISNHWIQYYRRIPHRKLLTALQLRANVYPTREFLAR GRQDQYIKACRHCDADIESCAHIIGNCPVTQDARIKRHNYICELLLEEAKKKDWVVFKEPHIRDSNKELYKPDL IFVKDARALVVDVTVRYEAAKSSLEEAAAEKVRKYKHLETEVRHLTNAKDVTFVGFPLGARGKWHQDNFKL LTELGLSKSRQVKMAETFSTVALFSSVDIVHMFASRARKSMVM |
| TD2+PM2 (SEQ ID No.66) | SKSWGKFIEEEEAEMASRRNLMIVDGTNLGFRFKHNNSKKPFASSYVSTIQSLAKSYSARTTIVLGDKGKSVFR LEHLPEYKGNRDEKYAQRTEEEKALDEQFFEYLKDAFELCKTTFPTFTIRGVEADDMAAYIVKLIGHLYDHV WLISTDGDWDTLLTDKVSRFSFTTRREYHLRDMYEHHNVDDVEQFISLKAIMGDLGDNIRGVEGIGAKRGYNI IREFGNVLDIIDQLPLPGKQKYIQNLNASEELLFRNLILVDLPTYCVDAIAAVGQDVLDKFTKDILEIAEQASCPK PGPPVSAGAMSLESGLTTHSVLAIERGPNSLANSGSDFGGGGLGLPLRLLRVSVGTQTSRSDWVDLVSWSHPG PTSKSQQVDLVSLFPKHRVDLLSKNDQVDLVAQFLPSKFPPNLAENDLALLVNLEFYRSDLHVYECVHFAAH WEGLSGLPEVYEQLAPQPCVGETLHSSLPRDSELFVPEEGSSEKESEDAPKTSPPTPGKHGLEQTGEEKVMVTV PDKNPPCPCCGTRVNSVLNLIEHLKVSHGKRGVCFRCAKCGKENSNYHSVVCHFPKCRGPETEKAPAGEWIC EVCNRDFTTKIGLGQHKRLAHPAVRNQERIVASQPKETSNRGAHKRCWTKEEEELLIRLEAQFEGNKNINKLI AEHITTKTAKQISDKRRLLSRKPAEEPREEPGTCHHTRRAAASLRTEPEMSHHAQAEDRDNGGGDILAEAHGT RPDLTDQPLPDADHTWYTDGSSLLQEGQRKAGAAVTTETEVIWAKALPAGTSAQRAELIALTQALKMAEGK KLNVYTDSRYAFATAHIHGEIYRRRGWLTSEGKEIKNKDEILALLKALFLPKRLSIIHCPGHQKGHSAEARGNR MADQAARKAAITETPDTSTLLIENSSPSGGSKRTADGSEFEGGPGRRPLPGRAAAGGRTMDEIRRHPDKGNGQ QRPTKQKSEEQLQAYYKKTLEERLSAGALNTFPRAFKQVMEGRDIKLVINQTAQDCFGCLESISQIRTATRDK KDTVTREKHPKKPFQKWMKDRAIKKGNYLRFQRLFYLDRGKLAKIILDDIECLSCDIPLSEIYSVFKTRWETTG SFKSLGDFKTYGKADNTAFRELITAKEIEKNVQEMSKGSAPGPDGITLGDVVKMDPEFSRTMEIFNLWLTTGKI PDMVRGCRTVLIPKSSKPDRLKDINNWRPITIGSILLRLFSRIVTARLSKACPLNPRQRGFIRAAGCSENLKLLQT IIQSAKREHRPLGVVFVDIAKAFDTVSHQHIIHVLQQREVDPHIVGLVSNMYENISTYITTKRNTHTDKIQIRVG VKQGDPMSPLLFNLAMDPLLCKLEESGKGYHRGQSSITAMAFADDLVLLSDSWENMNTNISILETFCNLTGLK TQGQKCHGFYIKPTKDSYTINDCAAWTINGTPLNMIDPGESEKYLGLRFDPWIGIARSGLSTKLDFWLQRIDQA PLKPLQKTDILKTYTIPRLIYIADHSEVKTALLETLDQKIRTAVKEWLHLPPCTCDAILYSSTRDGGLGITKLAGL IPSVQARRLHRIAQSSDDTMKCFMEKEKMEQLHKKLWIQARGDRENIPSIWEAPPSSEPPNNVSTNSEWEAPT QKDKFPKPCNWRKNEFKKWTKLASQGRGIVNFERDKISNHWIQYYRRIPHRKLLTALQLRANVYPTREFLAR GRQDQYIKACRHCDADIESCAHIIGNCPVTQDARIKRHNYICELLLEEAKKKDWVVFKEPHIRDSNKELYKPDL IFVKDARALVVDVTVRYEAAKSSLEEAAAEKVRKYKHLETEVRHLTNAKDVTFVGFPLGARGKWHQDNFKL LTELGLSKSRQVKMAETFSTVALFSSVDIVHMFASRARKSMVM |
| TD2+PM3 (SEQ ID No.67) | SKSWGKFIEEEEAEMASRRNLMIVDGTNLGFRFKHNNSKKPFASSYVSTIQSLAKSYSARTTIVLGDKGKSVFR LEHLPEYKGNRDEKYAQRTEEEKALDEQFFEYLKDAFELCKTTFPTFTIRGVEADDMAAYIVKLIGHLYDHV WLISTDGDWDTLLTDKVSRFSFTTRREYHLRDMYEHHNVDDVEQFISLKAIMGDLGDNIRGVEGIGAKRGYNI IREFGNVLDIIDQLPLPGKQKYIQNLNASEELLFRNLILVDLPTYCVDAIAAVGQDVLDKFTKDILEIAEQASCPK PGPPVSAGAMSLESGLTTHSVLAIERGPNSLANSGSDFGGGGLGLPLRLLRVSVGTQTSRSDWVDLVSWSHPG PTSKSQQVDLVSLFPKHRVDLLSKNDQVDLVAQFLPSKFPPNLAENDLALLVNLEFYRSDLHVYECVHFAAH WEGLSGLPEVYEQLAPQPCVGETLHSSLPRDSELFVPEEGSSEKESEDAPKTSPPTPGKHGLEQTGEEKVMVTV PDKNPPCPCCGTRVNSVLNLIEHLKVSHGKRGVCFRCAKCGKENSNYHSVVCHFPKCRGPETEKAPAGEWIC EVCNRDFTTKIGLGQHKRLAHPAVRNQERIVASQPKETSNRGARKRCWTKEEEELLIRLEAQFEGNKNINKLIA EHITTKTAKQISDKRRLLSRKPAEEPREEPGTCHHTRRAAASLRTEPEMSHHAQAEDRDNGGGDILAEAHGTR PDLTDQPLPDADHTWYTDGSSLLQEGQRKAGAAVTTETEVIWAKALPAGTSAQRAELIALTQALKMAEGKK LNVYTDSRYAFATAHIHGEIYRRRGWLTSEGKEIKNKDEILALLKALFLPKRLSIIHCPGHQKGHSAEARGNRM ADQAARKAAITETPDTSTLLIENSSPSGGSKRTADGSEFEGGPGRRPLPGRAAAGGRTMDEIRRHPDKGNGQQ RPTKQKSEEQLQAYYKKTLEERLSAGALNTFPRAFKQVMEGRDIKLVINQTAQDCFGCLESISQIRTATRDKK DTVTREKHPKKPFQKWMKDRAIKKGNYLRFQRLFYLDRGKLAKIILDDIECLSCDIPLSEIYSVFKTRWETTGS FKSLGDFKTYGKADNTAFRELITAKEIEKNVQEMSKGSAPGPDGITLGDVVKMDPEFSRTMEIFNLWLTTGKIP DMVRGCRTVLIPKSSKPDRLKDINNWRPITIGSILLRLFSRIVTARLSKACPLNPRQRGFIRAAGCSENLKLLQTII QSAKREHRPLGVVFVDIAKAFDTVSHQHIIHVLQQREVDPHIVGLVSNMYENISTYITTKRNTHTDKIQIRVGV KQGDPMSPLLFNLAMDPLLCKLEESGKGYHRGQSSITAMAFADDLVLLSDSWENMNTNISILETFCNLTGLKT QGQKCHGFYIKPTKDSYTINDCAAWTINGTPLNMIDPGESEKYLGLRFDPWIGIARSGLSTKLDFWLQRIDQAP LKPLQKTDILKTYTIPRLIYIADHSEVKTALLETLDQKIRTAVKEWLHLPPCTCDAILYSSTRDGGLGITKLAGLI PSVQARRLHRIAQSSDDTMKCFMEKEKMEQLHKKLWIQARGDRENIPSIWEAPPSSEPPNNVSTNSEWEAPTQ KDKFPKPCNWRKNEFKKWTKLASQGRGIVNFERDKISNHWIQYYRRIPHRKLLTALQLRANVYPTREFLARG RQDQYIKACRHCDADIESCAHIIGNCPVTQDARIKRHNYICELLLEEAKKKDWVVFKEPHIRDSNKELYKPDLI FVKDARALVVDVTVRYEAAKSSLEEAAAEKVRKYKHLETEVRHLTNAKDVTFVGFPLGARGKWHQDNFKL LTELGLSKSRQVKMAETFSTVALFSSVDIVHMFASRARKSMVM |
| TD2+PM4 (SEQ ID No.68) | SKSWGKFIEEEEAEMASRRNLMIVDGTNLGFRFKHNNSKKPFASSYVSTIQSLAKSYSARTTIVLGDKGKSVFR LEHLPEYKGNRDEKYAQRTEEEKALDEQFFEYLKDAFELCKTTFPTFTIRGVEADDMAAYIVKLIGHLYDHV WLISTDGDWDTLLTDKVSRFSFTTRREYHLRDMYEHHNVDDVEQFISLKAIMGDLGDNIRGVEGIGAKRGYNI IREFGNVLDIIDQLPLPGKQKYIQNLNASEELLFRNLILVDLPTYCVDAIAAVGQDVLDKFTKDILEIAEQASCPK PGPPVSAGAMSLESGLTTHSVLAIERGPNSLANSGSDFGGGGLGLPLRLLRVSVGTQTSRSDWVDLVSWSHPG PTSKSQQVDLVSLFPKHRVDLLSKNDQVDLVAQFLPSKFPPNLAENDLALLVNLEFYRSDLHVYECVHFAAH WEGLSGLPEVYEQLAPQPCVGETLHSSLPRDSELFVPEEGSSEKESEDAPKTSPPTPGKHGLEQTGEEKVMVTV PDKNPPCPCCGTRVNSVLNLIEHLKVSHGKRGVCFRCAKCGKENSNYHSVVCHFPKCRGPETEKAPAGEWIC EVCNRDFTTKIGLGQHKRLAHPAVRNQERIVASQPKETSNRGARKRCWTKEEEELLIRLEAQFEGNKNINKLIA EHITTKTAKQISDKRRLLSRKPAEEPREEPGTCHHTRRAAASLRTEPEMSHHAQAEDRDNGGGDILAEAHGTR PDLTDQPLPDADHTWYTDGSSLLQEGQRKAGAAVTTETEVIWAKALPAGTSAQRAELIALTQALKMAEGKK LNVYTDSRYAFATAHIHGEIYRRRGWLTSEGKEIKNKDEILALLKALFLPKRLSIIHCPGHQKGHSAEARGNRM ADQAARKAAITETPDTSTLLIENSSPSGGSKRTADGSEFEGGPGRRPLPGRAAAGGRTMDEIRRHPDKGNGQQ RPTKQKSEEQLQAYYKKTLEERLSAGALNTFPRAFKQVMEGRDIKLVINQTAQDCFGCLESISQIRTATRDKK DTVTREKHPKKPFQKWMKDRAIKKGNYLRFQRLFYLDRGKLAKIILDDIECLSCDIPLSEIYSVFKTRWETTGS FKSLGDFKTYGKADNTAFRELITAKEIEKNVQEMSKGSAPGPDGITLGDVVKMDPEFSRTMEIFNLWLTTGKIP DMVRGCRTVLIPKSSKPDRLKDINNWRPITIGSILLRLFSRIVTARLSKACPLNPRQRGFIRAAGCSENLKLLQTII WSAKREHRPLGVVFVDIAKAFDTVSHQHIIHVLQQREVDPHIVGLVSNMYENISTYITTKRNTHTDKIQIRVGV KQGDPMSPLLFNLAMDPLLCKLEESGKGYHRGQSSITAMAFADDLVLLSDSWENMNTNISILETFCNLTGLKT QGQKCHGFYIKPTKDSYTINDCAAWTINGTPLNMIDPGESEKYLGLRFDPWIGIARSGLSTKLDFWLQRIDQAP LKPLQKTDILKTYTIPRLIYIADHSEVKTALLETLDQKIRTAVKEWLHLPPCTCDAILYSSTRDGGLGITKLAGLI PSVQARRLHRIAQSSDDTMKCFMEKEKMEQLHKKLWIQARGDRENIPSIWEAPPSSEPPNNVSTNSEWEAPTQ KDKFPKPCNWRKNEFKKWTKLASQGRGIVNFERDKISNHWIQYYRRIPHRKLLTALQLRANVYPTREFLARG RQDQYIKACRHCDADIESCAHIIGNCPVTQDARIKRHNYICELLLEEAKKKDWVVFKEPHIRDSNKELYKPDLI FVKDARALVVDVTVRYEAAKSSLEEAAAEKVRKYKHLETEVRHLTNAKDVTFVGFPLGARGKWHQDNFKL LTELGLSKSRQVKMAETFSTVALFSSVDIVHMFASRARKSMVM |
| TD3+PM1 (SEQ ID No.69) | VTVKFKYKGEELEVDISKIKKVWRVGKMISFTYDDNGKTGRGAVSEKDAPKELLQMLEKSGKKASCPKPGPP VSAGAMSLESGLTTHSVLAIERGPNSLANSGSDFGGGGLGLPLRLLRVSVGTQTSRSDWVDLVSWSHPGPTSK SQQVDLVSLFPKHRVDLLSKNDQVDLVAQFLPSKFPPNLAENDLALLVNLEFYRSDLHVYECVHFAAHWEGL SGLPEVYEQLAPQPCVGETLHSSLPRDSELFVPEEGSSEKESEDAPKTSPPTPGKHGLEQTGEEKVMVTVPDKN PPCPCCGTRVNSVLNLIEHLKVSHGKRGVCFRCAKCGKENSNYHSVVCHFPKCRGPETEKAPAGEWICEVCN RDFTTKIGLGQHKRLAHPAVRNQERIVASQPKETSNRGAHKRCWTKEEEELLIRLEAQFEGNKNINKLIAEHIT TKTAKQISDKRRLLSRKPAEEPREEPGTCHHTRRAAASLRTEPEMSHHAQAEDRDNGGGMPKRKVSSAEGAA KEEPKRRSARLSAKPPAKVEAKPKKAAAKDKSSDKKVQTKGKRGAKGKQAEVANQETKEDLPAENGETKTE ESPASDEAGEKEAKSDGGPGRRPLPGRAAAGGRTMDEIRRHPDKGNGQQRPTKQKSEEQLQAYYKKTLEERL SAGALNTFPRAFKQVMEGRDIKLVINQTAQDCFGCLESISQIRTATRDKKDTVTREKHPKKPFQKWMKDRAIK KGNYLRFQRLFYLDRGKLAKIILDDIECLSCDIPLSEIYSVFKTRWETTGSFKSLGDFKTYGKADNTAFRELITA KEIEKNVQEMSKGSAPGPDGITLGDVVKMDPEFSRTMEIFNLWLTTGKIPDMVRGCRTVLIPKSSKPDRLKDIN NWRPITIGSILLRLFSRIVTARLSKACPLNPRQRGFIRAAGCSENLKLLQTIIWSAKREHRPLGVVFVDIAKAFDT VSHQHIIHVLQQREVDPHIVGLVSNMYENISTYITTKRNTHTDKIQIRVGVKQGDPMSPLLFNLAMDPLLCKLE ESGKGYHRGQSSITAMAFADDLVLLSDSWENMNTNISILETFCNLTGLKTQGQKCHGFYIKPTKDSYTINDCA AWTINGTPLNMIDPGESEKYLGLRFDPWIGIARSGLSTKLDFWLQRIDQAPLKPLQKTDILKTYTIPRLIYIADHS EVKTALLETLDQKIRTAVKEWLHLPPCTCDAILYSSTRDGGLGITKLAGLIPSVQARRLHRIAQSSDDTMKCFM EKEKMEQLHKKLWIQARGDRENIPSIWEAPPSSEPPNNVSTNSEWEAPTQKDKFPKPCNWRKNEFKKWTKLA SQGRGIVNFERDKISNHWIQYYRRIPHRKLLTALQLRANVYPTREFLARGRQDQYIKACRHCDADIESCAHIIG NCPVTQDARIKRHNYICELLLEEAKKKDWVVFKEPHIRDSNKELYKPDLIFVKDARALVVDVTVRYEAAKSSL EEAAAEKVRKYKHLETEVRHLTNAKDVTFVGFPLGARGKWHQDNFKLLTELGLSKSRQVKMAETFSTVALF SSVDIVHMFASRARKSMVM |
| TD3+PM2 (SEQ | VTVKFKYKGEELEVDISKIKKVWRVGKMISFTYDDNGKTGRGAVSEKDAPKELLQMLEKSGKKASCPKPGPP |
| ID No.70) | VSAGAMSLESGLTTHSVLAIERGPNSLANSGSDFGGGGLGLPLRLLRVSVGTQTSRSDWVDLVSWSHPGPTSK SQQVDLVSLFPKHRVDLLSKNDQVDLVAQFLPSKFPPNLAENDLALLVNLEFYRSDLHVYECVHFAAHWEGL SGLPEVYEQLAPQPCVGETLHSSLPRDSELFVPEEGSSEKESEDAPKTSPPTPGKHGLEQTGEEKVMVTVPDKN PPCPCCGTRVNSVLNLIEHLKVSHGKRGVCFRCAKCGKENSNYHSVVCHFPKCRGPETEKAPAGEWICEVCN RDFTTKIGLGQHKRLAHPAVRNQERIVASQPKETSNRGAHKRCWTKEEEELLIRLEAQFEGNKNINKLIAEHIT TKTAKQISDKRRLLSRKPAEEPREEPGTCHHTRRAAASLRTEPEMSHHAQAEDRDNGGGMPKRKVSSAEGAA KEEPKRRSARLSAKPPAKVEAKPKKAAAKDKSSDKKVQTKGKRGAKGKQAEVANQETKEDLPAENGETKTE ESPASDEAGEKEAKSDGGPGRRPLPGRAAAGGRTMDEIRRHPDKGNGQQRPTKQKSEEQLQAYYKKTLEERL SAGALNTFPRAFKQVMEGRDIKLVINQTAQDCFGCLESISQIRTATRDKKDTVTREKHPKKPFQKWMKDRAIK KGNYLRFQRLFYLDRGKLAKIILDDIECLSCDIPLSEIYSVFKTRWETTGSFKSLGDFKTYGKADNTAFRELITA KEIEKNVQEMSKGSAPGPDGITLGDVVKMDPEFSRTMEIFNLWLTTGKIPDMVRGCRTVLIPKSSKPDRLKDIN NWRPITIGSILLRLFSRIVTARLSKACPLNPRQRGFIRAAGCSENLKLLQTIIQSAKREHRPLGVVFVDIAKAFDT VSHQHIIHVLQQREVDPHIVGLVSNMYENISTYITTKRNTHTDKIQIRVGVKQGDPMSPLLFNLAMDPLLCKLE ESGKGYHRGQSSITAMAFADDLVLLSDSWENMNTNISILETFCNLTGLKTQGQKCHGFYIKPTKDSYTINDCA AWTINGTPLNMIDPGESEKYLGLRFDPWIGIARSGLSTKLDFWLQRIDQAPLKPLQKTDILKTYTIPRLIYIADHS EVKTALLETLDQKIRTAVKEWLHLPPCTCDAILYSSTRDGGLGITKLAGLIPSVQARRLHRIAQSSDDTMKCFM EKEKMEQLHKKLWIQARGDRENIPSIWEAPPSSEPPNNVSTNSEWEAPTQKDKFPKPCNWRKNEFKKWTKLA SQGRGIVNFERDKISNHWIQYYRRIPHRKLLTALQLRANVYPTREFLARGRQDQYIKACRHCDADIESCAHIIG NCPVTQDARIKRHNYICELLLEEAKKKDWVVFKEPHIRDSNKELYKPDLIFVKDARALVVDVTVRYEAAKSSL EEAAAEKVRKYKHLETEVRHLTNAKDVTFVGFPLGARGKWHQDNFKLLTELGLSKSRQVKMAETFSTVALF SSVDIVHMFASRARKSMVM |
| TD3+PM3 (SEQ ID No.71) | VTVKFKYKGEELEVDISKIKKVWRVGKMISFTYDDNGKTGRGAVSEKDAPKELLQMLEKSGKKASCPKPGPP VSAGAMSLESGLTTHSVLAIERGPNSLANSGSDFGGGGLGLPLRLLRVSVGTQTSRSDWVDLVSWSHPGPTSK SQQVDLVSLFPKHRVDLLSKNDQVDLVAQFLPSKFPPNLAENDLALLVNLEFYRSDLHVYECVHFAAHWEGL SGLPEVYEQLAPQPCVGETLHSSLPRDSELFVPEEGSSEKESEDAPKTSPPTPGKHGLEQTGEEKVMVTVPDKN PPCPCCGTRVNSVLNLIEHLKVSHGKRGVCFRCAKCGKENSNYHSVVCHFPKCRGPETEKAPAGEWICEVCN RDFTTKIGLGQHKRLAHPAVRNQERIVASQPKETSNRGARKRCWTKEEEELLIRLEAQFEGNKNINKLIAEHIT TKTAKQISDKRRLLSRKPAEEPREEPGTCHHTRRAAASLRTEPEMSHHAQAEDRDNGGGMPKRKVSSAEGAA KEEPKRRSARLSAKPPAKVEAKPKKAAAKDKSSDKKVQTKGKRGAKGKQAEVANQETKEDLPAENGETKTE ESPASDEAGEKEAKSDGGPGRRPLPGRAAAGGRTMDEIRRHPDKGNGQQRPTKQKSEEQLQAYYKKTLEERL SAGALNTFPRAFKQVMEGRDIKLVINQTAQDCFGCLESISQIRTATRDKKDTVTREKHPKKPFQKWMKDRAIK KGNYLRFQRLFYLDRGKLAKIILDDIECLSCDIPLSEIYSVFKTRWETTGSFKSLGDFKTYGKADNTAFRELITA KEIEKNVQEMSKGSAPGPDGITLGDVVKMDPEFSRTMEIFNLWLTTGKIPDMVRGCRTVLIPKSSKPDRLKDIN NWRPITIGSILLRLFSRIVTARLSKACPLNPRQRGFIRAAGCSENLKLLQTIIQSAKREHRPLGVVFVDIAKAFDT VSHQHIIHVLQQREVDPHIVGLVSNMYENISTYITTKRNTHTDKIQIRVGVKQGDPMSPLLFNLAMDPLLCKLE ESGKGYHRGQSSITAMAFADDLVLLSDSWENMNTNISILETFCNLTGLKTQGQKCHGFYIKPTKDSYTINDCA AWTINGTPLNMIDPGESEKYLGLRFDPWIGIARSGLSTKLDFWLQRIDQAPLKPLQKTDILKTYTIPRLIYIADHS EVKTALLETLDQKIRTAVKEWLHLPPCTCDAILYSSTRDGGLGITKLAGLIPSVQARRLHRIAQSSDDTMKCFM EKEKMEQLHKKLWIQARGDRENIPSIWEAPPSSEPPNNVSTNSEWEAPTQKDKFPKPCNWRKNEFKKWTKLA SQGRGIVNFERDKISNHWIQYYRRIPHRKLLTALQLRANVYPTREFLARGRQDQYIKACRHCDADIESCAHIIG NCPVTQDARIKRHNYICELLLEEAKKKDWVVFKEPHIRDSNKELYKPDLIFVKDARALVVDVTVRYEAAKSSL EEAAAEKVRKYKHLETEVRHLTNAKDVTFVGFPLGARGKWHQDNFKLLTELGLSKSRQVKMAETFSTVALF SSVDIVHMFASRARKSMVM |
| TD3+PM4 (SEQ | VTVKFKYKGEELEVDISKIKKVWRVGKMISFTYDDNGKTGRGAVSEKDAPKELLQMLEKSGKKASCPKPGPP |
| ID No.72) | VSAGAMSLESGLTTHSVLAIERGPNSLANSGSDFGGGGLGLPLRLLRVSVGTQTSRSDWVDLVSWSHPGPTSK SQQVDLVSLFPKHRVDLLSKNDQVDLVAQFLPSKFPPNLAENDLALLVNLEFYRSDLHVYECVHFAAHWEGL SGLPEVYEQLAPQPCVGETLHSSLPRDSELFVPEEGSSEKESEDAPKTSPPTPGKHGLEQTGEEKVMVTVPDKN PPCPCCGTRVNSVLNLIEHLKVSHGKRGVCFRCAKCGKENSNYHSVVCHFPKCRGPETEKAPAGEWICEVCN RDFTTKIGLGQHKRLAHPAVRNQERIVASQPKETSNRGARKRCWTKEEEELLIRLEAQFEGNKNINKLIAEHIT TKTAKQISDKRRLLSRKPAEEPREEPGTCHHTRRAAASLRTEPEMSHHAQAEDRDNGGGMPKRKVSSAEGAA KEEPKRRSARLSAKPPAKVEAKPKKAAAKDKSSDKKVQTKGKRGAKGKQAEVANQETKEDLPAENGETKTE ESPASDEAGEKEAKSDGGPGRRPLPGRAAAGGRTMDEIRRHPDKGNGQQRPTKQKSEEQLQAYYKKTLEERL SAGALNTFPRAFKQVMEGRDIKLVINQTAQDCFGCLESISQIRTATRDKKDTVTREKHPKKPFQKWMKDRAIK KGNYLRFQRLFYLDRGKLAKIILDDIECLSCDIPLSEIYSVFKTRWETTGSFKSLGDFKTYGKADNTAFRELITA KEIEKNVQEMSKGSAPGPDGITLGDVVKMDPEFSRTMEIFNLWLTTGKIPDMVRGCRTVLIPKSSKPDRLKDIN NWRPITIGSILLRLFSRIVTARLSKACPLNPRQRGFIRAAGCSENLKLLQTIIWSAKREHRPLGVVFVDIAKAFDT VSHQHIIHVLQQREVDPHIVGLVSNMYENISTYITTKRNTHTDKIQIRVGVKQGDPMSPLLFNLAMDPLLCKLE ESGKGYHRGQSSITAMAFADDLVLLSDSWENMNTNISILETFCNLTGLKTQGQKCHGFYIKPTKDSYTINDCA AWTINGTPLNMIDPGESEKYLGLRFDPWIGIARSGLSTKLDFWLQRIDQAPLKPLQKTDILKTYTIPRLIYIADHS EVKTALLETLDQKIRTAVKEWLHLPPCTCDAILYSSTRDGGLGITKLAGLIPSVQARRLHRIAQSSDDTMKCFM EKEKMEQLHKKLWIQARGDRENIPSIWEAPPSSEPPNNVSTNSEWEAPTQKDKFPKPCNWRKNEFKKWTKLA SQGRGIVNFERDKISNHWIQYYRRIPHRKLLTALQLRANVYPTREFLARGRQDQYIKACRHCDADIESCAHIIG NCPVTQDARIKRHNYICELLLEEAKKKDWVVFKEPHIRDSNKELYKPDLIFVKDARALVVDVTVRYEAAKSSL EEAAAEKVRKYKHLETEVRHLTNAKDVTFVGFPLGARGKWHQDNFKLLTELGLSKSRQVKMAETFSTVALF SSVDIVHMFASRARKSMVM |
| TD4+PM1 (SEQ ID No.73) | VTVKFKYKGEELEVDISKIKKVWRVGKMISFTYDDNGKTGRGAVSEKDAPKELLQMLEKSGKKASCPKPGPP VSAGAMSLESGLTTHSVLAIERGPNSLANSGSDFGGGGLGLPLRLLRVSVGTQTSRSDWVDLVSWSHPGPTSK SQQVDLVSLFPKHRVDLLSKNDQVDLVAQFLPSKFPPNLAENDLALLVNLEFYRSDLHVYECVHFAAHWEGL SGLPEVYEQLAPQPCVGETLHSSLPRDSELFVPEEGSSEKESEDAPKTSPPTPGKHGLEQTGEEKVMVTVPDKN PPCPCCGTRVNSVLNLIEHLKVSHGKRGVCFRCAKCGKENSNYHSVVCHFPKCRGPETEKAPAGEWICEVCN RDFTTKIGLGQHKRLAHPAVRNQERIVASQPKETSNRGAHKRCWTKEEEELLIRLEAQFEGNKNINKLIAEHIT TKTAKQISDKRRLLSRKPAEEPREEPGTCHHTRRAAASLRTEPEMSHHAQAEDRDNGGGDILAEAHGTRPDLT DQPLPDADHTWYTDGSSLLQEGQRKAGAAVTTETEVIWAKALPAGTSAQRAELIALTQALKMAEGKKLNVY TDSRYAFATAHIHGEIYRRRGWLTSEGKEIKNKDEILALLKALFLPKRLSIIHCPGHQKGHSAEARGNRMADQA ARKAAITETPDTSTLLIENSSPSGGSKRTADGSEFEGGPGRRPLPGRAAAGGRTMDEIRRHPDKGNGQQRPTKQ KSEEQLQAYYKKTLEERLSAGALNTFPRAFKQVMEGRDIKLVINQTAQDCFGCLESISQIRTATRDKKDTVTRE KHPKKPFQKWMKDRAIKKGNYLRFQRLFYLDRGKLAKIILDDIECLSCDIPLSEIYSVFKTRWETTGSFKSLGD FKTYGKADNTAFRELITAKEIEKNVQEMSKGSAPGPDGITLGDVVKMDPEFSRTMEIFNLWLTTGKIPDMVRG CRTVLIPKSSKPDRLKDINNWRPITIGSILLRLFSRIVTARLSKACPLNPRQRGFIRAAGCSENLKLLQTIIWSAKR EHRPLGVVFVDIAKAFDTVSHQHIIHVLQQREVDPHIVGLVSNMYENISTYITTKRNTHTDKIQIRVGVKQGDP MSPLLFNLAMDPLLCKLEESGKGYHRGQSSITAMAFADDLVLLSDSWENMNTNISILETFCNLTGLKTQGQKC HGFYIKPTKDSYTINDCAAWTINGTPLNMIDPGESEKYLGLRFDPWIGIARSGLSTKLDFWLQRIDQAPLKPLQ KTDILKTYTIPRLIYIADHSEVKTALLETLDQKIRTAVKEWLHLPPCTCDAILYSSTRDGGLGITKLAGLIPSVQA RRLHRIAQSSDDTMKCFMEKEKMEQLHKKLWIQARGDRENIPSIWEAPPSSEPPNNVSTNSEWEAPTQKDKFP KPCNWRKNEFKKWTKLASQGRGIVNFERDKISNHWIQYYRRIPHRKLLTALQLRANVYPTREFLARGRQDQY IKACRHCDADIESCAHIIGNCPVTQDARIKRHNYICELLLEEAKKKDWVVFKEPHIRDSNKELYKPDLIFVKDA RALVVDVTVRYEAAKSSLEEAAAEKVRKYKHLETEVRHLTNAKDVTFVGFPLGARGKWHQDNFKLLTELGL SKSRQVKMAETFSTVALFSSVDIVHMFASRARKSMVM |
| TD4+PM2 (SEQ ID No.74) | VTVKFKYKGEELEVDISKIKKVWRVGKMISFTYDDNGKTGRGAVSEKDAPKELLQMLEKSGKKASCPKPGPP VSAGAMSLESGLTTHSVLAIERGPNSLANSGSDFGGGGLGLPLRLLRVSVGTQTSRSDWVDLVSWSHPGPTSK SQQVDLVSLFPKHRVDLLSKNDQVDLVAQFLPSKFPPNLAENDLALLVNLEFYRSDLHVYECVHFAAHWEGL SGLPEVYEQLAPQPCVGETLHSSLPRDSELFVPEEGSSEKESEDAPKTSPPTPGKHGLEQTGEEKVMVTVPDKN PPCPCCGTRVNSVLNLIEHLKVSHGKRGVCFRCAKCGKENSNYHSVVCHFPKCRGPETEKAPAGEWICEVCN RDFTTKIGLGQHKRLAHPAVRNQERIVASQPKETSNRGAHKRCWTKEEEELLIRLEAQFEGNKNINKLIAEHIT TKTAKQISDKRRLLSRKPAEEPREEPGTCHHTRRAAASLRTEPEMSHHAQAEDRDNGGGDILAEAHGTRPDLT DQPLPDADHTWYTDGSSLLQEGQRKAGAAVTTETEVIWAKALPAGTSAQRAELIALTQALKMAEGKKLNVY TDSRYAFATAHIHGEIYRRRGWLTSEGKEIKNKDEILALLKALFLPKRLSIIHCPGHQKGHSAEARGNRMADQA ARKAAITETPDTSTLLIENSSPSGGSKRTADGSEFEGGPGRRPLPGRAAAGGRTMDEIRRHPDKGNGQQRPTKQ KSEEQLQAYYKKTLEERLSAGALNTFPRAFKQVMEGRDIKLVINQTAQDCFGCLESISQIRTATRDKKDTVTRE KHPKKPFQKWMKDRAIKKGNYLRFQRLFYLDRGKLAKIILDDIECLSCDIPLSEIYSVFKTRWETTGSFKSLGD FKTYGKADNTAFRELITAKEIEKNVQEMSKGSAPGPDGITLGDVVKMDPEFSRTMEIFNLWLTTGKIPDMVRG CRTVLIPKSSKPDRLKDINNWRPITIGSILLRLFSRIVTARLSKACPLNPRQRGFIRAAGCSENLKLLQTIIQSAKR EHRPLGVVFVDIAKAFDTVSHQHIIHVLQQREVDPHIVGLVSNMYENISTYITTKRNTHTDKIQIRVGVKQGDP MSPLLFNLAMDPLLCKLEESGKGYHRGQSSITAMAFADDLVLLSDSWENMNTNISILETFCNLTGLKTQGQKC HGFYIKPTKDSYTINDCAAWTINGTPLNMIDPGESEKYLGLRFDPWIGIARSGLSTKLDFWLQRIDQAPLKPLQ KTDILKTYTIPRLIYIADHSEVKTALLETLDQKIRTAVKEWLHLPPCTCDAILYSSTRDGGLGITKLAGLIPSVQA RRLHRIAQSSDDTMKCFMEKEKMEQLHKKLWIQARGDRENIPSIWEAPPSSEPPNNVSTNSEWEAPTQKDKFP KPCNWRKNEFKKWTKLASQGRGIVNFERDKISNHWIQYYRRIPHRKLLTALQLRANVYPTREFLARGRQDQY IKACRHCDADIESCAHIIGNCPVTQDARIKRHNYICELLLEEAKKKDWVVFKEPHIRDSNKELYKPDLIFVKDA RALVVDVTVRYEAAKSSLEEAAAEKVRKYKHLETEVRHLTNAKDVTFVGFPLGARGKWHQDNFKLLTELGL SKSRQVKMAETFSTVALFSSVDIVHMFASRARKSMVM |
| TD4+PM3 (SEQ ID No.75) | VTVKFKYKGEELEVDISKIKKVWRVGKMISFTYDDNGKTGRGAVSEKDAPKELLQMLEKSGKKASCPKPGPP VSAGAMSLESGLTTHSVLAIERGPNSLANSGSDFGGGGLGLPLRLLRVSVGTQTSRSDWVDLVSWSHPGPTSK SQQVDLVSLFPKHRVDLLSKNDQVDLVAQFLPSKFPPNLAENDLALLVNLEFYRSDLHVYECVHFAAHWEGL SGLPEVYEQLAPQPCVGETLHSSLPRDSELFVPEEGSSEKESEDAPKTSPPTPGKHGLEQTGEEKVMVTVPDKN PPCPCCGTRVNSVLNLIEHLKVSHGKRGVCFRCAKCGKENSNYHSVVCHFPKCRGPETEKAPAGEWICEVCN RDFTTKIGLGQHKRLAHPAVRNQERIVASQPKETSNRGARKRCWTKEEEELLIRLEAQFEGNKNINKLIAEHIT TKTAKQISDKRRLLSRKPAEEPREEPGTCHHTRRAAASLRTEPEMSHHAQAEDRDNGGGDILAEAHGTRPDLT DQPLPDADHTWYTDGSSLLQEGQRKAGAAVTTETEVIWAKALPAGTSAQRAELIALTQALKMAEGKKLNVY TDSRYAFATAHIHGEIYRRRGWLTSEGKEIKNKDEILALLKALFLPKRLSIIHCPGHQKGHSAEARGNRMADQA ARKAAITETPDTSTLLIENSSPSGGSKRTADGSEFEGGPGRRPLPGRAAAGGRTMDEIRRHPDKGNGQQRPTKQ KSEEQLQAYYKKTLEERLSAGALNTFPRAFKQVMEGRDIKLVINQTAQDCFGCLESISQIRTATRDKKDTVTRE KHPKKPFQKWMKDRAIKKGNYLRFQRLFYLDRGKLAKIILDDIECLSCDIPLSEIYSVFKTRWETTGSFKSLGD FKTYGKADNTAFRELITAKEIEKNVQEMSKGSAPGPDGITLGDVVKMDPEFSRTMEIFNLWLTTGKIPDMVRG CRTVLIPKSSKPDRLKDINNWRPITIGSILLRLFSRIVTARLSKACPLNPRQRGFIRAAGCSENLKLLQTIIQSAKR EHRPLGVVFVDIAKAFDTVSHQHIIHVLQQREVDPHIVGLVSNMYENISTYITTKRNTHTDKIQIRVGVKQGDP MSPLLFNLAMDPLLCKLEESGKGYHRGQSSITAMAFADDLVLLSDSWENMNTNISILETFCNLTGLKTQGQKC HGFYIKPTKDSYTINDCAAWTINGTPLNMIDPGESEKYLGLRFDPWIGIARSGLSTKLDFWLQRIDQAPLKPLQ KTDILKTYTIPRLIYIADHSEVKTALLETLDQKIRTAVKEWLHLPPCTCDAILYSSTRDGGLGITKLAGLIPSVQA RRLHRIAQSSDDTMKCFMEKEKMEQLHKKLWIQARGDRENIPSIWEAPPSSEPPNNVSTNSEWEAPTQKDKFP KPCNWRKNEFKKWTKLASQGRGIVNFERDKISNHWIQYYRRIPHRKLLTALQLRANVYPTREFLARGRQDQY IKACRHCDADIESCAHIIGNCPVTQDARIKRHNYICELLLEEAKKKDWVVFKEPHIRDSNKELYKPDLIFVKDA RALVVDVTVRYEAAKSSLEEAAAEKVRKYKHLETEVRHLTNAKDVTFVGFPLGARGKWHQDNFKLLTELGL SKSRQVKMAETFSTVALFSSVDIVHMFASRARKSMVM |
| TD4+PM4 (SEQ ID No.76) | VTVKFKYKGEELEVDISKIKKVWRVGKMISFTYDDNGKTGRGAVSEKDAPKELLQMLEKSGKKASCPKPGPP VSAGAMSLESGLTTHSVLAIERGPNSLANSGSDFGGGGLGLPLRLLRVSVGTQTSRSDWVDLVSWSHPGPTSK SQQVDLVSLFPKHRVDLLSKNDQVDLVAQFLPSKFPPNLAENDLALLVNLEFYRSDLHVYECVHFAAHWEGL SGLPEVYEQLAPQPCVGETLHSSLPRDSELFVPEEGSSEKESEDAPKTSPPTPGKHGLEQTGEEKVMVTVPDKN PPCPCCGTRVNSVLNLIEHLKVSHGKRGVCFRCAKCGKENSNYHSVVCHFPKCRGPETEKAPAGEWICEVCN RDFTTKIGLGQHKRLAHPAVRNQERIVASQPKETSNRGARKRCWTKEEEELLIRLEAQFEGNKNINKLIAEHIT TKTAKQISDKRRLLSRKPAEEPREEPGTCHHTRRAAASLRTEPEMSHHAQAEDRDNGGGDILAEAHGTRPDLT DQPLPDADHTWYTDGSSLLQEGQRKAGAAVTTETEVIWAKALPAGTSAQRAELIALTQALKMAEGKKLNVY TDSRYAFATAHIHGEIYRRRGWLTSEGKEIKNKDEILALLKALFLPKRLSIIHCPGHQKGHSAEARGNRMADQA ARKAAITETPDTSTLLIENSSPSGGSKRTADGSEFEGGPGRRPLPGRAAAGGRTMDEIRRHPDKGNGQQRPTKQ KSEEQLQAYYKKTLEERLSAGALNTFPRAFKQVMEGRDIKLVINQTAQDCFGCLESISQIRTATRDKKDTVTRE KHPKKPFQKWMKDRAIKKGNYLRFQRLFYLDRGKLAKIILDDIECLSCDIPLSEIYSVFKTRWETTGSFKSLGD FKTYGKADNTAFRELITAKEIEKNVQEMSKGSAPGPDGITLGDVVKMDPEFSRTMEIFNLWLTTGKIPDMVRG CRTVLIPKSSKPDRLKDINNWRPITIGSILLRLFSRIVTARLSKACPLNPRQRGFIRAAGCSENLKLLQTIIWSAKR EHRPLGVVFVDIAKAFDTVSHQHIIHVLQQREVDPHIVGLVSNMYENISTYITTKRNTHTDKIQIRVGVKQGDP MSPLLFNLAMDPLLCKLEESGKGYHRGQSSITAMAFADDLVLLSDSWENMNTNISILETFCNLTGLKTQGQKC HGFYIKPTKDSYTINDCAAWTINGTPLNMIDPGESEKYLGLRFDPWIGIARSGLSTKLDFWLQRIDQAPLKPLQ KTDILKTYTIPRLIYIADHSEVKTALLETLDQKIRTAVKEWLHLPPCTCDAILYSSTRDGGLGITKLAGLIPSVQA RRLHRIAQSSDDTMKCFMEKEKMEQLHKKLWIQARGDRENIPSIWEAPPSSEPPNNVSTNSEWEAPTQKDKFP KPCNWRKNEFKKWTKLASQGRGIVNFERDKISNHWIQYYRRIPHRKLLTALQLRANVYPTREFLARGRQDQY IKACRHCDADIESCAHIIGNCPVTQDARIKRHNYICELLLEEAKKKDWVVFKEPHIRDSNKELYKPDLIFVKDA RALVVDVTVRYEAAKSSLEEAAAEKVRKYKHLETEVRHLTNAKDVTFVGFPLGARGKWHQDNFKLLTELGL SKSRQVKMAETFSTVALFSSVDIVHMFASRARKSMVM |
| TD5+PM1 (SEQ ID No.77) | MPKRKVSSAEGAAKEEPKRRSARLSAKPPAKVEAKPKKAAAKDKSSDKKVQTKGKRGAKGKQAEVANQET KEDLPAENGETKTEESPASDEAGEKEAKSDASCPKPGPPVSAGAMSLESGLTTHSVLAIERGPNSLANSGSDFG GGGLGLPLRLLRVSVGTQTSRSDWVDLVSWSHPGPTSKSQQVDLVSLFPKHRVDLLSKNDQVDLVAQFLPSK FPPNLAENDLALLVNLEFYRSDLHVYECVHFAAHWEGLSGLPEVYEQLAPQPCVGETLHSSLPRDSELFVPEE GSSEKESEDAPKTSPPTPGKHGLEQTGEEKVMVTVPDKNPPCPCCGTRVNSVLNLIEHLKVSHGKRGVCFRCA KCGKENSNYHSVVCHFPKCRGPETEKAPAGEWICEVCNRDFTTKIGLGQHKRLAHPAVRNQERIVASQPKETS NRGAHKRCWTKEEEELLIRLEAQFEGNKNINKLIAEHITTKTAKQISDKRRLLSRKPAEEPREEPGTCHHTRRA AASLRTEPEMSHHAQAEDRDNGGGMPKRKVSSAEGAAKEEPKRRSARLSAKPPAKVEAKPKKAAAKDKSSD KKVQTKGKRGAKGKQAEVANQETKEDLPAENGETKTEESPASDEAGEKEAKSDGGPGRRPLPGRAAAGGRT MDEIRRHPDKGNGQQRPTKQKSEEQLQAYYKKTLEERLSAGALNTFPRAFKQVMEGRDIKLVINQTAQDCFG CLESISQIRTATRDKKDTVTREKHPKKPFQKWMKDRAIKKGNYLRFQRLFYLDRGKLAKIILDDIECLSCDIPLS EIYSVFKTRWETTGSFKSLGDFKTYGKADNTAFRELITAKEIEKNVQEMSKGSAPGPDGITLGDVVKMDPEFSR TMEIFNLWLTTGKIPDMVRGCRTVLIPKSSKPDRLKDINNWRPITIGSILLRLFSRIVTARLSKACPLNPRQRGFI RAAGCSENLKLLQTIIWSAKREHRPLGVVFVDIAKAFDTVSHQHIIHVLQQREVDPHIVGLVSNMYENISTYITT KRNTHTDKIQIRVGVKQGDPMSPLLFNLAMDPLLCKLEESGKGYHRGQSSITAMAFADDLVLLSDSWENMNT NISILETFCNLTGLKTQGQKCHGFYIKPTKDSYTINDCAAWTINGTPLNMIDPGESEKYLGLRFDPWIGIARSGL STKLDFWLQRIDQAPLKPLQKTDILKTYTIPRLIYIADHSEVKTALLETLDQKIRTAVKEWLHLPPCTCDAILYS STRDGGLGITKLAGLIPSVQARRLHRIAQSSDDTMKCFMEKEKMEQLHKKLWIQARGDRENIPSIWEAPPSSEP PNNVSTNSEWEAPTQKDKFPKPCNWRKNEFKKWTKLASQGRGIVNFERDKISNHWIQYYRRIPHRKLLTALQ LRANVYPTREFLARGRQDQYIKACRHCDADIESCAHIIGNCPVTQDARIKRHNYICELLLEEAKKKDWVVFKE PHIRDSNKELYKPDLIFVKDARALVVDVTVRYEAAKSSLEEAAAEKVRKYKHLETEVRHLTNAKDVTFVGFP LGARGKWHQDNFKLLTELGLSKSRQVKMAETFSTVALFSSVDIVHMFASRARKSMVM |
| TD5+PM2 (SEQ ID No.78) | MPKRKVSSAEGAAKEEPKRRSARLSAKPPAKVEAKPKKAAAKDKSSDKKVQTKGKRGAKGKQAEVANQET KEDLPAENGETKTEESPASDEAGEKEAKSDASCPKPGPPVSAGAMSLESGLTTHSVLAIERGPNSLANSGSDFG GGGLGLPLRLLRVSVGTQTSRSDWVDLVSWSHPGPTSKSQQVDLVSLFPKHRVDLLSKNDQVDLVAQFLPSK FPPNLAENDLALLVNLEFYRSDLHVYECVHFAAHWEGLSGLPEVYEQLAPQPCVGETLHSSLPRDSELFVPEE GSSEKESEDAPKTSPPTPGKHGLEQTGEEKVMVTVPDKNPPCPCCGTRVNSVLNLIEHLKVSHGKRGVCFRCA KCGKENSNYHSVVCHFPKCRGPETEKAPAGEWICEVCNRDFTTKIGLGQHKRLAHPAVRNQERIVASQPKETS NRGAHKRCWTKEEEELLIRLEAQFEGNKNINKLIAEHITTKTAKQISDKRRLLSRKPAEEPREEPGTCHHTRRA AASLRTEPEMSHHAQAEDRDNGGGMPKRKVSSAEGAAKEEPKRRSARLSAKPPAKVEAKPKKAAAKDKSSD KKVQTKGKRGAKGKQAEVANQETKEDLPAENGETKTEESPASDEAGEKEAKSDGGPGRRPLPGRAAAGGRT MDEIRRHPDKGNGQQRPTKQKSEEQLQAYYKKTLEERLSAGALNTFPRAFKQVMEGRDIKLVINQTAQDCFG CLESISQIRTATRDKKDTVTREKHPKKPFQKWMKDRAIKKGNYLRFQRLFYLDRGKLAKIILDDIECLSCDIPLS EIYSVFKTRWETTGSFKSLGDFKTYGKADNTAFRELITAKEIEKNVQEMSKGSAPGPDGITLGDVVKMDPEFSR TMEIFNLWLTTGKIPDMVRGCRTVLIPKSSKPDRLKDINNWRPITIGSILLRLFSRIVTARLSKACPLNPRQRGFI RAAGCSENLKLLQTIIQSAKREHRPLGVVFVDIAKAFDTVSHQHIIHVLQQREVDPHIVGLVSNMYENISTYITT KRNTHTDKIQIRVGVKQGDPMSPLLFNLAMDPLLCKLEESGKGYHRGQSSITAMAFADDLVLLSDSWENMNT NISILETFCNLTGLKTQGQKCHGFYIKPTKDSYTINDCAAWTINGTPLNMIDPGESEKYLGLRFDPWIGIARSGL STKLDFWLQRIDQAPLKPLQKTDILKTYTIPRLIYIADHSEVKTALLETLDQKIRTAVKEWLHLPPCTCDAILYS STRDGGLGITKLAGLIPSVQARRLHRIAQSSDDTMKCFMEKEKMEQLHKKLWIQARGDRENIPSIWEAPPSSEP PNNVSTNSEWEAPTQKDKFPKPCNWRKNEFKKWTKLASQGRGIVNFERDKISNHWIQYYRRIPHRKLLTALQ LRANVYPTREFLARGRQDQYIKACRHCDADIESCAHIIGNCPVTQDARIKRHNYICELLLEEAKKKDWVVFKE PHIRDSNKELYKPDLIFVKDARALVVDVTVRYEAAKSSLEEAAAEKVRKYKHLETEVRHLTNAKDVTFVGFP LGARGKWHQDNFKLLTELGLSKSRQVKMAETFSTVALFSSVDIVHMFASRARKSMVM |
| TD5+PM3 (SEQ ID No.79) | MPKRKVSSAEGAAKEEPKRRSARLSAKPPAKVEAKPKKAAAKDKSSDKKVQTKGKRGAKGKQAEVANQET KEDLPAENGETKTEESPASDEAGEKEAKSDASCPKPGPPVSAGAMSLESGLTTHSVLAIERGPNSLANSGSDFG GGGLGLPLRLLRVSVGTQTSRSDWVDLVSWSHPGPTSKSQQVDLVSLFPKHRVDLLSKNDQVDLVAQFLPSK FPPNLAENDLALLVNLEFYRSDLHVYECVHFAAHWEGLSGLPEVYEQLAPQPCVGETLHSSLPRDSELFVPEE GSSEKESEDAPKTSPPTPGKHGLEQTGEEKVMVTVPDKNPPCPCCGTRVNSVLNLIEHLKVSHGKRGVCFRCA KCGKENSNYHSVVCHFPKCRGPETEKAPAGEWICEVCNRDFTTKIGLGQHKRLAHPAVRNQERIVASQPKETS NRGARKRCWTKEEEELLIRLEAQFEGNKNINKLIAEHITTKTAKQISDKRRLLSRKPAEEPREEPGTCHHTRRA AASLRTEPEMSHHAQAEDRDNGGGMPKRKVSSAEGAAKEEPKRRSARLSAKPPAKVEAKPKKAAAKDKSSD KKVQTKGKRGAKGKQAEVANQETKEDLPAENGETKTEESPASDEAGEKEAKSDGGPGRRPLPGRAAAGGRT MDEIRRHPDKGNGQQRPTKQKSEEQLQAYYKKTLEERLSAGALNTFPRAFKQVMEGRDIKLVINQTAQDCFG CLESISQIRTATRDKKDTVTREKHPKKPFQKWMKDRAIKKGNYLRFQRLFYLDRGKLAKIILDDIECLSCDIPLS EIYSVFKTRWETTGSFKSLGDFKTYGKADNTAFRELITAKEIEKNVQEMSKGSAPGPDGITLGDVVKMDPEFSR TMEIFNLWLTTGKIPDMVRGCRTVLIPKSSKPDRLKDINNWRPITIGSILLRLFSRIVTARLSKACPLNPRQRGFI RAAGCSENLKLLQTIIQSAKREHRPLGVVFVDIAKAFDTVSHQHIIHVLQQREVDPHIVGLVSNMYENISTYITT KRNTHTDKIQIRVGVKQGDPMSPLLFNLAMDPLLCKLEESGKGYHRGQSSITAMAFADDLVLLSDSWENMNT NISILETFCNLTGLKTQGQKCHGFYIKPTKDSYTINDCAAWTINGTPLNMIDPGESEKYLGLRFDPWIGIARSGL STKLDFWLQRIDQAPLKPLQKTDILKTYTIPRLIYIADHSEVKTALLETLDQKIRTAVKEWLHLPPCTCDAILYS STRDGGLGITKLAGLIPSVQARRLHRIAQSSDDTMKCFMEKEKMEQLHKKLWIQARGDRENIPSIWEAPPSSEP PNNVSTNSEWEAPTQKDKFPKPCNWRKNEFKKWTKLASQGRGIVNFERDKISNHWIQYYRRIPHRKLLTALQ LRANVYPTREFLARGRQDQYIKACRHCDADIESCAHIIGNCPVTQDARIKRHNYICELLLEEAKKKDWVVFKE PHIRDSNKELYKPDLIFVKDARALVVDVTVRYEAAKSSLEEAAAEKVRKYKHLETEVRHLTNAKDVTFVGFP LGARGKWHQDNFKLLTELGLSKSRQVKMAETFSTVALFSSVDIVHMFASRARKSMVM |
| TD5+PM4 (SEQ ID No.80) | MPKRKVSSAEGAAKEEPKRRSARLSAKPPAKVEAKPKKAAAKDKSSDKKVQTKGKRGAKGKQAEVANQET KEDLPAENGETKTEESPASDEAGEKEAKSDASCPKPGPPVSAGAMSLESGLTTHSVLAIERGPNSLANSGSDFG GGGLGLPLRLLRVSVGTQTSRSDWVDLVSWSHPGPTSKSQQVDLVSLFPKHRVDLLSKNDQVDLVAQFLPSK FPPNLAENDLALLVNLEFYRSDLHVYECVHFAAHWEGLSGLPEVYEQLAPQPCVGETLHSSLPRDSELFVPEE GSSEKESEDAPKTSPPTPGKHGLEQTGEEKVMVTVPDKNPPCPCCGTRVNSVLNLIEHLKVSHGKRGVCFRCA KCGKENSNYHSVVCHFPKCRGPETEKAPAGEWICEVCNRDFTTKIGLGQHKRLAHPAVRNQERIVASQPKETS NRGARKRCWTKEEEELLIRLEAQFEGNKNINKLIAEHITTKTAKQISDKRRLLSRKPAEEPREEPGTCHHTRRA AASLRTEPEMSHHAQAEDRDNGGGMPKRKVSSAEGAAKEEPKRRSARLSAKPPAKVEAKPKKAAAKDKSSD KKVQTKGKRGAKGKQAEVANQETKEDLPAENGETKTEESPASDEAGEKEAKSDGGPGRRPLPGRAAAGGRT MDEIRRHPDKGNGQQRPTKQKSEEQLQAYYKKTLEERLSAGALNTFPRAFKQVMEGRDIKLVINQTAQDCFG CLESISQIRTATRDKKDTVTREKHPKKPFQKWMKDRAIKKGNYLRFQRLFYLDRGKLAKIILDDIECLSCDIPLS EIYSVFKTRWETTGSFKSLGDFKTYGKADNTAFRELITAKEIEKNVQEMSKGSAPGPDGITLGDVVKMDPEFSR TMEIFNLWLTTGKIPDMVRGCRTVLIPKSSKPDRLKDINNWRPITIGSILLRLFSRIVTARLSKACPLNPRQRGFI RAAGCSENLKLLQTIIWSAKREHRPLGVVFVDIAKAFDTVSHQHIIHVLQQREVDPHIVGLVSNMYENISTYITT KRNTHTDKIQIRVGVKQGDPMSPLLFNLAMDPLLCKLEESGKGYHRGQSSITAMAFADDLVLLSDSWENMNT NISILETFCNLTGLKTQGQKCHGFYIKPTKDSYTINDCAAWTINGTPLNMIDPGESEKYLGLRFDPWIGIARSGL STKLDFWLQRIDQAPLKPLQKTDILKTYTIPRLIYIADHSEVKTALLETLDQKIRTAVKEWLHLPPCTCDAILYS STRDGGLGITKLAGLIPSVQARRLHRIAQSSDDTMKCFMEKEKMEQLHKKLWIQARGDRENIPSIWEAPPSSEP PNNVSTNSEWEAPTQKDKFPKPCNWRKNEFKKWTKLASQGRGIVNFERDKISNHWIQYYRRIPHRKLLTALQ LRANVYPTREFLARGRQDQYIKACRHCDADIESCAHIIGNCPVTQDARIKRHNYICELLLEEAKKKDWVVFKE PHIRDSNKELYKPDLIFVKDARALVVDVTVRYEAAKSSLEEAAAEKVRKYKHLETEVRHLTNAKDVTFVGFP LGARGKWHQDNFKLLTELGLSKSRQVKMAETFSTVALFSSVDIVHMFASRARKSMVM |
| TD6+PM1 (SEQ ID No.81) | MPKRKVSSAEGAAKEEPKRRSARLSAKPPAKVEAKPKKAAAKDKSSDKKVQTKGKRGAKGKQAEVANQET KEDLPAENGETKTEESPASDEAGEKEAKSDASCPKPGPPVSAGAMSLESGLTTHSVLAIERGPNSLANSGSDFG GGGLGLPLRLLRVSVGTQTSRSDWVDLVSWSHPGPTSKSQQVDLVSLFPKHRVDLLSKNDQVDLVAQFLPSK FPPNLAENDLALLVNLEFYRSDLHVYECVHFAAHWEGLSGLPEVYEQLAPQPCVGETLHSSLPRDSELFVPEE GSSEKESEDAPKTSPPTPGKHGLEQTGEEKVMVTVPDKNPPCPCCGTRVNSVLNLIEHLKVSHGKRGVCFRCA KCGKENSNYHSVVCHFPKCRGPETEKAPAGEWICEVCNRDFTTKIGLGQHKRLAHPAVRNQERIVASQPKETS NRGAHKRCWTKEEEELLIRLEAQFEGNKNINKLIAEHITTKTAKQISDKRRLLSRKPAEEPREEPGTCHHTRRA AASLRTEPEMSHHAQAEDRDNGGGMPKRKVSSAEGAAKEEPKRRSARLSAKPPAKVEAKPKKAAAKDKSSD KKVQTKGKRGAKGKQAEVANQETKEDLPAENGETKTEESPASDEAGEKEAKSDGGPGRRPLPGRAAAGGRT MDEIRRHPDKGNGQQRPTKQKSEEQLQAYYKKTLEERLSAGALNTFPRAFKQVMEGRDIKLVINQTAQDCFG CLESISQIRTATRDKKDTVTREKHPKKPFQKWMKDRAIKKGNYLRFQRLFYLDRGKLAKIILDDIECLSCDIPLS EIYSVFKTRWETTGSFKSLGDFKTYGKADNTAFRELITAKEIEKNVQEMSKGSAPGPDGITLGDVVKMDPEFSR TMEIFNLWLTTGKIPDMVRGCRTVLIPKSSKPDRLKDINNWRPITIGSILLRLFSRIVTARLSKACPLNPRQRGFI RAAGCSENLKLLQTIIWSAKREHRPLGVVFVDIAKAFDTVSHQHIIHVLQQREVDPHIVGLVSNMYENISTYITT KRNTHTDKIQIRVGVKQGDPMSPLLFNLAMDPLLCKLEESGKGYHRGQSSITAMAFADDLVLLSDSWENMNT NISILETFCNLTGLKTQGQKCHGFYIKPTKDSYTINDCAAWTINGTPLNMIDPGESEKYLGLRFDPWIGIARSGL STKLDFWLQRIDQAPLKPLQKTDILKTYTIPRLIYIADHSEVKTALLETLDQKIRTAVKEWLHLPPCTCDAILYS STRDGGLGITKLAGLIPSVQARRLHRIAQSSDDTMKCFMEKEKMEQLHKKLWIQARGDRENIPSIWEAPPGGM PKRKVSSAEGAAKEEPKRRSARLSAKPPAKVEAKPKKAAAKDKSSDKKVQTKGKRGAKGKQAEVANQETKE DLPAENGETKTEESPASDEAGEKEAKSDGGSSEPPNNVSTNSEWEAPTQKDKFPKPCNWRKNEFKKWTKLAS QGRGIVNFERDKISNHWIQYYRRIPHRKLLTALQLRANVYPTREFLARGRQDQYIKACRHCDADIESCAHIIGN CPVTQDARIKRIINYICELLLEEAKKKDWVVFKEPIIRDSNKELYKPDLIFVKDARALVVDVTVRYEAAKSSLE EAAAEKVRKYKHLETEVRHLTNAKDVTFVGFPLGARGKWHQDNFKLLTELGLSKSRQVKMAETFSTVALFS SVDIVHMFASRARKSMVM |
| TD6+PM2 (SEQ ID No.82) | MPKRKVSSAEGAAKEEPKRRSARLSAKPPAKVEAKPKKAAAKDKSSDKKVQTKGKRGAKGKQAEVANQET KEDLPAENGETKTEESPASDEAGEKEAKSDASCPKPGPPVSAGAMSLESGLTTHSVLAIERGPNSLANSGSDFG GGGLGLPLRLLRVSVGTQTSRSDWVDLVSWSHPGPTSKSQQVDLVSLFPKHRVDLLSKNDQVDLVAQFLPSK FPPNLAENDLALLVNLEFYRSDLHVYECVHFAAHWEGLSGLPEVYEQLAPQPCVGETLHSSLPRDSELFVPEE GSSEKESEDAPKTSPPTPGKHGLEQTGEEKVMVTVPDKNPPCPCCGTRVNSVLNLIEHLKVSHGKRGVCFRCA KCGKENSNYHSVVCHFPKCRGPETEKAPAGEWICEVCNRDFTTKIGLGQHKRLAHPAVRNQERIVASQPKETS NRGAHKRCWTKEEEELLIRLEAQFEGNKNINKLIAEHITTKTAKQISDKRRLLSRKPAEEPREEPGTCHHTRRA AASLRTEPEMSHHAQAEDRDNGGGMPKRKVSSAEGAAKEEPKRRSARLSAKPPAKVEAKPKKAAAKDKSSD KKVQTKGKRGAKGKQAEVANQETKEDLPAENGETKTEESPASDEAGEKEAKSDGGPGRRPLPGRAAAGGRT MDEIRRHPDKGNGQQRPTKQKSEEQLQAYYKKTLEERLSAGALNTFPRAFKQVMEGRDIKLVINQTAQDCFG CLESISQIRTATRDKKDTVTREKHPKKPFQKWMKDRAIKKGNYLRFQRLFYLDRGKLAKIILDDIECLSCDIPLS EIYSVFKTRWETTGSFKSLGDFKTYGKADNTAFRELITAKEIEKNVQEMSKGSAPGPDGITLGDVVKMDPEFSR TMEIFNLWLTTGKIPDMVRGCRTVLIPKSSKPDRLKDINNWRPITIGSILLRLFSRIVTARLSKACPLNPRQRGFI RAAGCSENLKLLQTIIQSAKREHRPLGVVFVDIAKAFDTVSHQHIIHVLQQREVDPHIVGLVSNMYENISTYITT KRNTHTDKIQIRVGVKQGDPMSPLLFNLAMDPLLCKLEESGKGYHRGQSSITAMAFADDLVLLSDSWENMNT NISILETFCNLTGLKTQGQKCHGFYIKPTKDSYTINDCAAWTINGTPLNMIDPGESEKYLGLRFDPWIGIARSGL STKLDFWLQRIDQAPLKPLQKTDILKTYTIPRLIYIADHSEVKTALLETLDQKIRTAVKEWLHLPPCTCDAILYS STRDGGLGITKLAGLIPSVQARRLHRIAQSSDDTMKCFMEKEKMEQLHKKLWIQARGDRENIPSIWEAPPGGM PKRKVSSAEGAAKEEPKRRSARLSAKPPAKVEAKPKKAAAKDKSSDKKVQTKGKRGAKGKQAEVANQETKE DLPAENGETKTEESPASDEAGEKEAKSDGGSSEPPNNVSTNSEWEAPTQKDKFPKPCNWRKNEFKKWTKLAS QGRGIVNFERDKISNHWIQYYRRIPHRKLLTALQLRANVYPTREFLARGRQDQYIKACRHCDADIESCAHIIGN CPVTQDARIKRHNYICELLLEEAKKKDWVVFKEPHIRDSNKELYKPDLIFVKDARALVVDVTVRYEAAKSSLE EAAAEKVRKYKHLETEVRHLTNAKDVTFVGFPLGARGKWHQDNFKLLTELGLSKSRQVKMAETFSTVALFS SVDIVHMFASRARKSMVM |
| TD6+PM3 (SEQ ID No.83) | MPKRKVSSAEGAAKEEPKRRSARLSAKPPAKVEAKPKKAAAKDKSSDKKVQTKGKRGAKGKQAEVANQET KEDLPAENGETKTEESPASDEAGEKEAKSDASCPKPGPPVSAGAMSLESGLTTHSVLAIERGPNSLANSGSDFG GGGLGLPLRLLRVSVGTQTSRSDWVDLVSWSHPGPTSKSQQVDLVSLFPKHRVDLLSKNDQVDLVAQFLPSK FPPNLAENDLALLVNLEFYRSDLHVYECVHFAAHWEGLSGLPEVYEQLAPQPCVGETLHSSLPRDSELFVPEE GSSEKESEDAPKTSPPTPGKHGLEQTGEEKVMVTVPDKNPPCPCCGTRVNSVLNLIEHLKVSHGKRGVCFRCA KCGKENSNYHSVVCHFPKCRGPETEKAPAGEWICEVCNRDFTTKIGLGQHKRLAHPAVRNQERIVASQPKETS NRGARKRCWTKEEEELLIRLEAQFEGNKNINKLIAEHITTKTAKQISDKRRLLSRKPAEEPREEPGTCHHTRRA AASLRTEPEMSHHAQAEDRDNGGGMPKRKVSSAEGAAKEEPKRRSARLSAKPPAKVEAKPKKAAAKDKSSD KKVQTKGKRGAKGKQAEVANQETKEDLPAENGETKTEESPASDEAGEKEAKSDGGPGRRPLPGRAAAGGRT MDEIRRHPDKGNGQQRPTKQKSEEQLQAYYKKTLEERLSAGALNTFPRAFKQVMEGRDIKLVINQTAQDCFG CLESISQIRTATRDKKDTVTREKHPKKPFQKWMKDRAIKKGNYLRFQRLFYLDRGKLAKIILDDIECLSCDIPLS EIYSVFKTRWETTGSFKSLGDFKTYGKADNTAFRELITAKEIEKNVQEMSKGSAPGPDGITLGDVVKMDPEFSR TMEIFNLWLTTGKIPDMVRGCRTVLIPKSSKPDRLKDINNWRPITIGSILLRLFSRIVTARLSKACPLNPRQRGFI RAAGCSENLKLLQTIIQSAKREHRPLGVVFVDIAKAFDTVSHQHIIHVLQQREVDPHIVGLVSNMYENISTYITT KRNTHTDKIQIRVGVKQGDPMSPLLFNLAMDPLLCKLEESGKGYHRGQSSITAMAFADDLVLLSDSWENMNT NISILETFCNLTGLKTQGQKCHGFYIKPTKDSYTINDCAAWTINGTPLNMIDPGESEKYLGLRFDPWIGIARSGL STKLDFWLQRIDQAPLKPLQKTDILKTYTIPRLIYIADHSEVKTALLETLDQKIRTAVKEWLHLPPCTCDAILYS STRDGGLGITKLAGLIPSVQARRLHRIAQSSDDTMKCFMEKEKMEQLHKKLWIQARGDRENIPSIWEAPPGGM PKRKVSSAEGAAKEEPKRRSARLSAKPPAKVEAKPKKAAAKDKSSDKKVQTKGKRGAKGKQAEVANQETKE DLPAENGETKTEESPASDEAGEKEAKSDGGSSEPPNNVSTNSEWEAPTQKDKFPKPCNWRKNEFKKWTKLAS QGRGIVNFERDKISNHWIQYYRRIPHRKLLTALQLRANVYPTREFLARGRQDQYIKACRHCDADIESCAHIIGN CPVTQDARIKRHNYICELLLEEAKKKDWVVFKEPHIRDSNKELYKPDLIFVKDARALVVDVTVRYEAAKSSLE EAAAEKVRKYKHLETEVRHLTNAKDVTFVGFPLGARGKWHQDNFKLLTELGLSKSRQVKMAETFSTVALFS SVDIVHMFASRARKSMVM |
| TD6+PM4 (SEQ ID No.84) | MPKRKVSSAEGAAKEEPKRRSARLSAKPPAKVEAKPKKAAAKDKSSDKKVQTKGKRGAKGKQAEVANQET KEDLPAENGETKTEESPASDEAGEKEAKSDASCPKPGPPVSAGAMSLESGLTTHSVLAIERGPNSLANSGSDFG GGGLGLPLRLLRVSVGTQTSRSDWVDLVSWSHPGPTSKSQQVDLVSLFPKHRVDLLSKNDQVDLVAQFLPSK FPPNLAENDLALLVNLEFYRSDLHVYECVHFAAHWEGLSGLPEVYEQLAPQPCVGETLHSSLPRDSELFVPEE GSSEKESEDAPKTSPPTPGKHGLEQTGEEKVMVTVPDKNPPCPCCGTRVNSVLNLIEHLKVSHGKRGVCFRCA KCGKENSNYHSVVCHFPKCRGPETEKAPAGEWICEVCNRDFTTKIGLGQHKRLAHPAVRNQERIVASQPKETS NRGARKRCWTKEEEELLIRLEAQFEGNKNINKLIAEHITTKTAKQISDKRRLLSRKPAEEPREEPGTCHHTRRA AASLRTEPEMSHHAQAEDRDNGGGMPKRKVSSAEGAAKEEPKRRSARLSAKPPAKVEAKPKKAAAKDKSSD KKVQTKGKRGAKGKQAEVANQETKEDLPAENGETKTEESPASDEAGEKEAKSDGGPGRRPLPGRAAAGGRT MDEIRRHPDKGNGQQRPTKQKSEEQLQAYYKKTLEERLSAGALNTFPRAFKQVMEGRDIKLVINQTAQDCFG CLESISQIRTATRDKKDTVTREKHPKKPFQKWMKDRAIKKGNYLRFQRLFYLDRGKLAKIILDDIECLSCDIPLS EIYSVFKTRWETTGSFKSLGDFKTYGKADNTAFRELITAKEIEKNVQEMSKGSAPGPDGITLGDVVKMDPEFSR TMEIFNLWLTTGKIPDMVRGCRTVLIPKSSKPDRLKDINNWRPITIGSILLRLFSRIVTARLSKACPLNPRQRGFI RAAGCSENLKLLQTIIWSAKREHRPLGVVFVDIAKAFDTVSHQHIIHVLQQREVDPHIVGLVSNMYENISTYITT KRNTHTDKIQIRVGVKQGDPMSPLLFNLAMDPLLCKLEESGKGYHRGQSSITAMAFADDLVLLSDSWENMNT NISILETFCNLTGLKTQGQKCHGFYIKPTKDSYTINDCAAWTINGTPLNMIDPGESEKYLGLRFDPWIGIARSGL STKLDFWLQRIDQAPLKPLQKTDILKTYTIPRLIYIADHSEVKTALLETLDQKIRTAVKEWLHLPPCTCDAILYS STRDGGLGITKLAGLIPSVQARRLHRIAQSSDDTMKCFMEKEKMEQLHKKLWIQARGDRENIPSIWEAPPGGM PKRKVSSAEGAAKEEPKRRSARLSAKPPAKVEAKPKKAAAKDKSSDKKVQTKGKRGAKGKQAEVANQETKE DLPAENGETKTEESPASDEAGEKEAKSDGGSSEPPNNVSTNSEWEAPTQKDKFPKPCNWRKNEFKKWTKLAS QGRGIVNFERDKISNHWIQYYRRIPHRKLLTALQLRANVYPTREFLARGRQDQYIKACRHCDADIESCAHIIGN CPVTQDARIKRHNYICELLLEEAKKKDWVVFKEPHIRDSNKELYKPDLIFVKDARALVVDVTVRYEAAKSSLE EAAAEKVRKYKHLETEVRHLTNAKDVTFVGFPLGARGKWHQDNFKLLTELGLSKSRQVKMAETFSTVALFS SVDIVHMFASRARKSMVM |
| SIV-VPX (SEQ ID No.85) | MASGRDPREPLPGWLEIWDLDREPWDEWLQDMLRDLNEEARRHFGMNMLIRVWNYCVEEGRRHNTPWNEI GYKYYRIVQKSMFVHFRCGCRRRGPFSPYEERRNGQGGGAPPPPPGLA |
| SIV2-VPX (SEQ ID No.86) | MAEGAPEIPEGAGEVDLNTWLERSLEKINQEARLHFHPEFLFRLWNACIEHWDRHQRSLSYAKYRYLLLMN KAMFTHMQQECPCRSGHPRGPPPPGMV |
| HIV2-VPX (SEQ ID No.87) | MTDPRERVPPGNSGEETIGEAFEWLERTIEALNREAVNHLPRELIFQVWQRSWRYWHDEQGMSASYTKYRYL CLMQKAIFTHFKRGCTCWGEDMGREGLEDQGPPPPPPPGLV |
| GDNF (SEQ ID No.88) | KRSPDKQMAVLPRRERNRQAAAANPENSRGKGRRGQRGKNRGCVLTAIHLNVTDLGLGYETKEELIFRYCSG SCDAAETTYDKILKNLSRNRRLVSDKVGQACCRPIAFDDDLSFLDDNLVYHILRKHSAKRCGCI |
| EPO (SEQ ID No.89) | MGVHECPAWLWLLLSLLSLPLGLPVLGAPPRLICDSRVLERYLLEAKEAENITTGCAEHCSLNENITVPDTKVN FYAWKRMEVGQQAVEVWQGLALLSEAVLRGQALLVNSSQPWEPLQLHVDKAVSGLRSLTTLLRALGAQKE AISPPDAASAAPLRTITADTFRKLFRVYSNFLRGKLKLYTGEACRTGDR |

## Claims

1. An engineered retrotransposase, comprising one, two, or three mutations of the wild-type retrotransposase TgR2, or is a truncated form of the wild-type retrotransposase TgR2; or comprising one, two, or three mutations of the wild-type retrotransposase TgR2 on a truncated form of the wild-type retrotransposase TgR2, wherein the mutations are selected from the group consisting of:
(i) the amino acids in the wild-type retrotransposase TgR2 are mutated to other amino acids at the corresponding site(s) in a multiple sequence alignment;
(ii) the amino acids in the wild-type retrotransposase TgR2 that potentially interact with DNA or RNA in the tertiary structure are mutated to positively charged amino acids; and
(iii) cysteine residues in the wild-type retrotransposase TgR2 are mutated to serine.

2. The engineered retrotransposase according to claim 1, wherein the amino acid sequence of the wild-type retrotransposase TgR2 is shown as SEQ ID NO.1 or the amino acid position numbering of the engineered retrotransposase is defined as SEQ ID NO.1; or
the truncated form of the wild-type retrotransposase TgR2 is an N-terminal truncated form, a C-terminal truncated form and/or an internal sequence truncated form of the wild-type retrotransposase TgR2,
preferably, the N-terminal truncated form is a truncated form in which the 1^{st} to 500^{th} amino acids, e.g., the 1^{st} to 80^{th} amino acids, the 1^{st} to 190^{th} amino acids, or the 1^{st} to 220^{th} amino acids, of the wild-type retrotransposase TgR2 are truncated;
preferably, the internal sequence truncated form is a truncated form in which the 300^{th} to 1200^{th} amino acids, e.g., the 390^{th} to 500^{th} amino acids, the 1100^{th} to 1120^{th} amino acids, of the wild-type retrotransposase TgR2 are truncated.

3. The engineered retrotransposase according to claim 1, wherein
the amino acids in the wild-type retrotransposase TgR2 at the corresponding site(s) in the multiple sequence alignment are identified by using multiple sequence alignment softwares (such as MAFFT and the like), and the amino acid position numbering is defined as SEQ ID NO.1;
preferably, the corresponding site(s) of the wild-type retrotransposase TgR2 in the multiple sequence alignment is any one or are two, three, four, five, six, seven, eight, nine or ten or more sites selected from the group consisting of:
K700, L704, V727, S732, L737, A747, A748, T759, 1760, W762, S763, H792, A793, Q795, E798, P801, V807, S808, E812, S815, T819, T820, H825, K828, S860, Y864, H865, R866, Q868, S869, N889, N891, T892, S895, Q911 and D957;
more preferably, the amino acids in the wild-type retrotransposase TgR2 at the corresponding site(s) in the multiple sequence alignment are mutated into other amino acids at the corresponding site(s), wherein the site(s) is any one or are two, three, four, five, six, seven, eight, nine, ten or more selected from the group consisting of:
K700D, L704Q, V727L, S732T, L7371, A747S, A748S, A748P, T759L, I760L, W762R, W762K, W762Q, S763N, S763R, H792T, H792W, A793V, Q795K, Q795R, E798G, P801Q, V807I, S808R, E812K, E812R, S815N, T819D, T820L, H825Q, K828P, S860E, Y864F, H865Q, H865R, R866V, Q868S, S869K, N889G, N891Q, T892K, S895K, Q911E and D957G;
more preferably, the amino acids in the wild-type retrotransposase TgR2 at the corresponding site(s) in the multiple sequence alignment are mutated into other amino acids at the corresponding site(s), wherein the site(s) is any one or are more selected from the group consisting of:
K700D, W762Q, H792W, A793V, R866V and D957G.

4. The engineered retrotransposase according to claim 1, wherein
the amino acids in the wild-type retrotransposase TgR2 that potentially interact with DNA or RNA in the tertiary structure are mutated to arginine; the amino acid position numbering is defined as SEQ ID NO.1;
preferably, the amino acid(s) in the wild-type retrotransposase TgR2 that potentially interact with DNA or RNA in the tertiary structure is any one or are two, three, four, five, six, seven, eight, nine, ten or more selected from the group consisting of:
E153, G154, E160, E163, Q164, Q168, D227, K228, N229, N240, K267, E268, N269, P291, E294, H341, K342, C344, E350, E361, N363, N365, N367, A379, Q381, S383, D384, S390, D573, G575, K576, K579, 1585, L588, K635, E636, E638, K639, N640, Q642, E643, S645, K646, G647, S648, V661, V693, 1695, P696, S699, K700, 1713, 1715, T728, S732, K733, S751, E752, N753, L754, T819, N847, K950, Q955, D957, D990, K993, T994, Y995, P998, 11001, D1005, H1006, T1044, D1046, T1052, Q1062, Q1071, S1072, S1073, D1074, D1075, G1098, G1099, D1100, E1102, N1103, P1105, S1106, E1109, S1113, S1114, E1115, N1118, N1123, E1125, E1127, Q1131, D1133, K1134, K1137, K1210, D1216, D1218, E1220, A1223, H1224, G1227, N1228, Q1233, D1234, 11237, K1238, H1240, N1241, E1245, E1249, N1268, E1270, E1302, E1306, K1307 and K1310;
more preferably, the amino acid(s) in the wild-type retrotransposase TgR2 that potentially interact with DNA or RNA in the tertiary structure is any one or are two, three, four, five, six, seven, eight, nine, ten or more selected from the group consisting of:
D227, K228, N229, N240, K267, N269, P291, E294, H341, K342, C344, E361, N365, N367, A379, Q381, S383, D384, S390, K576, K579, K639, K646, K733, T819, K950, Q955, D990, K993, T1044, T1052, Q1062, Q1071, D1074, D1075, G1098, G1099, P1105, E1109, E1125, E1127, Q1131, A1223, 11237, E1245, E1249 and E1306.

5. The engineered retrotransposase according to claim 1, wherein
the cysteine(s) in the wild-type retrotransposase TgR2 is any one or are two, three, four, five, six, seven, eight, nine, ten or more selected from the group consisting of:
C4, C145, C170, C282, C344, C404, C518, C521, C690, C735, C750, C855, C901, C913, C930, C1034, C1036, C1079, C1139 and C1244, and the amino acid position numbering is defined as SEQ ID NO.1;
preferably, the cysteine(s) in the wild-type retrotransposase TgR2 is any one or are two, three or four selected from the group consisting of:
C344, C1034, C1036 and C1244.

6. The engineered retrotransposase according to any one of claims 1-5, wherein the amino acid position numbering is defined as SEQ ID NO.1; and the engineered retrotransposase comprises one or more sets of mutations selected from the group consisting of:
(1)G1098R; (2) G1098R+H341R; (3) G1098R+K700D; (4)G1098R+W762Q; (5)G1098R+H792W; (6)G1098R+A793V; (7)G1098R+R866V; (8)G1098R+Q955R; (9)G1098R+T1052R; (10)G1098R+Q1062R; (11)G1098R+P1105R; (12)G1098R+C1034S; (13)G1098R+I1237R; (14)G1098R+K1310R; (15)G1098R+D957G; (16)H341R+C1036S; (17)H341R+Q1062R; (18)H341R+11237R; (19)H341R+K1310R; (20)I1237R+K1310R; (21)C1034S+C1036S; (22)K700D+R866V; (23)W762Q+R866V; (24)H792W+R866V; (25)A793V+R866V; (26)G1098R+A793V+K700D; (27)G1098R+A793V+W762Q; (28)G1098R+A793V+H792W; (29)G1098R+A793V+Q955R; (30)G1098R+A793V+W762Q+Q955R; (31)G1098R+A793V+W762Q+H341R; (32)G1098R+A793V+Q955R+H341R; and (33)G1098R+A793V+W762Q+Q955R+H341R;
preferably, the engineered retrotransposase comprises one or more sets of mutations selected from the group consisting of:
G1098R+H341R; G1098R+K700D; G1098R+W762Q; G1098R+H792W, G1098R+A793V; G1098R+R866V; G1098R+Q955R; G1098R+A793V+W762Q; G1098R+A793V+Q955R; G1098R+A793V+Q955R+H341R; and G1098R+A793V+W762Q+Q955R+H341R.

7. An engineered retrotransposase, comprising any one or more sets of mutations selected from the group consisting of:
(1)G1098R; (2) G1098R+H341R; (3) G1098R+K700D; (4)G1098R+W762Q; (5)G1098R+H792W; (6)G1098R+A793V; (7)G1098R+R866V; (8)G1098R+Q955R; (9)G1098R+T1052R; (10)G1098R+Q1062R; (11)G1098R+P1105R; (12)G1098R+C1034S; (13)G1098R+I1237R; (14)G1098R+K1310R; (15)G1098R+D957G; (16)H341R+C1036S; (17)H341R+Q1062R; (18)H341R+11237R; (19)H341R+K1310R; (20)I1237R+K1310R; (21)C1034S+C1036S; (22)K700D+R866V; (23)W762Q+R866V; (24)H792W+R866V; (25)A793V+R866V; (26)G1098R+A793V+K700D; (27)G1098R+A793V+W762Q; (28)G1098R+A793V+H792W; (29)G1098R+A793V+Q955R; (30)G1098R+A793V+W762Q+Q955R; (31)G1098R+A793V+W762Q+H341R; and (32)G1098R+A793V+Q955R+H341R; (33)G1098R+A793V+W762Q+Q955R+H341R;
preferably, the engineered retrotransposase comprises one or more sets of mutations selected from the group consisting of:
G1098R+H341R; G1098R+K700D; G1098R+W762Q; G1098R+H792W, G1098R+A793V; G1098R+R866V; G1098R+Q955R; G1098R+A793V+W762Q; G1098R+A793V+Q955R; G1098R+A793V+Q955R+H341R; and G1098R+A793V+W762Q+Q955R+H341R; and
the amino acid position numbering is defined as SEQ ID NO.1.

8. An engineered retrotransposase, which is the engineered retrotransposase according to any one of claims 1 to 7, or an engineered retrotransposase obtained by further conjugating the wild-type retrotransposase TgR2 to other amino acid sequences.

9. The engineered retrotransposase according to claim 8, wherein the other amino acid sequence(s) is any one or are two, three, four, five, six, or seven selected from the group consisting of: an exonuclease, a single-stranded DNA-binding protein, a chromatin-modulating peptide segment, a high mobility peptide segment, an RNaseH domain, a nuclear localization signal (NLS) for nuclear import, and a nuclear localization signal for nuclear export.

10. The engineered retrotransposase according to claim 9, wherein the other amino acid sequence is an exonuclease, and the exonuclease is inserted at the N-terminal, after amino acid no. 191, after amino acid no. 432, after amino acid no. 1112, or at the C-terminal of the retrotransposase according to any one of claims 1 to 7 or the wild-type retrotransposase TgR2;
preferably, the exonuclease is an exonuclease from bacteriophage T5;
more preferably, the amino acid sequence of the exonuclease is shown as SEQ ID NO.8, or has at least 70%, 75%, 80%, 85%, 90%, 95%, 96%, 97%, 98%, or 99% identity with the amino acid sequence of SEQ ID No. 8.

11. The engineered retrotransposase according to claim 9, wherein the other amino acid sequence is a single-stranded DNA-binding protein, and the single-stranded DNA-binding protein is inserted at the N-terminal, after amino acid no. 191, after amino acid no. 432, after amino acid no. 1112, or at the C-terminal of the retrotransposase according to any one of claims 1 to 7 or the wild-type retrotransposase TgR2;
preferably, the single-stranded DNA-binding protein is a single-stranded DNA-binding protein from the bacterium *Sulfolobus tokodaii;*
more preferably, the sequence of the single-stranded DNA-binding protein is shown as SEQ ID NO.9, or has at least 70%, 75%, 80%, 85%, 90%, 95%, 96%, 97%, 98%, or 99% identity with the amino acid sequence of SEQ ID No. 9.

12. The engineered retrotransposase according to claim 9, wherein the other amino acid sequence is a chromatin-modulating peptide segment or a high mobility peptide, and the chromatin-modulating peptide segment or high mobility peptide segment is inserted at the N-terminal, after amino acid no. 191, after amino acid no. 432, after amino acid no. 1112, or at the C-terminal of the retrotransposase according to any one of claims 1 to 7 or the wild-type retrotransposase TgR2;
preferably, the chromatin-modulating peptide segment or high mobility peptide segment is a chromatin-modulating peptide segment or high mobility peptide segment from human;
preferably, the sequence of the chromatin-modulating peptide segment or high mobility peptide segment is shown as SEQ ID NO.10, or has at least 70%, 75%, 80%, 85%, 90%, 95%, 96%, 97%, 98%, or 99% identity with the amino acid sequence of SEQ ID No. 10.

13. The engineered retrotransposase according to claim 9, wherein the other amino acid sequence is an RNaseH domain, and the RNaseH domain is inserted at the N-terminal, after amino acid no. 191, after amino acid no. 432, after amino acid no. 1112, or at the C-terminal of the retrotransposase according to any one of claims 1 to 7 or the wild-type retrotransposase TgR2;
preferably, the RNaseH domain is an RNaseH domain from MLV reverse transcriptase;
more preferably, the sequence of the RNaseH domain from MLV reverse transcriptase is shown as SEQ ID NO.11, or has at least 70%, 75%, 80%, 85%, 90%, 95%, 96%, 97%, 98%, or 99% identity with the amino acid sequence of SEQ ID No.11.

14. The engineered retrotransposase according to claim 9, wherein
any two or three or four of the exonuclease and/or single-stranded DNA-binding protein and/or chromatin-modulating peptide segment (CMP) and/or RNaseH domain are inserted at the N-terminal, after amino acid no. 191, after amino acid no. 432, after amino acid no. 1112, or at the C-terminal of the retrotransposase according to any one of claims 1 to 7 or the wild-type retrotransposase TgR2;
more preferably, the exonuclease is inserted at the N-terminal of the retrotransposase according to any one of claims 1 to 7 or the wild-type retrotransposase TgR2, and the exonuclease or single-stranded DNA-binding protein or chromatin-modulating peptide segment (CMP) or RNaseH domain is inserted after amino acid no. 432 of the retrotransposase according to any one of claims 1 to 7 or the wild-type retrotransposase TgR2; or
more preferably, the single-stranded DNA-binding protein is inserted at the N-terminal of the retrotransposase according to any one of claims 1 to 7 or the wild-type retrotransposase TgR2, and the exonuclease or single-stranded DNA-binding protein or chromatin-modulating peptide segment (CMP) or RNaseH domain is inserted after amino acid no. 432 of the retrotransposase according to any one of claims 1 to 7 or the wild-type retrotransposase TgR2; or
more preferably, the chromatin-modulating peptide segment (CMP) is inserted at the N-terminal of the retrotransposase according to any one of claims 1 to 7 or the wild-type retrotransposase TgR2, and the exonuclease or single-stranded DNA-binding protein or chromatin-modulating peptide segment (CMP) or RNaseH domain is inserted after amino acid no. 432 of the retrotransposase according to any one of claims 1 to 7 or the wild-type retrotransposase TgR2; or
more preferably, the single-stranded DNA-binding protein is inserted at the N-terminal of the retrotransposase according to any one of claims 1 to 7 or the wild-type retrotransposase TgR2, and the chromatin-modulating peptide segment (CMP) is inserted after amino acid no. 432 of the retrotransposase according to any one of claims 1 to 7, and the chromatin-modulating peptide segment (CMP) or RNaseH domain is inserted after amino acid no. 1112 of the retrotransposase according to any one of claims 1 to 7 or the wild-type retrotransposase TgR2; or
more preferably, the single-stranded DNA-binding protein is inserted at the N-terminal of the retrotransposase according to any one of claims 1 to 7 or wild-type retrotransposase TgR2, and the RNaseH domain is inserted after amino acid no. 432 of the retrotransposase according to any one of claims 1 to 7 or wild-type retrotransposase TgR2, and the chromatin-modulating peptide segment (CMP) or RNaseH domain is inserted after amino acid no. 1112 of the retrotransposase according to any one of claims 1 to 7 or wild-type retrotransposase TgR2; or
more preferably, the chromatin-modulating peptide segment (CMP) is inserted at the N-terminal of the retrotransposase according to any one of claims 1 to 7 or wild-type retrotransposase TgR2, and the chromatin-modulating peptide segment (CMP) is inserted after amino acid no. 432 of the retrotransposase according to any one of claims 1 to 7 or wild-type retrotransposase TgR2, and the chromatin-modulating peptide segment (CMP) or RNaseH domain is inserted after amino acid no. 1112 of the retrotransposase according to any one of claims 1 to 7 or wild-type retrotransposase TgR2; or
more preferably, the chromatin-modulating peptide segment (CMP) is inserted at the N-terminal of the retrotransposase according to any one of claims 1 to 7 or wild-type retrotransposase TgR2, and the RNaseH domain is inserted after amino acid no. 432 of the retrotransposase according to any one of claims 1 to 7 or wild-type retrotransposase TgR2, and the chromatin-modulating peptide segment (CMP) or RNaseH domain is inserted after amino acid no. 1112 of the retrotransposase according to any one of claims 1 to 7 or wild-type retrotransposase TgR2.

15. The engineered retrotransposase accordimg to claim 14, wherein
the sequence of the exonuclease is shown as SEQ ID NO.8, or has at least 70%, 75%, 80%, 85%, 90%, 95%, 96%, 97%, 98%, or 99% identity with the amino acid sequence of SEQ ID No. 8; or
the sequence of the single-stranded DNA-binding protein is shown as SEQ ID NO.9, or has at least 70%, 75%, 80%, 85%, 90%, 95%, 96%, 97%, 98%, or 99% identity with the amino acid sequence of SEQ ID No. 9; or
the sequence of the chromatin-modulating peptide segment or high mobility peptide segment is shown as SEQ ID NO.10, or has at least 70%, 75%, 80%, 85%, 90%, 95%, 96%, 97%, 98%, or 99% identity with the amino acid sequence of SEQ ID No. 10; or
the sequence of the RNaseH domain is shown as SEQ ID NO.11, or has at least 70%, 75%, 80%, 85%, 90%, 95%, 96%, 97%, 98%, or 99% identity with the amino acid sequence of SEQ ID No.11.

16. The engineered retrotransposase according to any one of claims 1 to 15, wherein the engineered retrotransposase comprises an amino acid sequence as shown in any one of SEQ ID NOs.2-7, 12, 13, 38, 40, 42, 43, 45, 47, 49, 51, and 55-84; or comprises an amino acid sequence having at least 70%, 75%, 80%, 85%, 90%, 95%, 96%, 97%, 98%, or 99% identity with the amino acid sequence as shown in any one of SEQ ID NOs. 2-7, 12, 13, 38, 40, 42, 43, 45, 47, 49, 51, and 55-84; or
the engineered retrotransposase is a truncated form of any one of the amino acid sequences as shown in SEQ ID NOs. 1-7, 13, 38, 40, 42, 43, 45, 47, 49, 51, and 55-84, preferably a sequence obtained by truncating the corresponding 1^{st} to 80^{th}, or 1^{st} to 190^{th}, or 1^{st} to 220^{th}, or 390^{th} to 500^{th} or 1100^{th} to 1120^{th} amino acids in wild-type sequence SEQ ID NO. 1 from the amino acid as shown in any one of SEQ ID NOs. 1-7, 13, 38, 40, 42, 43, 45, 47, 49, 51, and 55~84.

17. A system for modifying DNA, comprising:
a retrotransposase, the retrotransposase is the wild-type retrotransposase TgR2 as shown in SEQ ID NO.1, or the retrotransposase according to any one of claims 1 to 16, or is a nucleic acid encoding the wild-type retrotransposase TgR2 as shown in SEQ ID NO. 1 or the retrotransposase according to any one of claims 1 to 16; and
a donor RNA or a nucleic acid encoding the donor RNA, and the donor RNA comprises: a sequence that binds to the retrotransposase and a heterologous sequence,
preferably, the heterologous sequence is of at least 1-50000 bases, e.g., 1nt and above, 10nt and above, 50nt and above, 60nt and above, 70nt and above, 80nt and above, 90nt and above, 100nt and above, 150nt and above, 200nt and above, 250nt and above, 300nt and above, 350nt and above, 400nt and above, 450nt and above, 500nt and above, 550nt and above, 600nt and above, 650nt and above, 700nt and above, 750nt and above, 800nt and above, 850nt and above, 900nt and above, 950nt and above, 1000nt and above, 1100nt and above, 1200nt and above, 1300nt and above, 1400nt and above, 1500nt and above, 1600nt and above, 1700nt and above, 1800nt and above, 1900nt and above, 2000nt and above, 2100nt and above, 2200nt and above, 2300nt and above, 2400nt and above, 2500nt and above, 2600nt and above, 2700nt and above, 2800nt and above, 2900nt and above, 3000nt and above, 3500nt and above, 4000nt and above, 4500nt and above, 5000nt and above, 5500nt and above, 6000nt and above, 6500nt and above, 7000nt and above, 7500nt and above, 8000nt and above, 8500nt and above, 9000nt and above, 9500nt and above, 10000nt and above, 15000nt and above, 20000nt and above, 25000nt and above, 30000nt and above, 35000nt and above, 40000nt and above, or 45000nt and above;
more preferably, the system comprises a nuleic acid encoding VPX protein, or the VPX protein itself.

18. The system according to claim 17, wherein the heterologous sequence comprises any one or more selected from the group consisting of: a sequence encoding a polypeptide or a non-coding RNA sequence, a sequence containing a promoter or an enhancer, a sequence encoding one or more introns, and a transcription termination sequence;
Preferably, the peptide is a therapeutic polypeptide or a mammalian polypeptide; more preferably the peptide is a therapeutic polypeptide, a membrane protein, an intracellular proteins an extracellular protein, a structural protein, a signaling protein, a regulatory protein, a transport protein, an organelle protein, a sensory protein, a motor protein, a defense protein, a storage protein, a reporter protein, an antibody, an enzyme, or a coagulation factor; more preferably the number of amino acids of the polypeptide is 20-10000, e.g., 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 550, 600, 650, 700, 750, 800, 850, 900, 950, 1000, 1100, 1200, 1300, 1400, 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 4000, 4100, 4200, 4300, 4400, 4500, 4600, 4700, 4800, 4900, 5000, 5100, 5200, 5300, 5400, 5500, 5600, 5700, 5800, 5900, 6000, 6100, 6200, 6300, 6400, 6500, 6600, 6700, 6800, 6900, 7000, 7100, 7200, 7300, 7400, 7500, 7600, 7700, 7800, 7900, 8000, 8100, 8200, 8300, 8400, 8500, 8600, 8700, 8800, 8900, 9000, 9100, 9200, 9300, 9400, 9500, 9600, 9700, 9800, or 9900;
preferably, the intracellular protein is selected from cytoplasmic proteins, nuclear proteins, organelle proteins, mitochondrial proteins, or lysosomal proteins,
more preferably, the sequence encoding the polypeptides contains one or more introns.

19. The system according to claim 17 or 18, wherein the donor RNA further comprises a homologous domain, preferably the homologous domain comprises a first homologous domain and a second homologous domain;
more preferably, the first homologous domain consists of 3 or more bases located at the 5' end of the donor RNA that has 100% identity with the target DNA strand, and the second homologous domain consists of 3 or more bases located at the 3' end of the donor RNA that has 100% identity with the target DNA strand; preferably the target DNA is a genomic safe harbor (GSH) site or a genomic Natural Harbor^{™} site.

20. The system according to any one of claims 17 to 19, wherein the nucleic acid encoding the retrotransposase according to any one of claims 1 to 16 and the donor RNA or the nucleic acid encoding the donor RNA are separated nuleic acids, preferably the donor RNA does not encode retrotransposase; more preferably, the donor RNA comprises one or more chemical modifications; or
the nucleic acid encoding the retrotransposase according to any one of claims 1 to 16 is covalently linked to the donor RNA or the nucleic acid encoding the donor RNA, preferably the nucleic acid encoding the retrotransposase according to any one of claims 1 to 16 and the donor RNA or the nucleic acid encoding the donor RNA form a fused nucleic acid, more preferably, the fused nucleic acid comprises RNA or DNA;
more preferably, the nucleic acid encoding the retrotransposase according to any one of claims 1 to 16, the donor RNA or the nucleic acid encoding the donor RNA, and the nucleic acid encoding VPX protein are separated nuleic acids; or
the nucleic acid encoding the retrotransposase according to any one of claims 1 to 16 and the nucleic acid encoding VPX protein are covanlently linked or form a fused nucleic acid, and is a nucleic acid separated from the donor RNA or the nucleic acid encoding the donor RNA;
the nucleic acid encoding the retrotransposase according to any one of claims 1 to 16 and the donor RNA or the nucleic acid encoding the donor RNA are covanlently linked or form a fused nucleic acids, and is a nucleic acid separated from the nucleic acid encoding the VPX protein;
the donor RNA or the nucleic acid encoding the donor RNA and the nucleic acid encoding VPX protein are covanlently linked or form a fused nucleic acids, and is a nucleic acid separated from the nucleic acid encoding the retrotransposase according to any one of claims 1 to 16; or
the nucleic acid encoding the retrotransposase according to any one of claims 1 to 16, the donor RNA or the nucleic acid encoding the donor RNA, and the nucleic acid encoding the VPX protein are covanlently linked or form a fused nucleic acid,
more preferably, the fused nucleic acid comprises RNA or DNA.

21. The system according to any one of claims 17 to 20, wherein the donor RNA comprises:
a 5'untranslated region (5'UTR) that optionally binds to the retrotransposase,
a 3' untranslated region (3'UTR) that binds to the retrotransposase,
a heterologous sequence, and
a promoter that operatively linked to the heterologous sequence,
preferably, the promoter is located between the 5' untranslated region (5'UTR) that binds to the retrotransposase and the heterologous sequence; or preferably, the promoter is located between the 3' untranslated region (3'UTR) that binds to the retrotransposase and the heterologous sequence.

22. The system according to claim 21, wherein the heterologous sequence comprises an open reading frame oriented in the 5' to 3' direction on the donor RNA or its reverse complementary sequence; or the heterologous sequence comprises an open reading frame oriented in the 3' to 5' direction on the donor RNA or its reverse complementary sequence.

23. The system according to any one of claims 17 to 14, wherein the donor RNA further comprises a nuclear localization signal, or the nucleic acid encoding the retrotransposase according to any one of claims 1 to 8 comprises a nuclear localization signal and/or a nucleolar localisation signal and/or a nuclear export signal.

24. The system according to any one of claims 9 to 23, wherein the nucleic acid encoding the retrotransposase according to any one of claims 1 to 16 and the nucleic acid encoding the donor RNA are present in a ratio of 10:1-1:10, e.g., in a ratio of 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, or 1:10.

25. The system according to any one of claims 17 to 24, wherein the donor RNA comprises a stem-loop sequence or helix located at 5' end of the pseudoknot sequence; preferably comprises one or more (e.g., 2, 3 or more) stem-loop sequences or helices located at 3' end of the pseudoknot sequence, for example, at 3' end of the pseudoknot sequence and 5' end of the heterologous sequence; more preferably, the donor RNA of the pseudoknot has catalytic activity, e.g., RNA cleavage activity, e.g., cis-RNA cleavage activity; or
the donor RNA comprises at least one stem-loop sequence or helix, for example 1, 2, 3, 4, 5 or more stem-loop sequences, hairpin loops, or helix sequences, located, for instance, at 3' end of the heterologous sequence.

26. The system according to any one of claims 17 to 25, wherein
the 5' untranslated region (5'UTR) in the donor RNA that binds to the retrotransposase has at least 70%, 75%, 80%, 85%, 90%, 95%, 96%, 97%, 98%, 99%, 100% identity with the nucleotide sequence as defined in any one of SEQ ID NOs. 16, 27, 29, 31, 33, 35, 37, 39, and 41;
the 3' untranslated region (3'UTR) in the donor RNA that binds to the retrotransposase has at least 70%, 75%, 80%, 85%, 90%, 95%, 96%, 97%, 98%, 99%, or 100% identity with the nucleotide sequence as defined in any one of SEQ ID NOs. 17, 32, 34, 44, 46, 48, 50, 52, and 54; or
the amino acid sequence of the VPX protein has at least 70%, 75%, 80%, 85%, 90%, 95%, 96%, 97%, 98%, 99%, or 100% identity with the amino acid sequence as defined in any one of SEQ ID NOs. 85, 86 and 87.

27. The system according to any one of claims 17 to 26, wherein the donor RNA comprises the following structures from its 5' end to 3' end:
a first homologous domain,
a 5' untranslated region (5'UTR) that binds to the retrotransposase,
a heterologous sequence,
a 3' untranslated region (3'UTR) that binds to the retrotransposase, and
a second homologous domain;
preferably, the first homologous domain is 1 and above, or 2 and above, or 5 and above, or 10 and above, or 20 and above, or 30 and above, or 40 and above, or 50 and above, or 60 and above, or 70 and above, or 80 and above, or 90 and above, or 100 and above, or 110 and above, or 120 and above, or 130 and above, or 140 and above, or 150 and above bases located at the 5' end of the donor RNA and has 100% identity with target DNA strand; the second homologous domain is 1 and above, or 2 and above, or 5 and above, or 10 and above, or 20 and above, or 30 and above, or 40 and above, or 50 and above, or 60 and above, or 70 and above, or 80 and above, or 90 and above, or 100 and above, or 110 and above, or 120 and above, or 130 and above, or 140 and above, or 150 and above bases located at the 3' end of the donor RNA and has 100% identity with target DNA strand;
more preferably, the 5' untranslated region (5'UTR) in the donor RNA that binds to the retrotransposase has at least 70%, 75%, 80%, 85%, 90%, 95%, 96%, 97%, 98%, 99%, or 100% identity with the nucleotide sequence as defined in any one of SEQ ID NOs. 16, 27, 29, 31, 33, 35, 37, 39, 41;
the 3' untranslated region (3'UTR) in the donor RNA that binds to the retrotransposase has at least 70%, 75%, 80%, 85%, 90%, 95%, 96%, 97%, 98%, 99%, 100% identity with the nucleotide sequence as defined in any one of SEQ ID NOs. 17, 32, 34, 44, 46, 48, 50, 52, and 54.

28. The system according to any one of claims 17 to 27, wherein
the 5' untranslated region (5'UTR) that binds to the retrotransposase is a non-natural 5' untranslated region (5'UTR); or
the 3' untranslated region (5'UTR) that binds to the retrotransposase is a non-natural 3' untranslated region (3'UTR);
more preferably, the non-natural 5' untranslated region (5'UTR) comprises nucleotide insertions, deletions, and/or substitutions, relative to the natural 5'UTR sequence;
more preferably, the non-natural 3' untranslated region (3'UTR) comprises nucleotide insertions, deletions, and/or substitutions, relative to the natural 3'UTR sequence;
more preferably, the non-natural 5' untranslated region (5'UTR) has at least 70%, 75%, 80%, 85%, 90%, 95%, 96%, 97%, 98%, 99%, or 100% identity with the nucleotide sequence as defined in any one of SEQ ID NOs. 16, 27, 29, 31, 33, 35, 37, 39, and 41;
more preferably, the non-natural 3' untranslated region (3'UTR) has at least 70%, 75%, 80%, 85%, 90%, 95%, 96%, 97%, 98%, 99%, or 100% identity with the nucleotide sequence as defined in any one of SEQ ID NOs. 17, 32, 34, 44, 46, 48, 50, 52, and 54.

29. The system according to any one of claims 17 to 27, wherein the heterologous sequence is inserted into target sites within the cellular genome at an average copy number of at least 0.01, 0.025, 0.05, 0.075, 0.1, 0.15, 0.2, 0.25, 0.3, 0.4, 0.5, 0.75, 1, 1.25, 1.5, 1.75, 2, 2.5, 3, 4 or 5 copies per genome, preferably inserted at only one target site within the genome.

30. The system according to any one of claims 17 to 29, wherein the system enables the heterologous sequences to be inserted into the the target sites of about 1%-100% of the cells (e.g., about 1%-10%, 10%-20%, 20%-30%, 30%-40%, 40%-50%, 50%-60%, 60%-70%, 70%-80%, 80%-90% or 90%-100% of the cells) within a cell population that contacts with the system (e.g., insterted with one or more than one copy), e.g., as measured by using single-cell ddPCR, or
the system enables the heterologous sequences to be inserted with one copy into the target sites of about 1%-100% of the cells (e.g., about 1%-10%, 10%-20%, 20%-30%, 30%-40%, 40%-50%, 50%-60%, 60%-70%, 70%-80%, 80%-90% or 90%-100% of the cells) withina cell population that contacts with the system, e.g., as measured by using colony isolation and ddPCR.

31. The system according to any one of claims 17 to 30, wherein the system enables the heterologous sequence to be inserted into the target sites (on-target insertion) within the cell population at a rate greater than into non-target sites (off-target insertion) within the cell population, wherein the ratio of the on-target insertion to the off-target insertion is greater than 10:1, 20:1, 30:1, 40:1, 50:1, 60:1, 70:1, 80:1, 90:1, 100:1, 200:1, 500:1 or 1,000:1.

32. The system according to any one of claims 17 to 31, wherein
the sequence of the first homologous domain is shown as SEQ ID NO.15, 19, 21, 23, 25 or 28, or has at least 70%, 75%, 80%, 85%, 90%, 95%, 96%, 97%, 98%, 99% identity with the nucleotide sequence shown as SEQ ID NO. 15, 19, 21, 23, 25 or 28; or
the sequence of the second homologous domain is shown as SEQ ID NO.18, 20, 22, 24, 26 or 36, or has at least 70%, 75%, 80%, 85%, 90%, 95%, 96%, 97%, 98%, 99% identity with the nucleotide sequence shown as SEQ ID NO.18, 20, 22, 24, 26 or 36.

33. A non-natural 5' untranslated region (5'UTR), wherein it has nucleotide insertions, deletions, and/or substitutions, relative to natural 5'UTR sequence; and preferably has at least 70%, 75%, 80%, 85%, 90%, 95%, 96%, 97%, 98%, 99%, or 100% identity with the nucleotide sequence shown as SEQ ID NO. 16, 27, 29, 31, 33, 35, 37, 39, or 41.

34. A non-natural 3' untranslated region (3'UTR), wherein it has nucleotide insertions, deletions, and/or substitutions, relative to natural 3'UTR sequence; and preferably has at least 70%, 75%, 80%, 85%, 90%, 95%, 96%, 97%, 98%, 99%, or 100% identity with the nucleotide sequence shown as SEQ ID NO. 17, 32, 34, 44, 46, 48, 50, 52, or 54.

35. An engineered transposable element, wherein it comprises the following sequences from the 5' end to 3' end:
a 5' untranslated region (5'UTR), a heterologous sequence and a 3' untranslated region (3'UTR),
wherein the 5' untranslated region comprises a nucleotide sequence having at least 70%, 75%, 80%, 85%, 90%, 95%, 96%, 97%, 98%, 99%, or 100% identity with any one selected from SEQ ID NOs. 16, 27, 29, 31, 33, 35, 37, 39, and 41; or
wherein the 3' untranslated region comprises a nucleotide sequence having at least 70%, 75%, 80%, 85%, 90%, 95%, 96%, 97%, 98%, 99%, or 100% identity with any one selected from SEQ ID NOs. 17, 32, 34, 44, 46, 48, 50, 52, and 54;
preferably, it comprises the following sequences from the 5' end to 3' end: a first homologous domain, a 5' untranslated region (5'UTR) that binds to the retrotransposase, a heterologous sequence, a 3' untranslated region (3'UTR) that binds to the retrotransposase, and a second homologous domain;
more preferably, the sequence of the first homologous domain has at least 70%, 75%, 80%, 85%, 90%, 95%, 96%, 97%, 98%, or 99% identity with the nucleotide sequence shown as SEQ ID NO.15, 19, 21, 23, 25 or 28; or
the sequence of the second homologous domain has at least 70%, 75%, 80%, 85%, 90%, 95%, 96%, 97%, 98%, or 99% identity with the nucleotide sequence shown as SEQ ID NO.18, 20, 22, 24, 26 or 36.

36. The element according to claim 35, wherein the element is the donor RNA or the nucleic acid encoding the donor RNA as defined in any one of claims 17 to 32.

37. A host cell, wherein it comprises the engineered retrotransposase according to any one of claims 1 to 16, or the element according to claim 35 or 36, or the system according to any one of claims 17 to 32; preferably the host cell is a mammalian cell or a plant cell; more preferably is a human cell.

38. A method for modifying a target DNA strand in a cell, tissue or subject, wherein it comprises administering to the cell, tissue or subject the engineered retrotransposase according to any one of claims 1 to 16, the element according to claim 35 or 36, or the system according to any one of claims 17 to 32, and wherein the system reverse transcribes the donor RNA sequence into the target DNA strand, thereby modifying the target DNA strand in the cell, tissue or subject.

39. The method according to claim 38, wherein the cell or tissue is a mammalian cell or tissue, preferably a human cell or tissue, and the subject is a mammal, preferably a human.

40. The method according to claim 39, wherein the cell is a fibroblast, a primary cell or a non-immortalized cell.

41. The method according to any one of claims 38 to 40, wherein the method is performed *in vivo* or *in vitro.*

42. A method for modifying the genome of a mammalian cell or inserting DNA into the mammalian genome, wherein it comprises applying the engineered retrotransposase according to any one of claims 1 to 16, the element according to claim 35 or 36, or the system according to any one of claims 17 to 32 to the cell, and preferably the mammalian is a human.

43. The method according to claim 42, wherein the method results in the addition of an exogenous DNA sequence of at least 5, 10, 20, 50, 100, 200, 500, 1000, 2000, 3000, 4000, 5000, 6000, 7000, 8000, 9000, or 10000 base pairs to the genome of the mammal.

44. The method according to any one of claims 38 to 43, wherein the cell is part of a tissue; or the mammalian cell is euploid, is not immortalized, is part of an organism, is a primary cell, is non-dividing, is a hepatocyte, or is derived from a subject with a genetic disease.

45. The method according to any one of claims 38 to 44, wherein
the method comprises contacting a cell, tissue or subject with the retrotransposase according to any one of claims 1 to 16 or a nucleic acid encoding the retrotransposase according to any one of claims 1 to 16, and a donor RNA or the nucleic acid encoding the donor RNA,
preferably, the contacting comprises contacting the cell, tissue or subject with a plasmid, virus, virus-like particle, virion, liposome, vesicle, exosome, or lipid nanoparticle;
more preferably, the contacting comprises using a non-viral delivery method, e.g., electroporation.

46. The method according to claim 45, wherein
the contacting comprises intravenous administration to the subject, preferably at least twice, the retrotransposase according to any one of claims 1 to 16 or a nucleic acid encoding the retrotransposase according to any one of claims 1 to 16, and a donor RNA or a nucleic acid encoding the donor RNA.

47. The method according to any one of claims 38 to 46, wherein
the retrotransposase according to any one of claims 1 to 16 or the nucleic acid encoding the retrotransposase according to any one of claims 1 to 16, and the donor RNA or the nucleic acid encoding the donor RNA, are administrated separately; or
the retrotransposase according to any one of claims 1 to 16 or the nucleic acid encoding the retrotransposase according to any one of claims 1 to 16, and the donor RNA or the nucleic acid encoding the donor RNA, are administrated together.

48. A nucleic acid, encoding the retrotransposase according to any one of claims 1 to 16.

49. A vector, comprising the nucleic acid according to claim 48.

50. A host cell, comprising the vector according to claim 49.

51. A pharmaceutical composition, wherein it comprises the system according to any one of claims 17 to 32, the nuleic acid according to claim 48, the vector according to claim 49, or the host cell according to claim 37 or 50; preferably the system is incorporated in a pharmaceutically acceptable vector; more preferably, the vector is a vesicle (including liposomes, natural or synthetic lipid bilayers, exosomes), a lipid nanoparticle, a virus or a plasmid vector.
